(19) Europäisches Patentamt
European Patent Office
Office européen des brevets

(11) Publication number: 0 050 856 B1

# (12) EUROPEAN PATENT SPECIFICATION

(45) Date of publication of patent specification: 27.12.84

(51) Int. Cl.$^3$: **C 07 C 103/52**, C 07 H 15/04, A 61 K 37/02

(21) Application number: 81108796.4

(22) Date of filing: 23.10.81

## (54) New peptide, process for its preparation and pharmaceutical composition containing it.

(30) Priority: 27.10.80 US 201241
28.01.81 US 229072

(43) Date of publication of application:
05.05.82 Bulletin 82/18

(45) Publication of the grant of the patent:
27.12.84 Bulletin 84/52

(84) Designated Contracting States:
AT BE CH DE FR GB IT LI LU NL SE

(56) References cited:
EP-A-0 011 283
EP-A-0 025 842
EP-A-0 027 260
GB-A-1 496 333
GB-A-2 033 906
GB-A-2 053 231

(73) Proprietor: Fujisawa Pharmaceutical Co., Ltd.
3, Doshomachi 4-chome Higashi-ku
Osaka-shi, Osaka 541 (JP)

(72) Inventor: Kitaura, Yoshihiko
No. 523, Oudono
Sakurai (JP)
Inventor: Nakaguchi, Osamu
Green-Heights-Sone, 418 No. 1-5-10
Shiroyamacho Toyonaka (JP)
Inventor: Hemmi, Keiji
No. S-501 Minami No. 8
Aobaoka Suita (JP)
Inventor: Aratani, Matsuhiko
No. 4, 1-chome
Satakedai Suita (JP)
Inventor: Takeno, Hidekazu
No. 67, Senzaicho
Tenri (JP)
Inventor: Okada, Satoshi
No. 2-21-7, Kawazoe
Takatsuki (JP)
Inventor: Tanaka, Hirokazu
No. 3-10-21, Hanayashikisouen
Takarazuka (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:
Chemical Abstracts vol. 72, no. 19, 1980 Columbus, Ohio, USA DEZELEE et al. "Structure of the peptidoglycan in Escherichia coli B and Bacillus megaterium K.M. Stereospecific synthesis of two meso-diaminopimelic acid peptides with the tetrapeptide subunit of bacterial cell wall peptidoglycan" abstract no. 97462z

(72) Inventor: Hashimoto, Masashi
No. 1-6-17, Satsukidai
Nakayama Takarazuka (JP)
Inventor: Kuroda, Yoshio
No. 4-2-8, Kosobecho
Takatsuki (JP)
Inventor: Iguchi, Eiko
No. 6-6-14, Hannancho
Abeno-ku Osaka (JP)
Inventor: Kohsaka, Masanobu
No. 5-26-8, Akasakadai
Sakai (JP)
Inventor: Aoki, Hatsuo
No. 4-13-3, Hata
Ikeda (JP)
Inventor: Imanaka, Hiroshi
No. 4-19-25, Sakurai Shimamotocho
Mishima-gun Osaka (JP)

(74) Representative: Türk, Dietmar, Dr. rer. nat. et al
Redies, Redies, Türk & Gille Patentanwälte
Brucknerstrasse 20
D-4000 Düsseldorf 13 (DE)

**Description**

This invention relates to a new peptide. More particularly, this invention relates to a new peptide and the pharmaceutically acceptable salt thereof, which have pharmacological activities, to processes for preparation thereof and a pharmaceutical composition comprising the same and a method of use thereof.

The inventors of this invention and other inventors had previously synthesized a lot of peptide compounds and a patent application covering the same was filed in many countries (e.g European patent application No. 80104502.2).

Thereafter, the inventors of this invention further continued to make an extensive synthetic study so that they have succeeded in synthesizing new peptides, which are generically disclosed but specifically not disclosed in the specification of said application and have protective efficacy in experimental infection and an antitumor activity.

A new peptide of this invention is represented by the following formula (I):

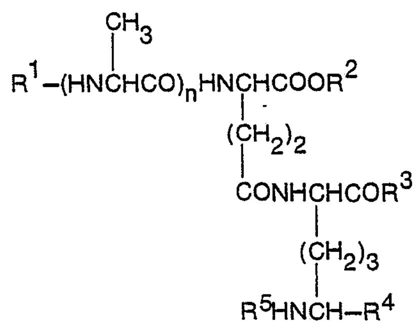

(I)

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is hydrogen, $R^3$ is 1-carboxyethylamino, $R^4$ is hydrogen, and $R^5$ is hydrogen; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is stearoyl, n is an integer of 0, $R^2$ is benzyl, $R^3$ is methoxy, $R^4$ is methoxycarbonyl and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is n-docosanoyl, n is an integer of 1, $R^2$ is benzyl, $R^3$ is 1-carboxyethylamino, $R^4$ is carboxy and $R^5$ is t-butoxycarbonyl; or

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is benzyl, $R^3$ is 1-carboxyethylamino, $R^4$ is carboxy and $R^5$ is t-butoxycarbonyl; or

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is 2-acetoxypropionyl, n is an integer of 1, $R^2$ is benzyl, $R^3$ is methoxycarbonylmethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is 2-acetoxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 2-acetoxypropionyl, n is an integer of 1, $R^2$ is hydrogen, $R^3$ is carboxymethylamino, $R^4$ is methoxycarbonyl and $R^5$ is t-butoxycarbonyl; or

$R^1$ is heptanoyl, n is an integerof 0, $R^2$ is hydrogen, $R^3$ is 1-carboxyethylamino, $R^4$ is carboxy and $R^5$ is t-butoxycarbonyl; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is octanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is 2-hydroxypropionyl, n is an integer of 1, $R^2$ is benzyl, $R^3$ is methoxycarbonylmethylamino, $R^4$ is carboxy, and $R^5$ is t-butoxycarbonyl; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is benzyl, $R^3$ is is carboxymethylamino, $R^4$ is methoxycarbonyl and $R^5$ is t-butoxycarbonyl; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-n-hexyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benyloxycarbonyl; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-n-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is methoxycarbonylmethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2-keto-L-gulonoyl, n is an integer of 0, $R^2$ is benzyl, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is 2-keto-L-gulonoyl, n is an integer of 0, $R^2$ is benzyl, $R^3$ is 1-carboxyethylamino, $R^4$ is carboxy and $R^5$ is hydrogen; or

$R^1$ is 2-keto-L-gulonoyl, n is an integer of 0, $R^2$ is hydrogen, $R^3$ is 1-carboxyethylamino, $R^4$ is carboxy and $R^5$ is hydrogen; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-2,3,4,5-O-tetracetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4,5,-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is t-butoxycarbonyl; or

$R^1$ is triacontanoyl, n is an integer of 1, $R^2$ is hydrogen, $R^3$ is 1-carboxyethylamino, $R^4$ is carboxy and $R^5$ is hydrogen; or

$R^1$ is heptanoyl, n is an integer of 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl; or

$R^1$ is heptanoyl, n is an integer of 0, $R^2$ is hydrogen, $R^3$ is 1-carboxyethylamino, $R^4$ is ethoxycarbonyl and $R^5$ is hydrogen.

A pharmaceutically acceptable salt of the new peptide of the formula (I) may include a salt with an inorganic or organic base such as an alkali metal salt (e.g. sodium salt, potassium salt, etc.), an alkaline earth metal salt (e.g. calcium salt, etc.), ammonium salt, organic amine salt (e.g. ethanolamine salt, triethylamine salt, dicyclohexylamine salt, etc.), or the like, and an acid addition salt with organic or inorganic acid such as methane sulfonate, hydrochloride, sulfate, nitrate, phosphate or the like.

The new peptide (I) of this invention can be prepared by various methods which are explained in detail as follows:

(I) Peptide bond formation:

(1)

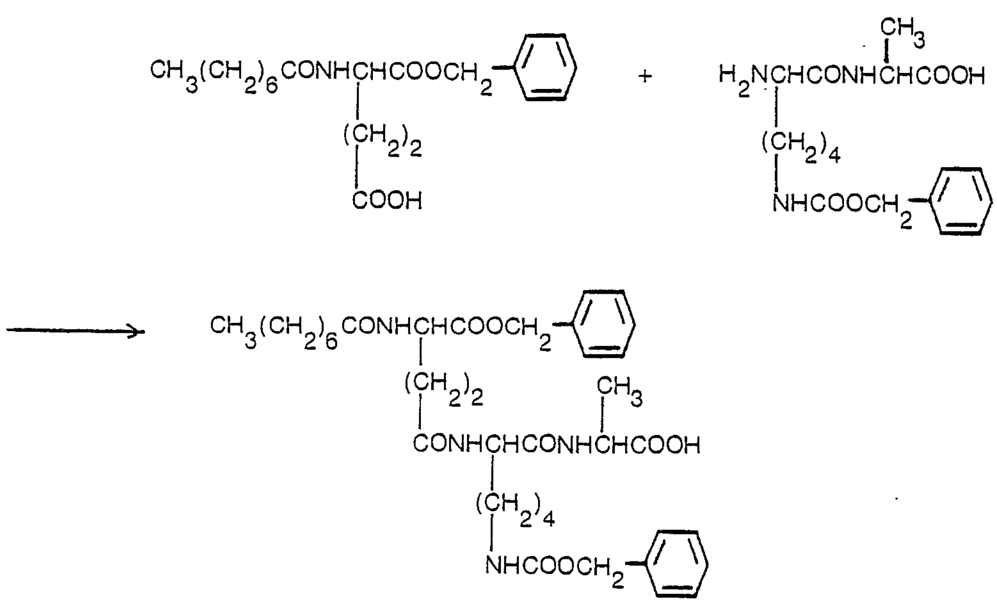

(2)

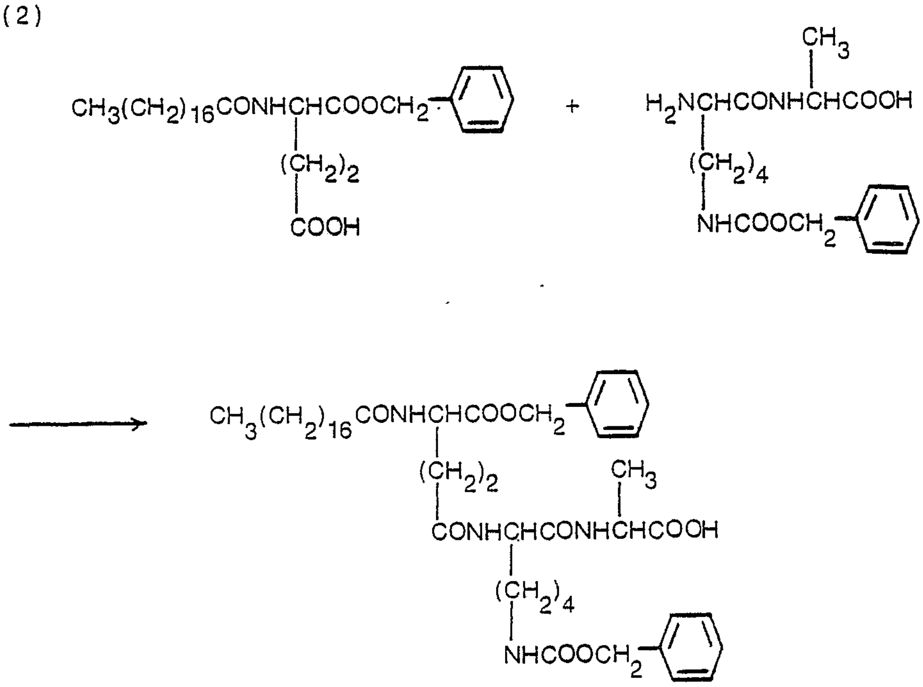

(3)

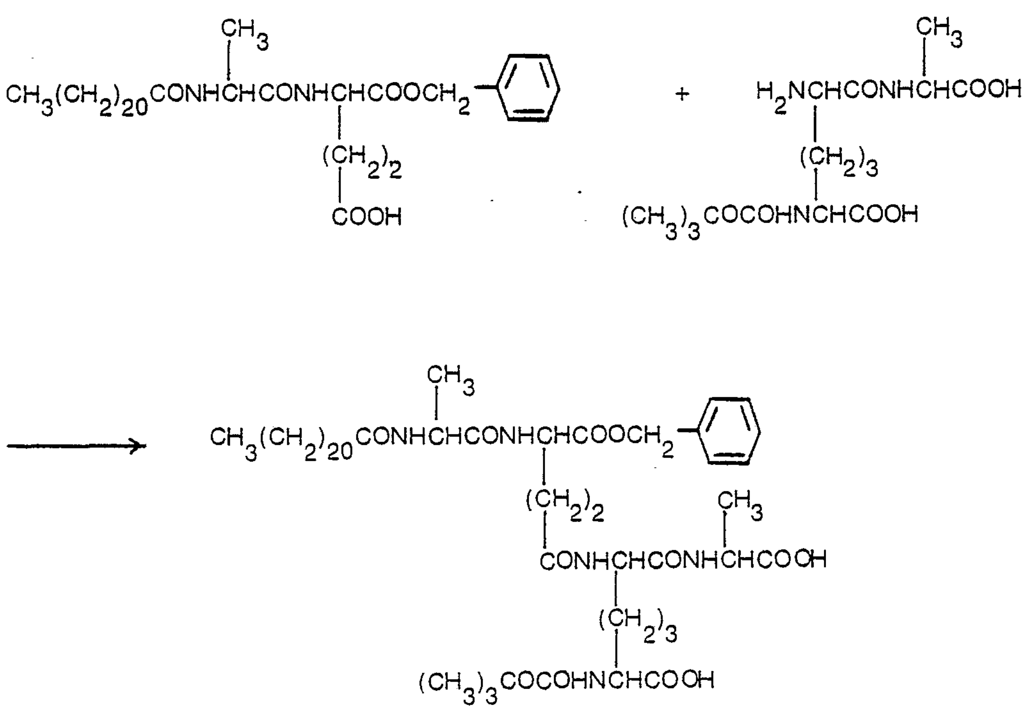

(4)

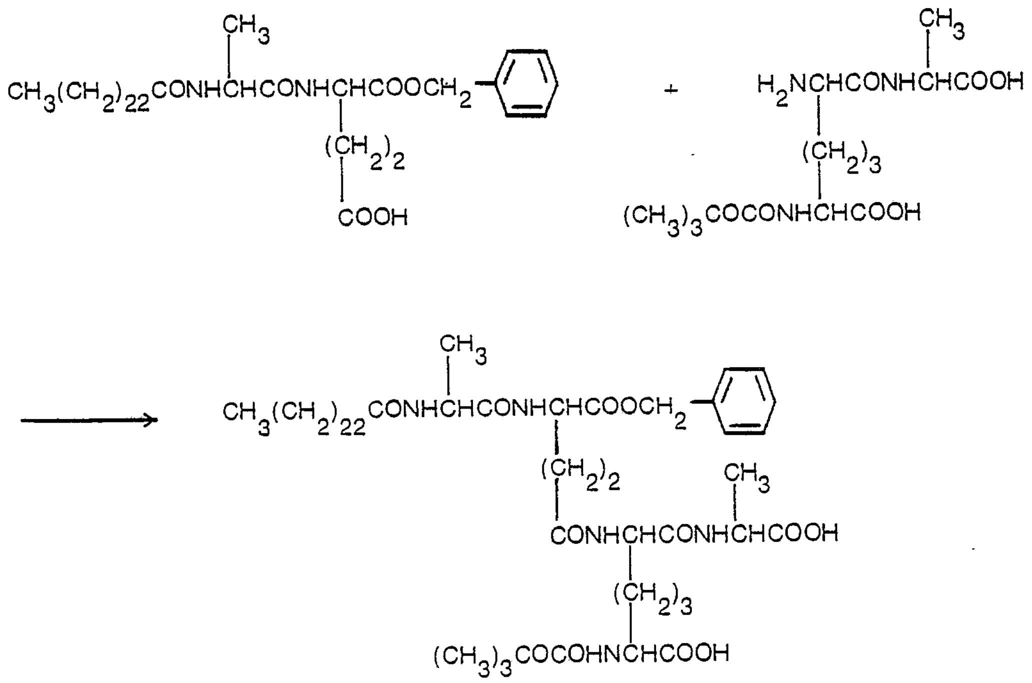

(5)

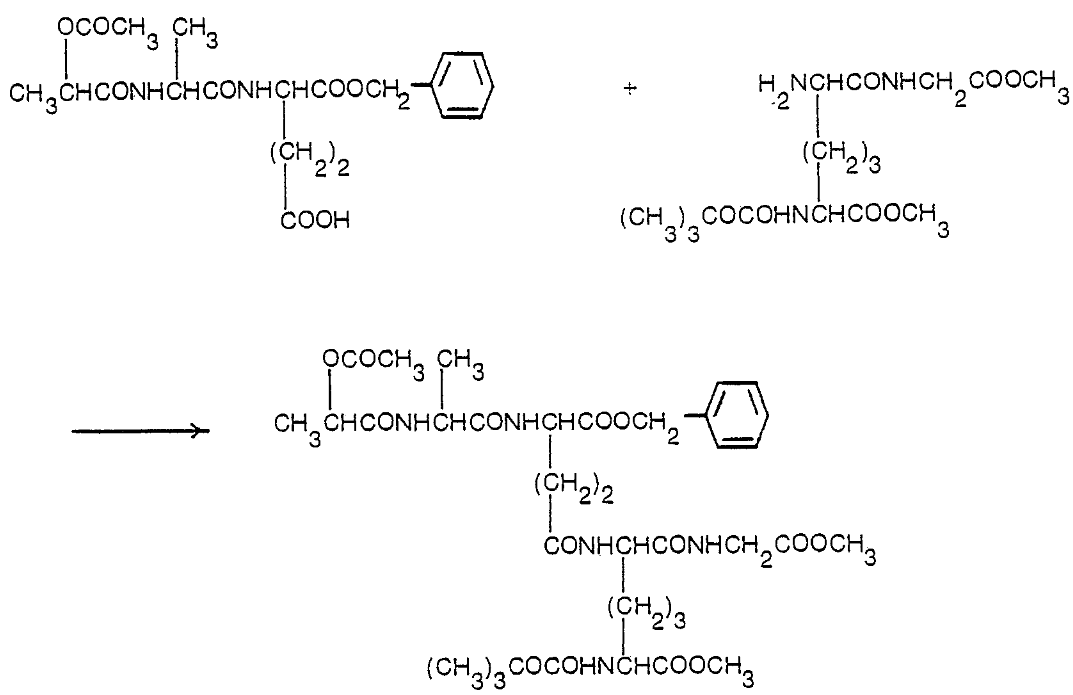

(6)

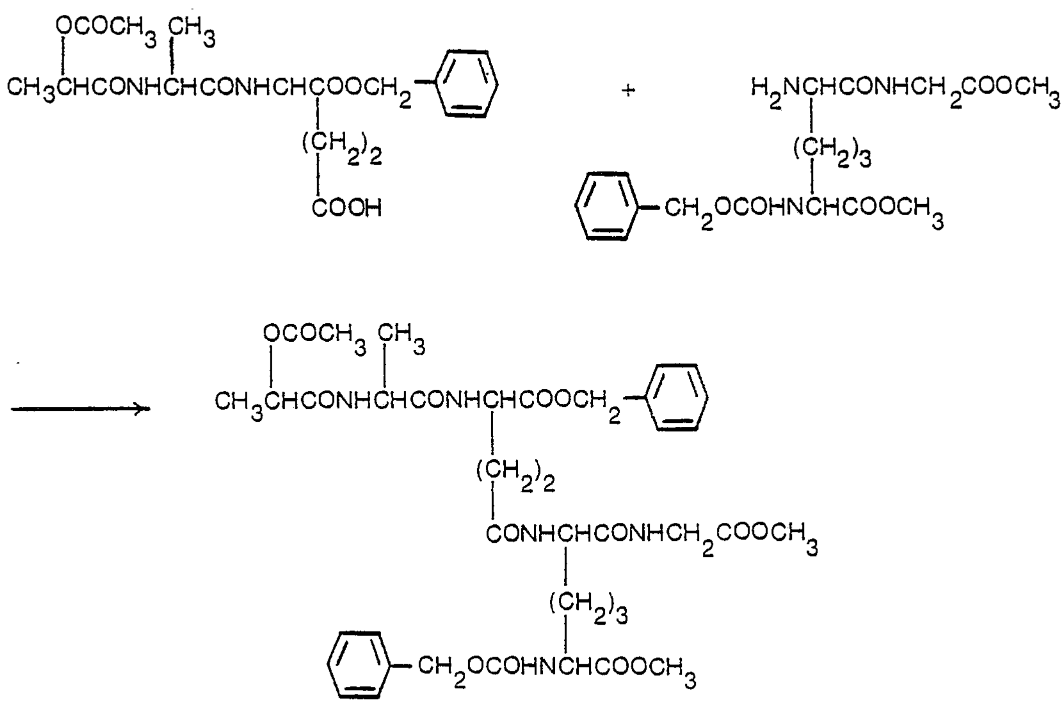

(7)

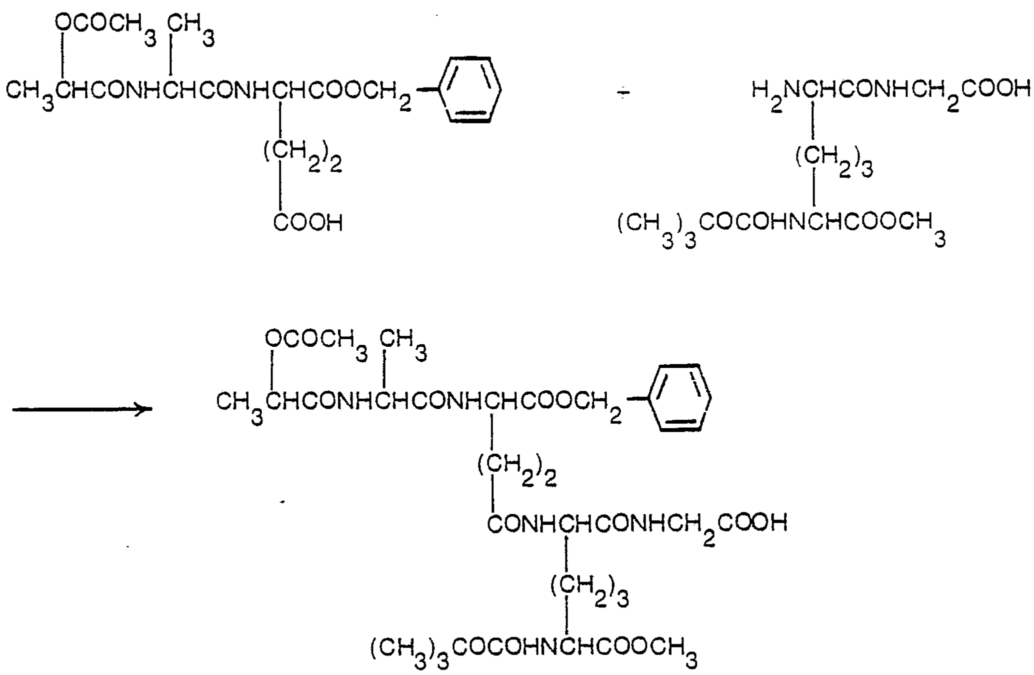

(8)

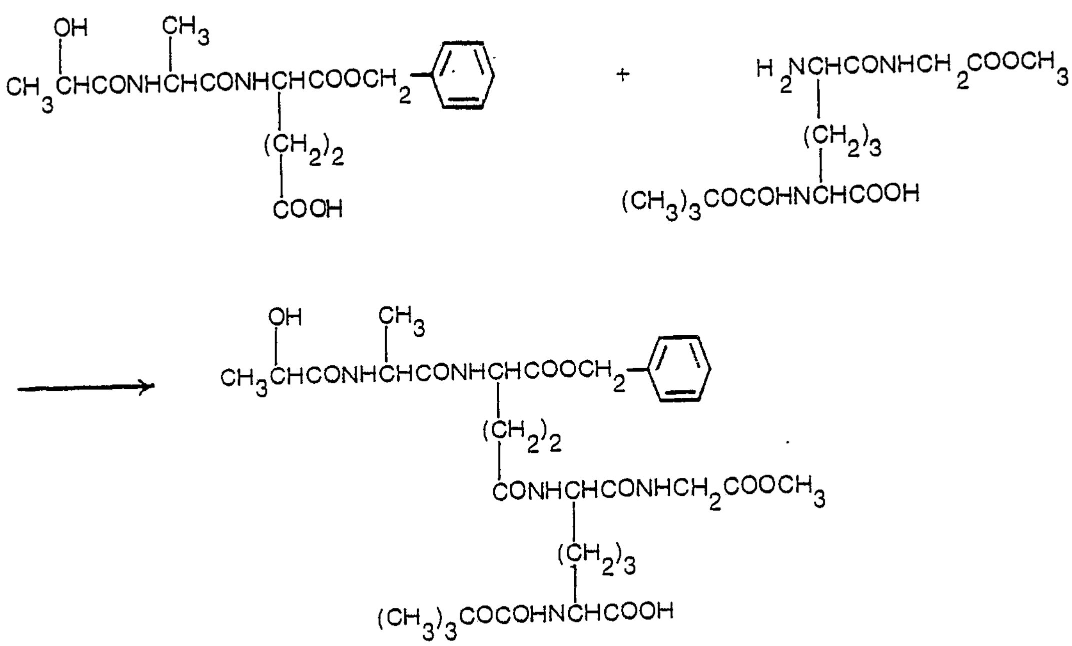

(9)

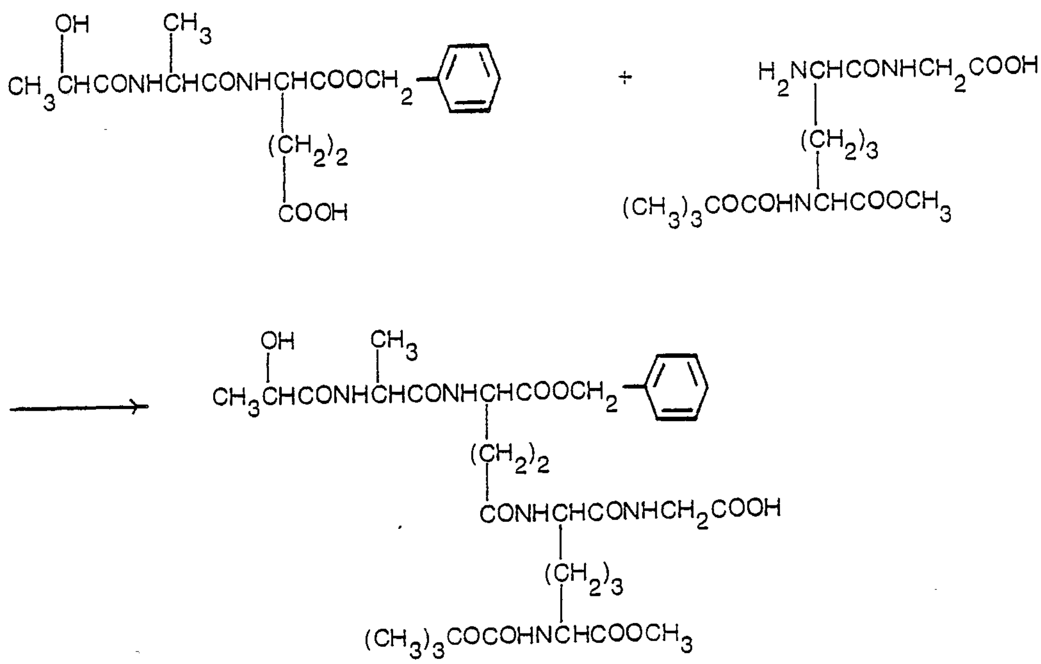

(10)

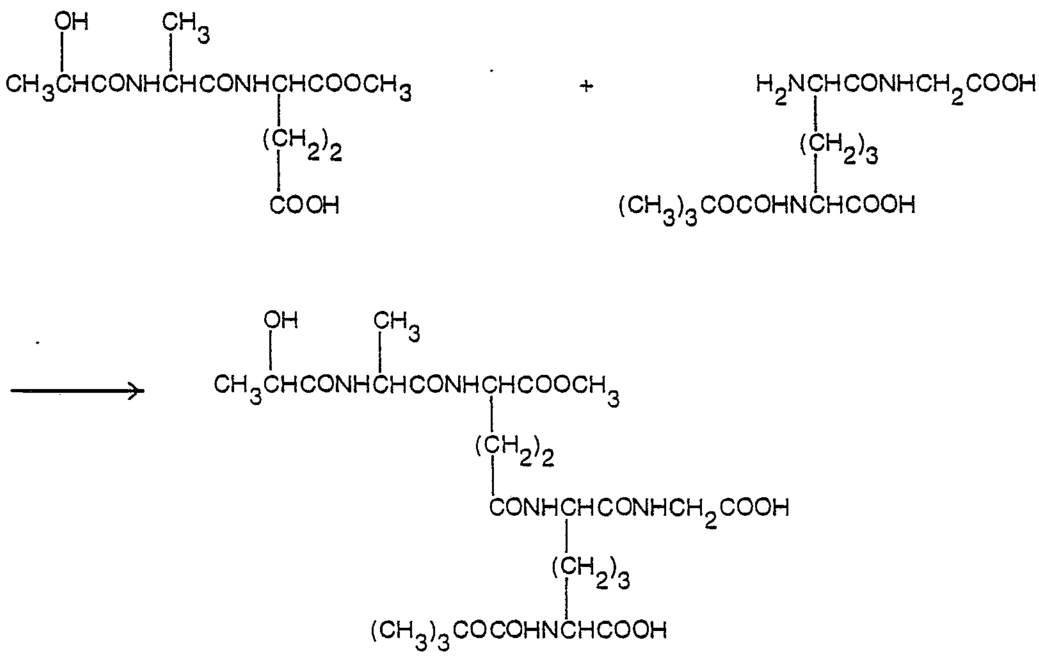

(11)

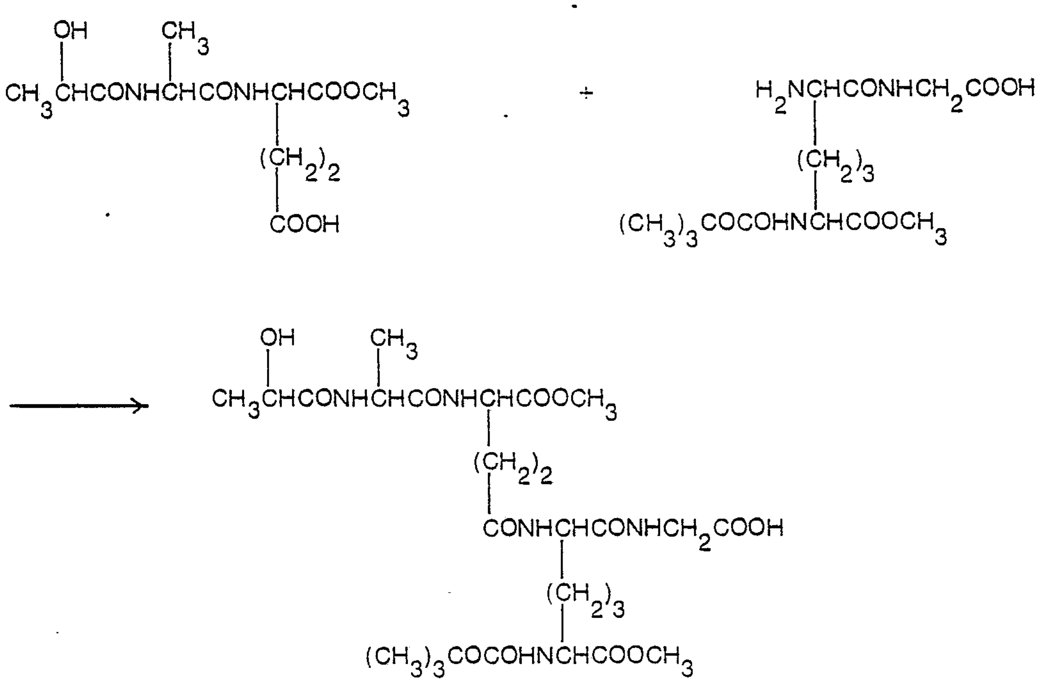

(12)

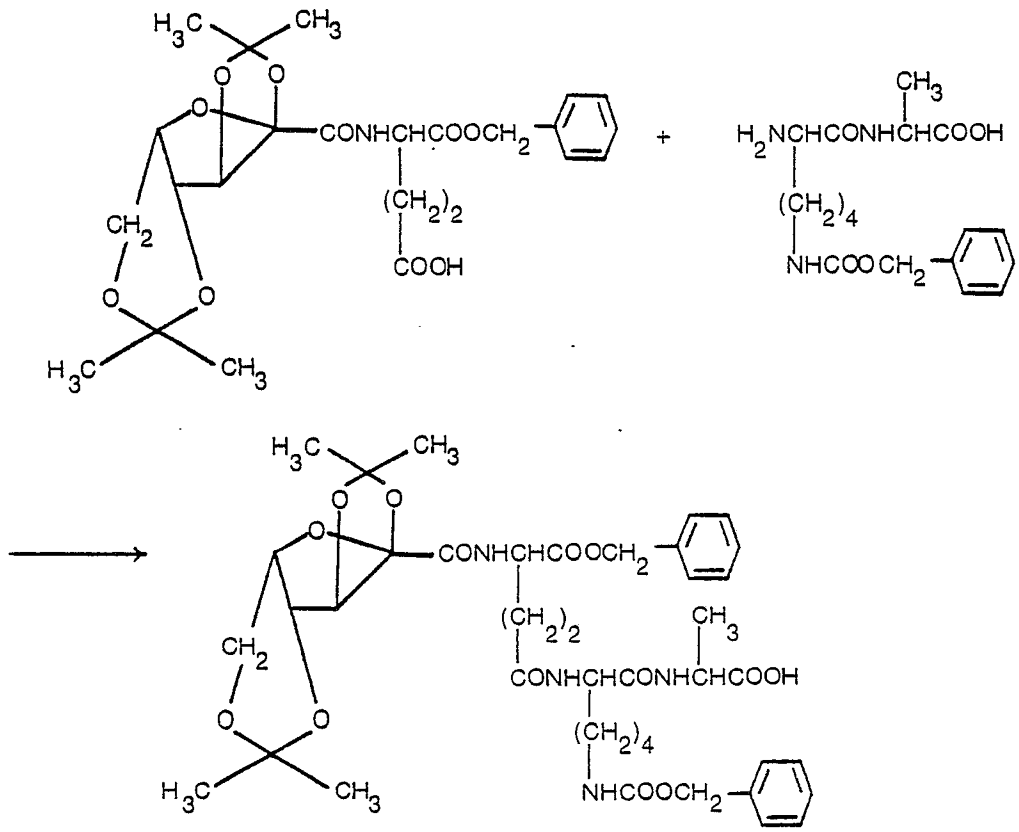

(13)

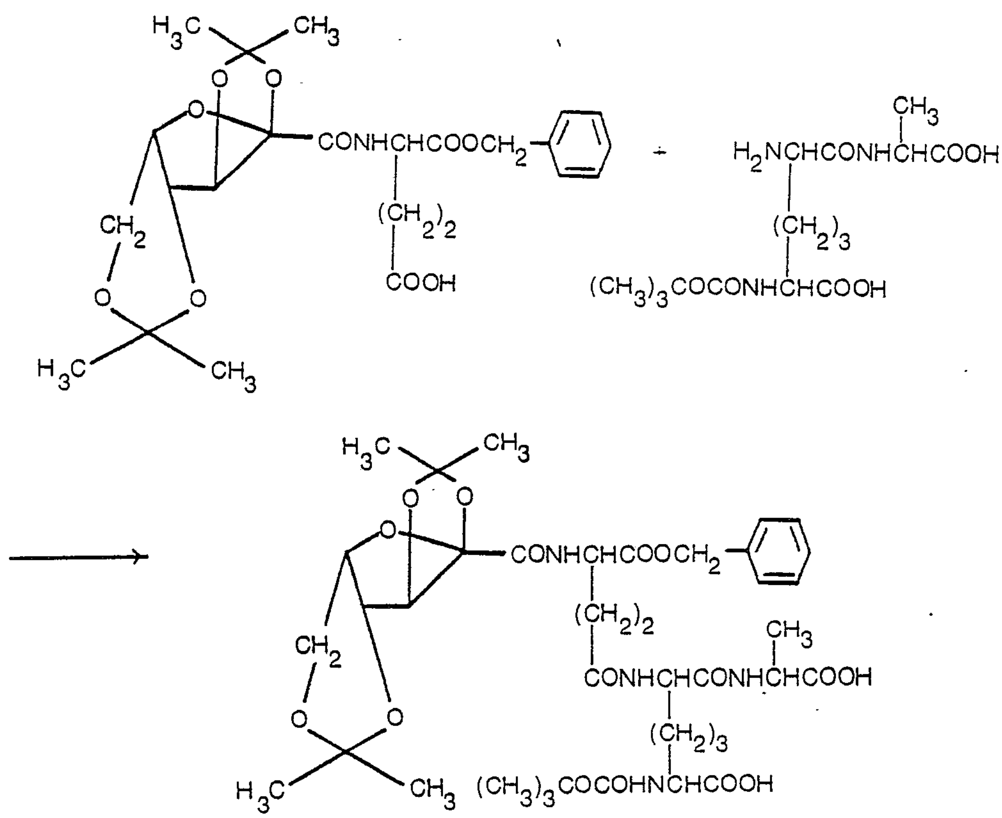

(14)

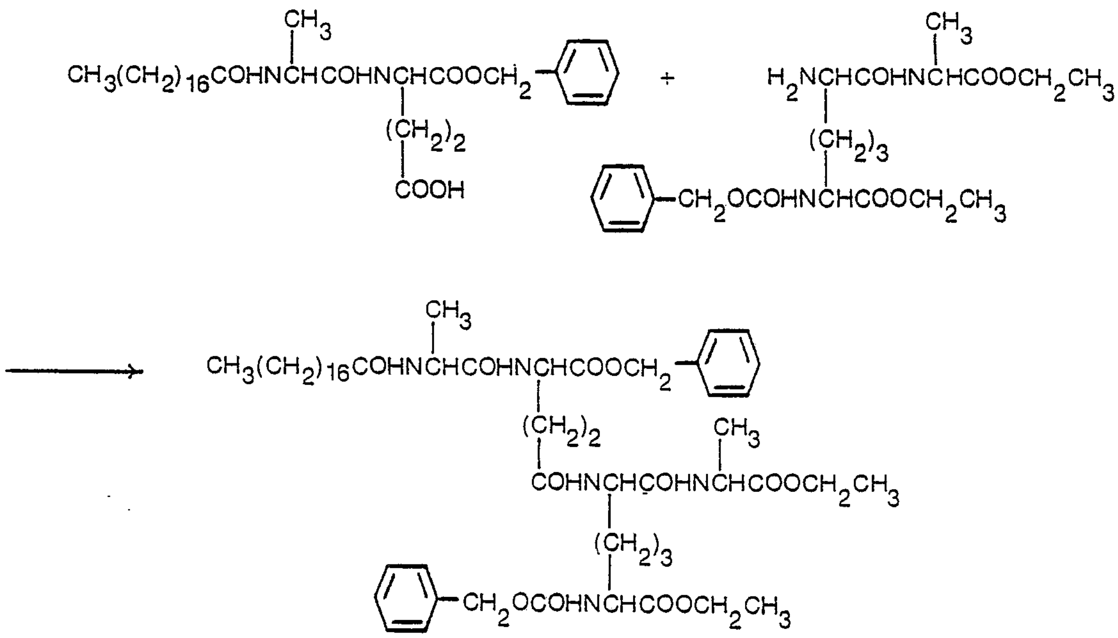

(15)

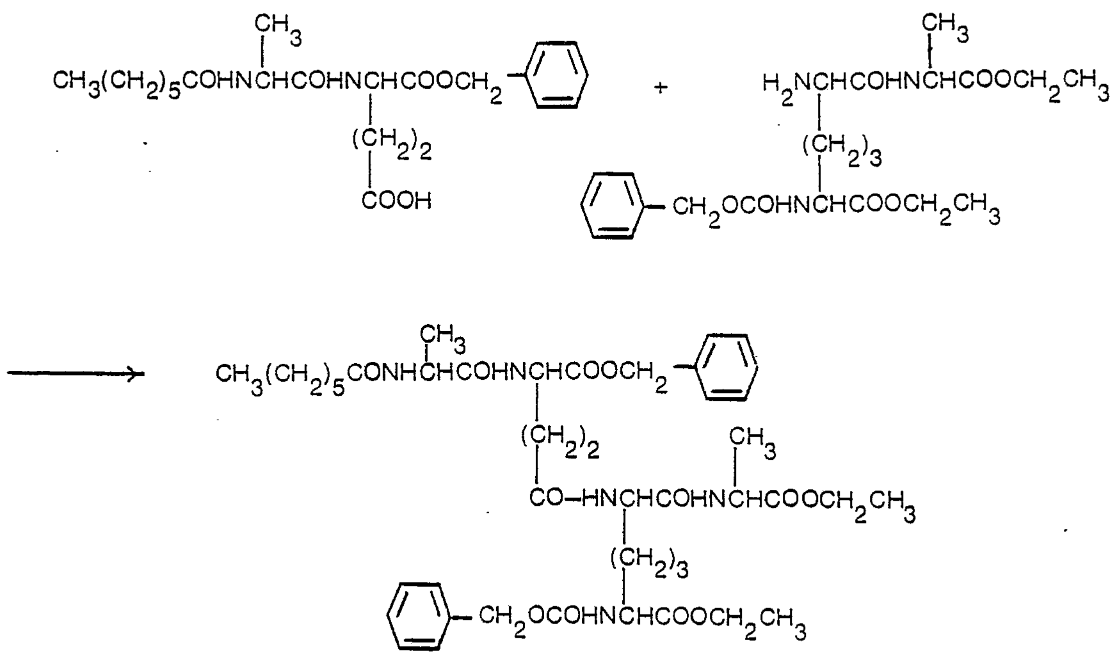

(16)

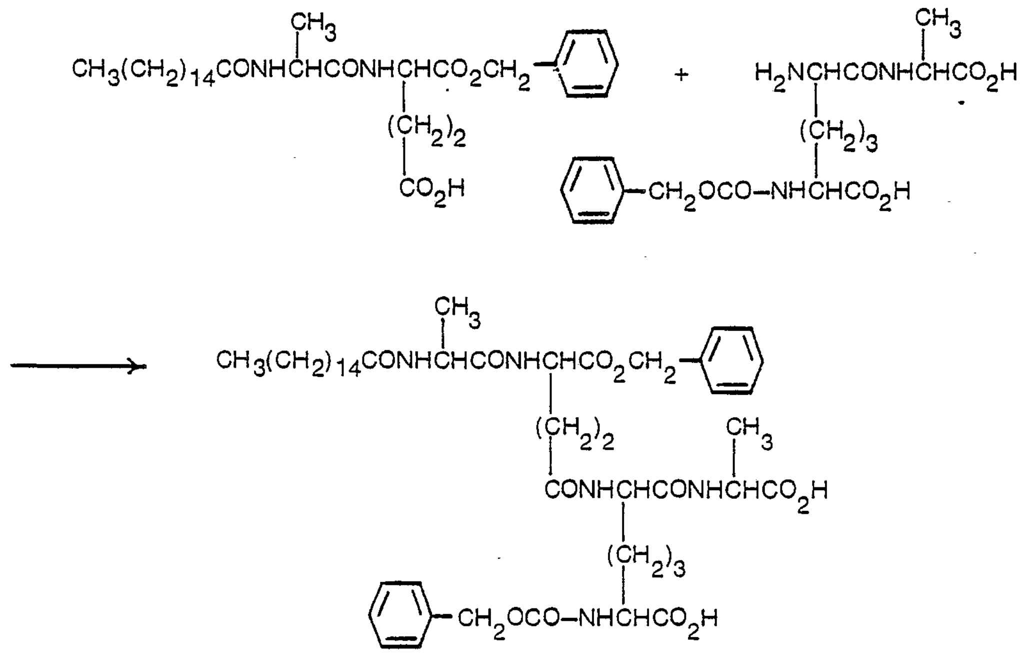

(17)

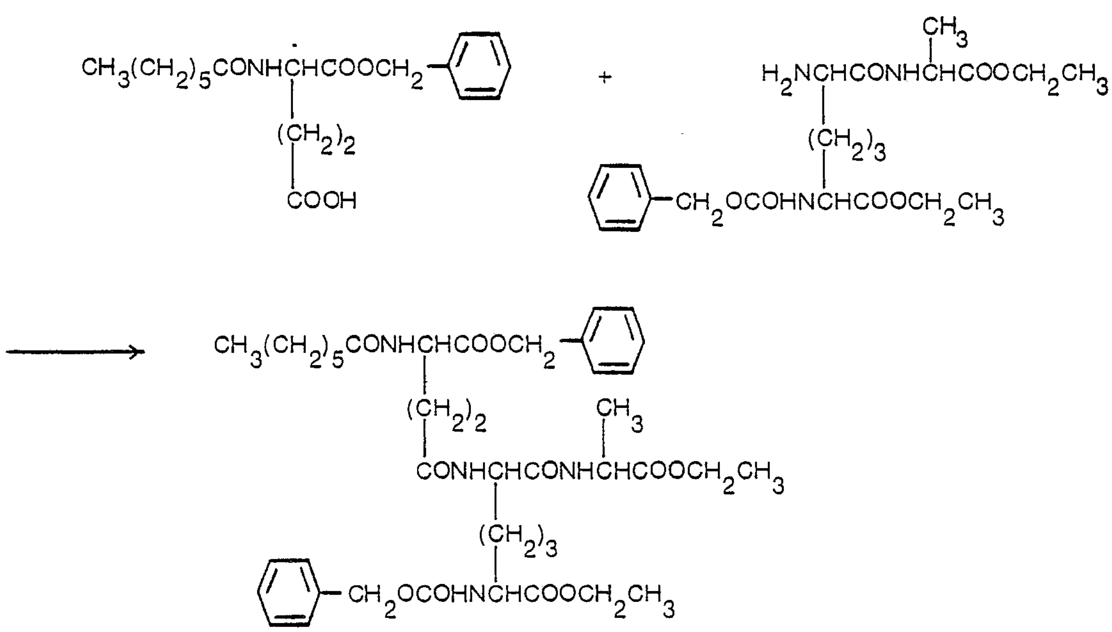

(18)

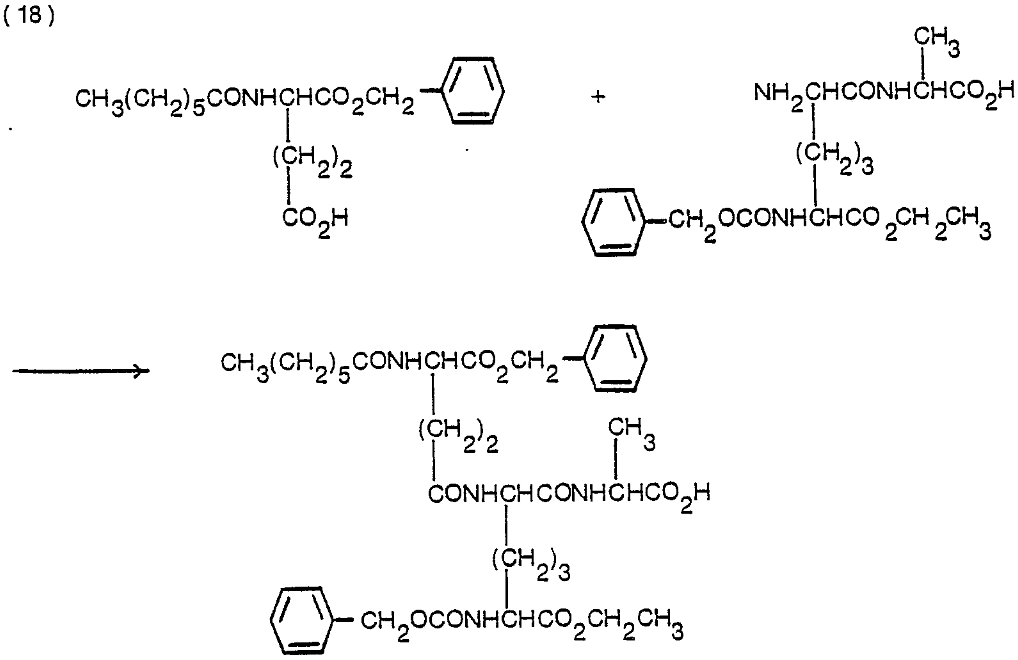

(II) Acylation:

(1)

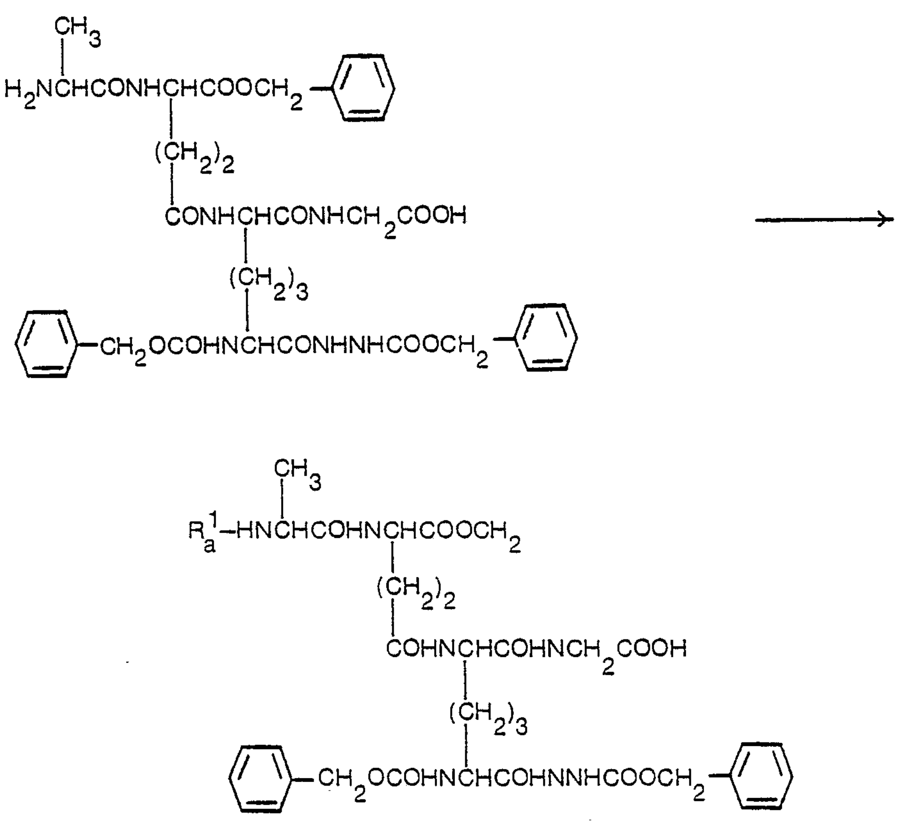

wherein

$R_a^1$ is 1-N-n-hexyl-2,3,4,5-O-tetraacetyl-D-glucaramoyl, 1-N-benzyl-2,3,4,5,-O-tetraacetyl-D-glucaramoyl or 1-N-lauryl-2,3,4,5-O-tetraacetyl-D-glucaramoyl.

(2)

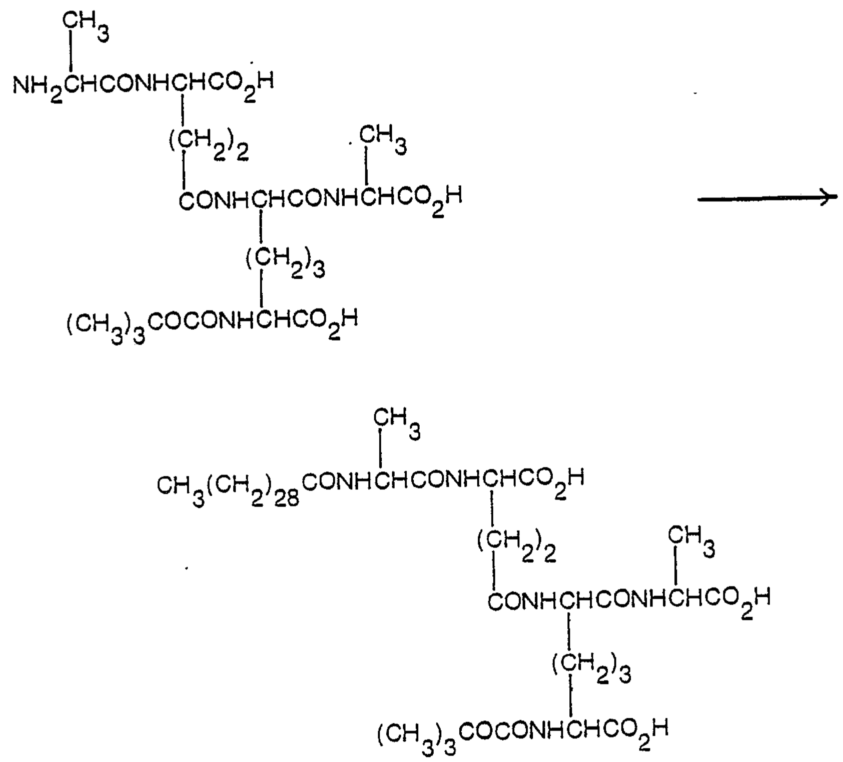

(III) Methylation:

(1)

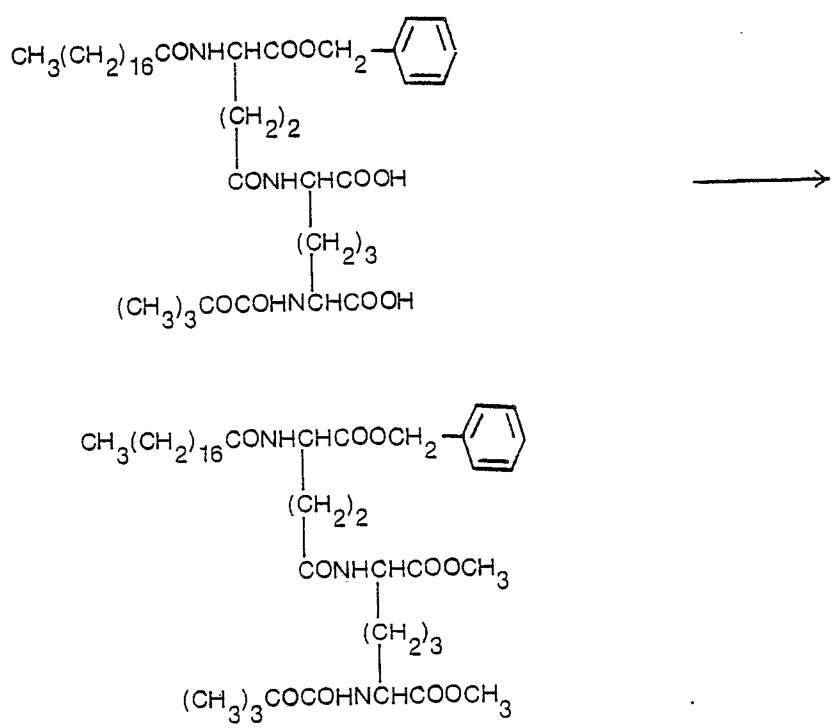

(2)

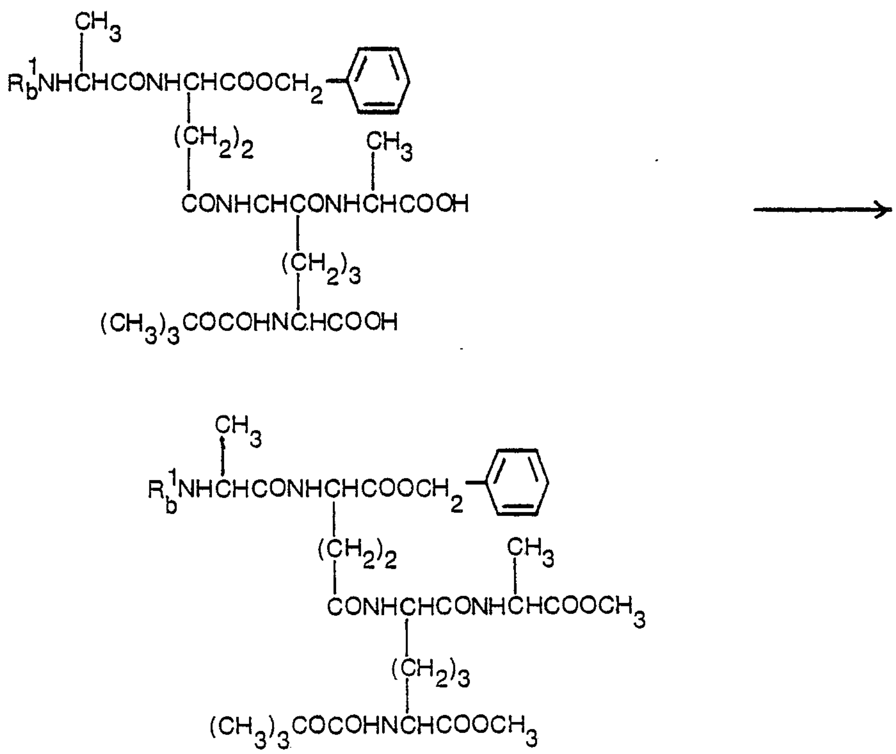

wherein $R_b^1$ is n-docosanoyl or n-tetracosanoyl,

(3)

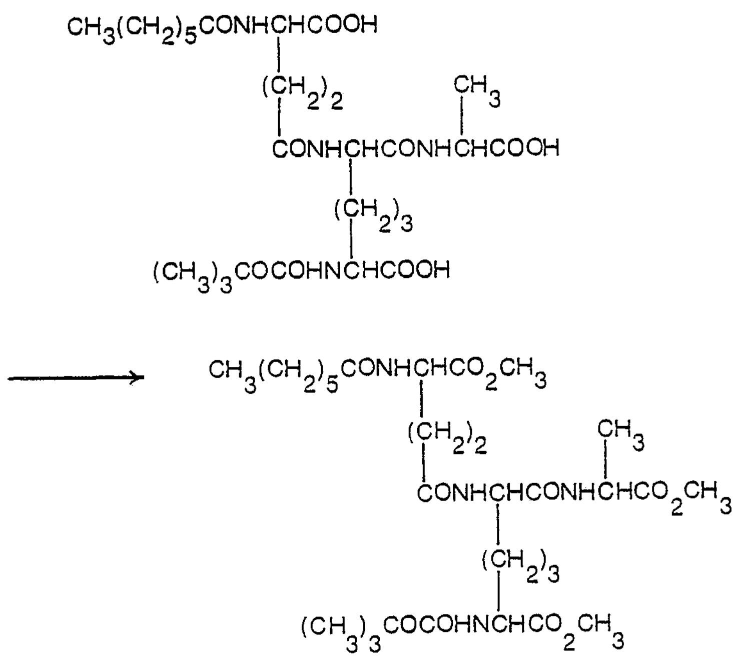

(4)

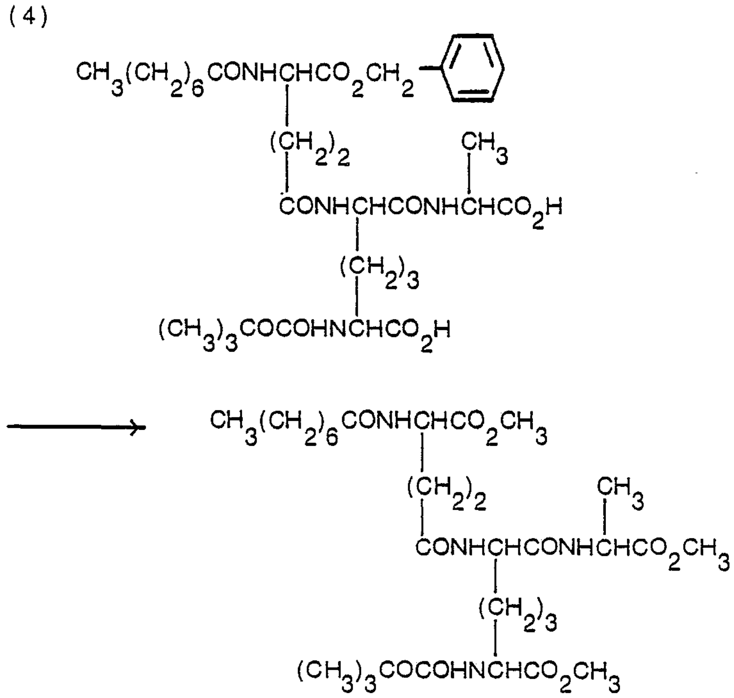

(IV) Elimination of protective groups:

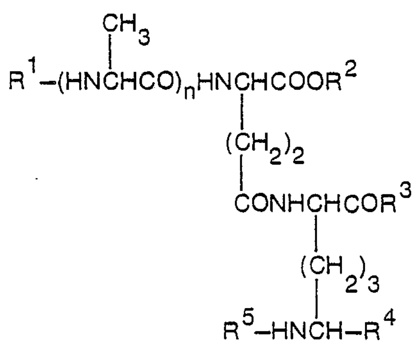

($I^a$)

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is stearoyl, n is an integer of 0, $R^2$ is benzyl, $R^3$ is methoxy, $R^4$ is methoxycarbonyl and $R^5$ is hydrogen; or

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-docosanoyl, n is an integer of 1, $R^2$ is benzyl, $R^3$ is 1-carboxyethylamino, $R^4$ is carboxy and $R^5$ is t-butoxycarbonyl;

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is octanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is 2-hydroxypropionyl, n is an integer of 1, $R^2$ is benzyl, $R^3$ is methoxycarbonylmethylamino, $R^4$ is carboxy and $R^5$ is t-butoxycarbonyl; or

$R^1$ is 2-hydroxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 1-N-n-hexyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2-keto-L-gulonoyl, n is an integer of 0, $R^2$ is benzyl, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is 2-keto-L-gulonoyl, n is an integer of 0, $R^2$ is benzyl, $R^3$ is 1-carboxyethylamino, $R^4$ is carboxy and $R^5$ is hydrogen; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is 1-N-benzyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is triacontanoyl, n is an integer of 1, $R^2$ is hydrogen, $R^3$ is 1-carboxyethylamino, $R^4$ is carboxy and $R^5$ is t-butoxycarbonyl;

$R^1$ is heptanoyl, n is an integer of 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl,

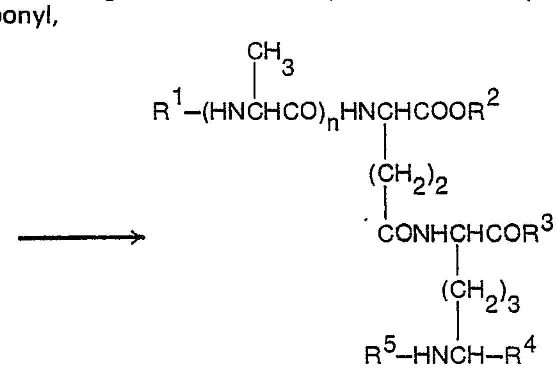

($I^b$)

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is benzyl, $R^3$ is methoxy, $R^4$ is methoxycarbonyl and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-tetracosanoyl, n is an integer of 1, $R^2$ is hydrogen, $R^3$ is 1-carboxyethylamino, $R^4$ is carboxy and $R^5$ is hydrogen; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is octanoyl, n is an integer of 0, $R^2$ is methyl, $R^3$ is 1-methoxycarbonylethylamino, $R^4$ is methoxycarbonyl and $R^5$ is hydrogen; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-n-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$ is 1-carboxyethylamino, $R^4$ is carboxy and $R^5$ is hydrogen; or

$R^1$ is stearoyl, n is an integer of 1, $R^2$ is hydrogen, $R^3$ is 1-ethoxycarbonylethylaminio, $R^4$ is ethoxycarbonyl and $R^5$ is hydrogen; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is hydrogen; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is hydrogen, $R^3$ is 1-carboxyethylamino, $R^4$ is ethoxycarbonyl and $R^5$ is hydrogen.

(I) Peptide bond formation [(1) to (18)]:

The reaction of this process can be conducted as follows.

That is, in one case, as the first step, the carboxy group of each carboxy compound or its salt is usually activated in a conventional manner, for example, in the form of its acid halide, azide, acid anhydride or a mixed anhydride, activated ester, and the like, and is reacted with each amino compound to give the object compound, and in the other case each carboxy compound or its salt is reacted with

each amino compound or its salt directly in the presence of a conventional condensing agent such as N,N-dicyclohexylcarbodiimide and the like. Among these activation methods, preferred activation method for the carboxy group of each carboxy compound into its activated form and preferred condensing agent as mentioned above are selected according to kind of the carboxy protective group(s) of each carboxy compound and each amino compound and to the reaction conditions (e.g. the kinds of the reaction solvent, reaction temperature and so on).

This reaction is preferably carried out in a solvent such as methylene chloride, chloroform, tetrahydrofuran, dioxane, ethyl acetate, methanol, ethanol, water or the like under at —20°C to at ambient temperature and the reaction in the presence of a condensing agent is usually carried out in an anhydrous, but not critical conditions.

(II) Acylation [(1) and (2)]:

The reaction of this process is conducted by reacting each starting compound or its salt with 1 - N - n - hexyl - 2,3,4,5 - O - tetraacetyl - D - glucaramic acid, 1 - N - benzyl - 2,3,4,5 - O - tetraacetyl - D - glucaramic acid, 1 - N - lauryl - 2,3,4,5 - O - tetraacetyl - D - glucaramic acid or triacontanoic acid, or their reactive derivatives.

As such reactive derivatives, there may be exemplified an acid halide, an acid azide, an acid anhydride, an activated amide, an activated ester, etc.

In the reaction, when a free acid is used as an acylating agent, the acylation may preferably be conducted in the presence of a conventional condensing agent.

The reaction is usually conducted in a solvent such as water, alcohol (e.g. methanol, ethanol, propanol, etc.), acetone, ethyl ether, dioxane, acetonitrile, ethyl acetate, methylene chloride or other conventional solvents, or a mixture thereof.

The reaction may also be preferably conducted in the presence of a conventional organic or inorganic base.

There is no limitation to this reaction temperature and this reaction may preferably be conducted within the range of cooling to ambient temperature.

(III) Methylation [(1) to (4)]:

The reaction of this process is conducted by reacting each starting compound with a methylating agent.

As such a methylating agent, there may be exemplified a conventional one such as methanol or its reactive equivalent (e.g. halide, sulfate, aliphatic or aromatic sulfonate or the corresponding diazo compound, etc.) or the like.

The reaction is usually conducted in a solvent such as tetrahydrofuran, chloroform or other conventional solvent, or a mixture thereof.

The reaction is usually conducted within the range of cooling to ambient temperature.

(IV) Elimination of protective groups:

This process relates to a method for preparing the compound ($I^b$) or its salt by subjecting the compound ($I^a$) or its salt to an elimination reaction of amino and (or) carboxy protective group(s).

(IV-1), Elimination of amino protective group:

This reaction is carried out by conventional methods such as catalytic reduction method, liquid-ammoniaalkalimetal method, acid method, zinc acid method, base method, hydrazine method or the like.

Among the above elimination methods, preferred one is selected according to kinds of the amino protective group.

This reaction is usually conducted under ice-cooling to ambient temperature.

(IV-2), Elimination of carboxy protective group:

This reaction is conducted by a conventional method such as hydrolysis and reduction or the like.

Hydrolysis is preferably conducted in the presence of an acid or base under somewhat milder conditions such as cooling or warming and usually in a conventional solvent.

Reduction, including chemical reduction and catalytic reduction, is conducted in a conventional manner.

The reduction is usually conducted in a conventional solvent under somewhat milder conditions such as cooling or warming.

(IV-3), Removal of hydrazino group:

A protected carbazoyl of the formula: —CONHNHY wherein Y is an amino protective group, can be removed by subjecting the compound ($I^a$) at first to the reaction of process (IV-1) for eliminating the amino protective group (i.e. Y) to give $—CONHNH_2$ group and then subjecting the reaction product to the reaction of this step to give —COOH group.

The reaction of this step is conducted in a conventional manner by treating the compound ($I^a$)

with a conventional oxidising agent which is capable of oxidising a group of the formula: —$CONHNH_2$ to form into a group of the formula: —COOH.

The reaction is usually conducted in a conventional solvent under ice-cooling to ambient temperature.

It is noted that this process includes the following cases of elimination of the carboxy protective and the amino protective group. That is, one case is that all of the carboxy protective groups and the amino protective group in the Compound ($I^a$) are simultaneously removed by a method to be employed to the reaction, and the other case is that the carboxy protective groups and the amino protective group are sequentially and stepwise removed by a method which is appropriately selected according to the kinds of the protective group to be removed.

As to Process (IV) for elimination of protective group(s) (i.e. Processes IV-1 to IV-3), the followings are to be noted. That is, in case that the acyl group for $R^1$ has one or more protective group(s) for hydroxy, such a hydroxy protective group such as alkanoyl (e.g. acetyl, etc.) may be previously or later removed by subjecting the compound ($I^a$) to elimination reaction of hydroxy protective group in a conventional manner such as reduction as illustrated in the Process IV-1 or IV-2.

The starting compounds include known compounds (e.g. European Patent publication Nos. 11283) and new compounds. Said new compounds can be prepared by the similar methods thereto.

As to the object compound (I) and starting compounds which are prepared according to the aforementioned processes, it is to be noted that each of said compounds includes one or more stereoisomers which is due to the asymmetric carbon atoms in their molecule and all of such isomers are included within the scope of this invention.

The new peptide (I) and its pharmaceutically acceptable salts of this invention have been found to possess protective efficacy in experimental infection and antitumor activity.

Accordingly, the new peptide (I) and its pharmaceutically acceptable salts are useful for the therapeutic treatment of infectious diseases caused by pathogenic microorganism, especially gram-negative bacteria and gram-positive bacteria and fungi, and of tumor in human being and animals.

Further, the starting compounds are useful as intermediate for preparing Compound (I) having biologically active properties as mentioned above.

For the purpose of showing pharmaceutical utility of the new peptide (I), pharmacological data thereof are illustrated in the following.

PROTECTIVE EFFICACY IN EXPERIMENTAL INFECTION IN MICE

In determining the protective efficacy against experimental infections in mice, the test compound was dissolved in and diluted with sterile saline to provide prescribed concentration of drug.

Male ICR-strain mice, aged 4 weeks were used in groups of ten mice. E. coli 22 was cultivated overnight at 37°C on trypticase soy agar and then were suspended in a sterile saline to obtain microbial cell concentration of $2.6 \times 10^9$ CFU/ml. Mice were inoculated intra-peritoneally with $8.7 \times 10^7$ CFU/mouse. Each of the test drugs was given intraperitoneally in various doses to a group of ten mice four days before challenge.

Several percent were found from the number of the surviving animals after three days of injection. Results are shown in Table.

| Test Compound (Example No.) | Survival (%) | | | |
|---|---|---|---|---|
| | Dose (mg/kg) | | | |
| | 0 | 0.01 | 0.1 | 1 |
| 1 (Step 2) | 10 | 70 | 50 | 90 |
| 2 (Step 2) | 10 | 50 | 70 | 90 |
| 3 (Step 2) | *0 | 30 | 70 | 100 |
| 4 (Step 3) | 10 | 40 | 80 | 80 |
| 5 | 10 | 40 | 50 | 70 |
| 6 (Step 3) | *0 | 20 | 50 | 80 |
| 10 (Step 3) | 20 | 60 | 80 | 80 |
| 12 (Step 2) | 10 | 70 | 80 | 80 |
| 13 (Step 2) | 10 | 20 | 80 | 90 |
| 14 (Step 3) | *0 | 40 | 70 | 60 |
| 14 (Step 4) | *0 | 30 | 70 | 60 |
| 17 (Step 2) | *0 | 10 | 0 | 50 |
| 19 (Step 3) | *0 | 30 | 30 | 70 |
| 20 (Step 2) | **10 | 60 | 80 | 100 |
| 22 (Step 3) | *0 | 10 | 30 | 50 |
| 22 (Step 4) | ***20 | 80 | 80 | 50 |
| 23 (Step 3) | *0 | 10 | 20 | 70 |
| 23 (Step 4) | 0 | 30 | 30 | 50 |
| 25 (Step 2) | 10 | 40 | 80 | 70 |
| 26 (Step 2) | 10 | 20 | 60 | 70 |

(continued)

| Test Compound (Example No.) | Survival (%) | | | |
|---|---|---|---|---|
| | Dose (mg/kg) | | | |
| | 0 | 0.01 | 0.1 | 1 |
| 27 (Step 2) | 20 | 40 | 80 | 80 |
| 28 (Step 2) | *0 **** | 70 | 90 | 90 |

Note:
- * Observation of surviving animals: 1 day after infection
- ** Observation of surviving animals: 2 days after infection
- *** Infection: $1.8 \times 10^7$ cells/mouse
- **** Infection: $7.5 \times 10^7$ cells/mouse

ANTITUMOR ACTIVITY

(1) Methylcholanthlene-induced fibrosarcoma (Meth-A) was used. A mixture of the tumor cells ($1 \times 10^5$) and the test compound was suspended in a 0.5% methylcellulose saline solution. The suspension was inoculated intradermally in male BALB/mice. Three weeks after inoculation, tumor size was measured.

Results are shown in the following table.

| Test compound (Example No.) | Dose µg/site | Suppression of Meth-A growth |
|---|---|---|
| 25 (Step 2) | 100 | 8/8 |
| Control | 0 | 0/8 |

Note:
Values gives Number of tumor-free mice/Number of mice tested, provided that when the perpendicular diameter of the tumor was less than 5 mm, the animal was defined as free of tumor.

(2) Methylcholanthlene-induced fibrosarcoma (Meth-A) was used. The test compound was given to BALB/C mice intrapleurally 14 days before, and 1 hour and 3 days after the intrapleural inoculation of Meth-A, respectively.

The antitumor effect of the test compound was estimated by the prolongation of life-span in mice bearing Meth-A.

Results are shown in the following table.

| Test compound (Example No.) | Dose µg/mouse | MST | T/C |
|---|---|---|---|
| 2 (Step 2) | 400 | 14.5 | 2.07 |
| Control | 0 | 7.0 | |
| 3 (Step 3) | 400 | 11.5 | 1.64 |
| Control | 0 | 7.0 | |

Note:
MST means median survival time.

The pharmaceutical composition of this invention can be used in the form of a pharmaceutical preparation, for example, in solid, semisolid or liquid form, which contains an active substance of this invention in admixture with an organic or inorganic carrier or excipient suitable for external, enteral or parenteral applications. The active ingredient may be compounded, for example, with the usual nontoxic, pharmaceutically acceptable carriers for tablets, pellets, capsules, suppositories, solutions, emulsions, suspensions, and any other form suitable for use. The carriers which can be used are water, glucose, lactose, gun acacia, gelatin, mannitol, starch paste, magnesium trisilicate, talc, corn starch, keratin, collidal silica, potato starch, urea and other carriers suitable for use in manufacturing preparations, in solid, semisolid, or liquid form, and in addition auxiliary, stabilizing, thickening and coloring agents and perfumes may be used. The pharmaceutical compositions can also contain preservative or bacteriostatic agents to keep the active ingredient in the desired preparations stable in activity. The active object compound is included in the pharmaceutical composition in an amount sufficient to produce the desired therapeutic effect upon the process or condition of diseases.

For applying this composition to humans, it is preferably to apply it by intravenous, intramuscular or oral administration. While the dosage or therapeutically effective amount of the object compound of this invention varies from and also depends upon the age and condition of each individual patient to be treated, a daily dose of about 0.1—100 mg of the active ingredient/kg of a human being or an animal is generally given for treating diseases, and an average single dose of about 50 mg, 100 mg, 250 mg, and 500 mg is generally administered.

The following examples are given for purpose of illustrating this invention.

In the following examples, starting compounds and object compounds are expressed by using the following abbreviations:

| | | |
|---|---|---|
| Lac | : | Lactoyl |
| Ala | : | Alanyl |
| Glu | : | Glutamyl |
| Gly | : | Glycyl |
| DAP | : | $\alpha$, $\varepsilon$-Diaminopimelyl |
| Z | : | benzyloxycarbonyl |
| Boc | : | t-butoxycarbonyl |
| Bzl | : | benzyl |
| Ac | : | acetyl |
| Su | : | N-hydroxysuccinimide |
| Me | : | methyl |
| Et | : | ethyl |
| Lys | : | Lysyl |

Preparation 1

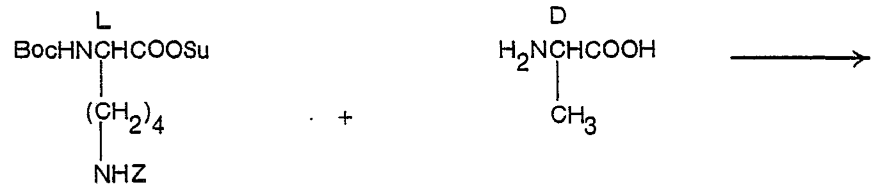

(1) (2)

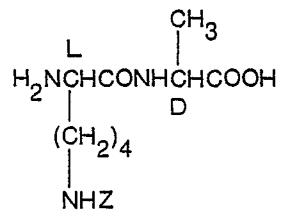

(3)

D-AlaOH (2) (1.78 g) was dissolved in a mixture of water (40 ml), dioxane (40 ml) and triethylamine (4.04 g).

To this solution was added Boc-L-Lys(ε-Z)OSu (1) (9.26 g) and the resulting solution was left overnight at ambient temperature and then filtered.

The filtrate was evaporated to give an oily residue which was dissolved in water. The solution was acidified with dil hydrochloric acid and then extracted with ethyl acetate. The organic layer was washed with water, dried over magnesium sulfate and then evaporated to give a white foam. The foam was dissolved in trifluoroacetic acid (30 ml) and reacted for 30 minutes at ambient temperature. Excess trifluoroacetic acid was evaporated to give a paste which was dissolved in water.

The solution was passed through HP20 column.

The column was eluted with water and water-methanol (1:1), successively. The latter fractions were combined and evaporated to give L-Lys(ε-Z)-D-AlaOH (3) (4.50 g).

IR (Nujol): 3350, 3300, 1685, 1660, 1640 $cm^{-1}$

NMR ($CD_3OD$) $\delta$: 1.30 (3H, d, J=7Hz), 1.20—1.70 (6H, m), 2.86—3.50 (2H, m), 4.16 (1H, q, J=7Hz), 5.00 (2H, s), 7.30 (5H, s)

Preparation 2

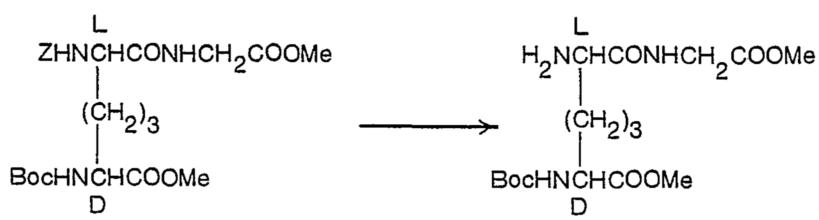

(1) (2)

Z-(L)-Boc-(D)-mesoDAP-(L)-GlyOMe-(D)-OMe (1) (0.66 g) was dissolved in methanol (10 ml) and hydrogenated over 10% palladium charcoal (0.12 g). After removal of the catalyst by filtration, the filtrate was concentrated under reduced pressure to give Boc-(D)-mesoDAP-(L)-GlyOMe-(D)-OMe (2) (0.48 g).

IR ($CH_2Cl_2$): 3400, 3360, 1735, 1705, 1670 $cm^{-1}$

NMR ($CDCl_3$) $\delta$: 1.42 (9H, s), 1.3—2.2 (6H, m), 3.3—3.6 (1H, m), 3.77 (6H, s), 4.05 (2H, d, J=5Hz), 4.1—4.5 (1H, m)

Preparation 3

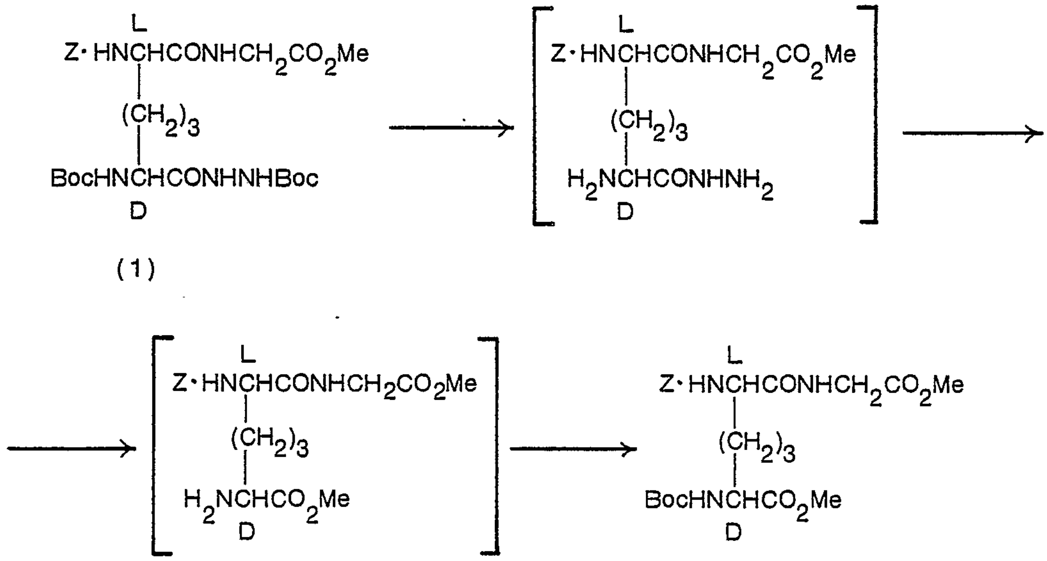

(1)

(2)

Z-(L)-Boc-(D)-mesoDAP-(L)-GlyOME-(D)-NHNHBoc (1) (1.8 g) was added to trifluoroacetic acid and the mixture was stirred for 15 minutes at room temperature. After evaporation of the solvent, the residue was dissolved in methanol (50 ml) and treated with N-bromosuccinimide (1.58 g), and the mixture was stirred for 10 minutes at 0°—5°C and concentrated in vacuo. The residue was dissolved in water (40 ml) and the excess reagent was decomposed by adding 5% sodium bisulfite. The mixture was neutralized to pH 9 with sodium bicarbonate, and extracted with, ethyl acetate (40 ml). The extract was washed with water and dried over magnesium sulfate, and the solvent was removed in vacuo. The residue was dissolved in a mixture of dioxane (20 ml) and water (10 ml). To this mixture triethylamine (0.66 g) and di-t-butyldicarbonate (1.42 g) was added and stirred for an hour at room temperature. After evaporation of the solvent, the residue was dissolved in ehtylacetate (40 ml), and washed successively with 2.5% hydrochloric acid (15 ml) and water (20 ml), dried over magnesium sulfate and evaporated. The residue was pulverized with isopropylether to give Z-(L)-Boc-(D)-mesoDAP-(L)-GlyOMe-(D)-OMe (2) (0.7 g).

NMR ($CD_3OD$) $\delta$: 1.43 (9H, s), 1.4—2.0 (6H, m), 3.72 (6H, s), 3.8—4.3 (4H, m), 5.12 (2H, s), 7.35 (5H, s)

Preparation 4

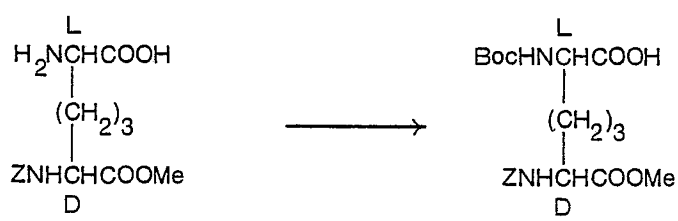

(1) (2)

To a mixture of Z-(D)-mesoDAP-(D)-OMe (1) (90 mg) and triethylamine (50 mg) in 50% aqueous dioxane was added di-t-butyldicarbonate (120 mg) and the mixture was stirred for overnight at room temperature. After evaporation of dioxane, the aqueous solution was washed with ether (10 ml) and acidified to pH 3 with 5% hydrochloric acid and extracted with ethyl acetate (30 ml). The extract was washed with water and dried over magnesium sulfate. The solvent was removed under reduced pressure to give Boc-(L)-Z-(D-mesoDAP-(D)-OMe (2) (110 mg).

IR $cm^{-1}$ in $CH_2Cl_2$: 3390, 1730 (shoulder), 1710

NMR ($CDCl_3$) $\delta$: 1.45 (9H, s), 1.4—2.0 (6H, m), 3.68 (3H, s), 4.0—4.4 (2H, m), 5.08 (2H, s), 7.30 (5H, s)

Preparation 5

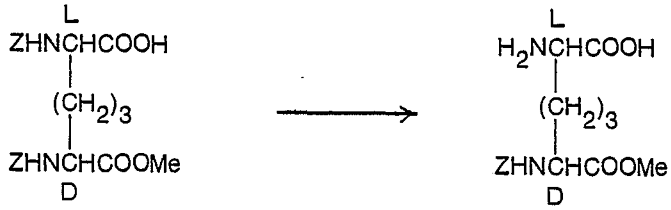

(1) (2)

To a solution of diZDAP(D)-OMe (1) (2.20 g) in benzene (50 ml) was added thionyl chloride (5 ml) and the mixture was refluxed for an hour. The reaction mixture was concentrated under reduced pressure.

The residual oil was dissolved in a mixture of dioxane (20 ml) and water (20 ml) and then stood overnight at room temperature. After concentration of dioxane, the concentrate was washed with ethyl acetate (20 ml). The aqueous layer was concentrated to about 10 ml and neutralized to pH 5 with 5% aqueous sodium bicarbonate. The resulting crystalline solid was filtered and washed with water to give Z(D)-mesoDAP-(D)-OMe (2) (0.58 g).

IR $cm^{-1}$ (Nujol): 3100, 1745, 1695, 1610

NMR ($DCl$-$D_2O$) $\delta$: 1.3—2.3 (6H, m), 3.73 (3H, s), 4.0—4.5 (2H, m), 5.15 (2H, s), 7.43 (5H, s)

Preparation 6

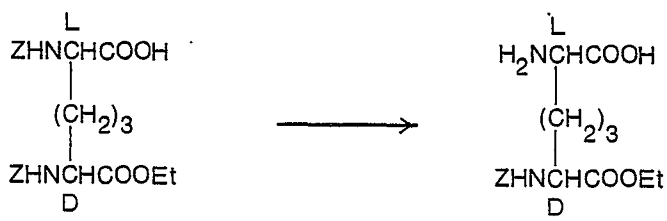

(1) (2)

Z-(D)-mesoDAP-(D)-OEt (2) was prepared substantially in the same manner as Preparation 5.

IR $cm^{-1}$ (Nujol): 3300—2200, 1725, 1690, 1615

NMR ($D_2O$-$DCl$), $\delta$: 1.25 (3H, t, J=7Hz), 1.5—2.2 (6H, m), 3.9—4.5 (4H, m), 5.13 (2H, s), 7.42 (5H, s)

Preparation 7

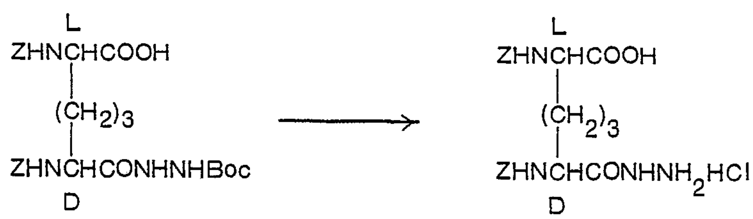

(1) (2)

Into a solution of diZ-mesoDAP-(D)-NHNHBoc (1) (30.3 g) in ethyl acetate (240 ml) was passed a stream of hydrogen chloride gas for 40 minutes under ice-bath cooling. The reaction mixture was stirred for an hour at the same temperature. The precipitated crystals were filtered and washed with ether to give diZ-mesoDAP-(D)-$NHNH_2$-HCl (2) (24.2 g).

IR $cm^{-1}$ (Nujol): 3280, 3400—2200, 1720, 1680, 1660

NMR (DMSO-$d_6$), $\delta$: 1.3—2.0 (6H, m), 3.8—4.2 (2H, m), 5.04 (4H, s), 7.36 (10H, s)

Preparation 8

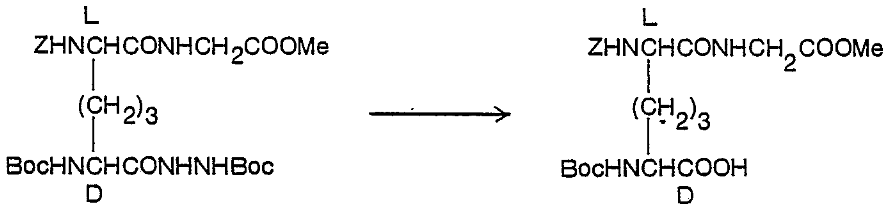

(1) (2)

Z-(L)-Boc-(D)-mesoDAP-(L)-GlyOMe-(D)-NHNHBoc (1) (9.57 g) was added to trifluoroacetic acid (96 ml) and the mixture was stirred for an hour at ambient temperature. After evaporation of trifluoroacetic acid, the residue was dissolved in 50% aqueous dioxane (180 ml) and the mixture was treated with N-bromosuccinimide (6.43 g). After stirring for an hour under ice-bath cooling, the excess reagent was decomposed with 10% sodium bisulfite. The resulting solution was adjusted to pH 8 with 50% sodium bicarbonate and then a solution of di-t-butylcarbonate (8.56 g) in dioxane (35 ml) was added thereto.

The resulting mixture was stirred for 20 hours at ambient temperature and evaporated. The aqueous solution was washed with ethyl acetate (100 ml) and adjusted to pH 3 with 5% hydrochloric acid and then extracted with ethyl acetate (250 ml). The extract was washed with water and dried over magnesium sulfate. The solvent was removed under reduced pressure to leave an oil, which was chromatographed on silica gel (220 g) eluting with a mixture of chloroform and methanol (20:1) to give Z-(L)-Boc-(D)-mesoDAP-(L)-GlyOMe (2) (4.78 g).

NMR ($CD_2OD$), δ: 1.47 (9H, s), 1.4—2.0 (6H, m), 3.73 (3H, s), 3.97 (2H, s), 4.0—4.35 (2H, m), 5.13 (2H, s), 7.37 (5H, s)

Preparation 9

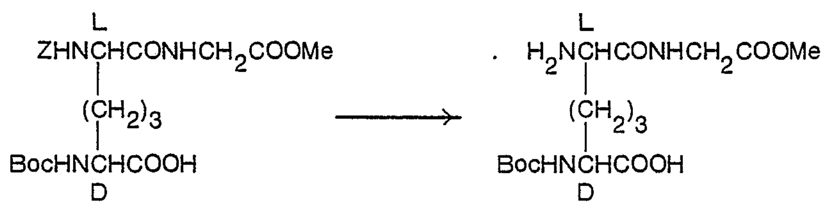

(1) (2)

A solution of Z-(L)-Boc-(D)-mesoDAP-(L)-GlyOMe (1) (4.78 g) in a mixture of methanol (100 ml) and water (15 ml) was hydrogenated over 10% palladium-charcoal (1.45 g). After removal of the catalyst, the filtrate was evaporated to dryness under reduced pressure. The residue was pulverized with ether to give Boc-(D)-mesoDAP-(L)-GlyOMe (2) (3.26 g).

IR $cm^{-1}$ (Nujol): 3600—2200, 1740, 1680, 1220, 1170, 1050, 1030, 860

Preparation 10

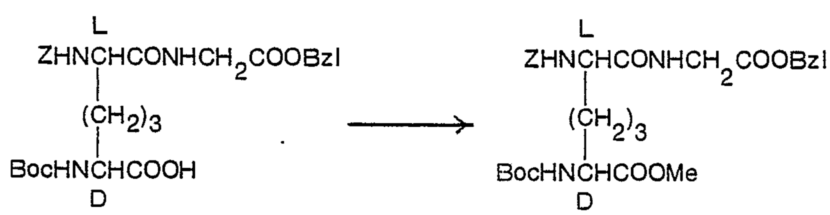

(1) (2)

A solution of Z-(L)-Boc-(D)-mesoDAP-(L)-GlyOBzl (1) (13.7 g) in methanol (140 ml) was treated with ethereal diazomethane under ice-bath cooling. The mixture was stirred for an hour at the same

temperature and concentrated under reduced pressure. The residue was pulverized with isopropylether to give Z-(L)-Boc-(D)-mesoDAP-(L)-GlyOBzl-(D)-OMe (2) (11.9 g).

NMR ($CD_3OD$), δ: 1.44 (9H, s), 1.3—2.0 (6H, m), 3.68 (3H, s), 3.96 (2H, d, J=4Hz), 4.0—4.3 (2H, m), 5.06 (2H, s), 5.14 (2H, s), 7.30 (10H, s)

Preparation 11

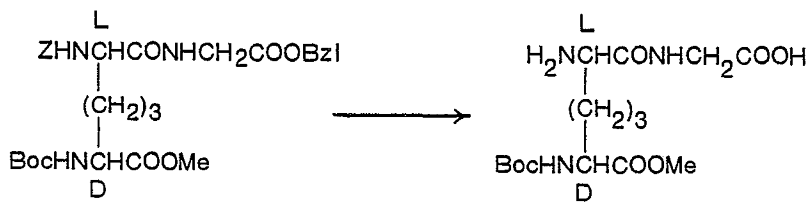

(1) (2)

A solution of Z-(L)-Boc-(D)-mesoDAP-(L)-GlyOBzl-(D)-OMe (1) (10.17 g) in a mixture of methanol (150 ml) and water (15 ml) was hydrogenated over 10% palladium charcoal (3.0 g) for 4 hours. After removal of the catalyst, the filtrate was evaporated to dryness under reduced pressure. The residue was pulverized with ether to give Boc-(D)-mesoDAP-(L)-GlyOH-(D)-OMe (2) (5.85 g).

IR $cm^{-1}$ (Nujol): 3325, 1750, 1690, 1640, 1610

NMR ($CD_3OD$), δ: 1.40 (9H, s), 1.5—2.0 (6H, m), 3.70 (3H, s), 3.82 (2H, s), 3.8—4.2 (2H, m)

Preparation 12

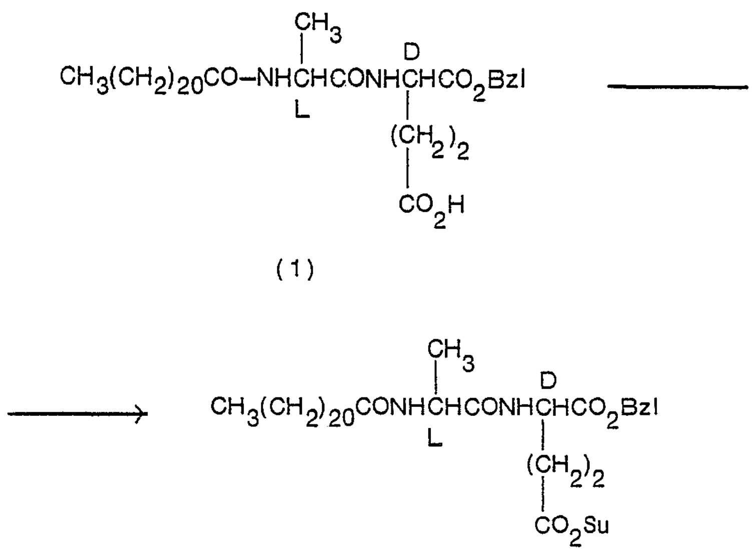

(1)

(2)

To a solution of n-docosonoyl-L-Ala-D-Glu(α-OBzl) (1) (4.76 g) in a mixture of tetrahydrofuran (50 ml) and chloroform (100 ml) were added dicyclohexylcarbodiimide (1.56 g) and N-hydroxysuccinimide (0.87 g). The mixture was kept for 17 hours at room temperature. The precipitate was filtered off and washed with chloroform (150 ml). The filtrate was concentrated in vacuo to give a crystal line residue, which was collected and washed with diisopropylether to give n-docosanoyl-L-Ala-D-Glu(α-OBzl)-OSu (2) (5.70 g).

IR (Nujol): 3380, 1820, 1790, 1750, 1640 $cm^{-1}$

NMR ($CDCl_3$-$CD_3OD$), δ: 0.91 (3H, m), 1.06—2.53 (47H, m), 2.84 (4H, s), 4.35—4.75 (2H, m), 5.19 (2H, s), 7.33 (5H, s)

Preparation 13

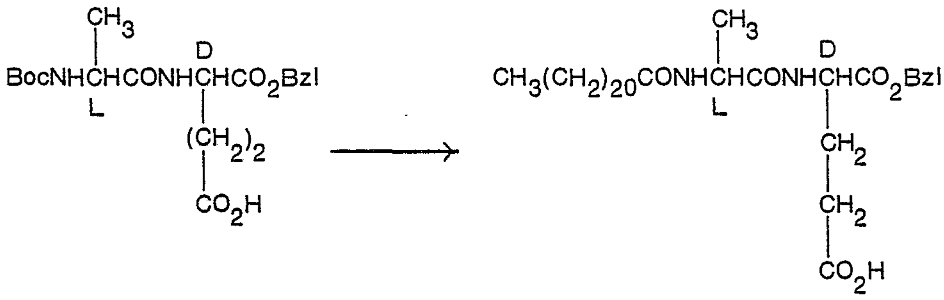

(1) (2)

Boc-L-Ala-D-Glu($\alpha$-OBzl) (1) (4.0 g) was dissolved in trifluoroacetic acid (30 ml) and stirred for 2 hours at room temperature. The recation mixture was concentrated in vacuo, and the residual oil was washed with ethylether. The oil was dissolved in methylenechloride (40 ml) and triethylamine (2.48 g) and methanol (12 ml) were added thereto. After the solution turned clean, n-docosanoic acid succinimido-ester (4.28 g) and another 0.5 equivalent mole of triethylamine were added. After stirring for 19 hours, the reaction mixture was concentrated in vacuo. Water (15 ml) and 1N-hydrochloric acid (30 ml) were added to the solution to give a crystalline mass. The precipitate was collected and washed with water (50 ml) to give a crude crystal (5.78 g), which was purified by washing with hot diisopropylether to give n-docosanoyl-L-Ala-D-Glu($\alpha$-OBzl) (2) (5.1 g).

IR (Nujol): 3300, 1725, 1700, 1650, 1630 $cm^{-1}$

NMR ($CDCl_3$), $\delta$: 0.88 (3H, m), 1.05—2.50 (47H, m), 4.3—4.8 (2H, m), 5.15 (2H, s), 7.32 (5H, s)

Preparation 14

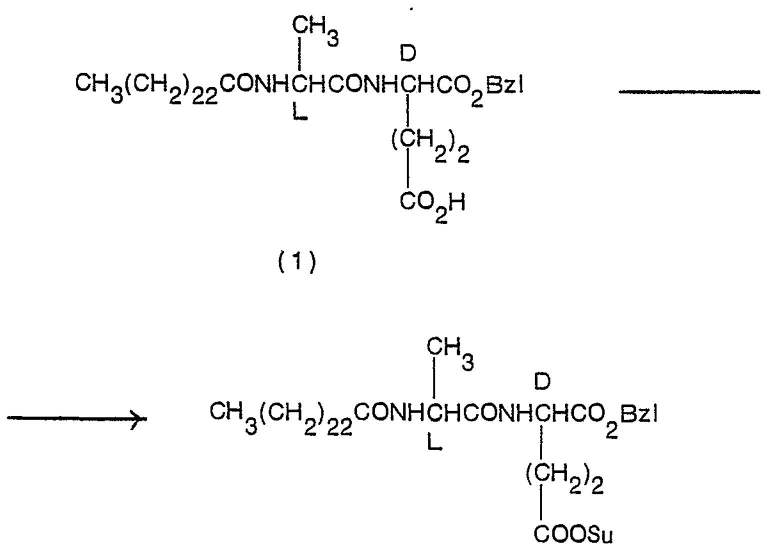

(1)

(2)

n-Tetracosanoyl-L-Ala-D-Glu($\alpha$-OBzl)OSu (2) was prepared substantially in the same manner as Preparation 12.

IR (Nujol): 3380, 1820, 1790, 1745, 1630 $cm^{-1}$

NMR ($CDCl_3$-$CD_3OD$), $\delta$: 0.87 (3H, m), 1.05—1.50 (51H, m), 2.82 (4H, s), 4.30—4.75 (2H, m), 5.17 (2H, s), 7.33 (5H, s)

Preparation 15

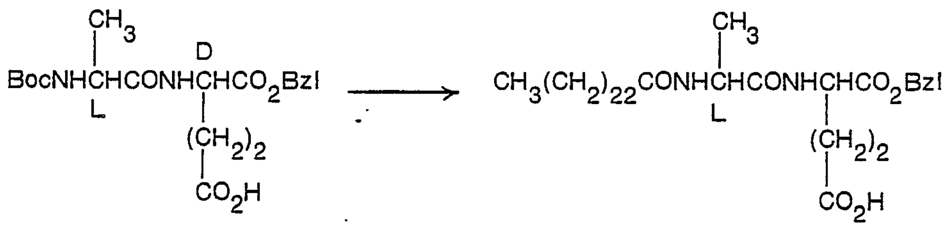

(1) (2)

n-Tetracosanoyl-L-Ala-D-Glu($\alpha$-OBzl) (2) was prepared substantially in the same manner as Preparation 13.

IR (Nujol): 3360, 1740, 1710, 1640 $cm^{-1}$

NMR ($CDCl_3$-$CD_3OD$), $\delta$: 0.88 (3H, m), 1.06—1.70 (47H, m), 2.00—2.50 (4H, m), 5.16 (2H, s), 7.36 (5H, s)

Preparation 16

(1) Step 1

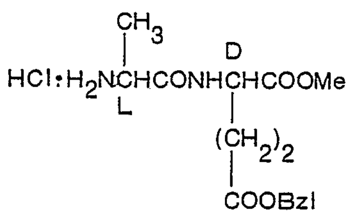

+

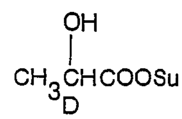

(1) (2)

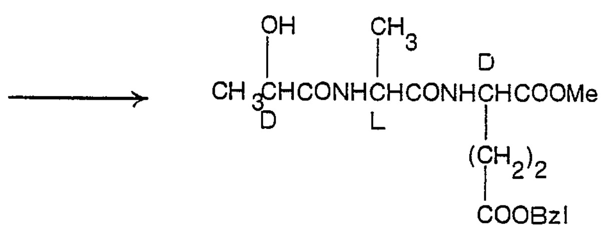

(3)

To a mixture of L-Ala-D-Glu($\gamma$-OBzl)OMe hydrochloric acid salt (1) (2.9 g) and triethylamine (0.91 g) in acetonitrile (30 ml) was added D-lactic acid N-hydroxysuccinimide ester (2) (2.53 g) under ice-bath cooling. The mixture wa stirred for 1.5 hours at ambient temperature. After evaporation of acetonitrile, the residue was dissolved in ethyl acetate (70 ml), washed successively with 2.5% hydrochloric acid (30 ml x 2), water (30 ml), 2.5% sodium bicarbonate (30 ml) and water (30 ml x 2), dried over magnesium sulfate and then evaporated. The residue was chromatographed on silica gel column (60 g) eluting with a mixture of chloroform and ethyl acetate (1:1) to give D-Lac-L-Ala-D-Gly($\gamma$-OBzl)OMe (3) (2.23 g).

NMR ($CDCl_3$), $\delta$: 1.30 (3H, d, J=7Hz), 1.43 (3H, d, J=7Hz), 2.0—2.7 (4H, m), 3.30 (1H, broad s), 4.73 (3H, s), 4.0—4.8 (3H, m), 5.13 (2H, s), 7.35 (5H, s)

(2) Step 2

Compound (3) 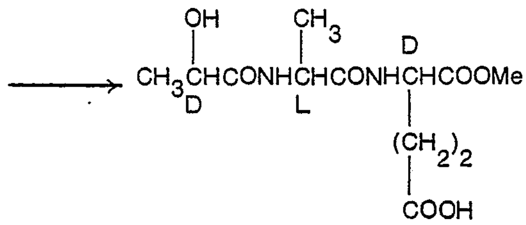

(4)

A solution of D-Lac-L-Ala-D-Glu($\gamma$-OBzl)OMe (3) (2.09 g) in methanol (100 ml) was hydrogenated over 10% palladium charcoal (0.6 g). After removal of the catalyst, the filtrate was evaporated under reduced pressure to give D-Lac-L-Ala-D-GluOMe (4) (1.6 g).

NMR ($CDCl_3$), $\delta$: 1.40 (3H, d, J=7Hz), 1.45 (3H, d, J=7Hz), 1.9—2.6 (4H, m), 4.75 (3H, s), 4.18 (1H, q, J=7Hz), 4.3—4.6 (2H, m)

Preparation 17

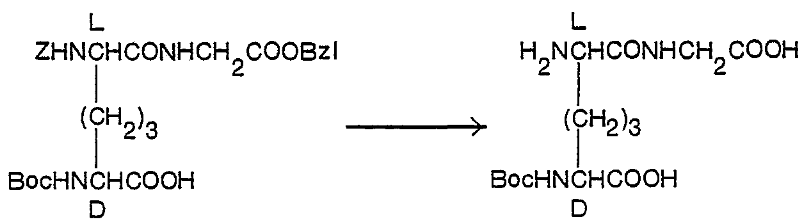

(1) (2)

A solution of Z-(L)-Boc-(D)-mesoDAP-(L)-GlyOBzl (1) (3.8 g) in a mixture of methanol (100 ml) and water (10 ml) was hydrogenated over 10% palladium charcoal (0.18 g). After removal of the catalysts, the filtrate was evaporated to dryness under reduced pressure. The residue was pulverized with ether to give Boc-(D)-mesoDAP-(L)-GlyOH (2) (2.10 g).

NMR ($CD_3OD$), $\delta$: 1.43 (9H, s), 1.3—2.1 (6H, m), 3.8—4.2 (4H, m)

Preparation 18

(1) Step 1

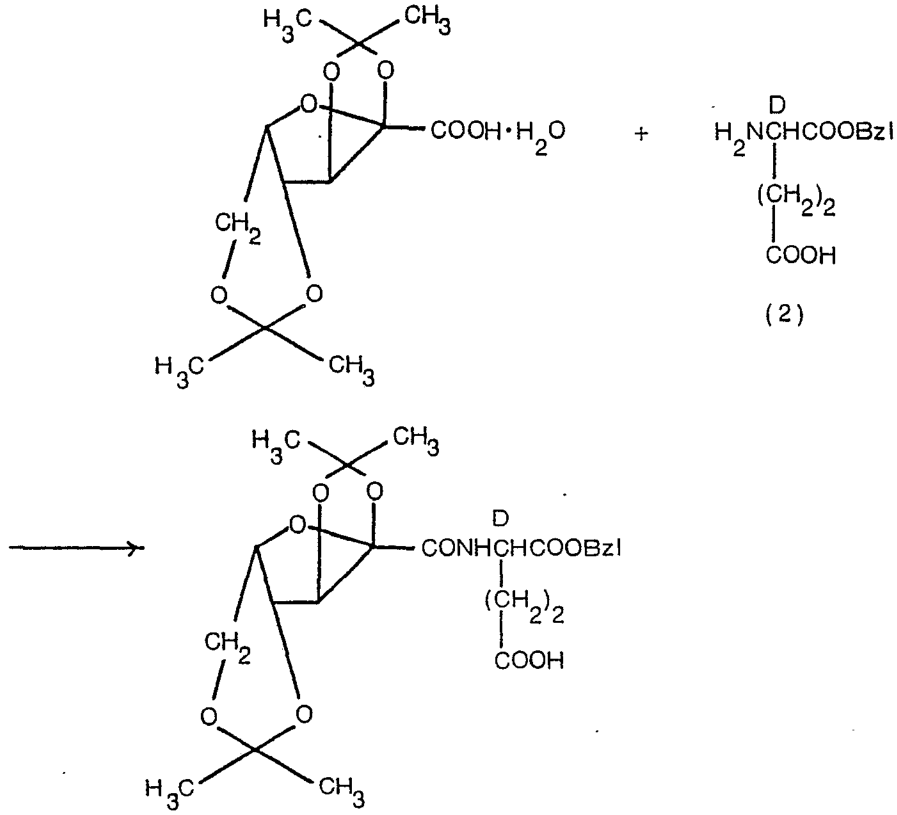

A mixture of phosphorus oxychloride (120 mg) and N,N-dimethylformamide (1 ml) was stirred at 25—30°C for 3 hours and then was cooled to 0°C. To the solution was added methylene chloride solution (2 ml) of 2,3; 4,6-di-isopropylidene-2-keto-L-gulonic acid mono hydrate (1) (150 mg), keeping the temperature at 0 to 5°C for 30 minutes.

The resulting solution was cooled at −20 to −30°C.

On the other hand, D-Glu(OBzl) (2) (120 mg) was dissolved in a mixture of methylene chloride (100 ml) and bis-trimethylsilyl-acetamide (800 mg).

The solution thus prepared was added to the above solution at −20 to −30°C. The temperature of the mixture was raised to ambient temperature and then the mixture was stirred for 15 hours. The reaction mixture was evaporated in vacuo and extracted with ethyl acetate. The organic layer was washed with water (×2) and brine and then dried over magnesium sulfate. The solvent was evaporated to give an oily residue which was subjected to silica gel column.

The column was eluted with chloroform and methanol (20:1). The solvent of the fraction containing, the object compound was evaporated to give 2,3;4,6-di-isopropylidene-2-keto-L-gulonoyl-$\gamma$-D-Glu($\alpha$-OBzl)OH (3) (220 mg).

NMR ($CDCl_3$), $\delta$: 1.33 (3H, s), 1.43 (3H, s), 1.53 (6H, s), 2.10—2.40 (4H, m), 4.15 (2H, s), 4.33—4.66 (3H, m), 4.73 (1H, t, J=7Hz), 5.16 (2H, s), 7.33 (5H, s)

(2) Step 2

Compound (3)

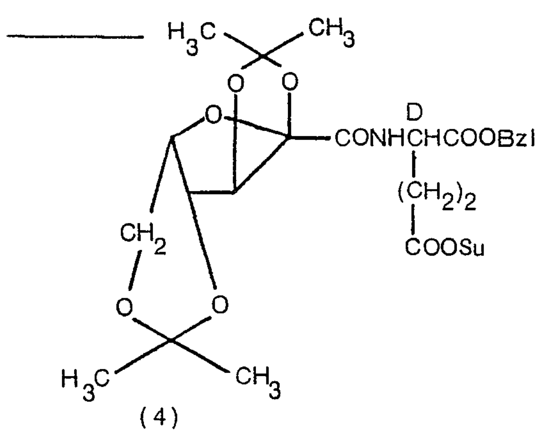

(4)

2,3;4,6-Di-isopropylidene-2-keto-L-gulonoyl-D-Glu(α-OBzl)OH (3) (950 mg) and N-hydroxysuccinimide (230 mg) was dissolved in tetrahydrofuran (10 ml). To the solution was added dicyclohexylcarbodiimide (410 mg) at 0°C. The reaction mixture was stirred at 0°C and stood overnight in a refrigerator. The precipitates were filtered and washed with tetrahydrofuran. The filtrate was evaporated to give a viscous oil which was pulverized with diethylether to give 2,3;4,6-di-isopropylidene-2-keto-L-gulonoyl-D-Glu(α-OBzl)OSu (4) (1.1 g).

NMR ($CDCl_3$), δ: 1.30 (3H, s), 1.40 (3H, s), 1.50 (6H, s), 2.77 (4H, s), 4.10—4.90 (6H, s), 5.16 (2H, s), 7.30 (5H, s)

Preparation 19

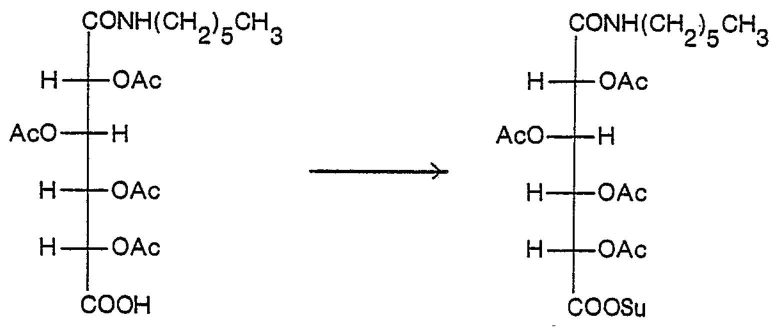

(1) (2)

ester of 1-N-n-hexyl-2,3,4,5-O-tetraacetyl-D-glucaramic acid with N-hydroxylsuccinimide (2) was prepared substantially in the same manner as step 2 of Preparation 18.

NMR ($CDCl_3$), δ: 0.7—1.1 (3H, m), 1.1—2.0 (8H, m), 2.10 (3H, s), 2.22 (9H, s), 2.88 (4H, s), 3.0—3.5 (2H, m), 5.2—6.0 (4H, m), 6.23 (1H, m)

Preparation 20

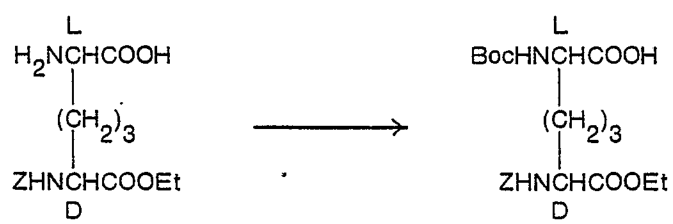

(1) (2)

Boc-(L)-Z-(D)-mesoDAP-(D)-OEt (2) was prepared substantially in the same manner as Preparation 4.

NMR ($CDCl_3$), δ: 1.17 (3H, t, J=7Hz), 1.40 (9H, s), 1.30—2.0 (6H, m), 4.13 (2H, q, J=7Hz), 4.25 (1H, m), 5.07 (2H, s), 5.30 (1H, broad), 7.25 (5H, s), 8.12 (1H, broad s)

Preparation 21

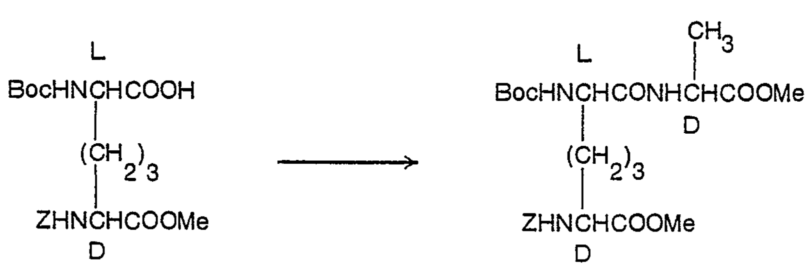

(1) (2)

Boc-(L)-Z-(D)-mesoDAP-(L)-D-AlaOMe-(D)-OMe (2) was prepared substantially in the same manner as Preparation 1.

IR (Nujol): 3300, 1740, 1685, 1655 $cm^{-1}$

Preparation 22

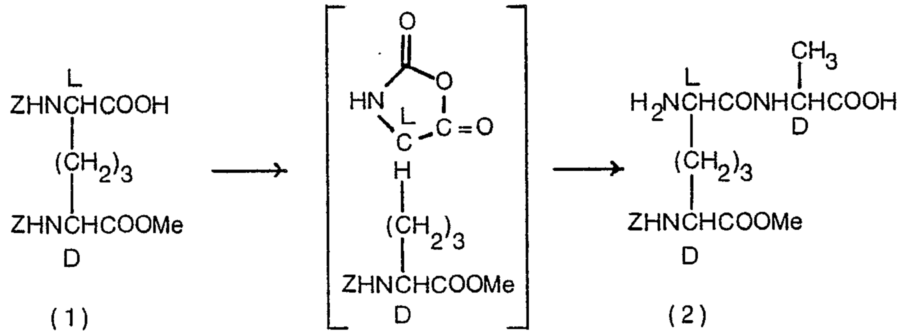

(1) (2)

To a solution of diZ-mesoDAP-(D)-OMe (1) (37.0 g) in methylene chloride (200 ml) was added thionyl chloride (74 ml) and the mixture was refluxed for an hour. After evaporation of the solvent, the residue was dissolved in acetonitrile (200 ml). This solution was added to a mixture of D-alanine (21.0 g) and sodium carbonate (8.3 g) in a mixture of 0.5N sodium hydride (400 ml) and acetonitrile (200 ml) under ice-bath cooling. After stirring for 30 minutes at the same temperature, the mixture was diluted with ethyl acetate (150 ml). The aqueous layer was evaporated and the organic layer was extracted with water (150 ml). The aqueous layer and the extract were combined, washed with ethyl acetate and then acidified to pH 4 with 20% hydrochloric acid. This solution was put on a column of HP 20 (700 ml). After washing with water, the column was eluted with a mixture of methanol and water (4:1) to give Z-(D)-mesoDAP-(L)-D-AlaOH-(D)-OMe (2) (20.0 g).

NMR ($D_2O$-$NaHCO_3$), $\delta$: 1.2—2.1 (6H, m), 1.35 (3H, d, J=7Hz), 3.73 (3H, s), 3.9—4.4 (3H, m), 5.10 (2H, s), 7.07 (5H, s)

Preparation 23

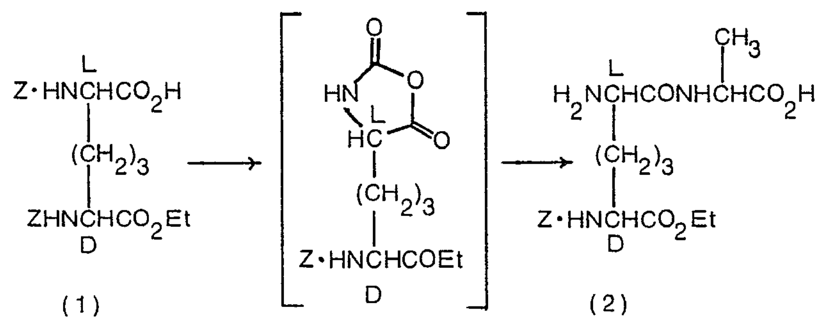

(1) (2)

Z-(D)-mesoDAP-(L)-L-AlaOH-(D)-OEt (2) was prepared substantially in the same manner as Preparation 22.

NMR ($D_2O$-$NaHCO_3$), $\delta$: 1.22 (3H, t, J=7Hz), 1.37 (3H, d, J=7Hz), 1.5—2.1 (6H, m), 3.8—4.4 (5H, m), 5.12 (2H, s), 7.40 (5H, s)

Preparation 24

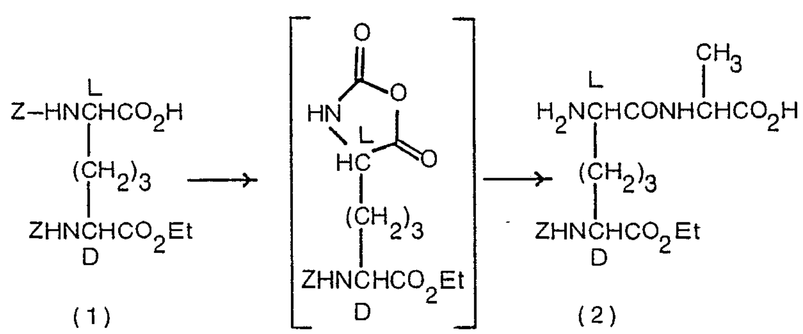

(1) (2)

Z-(D)-mesoDAP-(L)-D-AlaOH-(D)-OEt (2) was prepared substantially in the same manner as Preparation 22.

NMR ($D_2O$-$NaHCO_3$), $\delta$: 1.23 (3H, t, J=8Hz), 1.35 (3H, d, J=7Hz), 1.1—2.0 (6H, m), 3.9—4.5 (5H, m), 5.15 (2H, s), 7.34 (5H, s)

Preparation 25

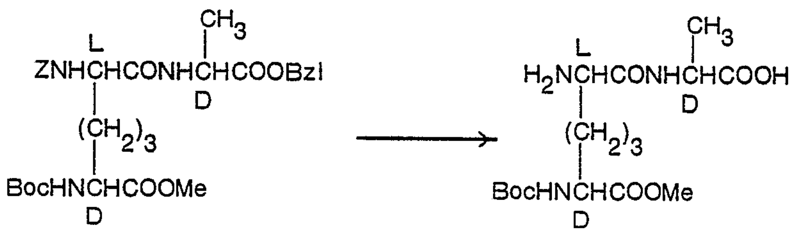

(1) (2)

Boc-(D)-mesoDAP-(L)-D-AlaOH-(D)-OMe (2) was prepared substantially in the same manner as Preparation 17.

Preparation 26

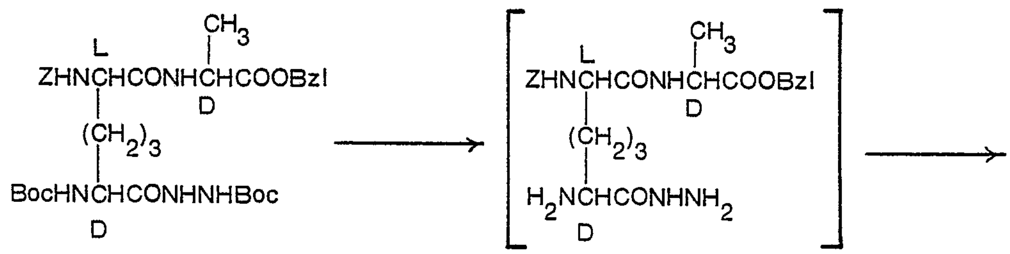

(1)

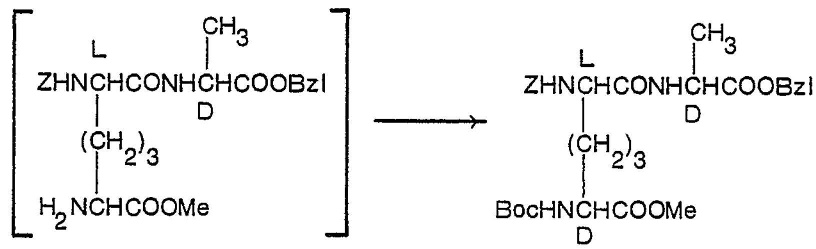

(2)

Z-(L)-Boc-(D)-mesoDAP-(L)-D-AlaOBzl-(D)-OMe (2) was prepared substantially in the same manner as Preparations 3.

NMR ($CDCl_3$), $\delta$: 1.42 (9H, s), 1.0—2.1 (9H, s), 3.66 (3H, s), 4.0—4.7 (3H, m), 5.07 (2H, s), 5.10 (2H, s), 7.29 (10H, s)

Preparation 27

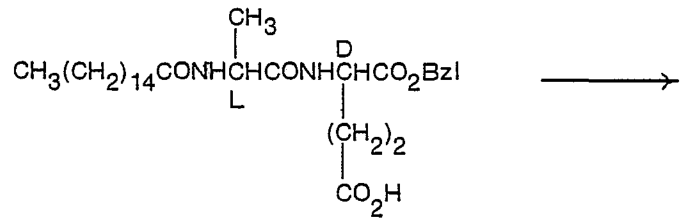

(1)

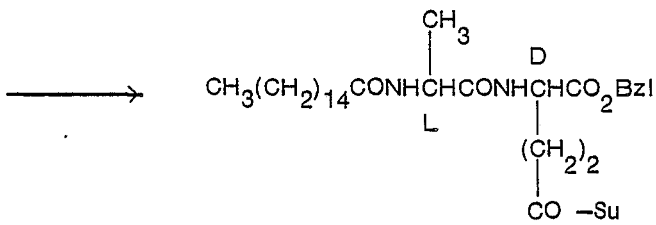

(2)

To a solution of palmitoyl-L-Ala-D-Glu(OH)OBzl (1) (1.84 g) in a mixture of tetrahydrofuran (20 ml) and chloroform (30 ml) were added N-hydroxysuccinimide (425 mg) and dicyclohexylcarbodiimide (728 mg). The reaction mixture was kept for 18 hours at room temperature and the precipitate was filtered off and washed with chloroform. The filtrate was concentrated in vacuo and the diisopropylether was added to the residue. The product was collected and dried to afford palmitoyl-L-Ala-D-Glu(OSu)OBzl (2) (1.70 g).

IR (Nujol): 3300, 1805, 1780, 1745, 1640 $cm^{-1}$

NMR ($CDCl_3$, $\delta$): 0.89 (3H, m), 1.05—2.70 (35H, m), 2.80 (4H, s), 4.20—4.83 (2H, m), 5.15 (2H, s), 6.23 (1H, d, J=8Hz), 7.30 (5H, s).

Preparation 28

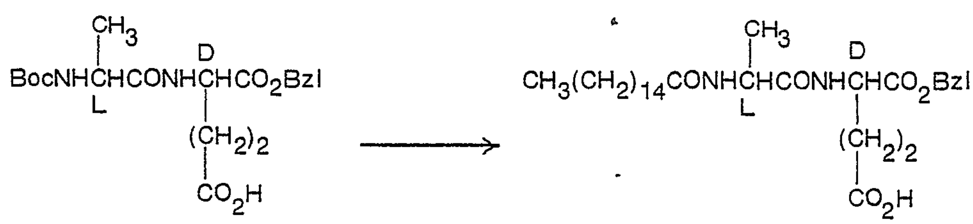

(1) (2)

Trifluoroacetic acid (15 ml) was added to Boc-L-Ala-D-Glu-(OH)OBzl (1) (3.26 g) and the mixture was stirred for 20 minutes at room temperature, concentrated in vacuo and washed with diisopropylether. The oil was dissolved in a mixture of water (15 ml) and sodium bicarbonate was added until the pH of the solution became 8—9. The mixture of water (6 ml) and acetone (20 ml) was added to the solution and palmitoyl chloride (2.20 g) was added in one portion. After stirring for 30 minutes at room temperature, the pH of the solution was adjusted to 3 with 1N-hydrochloric acid and the reaction mixture was concentrated. To the residue was added water (30 ml) and the precipitates was collected and dried to give a crude product, which was purified by column chromatography of Silicagel (90 g) with chloroform-methanol (30:1, v/v) as an eluent to give palmitoyl-L-Ala-D-Glu(OH)OBzl (2) (2.00 g). mp. 131—132°C.

$[\alpha]_D$ −23.38° (C=0.2, $CHCl_3$).

IR (Nujol): 3300, 1730, 1700, 1650, 1635 $cm^{-1}$

NMR ($CDCl_3$, $\delta$): 0.88 (3H, m), 1.1—1.75 (31H, m), 2.0—2.5 (4H, m), 4.4—4.8 (2H, m), 5.11 (2H, s), 7.34 (5H, s)

Preparation 29

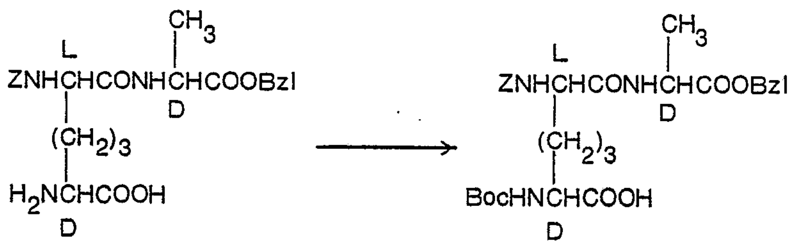

(1) (2)

To a solution of Z-(L)-mesoDAP-(L)-D-AlaOBzl (1) (5.97 g) in a mixture of water (60 ml), dioxane (60 ml) and triethylamine (2.73 g) was dropped a solution of di-tert-butyl carbonate (3.21 g) in dioxane (18 ml) at room temperature and the pH of the mixture was adjusted between 9 and 10 with triethylamine. After stirring for 1 hour, the mixture was diluted with water and ethyl acetate (100 ml) was added. The organic layer was extracted with water three times and the aqueous layers were combined. The pH of the aqueous layer was adjusted to 4—5 with 5% hydrochloric acid and extracted with ethyl acetate. The extract was washed with brine, dried over magnesium sulfate and concentrated in vacuo. The resulting residue was crystallized with diisopropylether to give Z-(L)-Boc-(D)-mesoDAP-(L)-D-AlaOBzl (2) (5.28 g) mp. —143°C (dec.).

NMR ($CD_3OD$): $\delta$1.33 (2H, d, J=8Hz), 1.40 (9H, s), 1.2—2.0 (6H, m), 3.9—4.2 (2H, m), 4.40 (1H, q, J=8Hz), 5.05 (2H, s), 5.10 (2H, s), 7.30 (10H, s).

Preparation 30

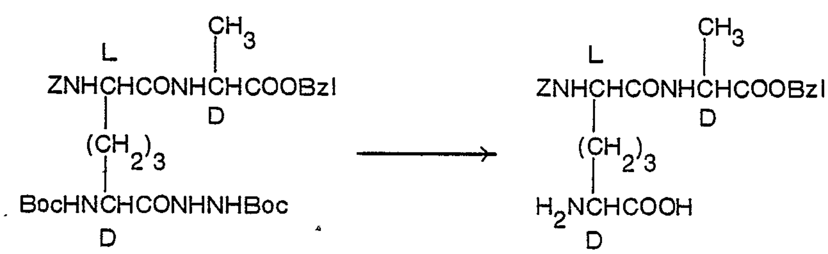

(1) (2)

Z-(L)-Boc-(D)-mesoDAP-(L)-D-AlaOBzl-(D)-NHNHBoc (1) (28 g) was added to trifluoroacetic acid (280 ml) and the mixture was stirred for 1 hour at room temperature. After evaporation of trifluoroacetic acid, the residue was dissolved in a mixture of water (240 ml), 1N hydrochloric acid (40 ml) and dioxane (200 ml), and N-bromosuccinimide (16.4 g) was added at 0°C. After stirring for 0.5 hours at the same temperature, the reaction mixture was treated with an aqueous solution of sodium sulfite and concentrated in vacuo. To the residue was added 30% aqueous sodium hydroxide until the pH of the mixture reached to 4—5. The resulting precipitate was collected and washed with water to give Z-(L)-mesoDAP-(L)-D-AlaOBzl (2).

IR (Nujol): 3280, 1725, 1685, 1640 $cm^{-1}$

NMR (DMSO-$d_6$): $\delta$ 1.34 (3H, d, J=8Hz), 1.2—2.0 (6H, m), 4.37 (1H, q, J=8Hz), 5.08 (2H, s), 5.16 (2H, s), 7.43 (10H, s)

Example 1

(1) Step 1

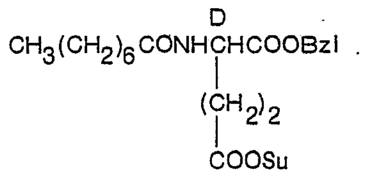

+

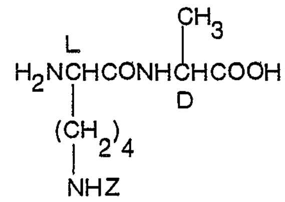

(1) (2)

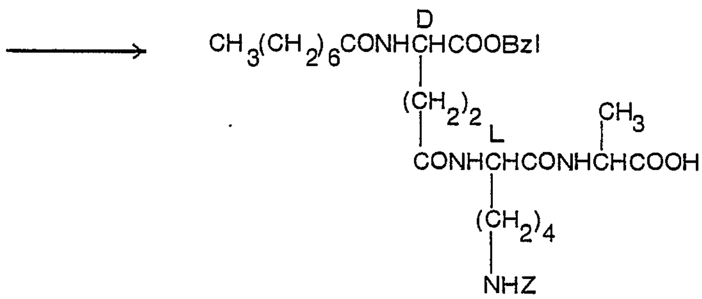

(3)

L-Lys($\varepsilon$-Z)-D-AlaOH (2) (0.915 g) was dissolved in a mixture of methylene chloride (40 ml) methanol (40 ml) and triethylamine (0.53 g).

To this solution was added n-octanoyl-D-Glu($\alpha$-OBzl)-$\gamma$-OSu (1) (1.20 g) and the resulting solution was left overnight at ambient temperature. The reaction mixture was evaporated to give a paste, to which were added water (50 ml), ether (50 ml) and 1N hydrogen chloride. The resulting mixture was stirred well and ether was evaporated to separate crystals from the aqueous layer. The crystals were filtered, washed with water and then dried to give n-octanoyl-$\gamma$-D-Glu($\alpha$-OBzl)-L-Lys($\varepsilon$-Z)-D-AlaOH (3) (1.50 g).

IR (Nujol): 3300, 1725, 1685, 1650, 1630 $cm^{-1}$

NMR (DMSO-$d_6$): $\delta$ 0.84 (3H, t, J=7Hz), 1.00—2.40 (25H, m), 2.84—3.12 (2H, m), 4.10—4.50 (3H, m), 5.00 (2H, s), 5.10 (2H, s), 7.32 (10H, s), 8.10 (2H, t, J=8Hz), 8.84 (1H, d, J=8Hz)

(2) Step 2

Compound (3)

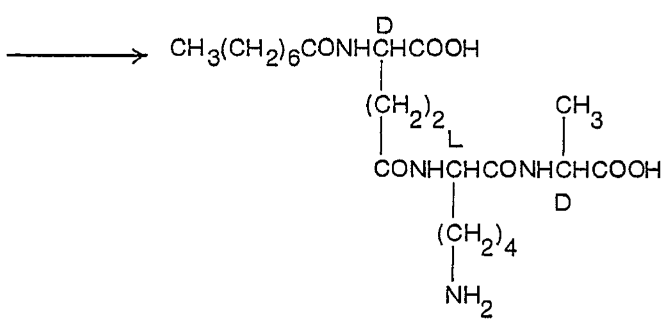

(4)

Octanoyl-$\gamma$-D-Glu($\alpha$-OBzl)-L-Lys($\varepsilon$-Z)-D-AlaOH (3) (1.20 g) was dissolved in acetic acid (50 ml) and hydrogenated over palladium black (150 mg). The catalyst was removed by filtration and the filtrate was evaporated to give a paste. The paste was allowed to stand to give crystals. The crystals were washed throughly with diethyl ether to give octanoyl-$\gamma$-D-Glu($\alpha$-OH)-L-Lys-D-AlaOH (4) (0.80 g).

$[\alpha]_D = +41.7$ (C=0.2, acetic acid)

IR (Nujol): 3360, 1710 (sh), 1640 $cm^{-1}$

NMR ($D_2O$): $\delta$ 0.84 (3H, obscure t, J=7Hz), 1.00—2.50 (25H, m), 2.80—3.10 (2H, m), 4.00—4.40 (3H, m)

Example 2

(1) Step 1

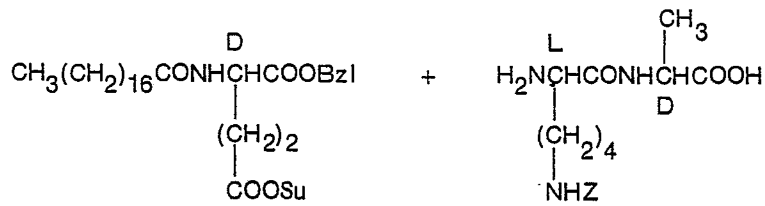

(1) (2)

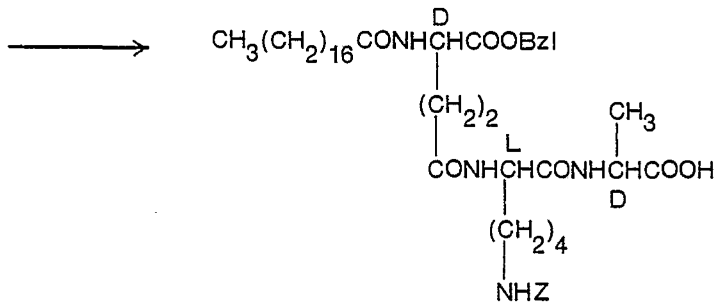

(3)

Stearoyl-γ-D-Glu(α-OBzl)-L-Lys(ε-Z)-D-AlaOH (3) was prepared substantially in the same manner as step (1) of Example 1.

NMR (DMSO-$d_6$): δ 0.84 (3H, t, J=7Hz), 1.00—2.40 (45H, m), 2.80—3.10 (2H, m), 4.00—4.80 (3H, m), 5.00 (2H, s), 5.08 (2H, s), 7.32 (10H, s), 7.80 (1H, d, J=8Hz), 8.08 (2H, t, J=8Hz)

(2) Step 2

Compound (3)

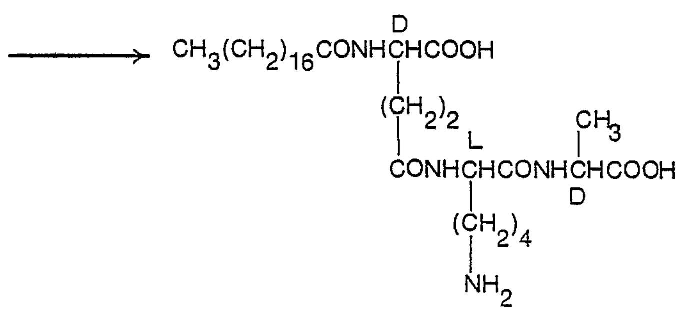

(4)

Stearoyl-γ-D-Glu(α-OH)-L-Lys-D-AlaOH (4) was prepared substantially in the same manner as step (2) of Example 1.

$[\alpha]_D = -11.10$ (C=0.21, acetic acid)

IR (Nujol): 3350, 1730, 1640 $cm^{-1}$

NMR (NaOD-$D_2O$), δ: 0.68—2.80 (50H, m), 4.10—4.50 (3H, m)

## Example 3

(1) Step 1

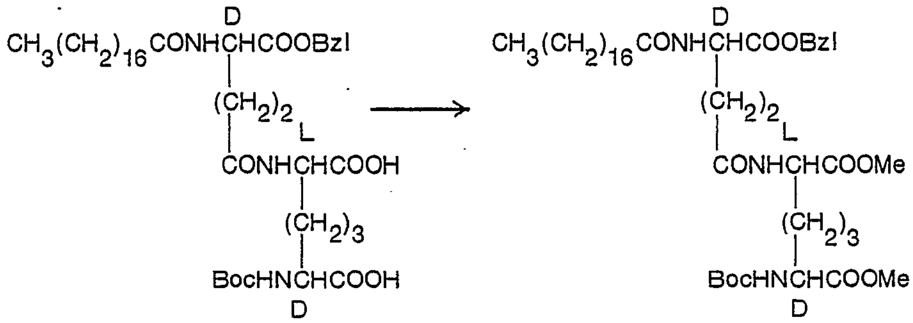

(1) (2)

Stearoyl-$\gamma$-D-Glu($\alpha$-OBzl)-(L)-Boc-(D)-mesoDAP-(1) (2.20 g) was dissolved in tetrahydrofuran (50 ml), and ethereal diazomethane solution was added thereto until pale yellow of the solution persisted. After 10 minutes, excess diazomethane was destroyed by adding acetic acid thereto. The resulting solution was evaporated to give a white crystalline residue, which was washed with diisopropyl ether to give stearoyl-$\gamma$-D-Glu($\alpha$-OBzl)-(L)-Boc-(D)-mesoDAP-(L)-(OMe)-D-(OMe) (2) (2.10 g).

IR (Nujol): 3300, 1740, 1680, 1640 $cm^{-1}$

NMR ($CDCl_3$), $\delta$: 0.83—2.50 (45H, m), 3.71 (3H, s), 3.75 (3H, s), 4.10—5.00 (3H, m), 5.20 (2H, s), 7.36 (5H, s)

(2) Step 2

Compound (2)

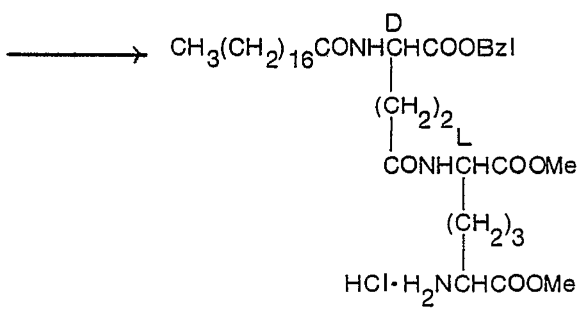

Stearoyl-$\gamma$-D-Glu($\alpha$-OBzl)-(L)-Boc-(D)-mesoDAP-(L)-(OMe)-(D)-(OMe) (2) (0.90 g) was dissolved in a mixture of acetic acid (10 ml) and hydrogen chloride-saturated acetic acid (4 ml). The solution was allowed to stand for 15 minutes at ambient temperature and then evaporated to give a crystalline residue.

The crystalline residue was throughly washed with isopropyl ether to give stearoyl-$\gamma$-D-Glu($\alpha$-OBzl)-(L)-mesoDAP-(L)-(OMe)-(D)-(OMe) hydrochloric acid salt (3).

IR (Nujol): 3300, 1740, 1640 $cm^{-1}$

NMR (DMSO-$d_6$), $\delta$: 0.76—2.40 (45H, m), 3.64 (3H, s), 3.76 (3H, s), 3.84—4.40 (3H, m), 5.12 (2H, s), 7.38 (5H, s)

(3) Step 3

Compound (3)

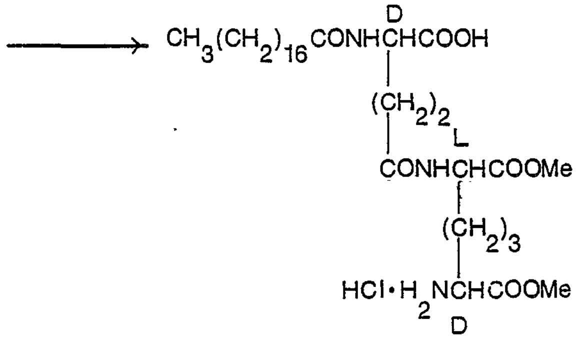

(4)

Stearoyl-$\gamma$-D-Glu($\alpha$-OH)-(L)-mesoDAP-(L)-(OMe)-(D)-(OMe) (4) was prepared substantially in the same manner as step (2) of Example 1.

$[\alpha]_D = +3.901$ (C=0.2, acetic acid)

IR (Nujol): 3300, 1730, 1640, 1600 $cm^{-1}$

NMR ($DMSO-d_6$), $\delta$: 0.70—2.32 (45H, m), 3.62 (3H, s), 3.74 (3H, s), 3.80—4.32 (3H, m), 8.02 (1H, d, J=8Hz), 8.30 (1H, d, J=8Hz)

## Example 4

(1) Step 1

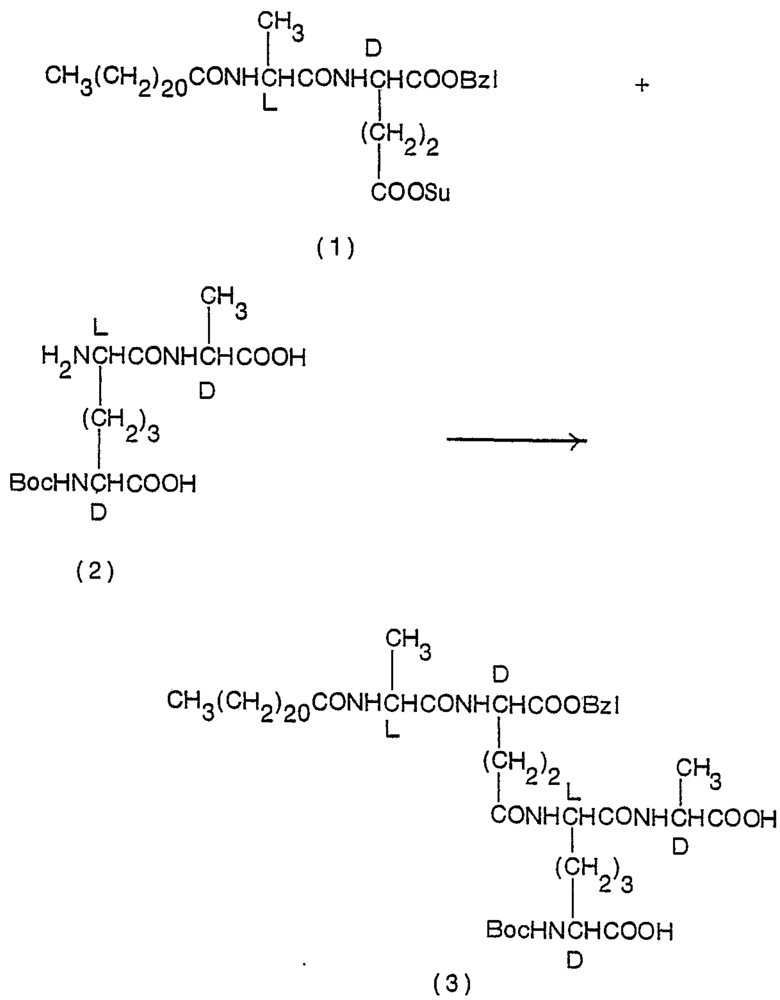

(1)

+

(2)

(3)

To s suspension of n-docosanoyl-L-Ala-D-Glu(α-OBzl)-γ-OSu (1) (2.91 g) in methylene chloride (50 ml) were added triethylamine (0.89 g) and Boc-(D)-mesoDAP-(L)-D-AlaOH (2)(1.44 g). The mixture was kept for 66 hours at ambient temperature and then concentrated in vacuo. To the residue were added water (100 ml), 1N hydrochloric acid (10 ml) and ethylether (30 ml). The resulting mixture was stirred for 15 minutes and ethylether was removed. The precipitate was collected by filtration and then washed with watger to give a crude product which was washed with diisopropylether to give n-docosanoyl-L-Ala-γ-D-Glu(α-OBzl)-(L)-Boc-(D)-mesoDAP-(L)-D-AlaOH (3)(3.60 g).

IR (Nujol): 3300, 1720, 1680, 1630 $cm^{-1}$

NMR ($CDCl_3$-$CD_3OD$), δ: 0.94 (3H, m), 1.1—2.5 (65H, m), 5.21 (2H, s), 7.38 (5H, s)

(2) Step 2

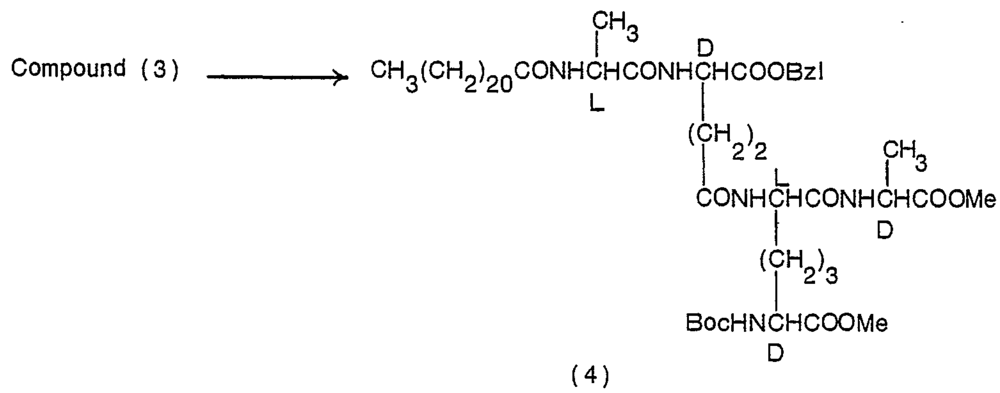

(4)

To a solution of n-docosanoyl-L-Ala-γ-D-Glu(α-OBzl)-(L)-Boc-(D)-mesoDAP-(L)-AlaOH (3)(1.5 g) in a mixture of tetrahydrofuran (40 ml) and chloroform (20 ml) was added 0.6 M diazomethane in ethylether (10 ml). The resulting mixture was stirred for 30 minutes at ambient temperature and then the excess reagent was decomposed with acetic acid. The reaction mixture was concentrated and the residue was washed with ethylether and collected by filtration to give n-docosanoyl-L-Ala-γ-D-Glu(α-OBzl)-(L)-Boc-(D)-mesoDAP-(L)-D-Ala(OMe)-(D)-OMe (4)(1.36 g).

NMR ($CDCl_3$-$CD_3OD$), δ: 0.90 (3H, m), 1.1-2.6 (65H, m), 3.71 (3H, s), 3.74 (3H, s), 5.19 (2H, s), 7.48 (5H, s)

(3) Step 3

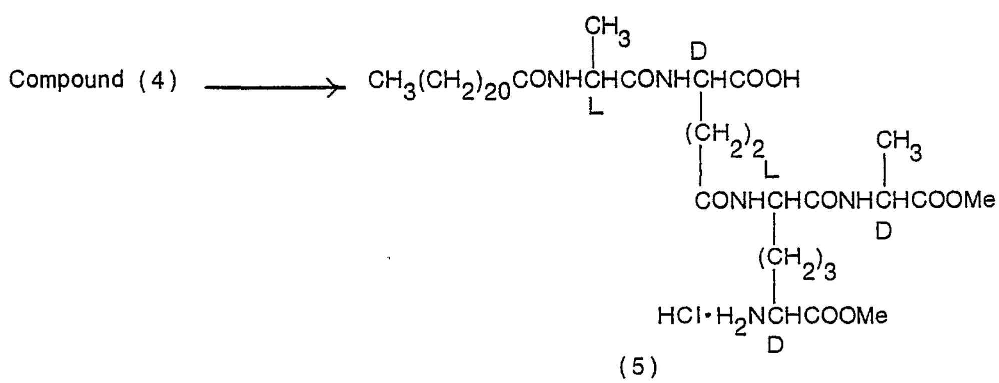

(5)

n-Docosanoyl-L-Ala-γ-D-Glu(α-OBzl)-(L)-Boc-(D)-mesoDAP-(L)-D-AlaOMe-(D)-OMe (4)(1.25 g) was dissolved in acetic acid (20 ml) and hydrogenated under an atomospheric pressure of hydrogen over 10% palladium charcoal. After removal of the catalyst, acetic acid (δ ml) saturated with hydrogen chloride was added to the filtrate. The resulting mixture was kept for 3 hours at ambient temperature and concentrated in vacuo to give a crystalline residue, which was collected and washed with diisopropylether to give n-docosanoyl-L-Ala-γ-D-Glu(α-OH)-(L)-(D)-AlaOMe-(D)-OMe hydrochloric acid salt (5) (1.08 g).

$[\alpha]_D = -11.42°$ (C=0.30, acetic acid)

IR (Nujol): 3380, 1745, 1630 $cm^{-1}$

NMR (DMSO-$d_6$), δ: 0.87 (3H, m), 1.05—2.45 (56H, m), 3.63 (3H, s), 3.75 (3H, s)

Example 5

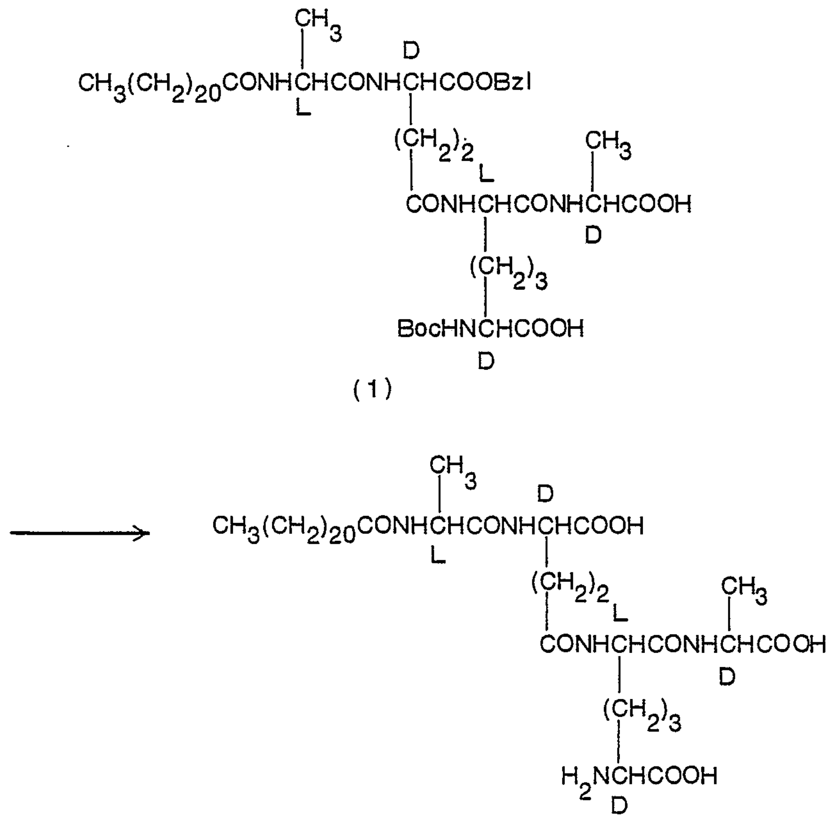

(1)

(2)

n-Docosanoyl-L-Ala-$\gamma$-D-Glu($\alpha$-OH)-(L)-mesoDAP-(L)-D-AlaOH (2) was prepared substantially in the same manner as step 3 of Example 4.

$[\alpha]_D = -19.34°$ (C=0.20, acetic acid)

IR (Nujol): 3380, 1740, 1630 $cm^{-1}$

NMR (NaOD-$D_2O$), $\delta$: 0.91 (3H, m) 3.25 (1H, m), 4.0-4.6 (5H, m)

Example 6

(1) Step 1

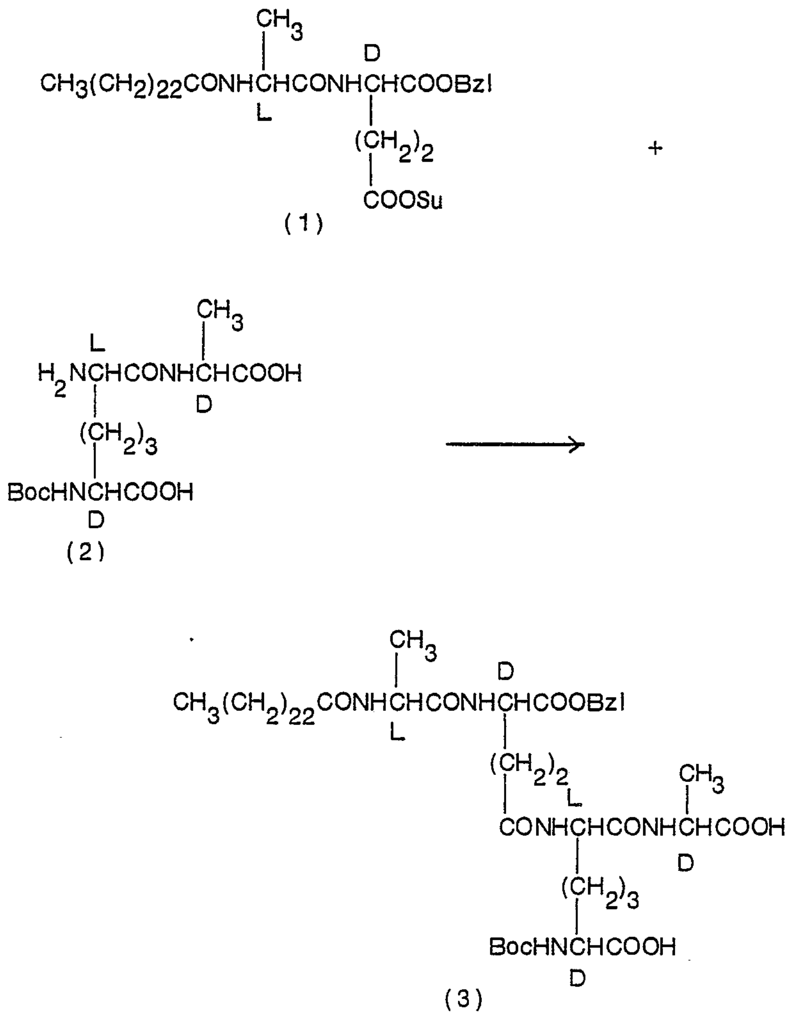

(1)

(2)

(3)

n-Tetracosanoyl-L-Ala-$\gamma$-D-Glu($\alpha$-OBzl)-(L)-Boc-(D)-mesoDAP-(L)-D-AlaOH (3) was prepared substantially in the same manner as step 1 of Example 4.

IR (Nujol): 3300, 1720, 1680, 1630 $cm^{-1}$

NMR ($CDCl_3CD_3OD$), $\delta$: 0.90 (3H, m), 1.05—2.50 (69H, m), 5.15 (2H, s), 7.34 (5H, s)

(2) Step 2

Compound (3) 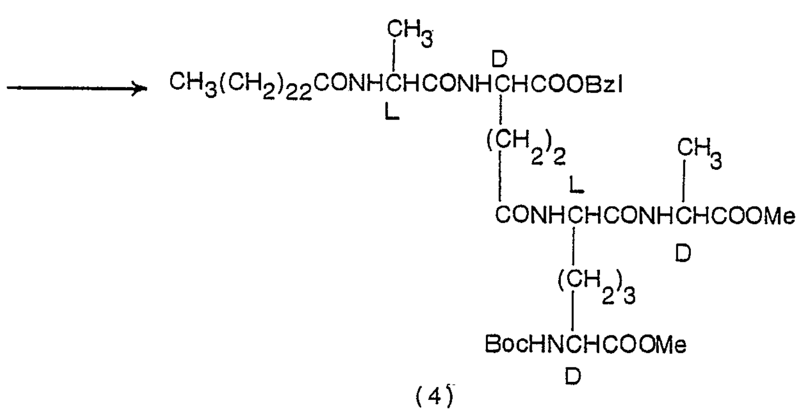

(4)

n-Tetracosanoyl-L-Ala-$\gamma$-D-Glu($\alpha$-OBzl)-(L)-Boc-(D)-mesoDAP-(L)-D-AlaOMe-(D)-OMe (4) was prepared substantially in the same manner as step 2 of Example 4.

IR (Nujol): 3300, 1735, 1685, 1630 $cm^{-1}$

NMR ($CDCl_3$-$CD_3OD$), $\delta$: 0.89 (3H, m), 1.05-2.50 (69H, m), 3.69 (3H, s), 3.75 (3H, s), 5.18 (2H, s), 7.42 (15H, s)

(3) Step 3

Compound (4)

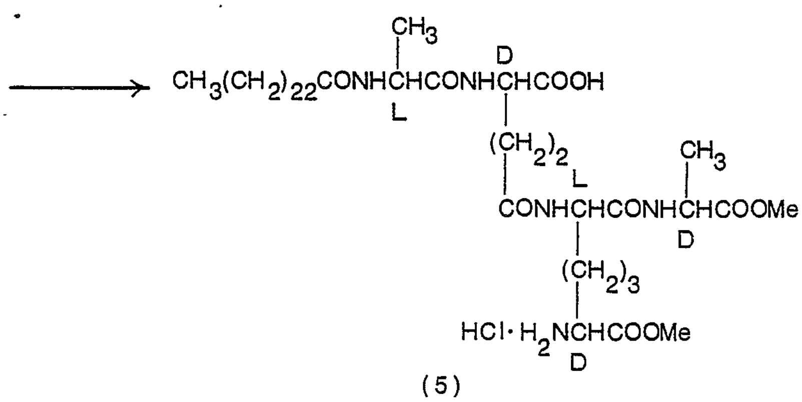

(5)

n-Tetracosanoyl-L-Ala-$\gamma$-D-Glu($\alpha$-OH)-(L)-mesoDAP-(L)-D-AlaOMe-(D)-OMe hydrochloric acid salt (5) was prepared substantially in the same manner as step 3 of Example 4.

$[\alpha]_D = -14.01$ (C=0.26, acetic acid)

IR (Nujol): 3380, 1745, 1630 $cm^{-1}$

NMR (DMSO-$d_6$), $\delta$: 0.85 (3H, m), 1.0—2.4 (60H, m), 3.60 (3H, s), 3.72 (3H, s)

(4) Step 4

Compound (3)

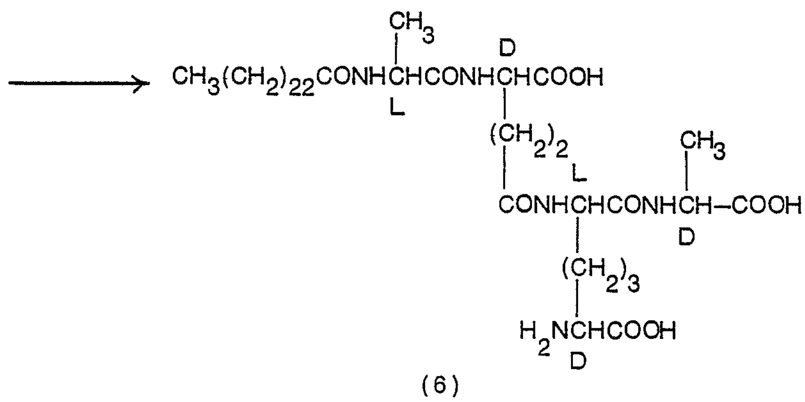

(6)

D-Tetracosanoyl-L-Ala-$\gamma$-D-Glu($\alpha$-OH)-(L)-mesoDAP-(L)-D-AlaOH (6) was prepared substantially in the same manner as step 3 of Example 4.

IR (Nujol): 3280, 1730, 1630 $cm^{-1}$

NMR ($D_2O$+$NaHCO_3$), $\delta$: 0.88 (3H, m), 3.72 (1H, m), 3.95—4.6 (4H, m)

Example 7

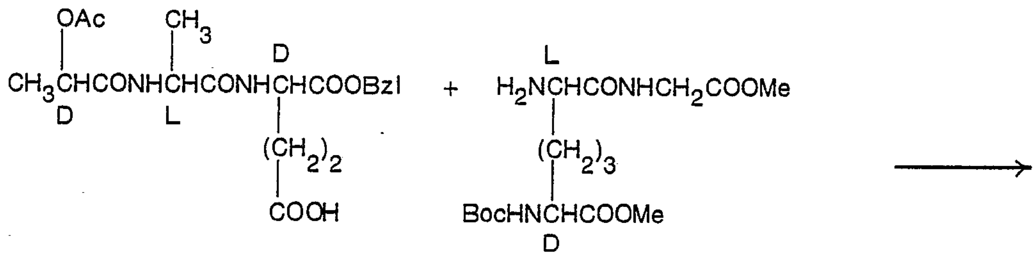

(1) (2)

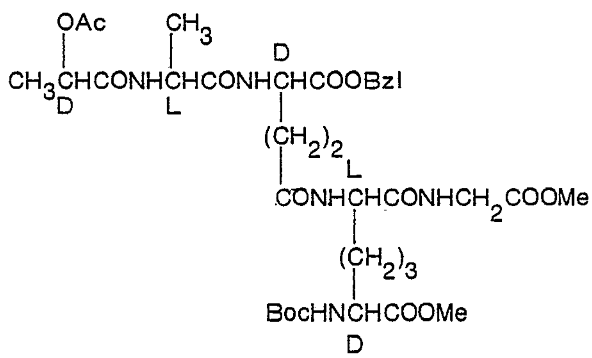

(3)

D-Lac(OAc)-L-Ala-$\gamma$-D-Glu($\alpha$-OBzl)-(L)-Boc-(D)-mesoDAP-(L)-GlyOMe-(D)-OMe (3) was prepared substantially in the same manner as step 1 of Example 4.

IR (Nujol): 3250, 1720, 1680, 1630 $cm^{-1}$

NMR ($CD_3OD$), $\delta$: 1.2—1.9 (17H, m), 2.12 (3H s), 1.9—2.6 (2H, m), 3.70 (6H, s), 3.97 (2H, s), 4.0—4.6 (4H, m), 4.98 (1H, q, J=7Hz), 5.18 (2H, s), 7.35 (5H, s)

Example 8

(1) Step 1

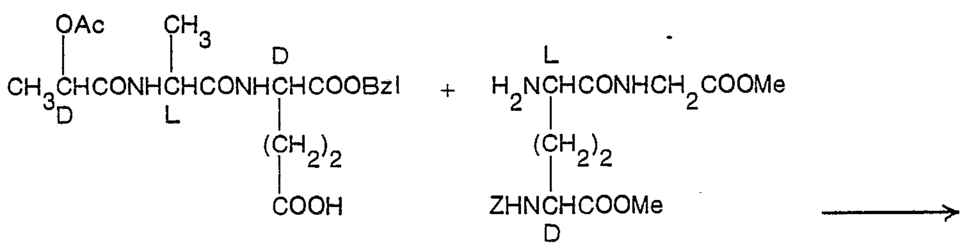

(1) (2)

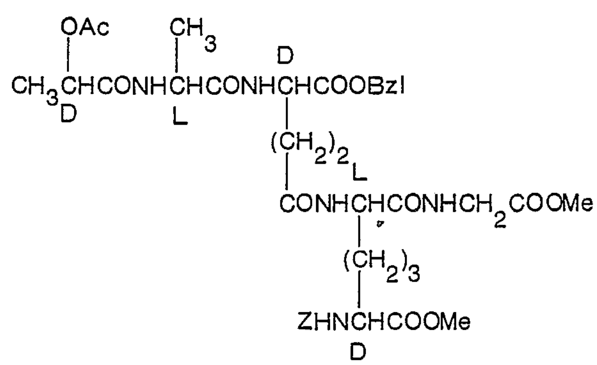

(3)

D-Lac(OAc)-L-Ala-γ-D-Glu(α-OBzl))-(L)-Z-(D)-mesoDAP-(L)-GlyOMe-(D)-OMe (3) was prepared substantially in the same manner as step 1 of Example 4.

IR (Nujol): 3260, 1735, 1635 $cm^{-1}$

NMR ($CDCl_3$), δ: 1.1—2.5 (10H, m), 1.35 (3H, d, J=7Hz), 1.42 (3H, d, J=7Hz), 2.08 (3H, s), 3.60 (3H, s), 3.67 (3H, s), 3.94 (2H, d, J=7Hz), 4.1—4.8 (4H, m), 4.9—5.2 (1H, m), 5.06 (2H, s), 5.09 (2H, s), 7.30 (10H, s)

(2) Step 2

Compound (3) 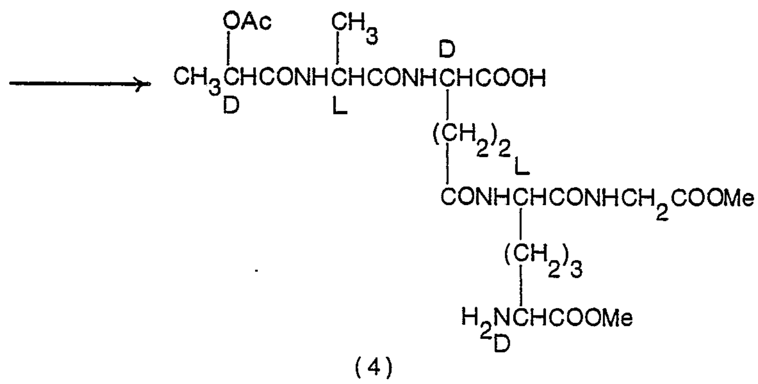

(4)

D-Lac(OAc)-L-Ala-γ-D-Glu(α-OBzl)-(L)-Z-(D)-mesoDAP)-(L)-GlyOMe-(D)-OMe (3) (1.32 g) was hydrogenated in acetic acid (15 ml) over 10% palladium charcoal (0.4 g) for 1.5 hours under an atmospheric pressure of hydrogen at ambient temperature. After completion of the reaction, the catalyst was filtered off and the filtrate was evaporated to dryness. The residue was pulverized with ether to give D-Lac(OAc)-L-Ala-γ-D-Glu(α-OH)-(L)-mesoDAP-(L)-GlyOMe-(D)-OMe (4)(1.1 g).

NMR ($D_2O$), δ: 1.2—2.3 (10H, m), 1.43 (3H, d, J=7Hz), 1.47 (3H, d, J=7Hz), 3.77 (3H, s), 3.87 (3H, s), 4.03 (2H, s), 4.1—4.5 (4H, m), 5.07 (1H, q, J=7Hz)

Example 9

(1) Step 1

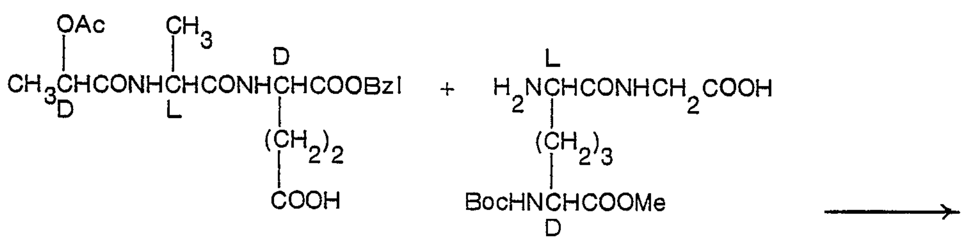

(1) (2)

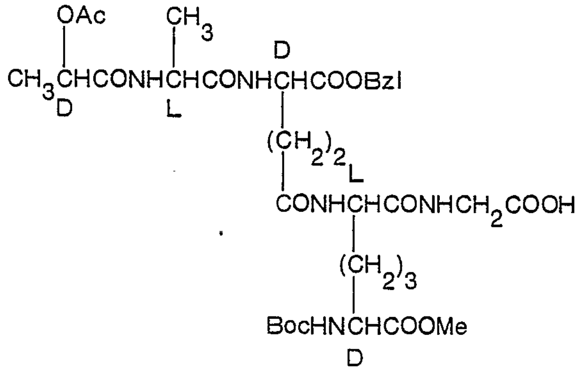

(3)

D-Lac(OAc)-L-Ala-$\gamma$-D-Glu($\alpha$-OBzl)-(L)-Boc-(D)-mesoDAP-(L)-GlyOH-(D)-OMe (3) was prepared substantially in the same manner as step 1 of Example 4.

IR (Nujol): 3270, 1735, 1650 $cm^{-1}$

NMR ($CDCl_3$), $\delta$: 1.2—2.7 (16H, m), 1.43 (9H, s), 2.12 (3H, s), 3.70 (3H, s), 3.8—4.5 (7H, m), 4.9—5.3 (1H, m), 5.13 (2H, s), 7.32 (5H, s)

(2) Step 2

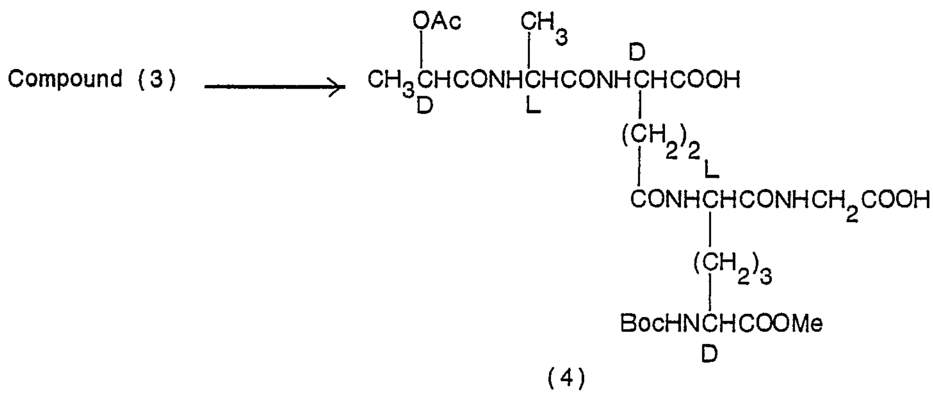

(4)

D-Lac(OAc)-L-Ala-$\gamma$-D-Glu($\alpha$-OH)-(L)-Boc-(D)-mesoDAP-(L)-GlyOH-(D)-OMe (4) was prepared substantially in the same manner as step 2 of Example 8.

IR (Nujol): 3300, 1725, 1650 $cm^{-1}$

NMR ($CD_3OD$), $\delta$: 1.3—2.6 (16H, m), 1.43 (9H, s), 2.11 (3H, s), 3.70 (3H, s), 3.93 (2H, s), 4.2—4.6 (4H, m), 5.08 (1H, q, J=7Hz)

## Example 10

(1) Step 1

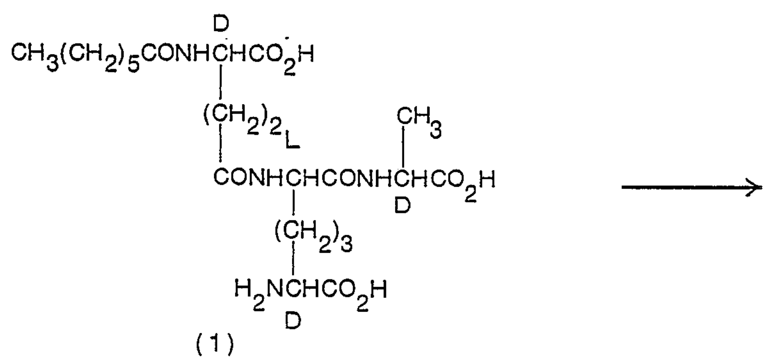

(1)

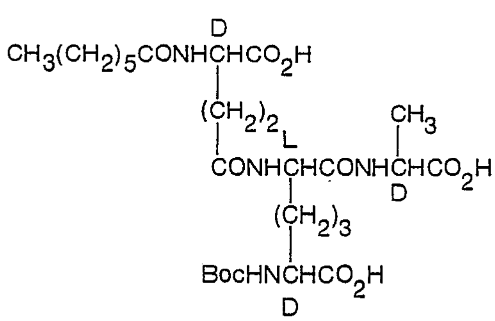

(2)

Heptanoyl-$\gamma$-D-Glu($\alpha$-OH)-(L)-mesoDAP-(L)-D-AlaOH (1) (1.00 g) was dissolved in the mixture of water (10 ml) and dioxane (20 ml). Triethylamine (730 mg) and 2-(tert-butoxycarbonyloxyimino)-2-phenylacetonitrile (640 mg) was added to the solution. After stirring for 16 hours at ambient temperature, the reaction mixture was concentrated. To the residue was added waters (30 ml), 1N hydrochloric acid (8 ml) and ethyl acetate. The organic layer was separated and washed with brine, dried over magnesium sulfate and concentrated in vacuo to give an oily residue, which was treated with ethylether and diisopropylether to give heptanoyl-γ-D-Glu(α-OH)-(L)-Boc-(D)-mesoDAP-(L)-D-AlaOH (2)(925 mg).

IR (Nujol): 3300, 1720, 1630 $cm^{-1}$

NMR ($CD_3OD$), δ: 0.90 (3H, m), 1.1—2.6 (23H, m), 3.70 (1H, t, J=6Hz), 3.90—4.65 (3H, m)

(2) Step 2

Compound (2) 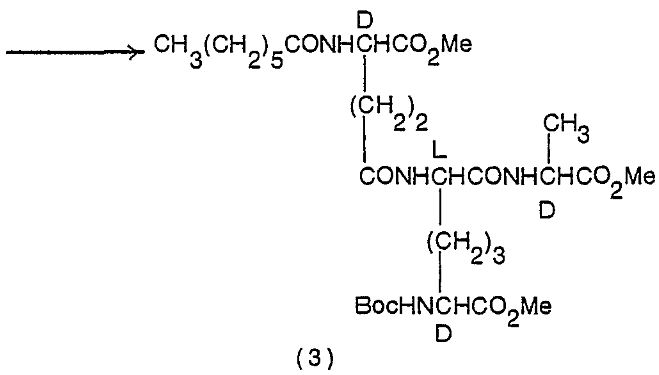

(3)

Heptanoyl-γ-D-Glu(α-OMe)-(L)-Boc-(D)-mesoDAP-(L)-D-AlaOMe-(D)-OMe (3) was prepared substantially in the same manner as step 2 of Example 4.

IR (Nujol): 3300, 1735, 1685, 1630 $cm^{-1}$

NMR ($CD_3OD$), δ: 0.91 (3H, m), 1.15—1.90 (16H, m), 2.00—2.45 (4H, m), 3.71 (9H, s), 4.06 (1H, m), 4.24—4.56 (3H, m)

(3) Step 3

Compound (3) 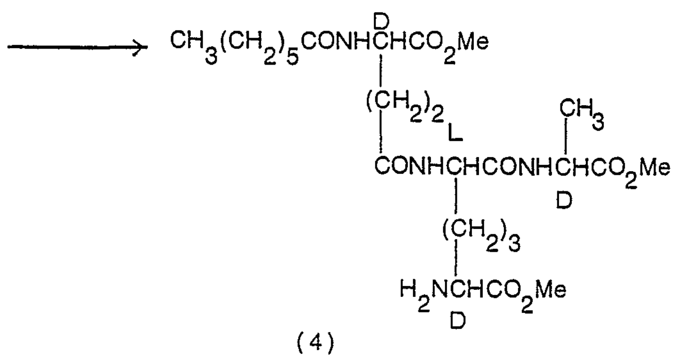

(4)

Heptanoyl-γ-D-Glu(α-OMe)-(L)-mesoDAP-(L)-D-AlaOMe-(D)-OMe (4) was prepared substantially in the same manner as step 3 of Example 4.

IR (Nujol): 3320, 3080, 1740, 1635 $cm^{-1}$

NMR ($CD_3OD$), δ: 0.92 (3H, m), 1.16—2.46 (26H, m), 3.74 (9H, s), 4.21—4.60 (4H, m)

Example 11

(1) Step 1

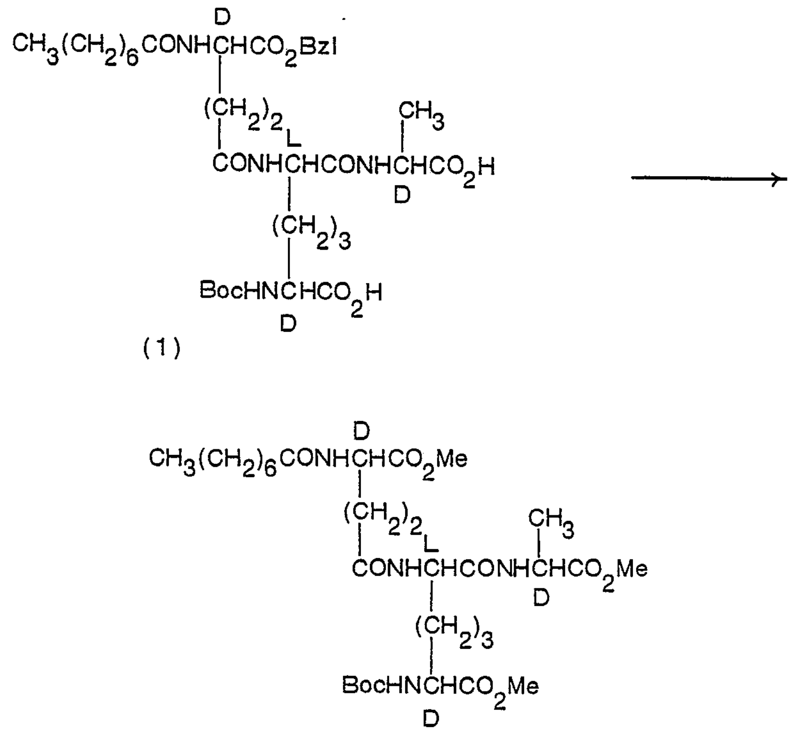

(1)

(2)

Octanoyl-$\gamma$-D-Glu($\alpha$-OMe)-(L)-Boc-(D)-mesoDAP-(L)-D-AlaOMe-(D)-OMe (2) was prepared substantially in the same manner as step 2 of Exaple 4.

IR (Nujol): 3370, 1750, 1690, 1640 $cm^{-1}$

NMR ($CDCl_3$), $\delta$: 0.89 (3H, m), 1.05—2.75 (37H, m), 3.72 (9H, s), 4.20 (1H, m), 4.35—4.75 (3H, m), 5.35 (1H, d, J=8Hz), 6.75 (1H, d, J=8Hz), 6.85 (1H, d, J=8Hz), 7.42 (1H, d, J=7Hz)

(2) Step 2

Compound (2) 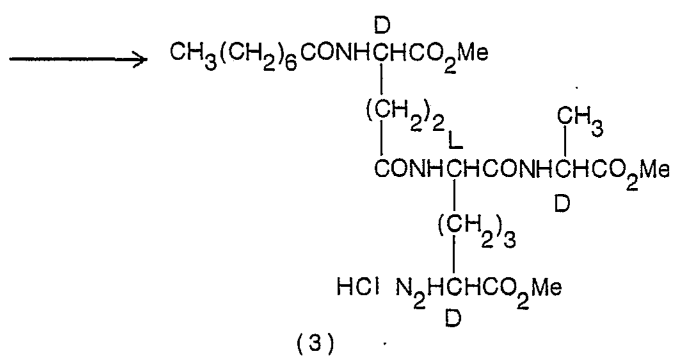

(3)

Octanoyl-$\gamma$-D-Glu($\alpha$-OMe)-(L)-mesoDAP-(L)-D-AlaOMe-(D)-OMe hydrochloric acid salt (3) was prepared substantially in the same manner as step 3 of Example 4.

IR (Nujol): 3280, 1735, 1630 $cm^{-1}$

NMR ($CD_3OD$), $\delta$: 0.89 (3H, m), 1.2—2.5 (22H, m), 3.72 (6H, s), 3.86 (3H, s), 4.07 (1H, t, J=6Hz), 4.19—4.65 (3H, m)

Example 12

(1) Step 1

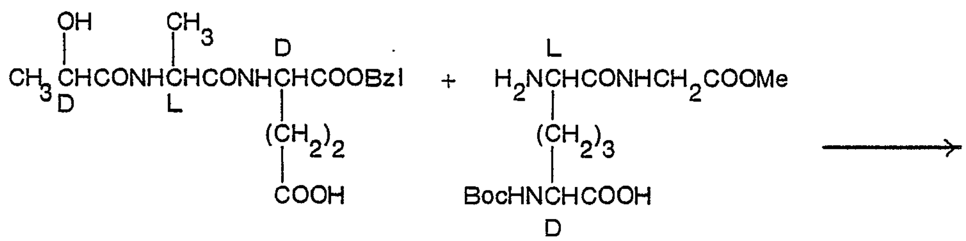

(1) (2)

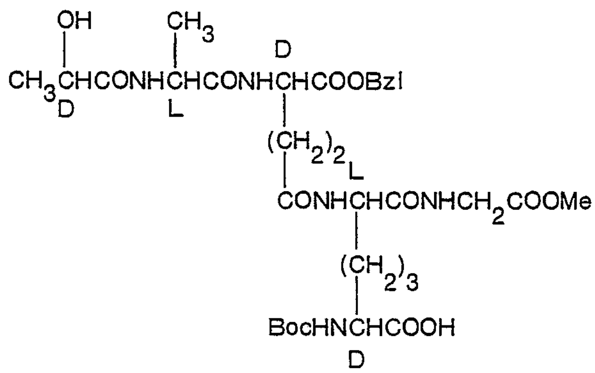

(3)

D-Lac-L-Ala-γ-D-Glu(α-OBzl)-(L)-Boc-(D)-mesoDAP-(L)-GlyOMe (3) was prepared substantially in the same manner as step 1 of Example 4.

NMR ($CD_3OD$), δ: 1.38 (6H, d, J=7Hz), 1.47 (9H, s), 1.3—2.5 (10H, m), 3.75 (3H, s), 4.00 (2H, s), 4.0—4.6 (5H, m), 5.20 (2H, s), 7.40 (5H, s)

(2) Step 2

Compound (3) 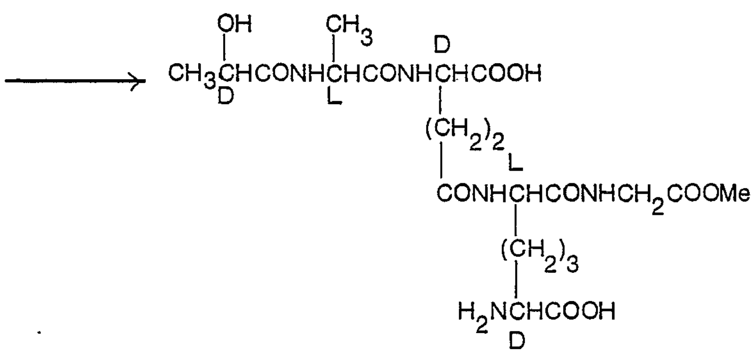

(4)

D-Lac-L-Ala-γ-D-Glu(α-OH)-(L)-mesoDAP-(L)-GlyOMe (4) was prepared substantially in the same manner as step 3 of Example 4.

IR $cm^{-1}$ Nujol: 3280, 1740, 1640

NMR ($D_2O$), δ: 1.38 (3H, d, J=7Hz), 1.41 (3H, d, J=7Hz), 1.3—2.5 (10H, m), 3.75 (3H, s), 3.98 (2H, s), 4.0—4.6 (5H, m)

Example 13

(1) Step 1

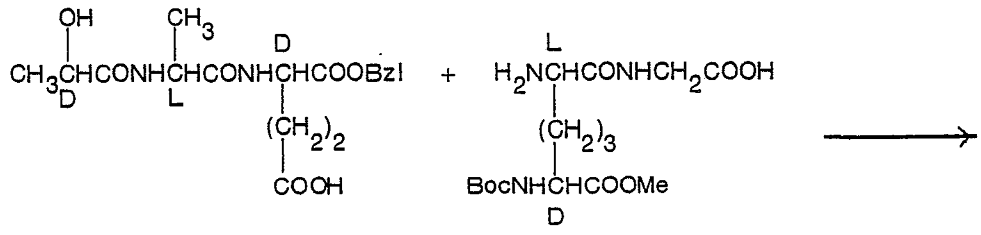

(1) (2)

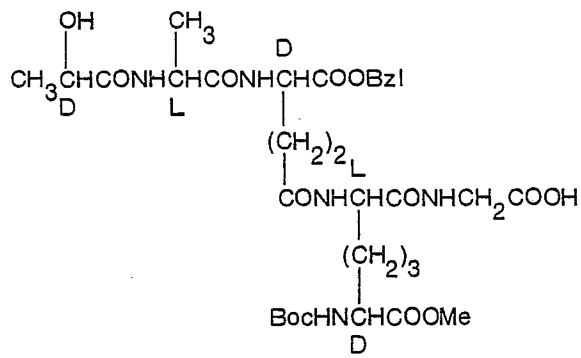

(3)

D-Lac-L-Ala-γ-D-Glu(α-OBzl)-(L)-Boc-(D)-mesoDAP-(L)-GlyOH-(D)-OMe (3) was prepared substantially in the same manner as step 1 of Example 4.

NMR ($CD_3OD$), δ: 1.42 (9H, s), 1.2—2.5 (16H, m), 3.70 (3H, s), 3.92 (2H, s), 4.0—4.6 (5H, m), 5.17 (2H, s), 7.35 (5H, s)

(2) Step 2

Compound (2)

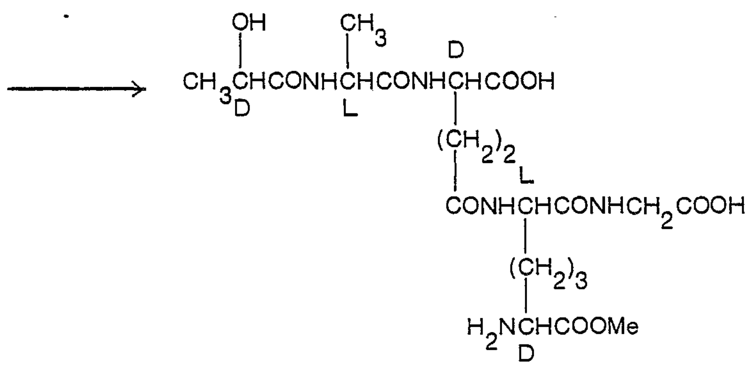

(3)

D-Lac-L-Ala-γ-D-Glu(α-OH)-(L)-mesoDAP-(L)-GlyOH-(D)-OMe (3) was prepared substantially in the same manner as step 3 of Example 4.

IR (KBr): 3700—2500, 1730, 1650 $cm^{-1}$

NMR ($D_2O$), δ: 1.37 (3H, d, J=7Hz), 1.40 (3H, d, J=7Hz), 1.3—2.5 (10H, m), 3.80 (2H, s), 3.83 (3H, s), 4.0—4.6 (5H, m)

Example 14

(1) Step 1

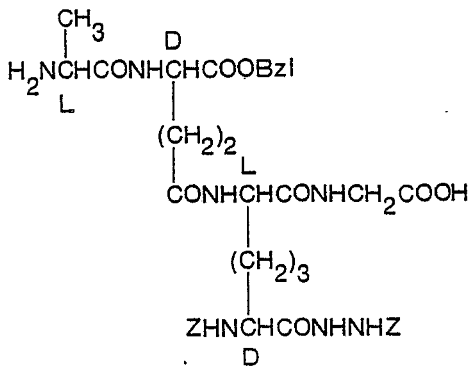

+

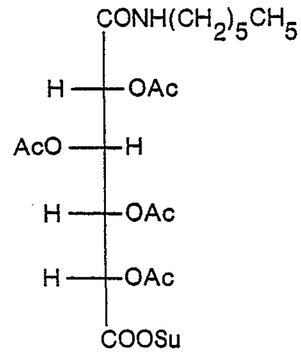

(1)

(2)

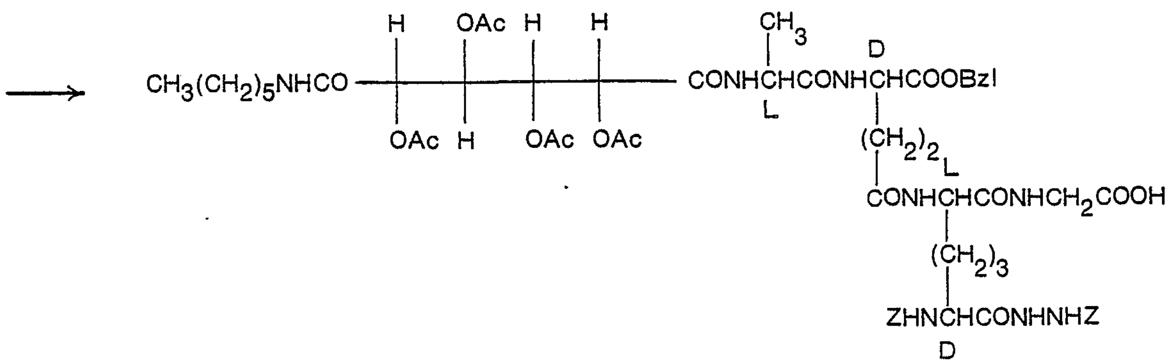

To an ice-cooling solution of L-Ala-$\gamma$-D-Glu($\alpha$-OBzl)-(L)-Z-(D)-mesoDAP-(L)-GlyOH-(D)-NHNHZ (1)(1.23 g) and triethylamine (0.30 g) in a mixture of acetone (12 ml) and water (2 ml) was added a solution of ester of 1-N-n-hexyl-2,3,4,5-O-tetraacetyl-D-glucaramic acid with N-hydroxylsuccinimide (2)(0.84 g) in acetone (12 ml). The mixture was stirred overnight, allowing the temperature of the mixture to reach to ambient temperature. After evaporation, the residue was added to a mixture of water (50 ml) and ethyl acetate (100 ml). The organic layer was washed with aqueous sodium bicarbonate (30 ml), water (30 ml), 10% hydrochloric acid (30 ml) and water (30 ml x 2) and then dried over magnesium sulfate. The solvent was removed in vacuo to give 1-N-n-hexyl-2,3,4,5-O-tetraacetyl-D-glucaramoyl-L-Ala-$\gamma$-D-Glu($\alpha$-OBzl)-(L)-Z-(D)-mesoDAP-(L)-GlyOH-(D)-NHNHZ (3)(1.15 g).

NMR (DMSO-$d_3$), $\delta$: 0.7—1.0 (3H, m), 1.1—1.7 (19H, m), 1.7—2.3 (2H, m), 1.96 (3H, s), 2.00 (3H, s), 2.07 (3H, s), 2.12 (3H, s), 2.8—3.1 (2H, m), 3.8—4.5 (10H, m), 5.00 (2H, s), 5.06 (2H, s), 5.10 (2H, s), 5.30 (1H, m), 5.60 (1H, m), 7.34 (15H, s), 7.7—8.2 (5H, m), 8.40 (1H, m), 9.98 (1H, s)

(2) Step 2

Compound (3) →

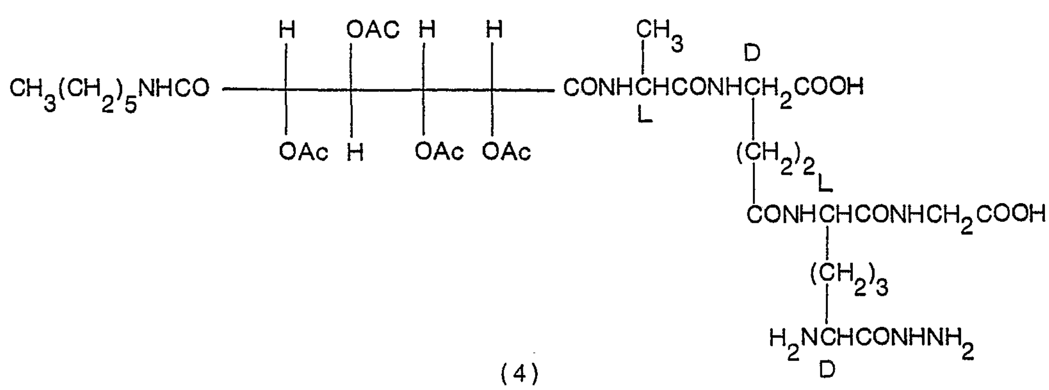

(4)

1-N-n-hexyl-2,3,4,5-O-tetraacetyl-D-Glucaramoyl-L-Ala-γ-D-Glu(αOH)-(L)-mesoDAP-(L)-GlyOH-(D)-$NHNH_2$ (4) was prepared substantially in the same manner as step 2 of Example 1.

NMR ($CD_3OD$), δ: 0.8—1.1 (3H, m), 1.1—2.5 (21H, m), 2.03 (6H, s), 2.17 (3H, s), 2.23 (3H, s), 3.0—3.6 (2H, m), 3.7—4.8 (6H, m), 5.1—6.0 (4H, m)

(3) Step 3

Compound (4)

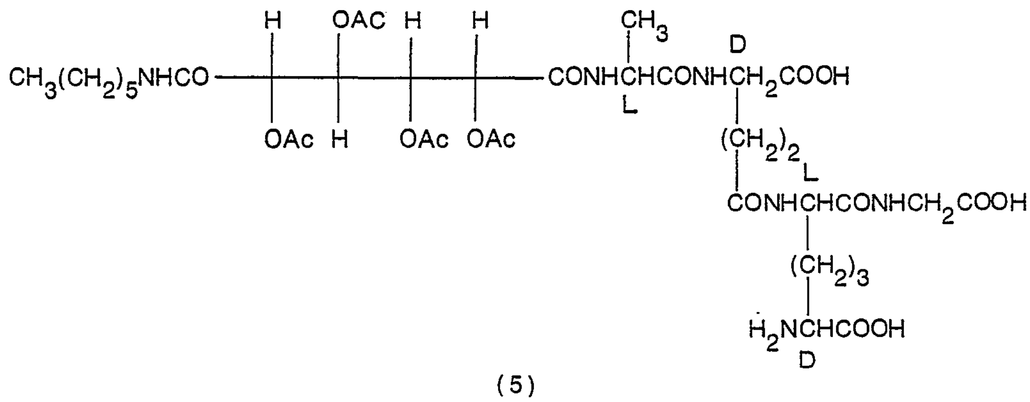

(5)

To an ice-cooling solution of 1-N-nhexyl-2,3,4,5-O-tetraacetyl-D-glucaramoyl -L-Ala-γ-D-Glu(α-OH)-(L)-mesoDAP-(L)-GlyOH-(D)-$NHNH_2$ (4)(0.64 g) in a mixture of 10% hydrochloric acid (3 ml) and 1,4-dioxane (6 ml) was added N-bromosuccinimide (0.2 g). After stirring for an hour at ambient temperature, the mixture was treated with sodium bisulfite and then adjusted to pH 4 with aqueous sodium carbonate. After evaporation, the residue was dissolved in 1N hydrochloric acid (2 ml) and put on a column of HP—20 (40 ml). After removal of inorganic salts with water, the product was obtained by eluting with 70% methanol. The eluate was lyophilized to give 1-N-n-hexyl-2,3,4,5-O-tetraacetyl-D-glucaramoyl-L-Ala-γ-D-Glu(α-OH)-(L)-mesoDAP-(L)-GlyOH (5) (0.43 g).

NMR ($D_2O$), δ: 0.7—1.0 (3H, m), 1.0—1.6 (19H, m), 1.6—2.5 (2H, m), 2.14 (3H, s), 2.16 (3H, s), 2.22 (3H, s), 2.24 (3H, s), 3.0—3.3 (2H, m), 3.76 (1H, m), 4.0—4.6 (5H, m), 5.1 —5.8 (4H, m)

(4) Step 4

Compound (5)

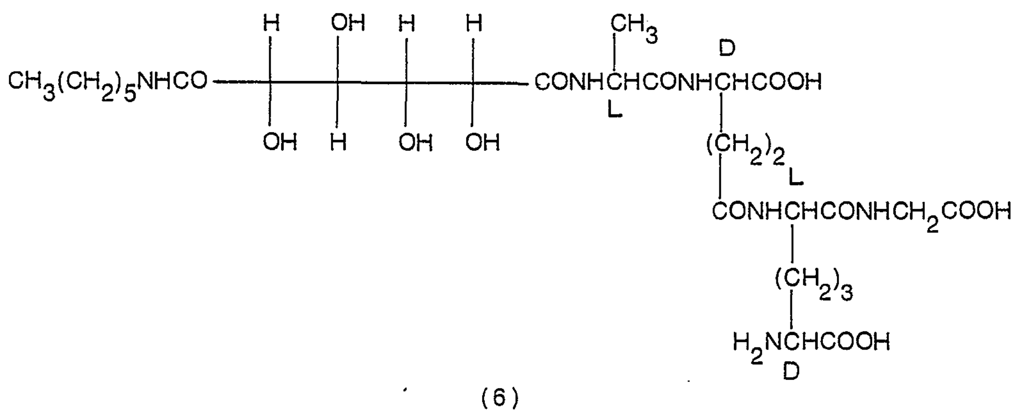

(6)

To an ice-cooling solution of 1-N-n-hexyl-2,3,4,5-O-tetraacetyl-D-glucaramoyl-L-Ala-γ-D-Glu(α-OH)-(L)-mesoDAP-(L)-GlyOH (5)(0.33 g) in 50% methanol was added 2N potassium carbonate (2 ml). The mixture was stirred for two hours at the same temperature and then acidified to pH 4 with 10% hydrochloric acid. After evaporation, the residue was dissolved in 1N hydrochloric acid (1 ml) and put on a column of HP—20 (15 ml). Inorganic salts were removed with water and then eluted with 80%

methanol. The eluate was lyophilized to give 1-N-n-hexyl-D-glucaramoyl-L-Ala-$\gamma$-D-Glu($\alpha$-OH)-(L)-mesoDAP-(L)-GlyOH (6)(0.26 g).

IR (Nujol): 3300, 1720, 1640, 1540, 1230 -m$^{-1}$

NMR ($D_2O$), $\delta$: 0.7—1.0 (3H, m), 1.0—2.5 (21H, m), 3.1—3.5 (2H, m), 3.5—4.5 (10H, m)

## Example 15

(1) Step 1

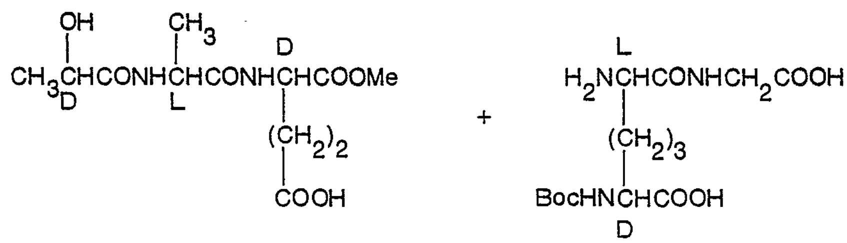

(1) (2)

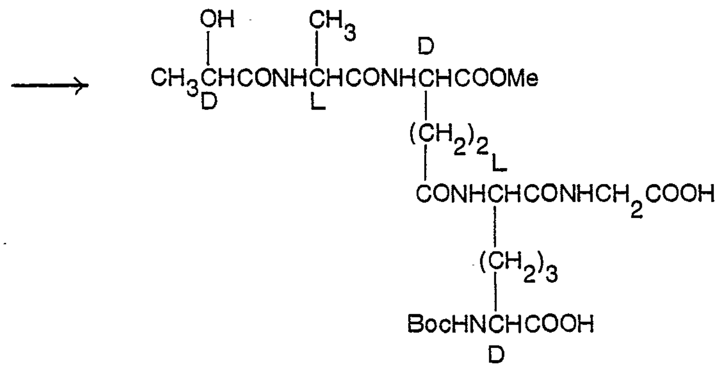

(3)

D-Lac-L-Ala-$\gamma$-D-Glu($\alpha$-OMe)-(L)-Boc-(D)-mesoDAP-(L)-GlyOH (3) was prepared substantially in the same manner as step 1 of Example 4.

NMR ($CD_3OD$), $\delta$: 1.46 (9H, s), 1.2—2.4 (10H, m), 3.72 (3H, s), 3.93 (2H, s), 4.0—4.6 (5H, m)

(2) Step 2

Compound (3)

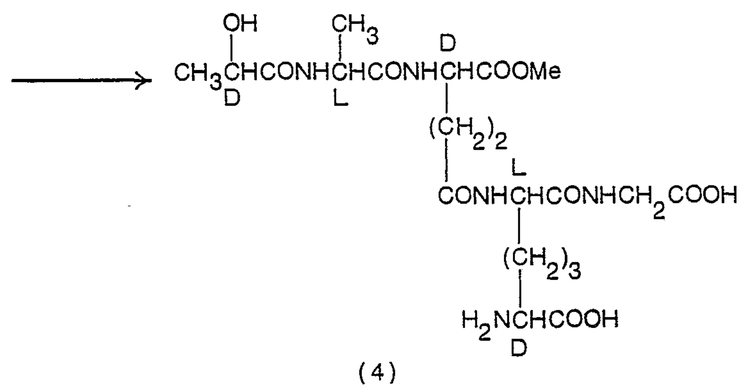

(4)

D-Lac-L-Ala-$\gamma$-D-Glu($\alpha$-OMe)-(L)-mesoDAP-(L)-GlyOH (4) was prepared substantially in the same manner as step 3 of Example 4.

NMR ($D_2O$), $\delta$: 1.40 (3H, d, J=7Hz), 1.45 (3H, d, J=7Hz), 1.3—2.6 (10H, m), 3.77 (3H, s), 3.87 (2H, s), 4.2—4.6 (4H, m)

Example 16

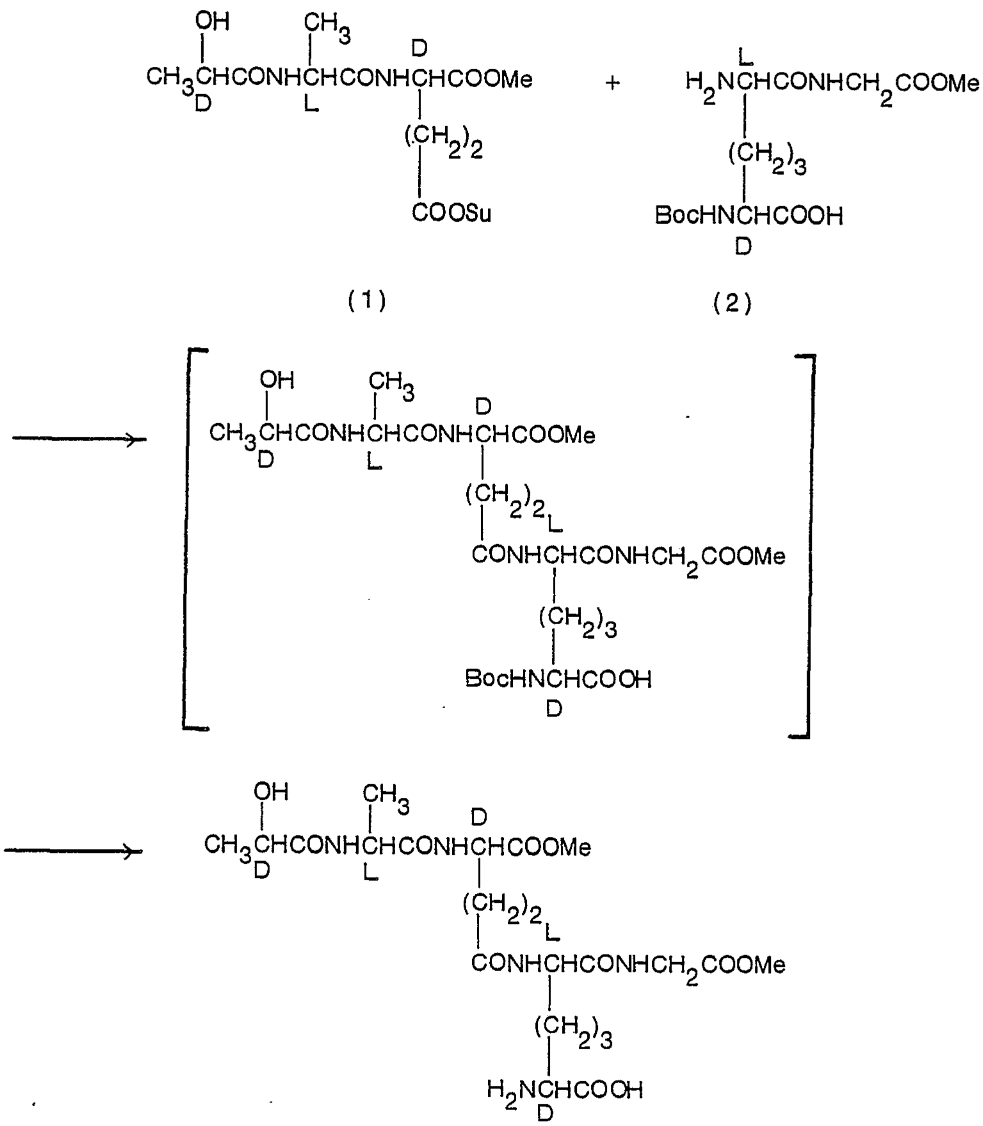

(1) (2)

(3)

D-Lac-L-Ala-$\gamma$-D-Glu($\alpha$-OMe)-(L)-mesoDAP-(L)-GlyOMe (3) was prepared substantially in the same manner as steps 1 and 3 of Example 4.

NMR ($D_2O$), $\delta$: 1.36 (3H, d, J=7Hz), 1.42 (3H, d, J=7Hz), 1.2—2.4 (10H, m), 3.72 (6H, s), 3.98 (2H, s), 4.2—4.5 (4H, m)

Example 17

(1) Step 1

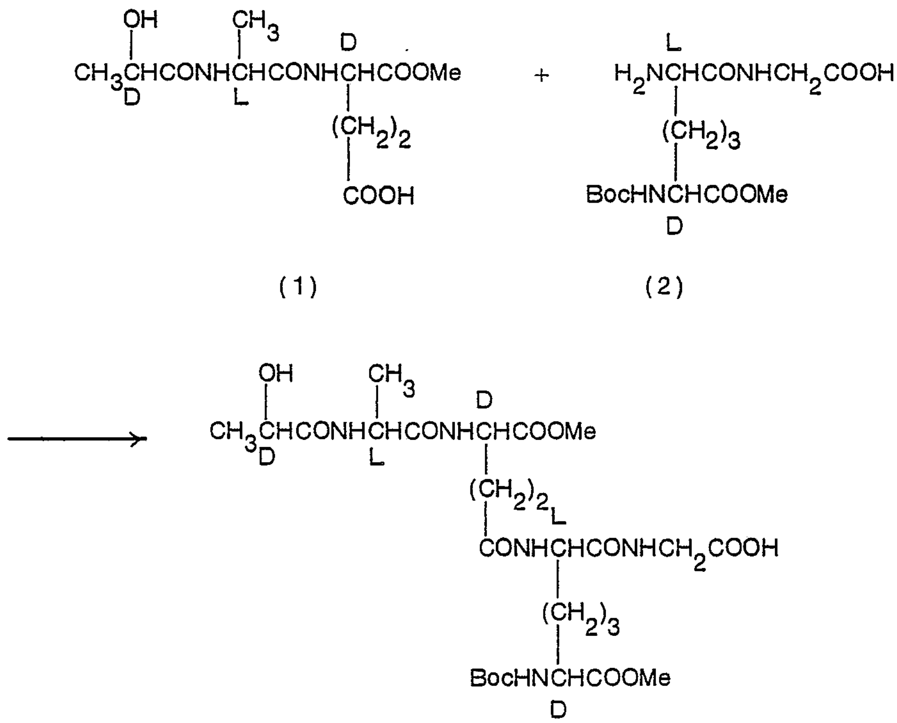

(1) (2)

(3)

D-Lac-L-Ala-γ-D-Glu(α-OMe)-(L)-Boc-(D)-mesoDAP-(D)-OMe-(L)-GlyOH (3) was prepared substantially in the same manner as step 1 of Example 4.

IR $cm^{-1}$ Nujol: 3300, 1730, 1660

NMR ($CD_3OD$), δ: 1.47 (9H, s), 1.4—2.5 (16H, m), 3.73 (6H, s), 3.95 (2H, s), 4.0—4.6 (5H)

(2) Step 2

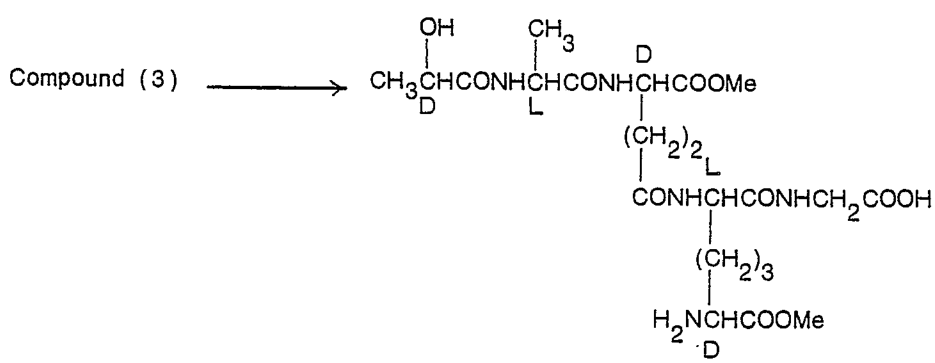


(4)

D-Lac-L-Ala-γ-D-Glu(α-OMe)-(L)-mesoDAP-(D)-OMe-(L)-GlyOH (4) was prepared substantially in the same manner as Step 3 of Example 4.

$[\alpha]_D = -22.9°$ (C=0.2, $H_2O$)

IR $cm^{-1}$ Nujol: 3400, 1745, 1650

NMR ($D_2O$), δ: 1.33 (3H, d, J=7Hz), 1.40 (3H, d, J=7Hz), 1.2—2.6 (10H, m), 3.77 (3h, s), 3.83 (3H, s), 4.0—4.6 (5H, m)

Example 18

(1) Step 1

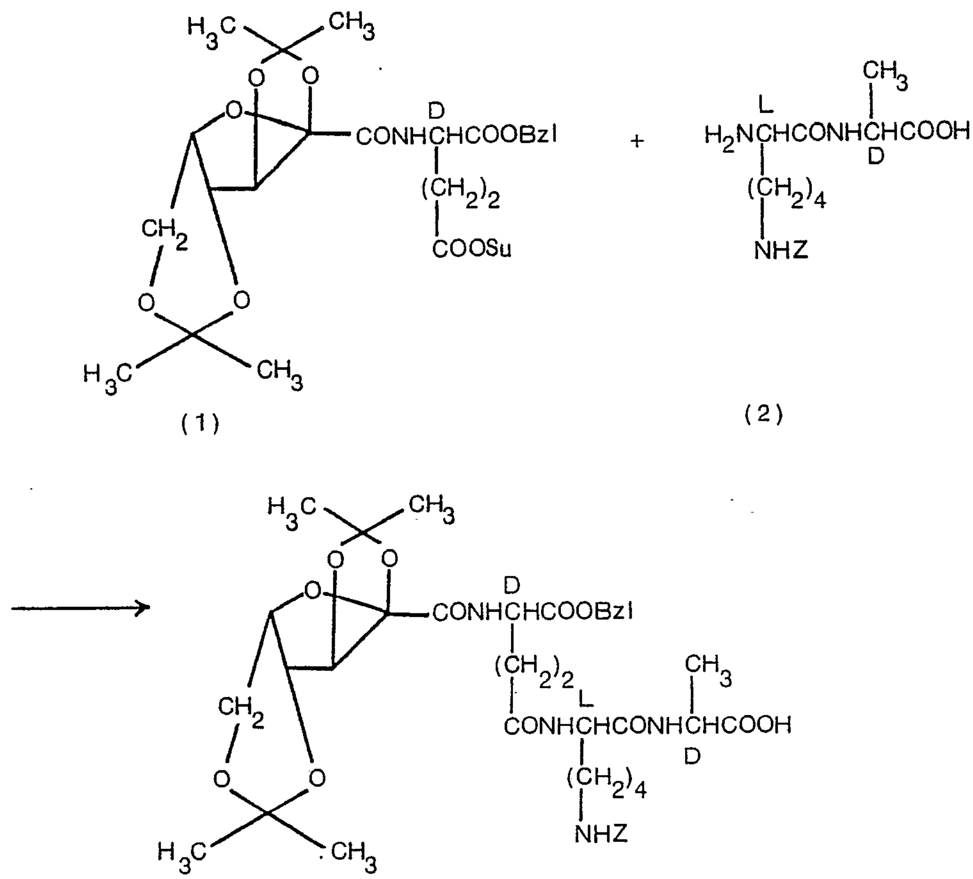

(1) (2)

(3)

2,3; 4,6-diisopropylidene-L-2-ketogulonoyl-γ-D-Glu(α-OBzl)-L-Lys(Z)-D-AlaOH (3) was prepared substantially in the same manner as step 1 of Example 1.

IR (Nujol), 3300, 1750—1620, 1250—1060$^{-1}$

(2) Step 2

Compound (3) 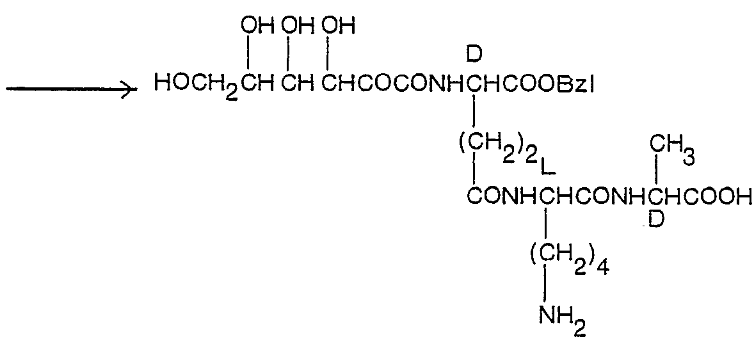

(4)

2,3;4,6-Diisopropylidene 2-ketogulonoyl-γ-D-Glu(α-OBzl)-L-Lys(Z)-D-AlaOH (3)(0.9 g) was dissolved in dioxane. To the solution was added conc. hydrochloric acid (5 ml), and the mixture was stirred for 20 hours and then adjusted to pH 6—7 with aqueous sodium bicarbonate solution. The resultant mixture was concentrated in vacuo and the residue was dissolved in water. The solution was subjected to carbon column (30 ml). The column was eluted with methanol and ethanol (1:1). The fractions

containing the object compound (4) were collected and the solvent was evaporated to give a foam which was lyophilized to give 2-keto-L-gulonoyl-γ-D-Glu(α-OBzl)-L-Lys-D-AlaOH (4)(400 mg).

NMR ($D_2O$), δ: 1.32 (3H, d, J=7Hz), 3.15 (2H, t, J=7Hz), 3.66—4.50 (8H, m), 5.10 (2H, s), 7.43 (5H, s)

(3) Step 3

Compound (4) 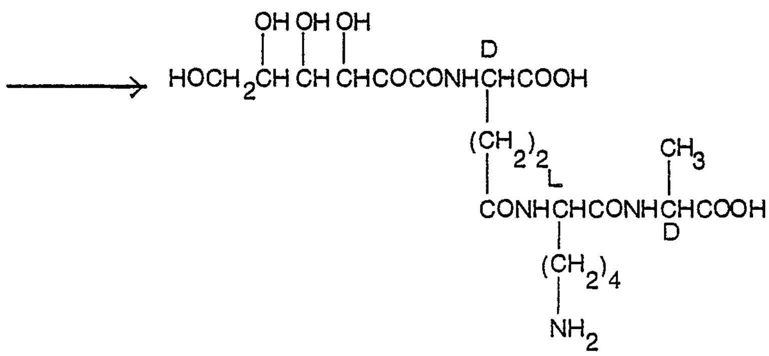

(5)

2-Keto-L-gulonoyl-γ-D-Glu(α-OH)-L-Lys-D-AlaOH (5) was prepared substantially in the same manner as step 2 of Example 1.

NMR($D_2O$), δ: 1.36 (3H, d, J=7Hz), 3.05 (2H, t, J=7Hz), 3.70—4.50 (8H, m)

Example 19

(1) Step 1

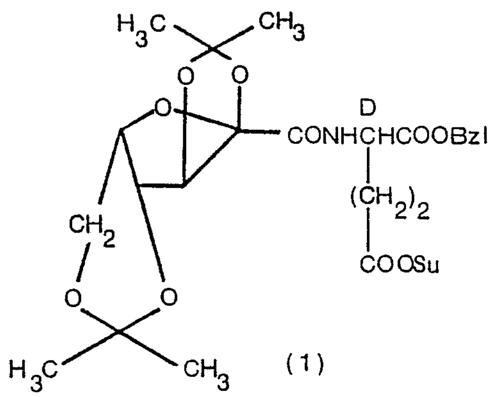

(1)

+

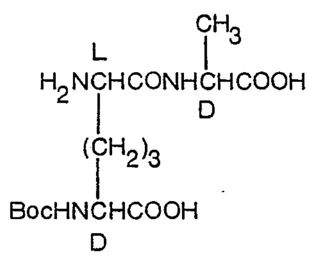

(2)

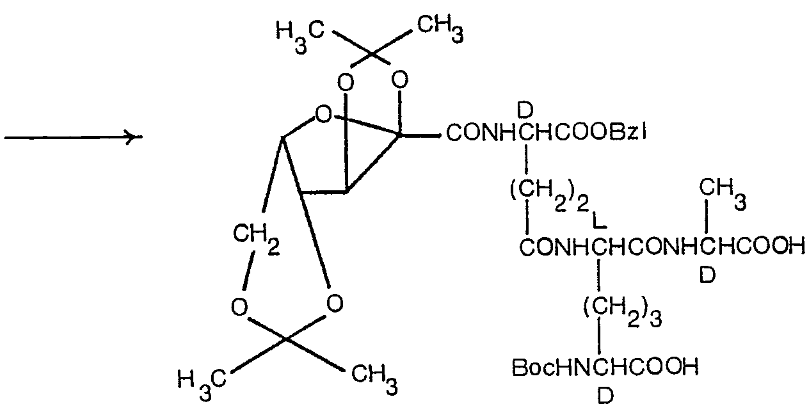

(3)

2,3;4,6-di-isopropylidene-2-keto-L-gulonoyl-$\gamma$-D-Glu($\alpha$-OBzl)-(L)-Boc (D)-mesoDAP-(L)-D-AlaOH (3) was prepared substantially in the same manner as step 1 of Example 1.

IR (Nujol): 3300, 1730, 1680, 1520, 1250—1060 $cm^{-1}$

NMR ($CD_3OD$), $\delta$: 1.06 (3H, s), 1.20 (3H, s), 1.47 (15H, s), 4.16—4.50 (9H, m), 5.20 (2H, s), 7.40 (5H, s)

(2) Step 2

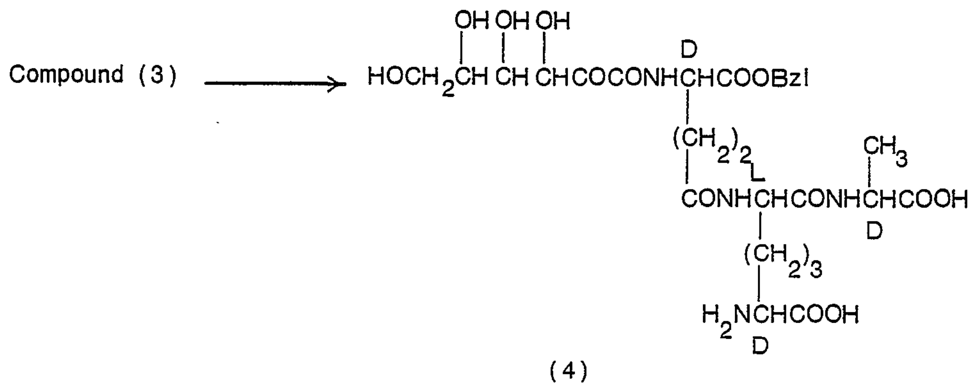


(4)

2-Keto-L-gulonoyl-$\gamma$-D-Glu($\alpha$-OBzl)-(L)-mesoDAP-(L)-D-AlaOH (4) was prepared substantially in the same manner as step 2 of Example 18.

IR (Nujol): 3300, 1730, 1650, 1530, 1220, 1180, 1050 $cm^{-1}$

NMR ($D_2O$), $\delta$: 1.33 (3H, d, J=8Hz), 3.66—4.50 (8H, m), 5.30 (2H, s), 7.47 (5H, s)

(3) Step 3

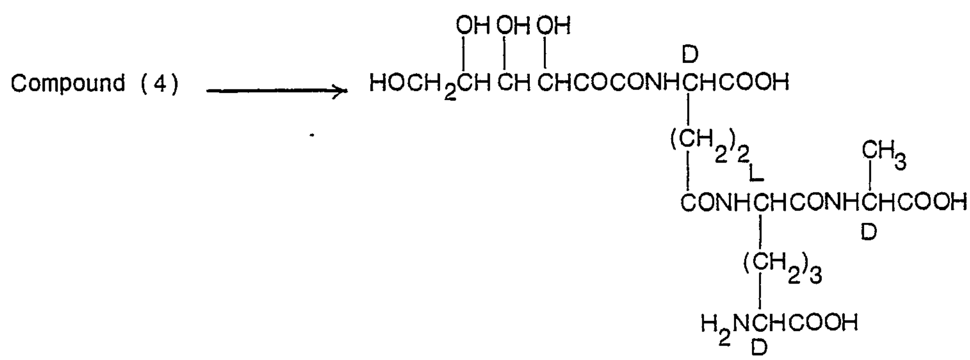


(5)

2-Keto-L-gulonoyl-$\gamma$-D-Glu($\alpha$-OH)-(L)-mesoDAP-(L)-D-AlaOH (5) was prepared substantially in the same manner as step 2 of Example 1.

IR (Nujol): 3300, 1720, 1670 $cm^{-1}$

NMR ($D_2O$), $\delta$: 1.38 (3H, d, J=7Hz), 1.70—2.50 (10H, m), 3.60—4.40 (8H, m)

Example 20

(1) Step 1

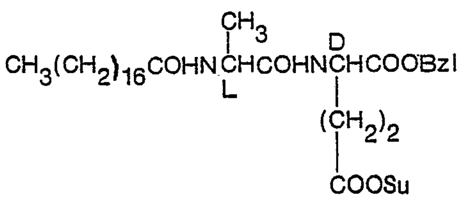

+

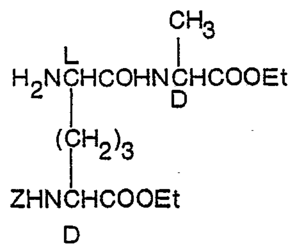

(1) (2)

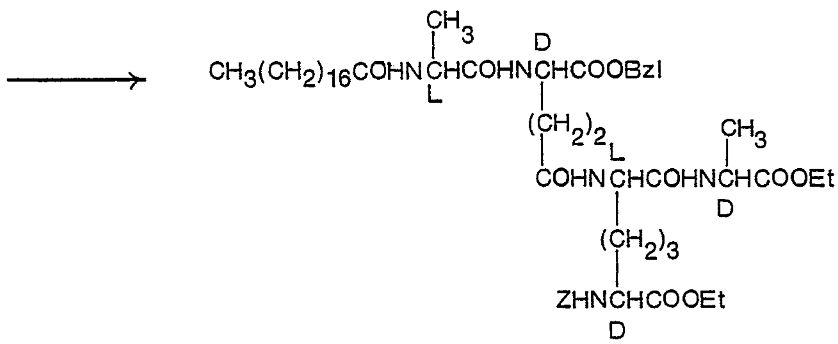

(3)

Stearoyl-L-Ala-$\gamma$-D-Glu($\alpha$-OBzl)-(L)-Z-(D)-mesoDAP-(D)-OEt-(L)-D-AlaOEt (3) was prepared substantially in the same manner as step 1 of Example 4.

IR (Nujol): 3280, .750, 1710, 1650 $cm^{-1}$

NMR ($CDCl_3$), $\delta$: 0.87 (3H, m), 3.96—4.60 (9H, m), 5.10 (2H, s), 5.13 (2H, s), 7.32 (10H, s)

(2) Step 2

Compound (3)

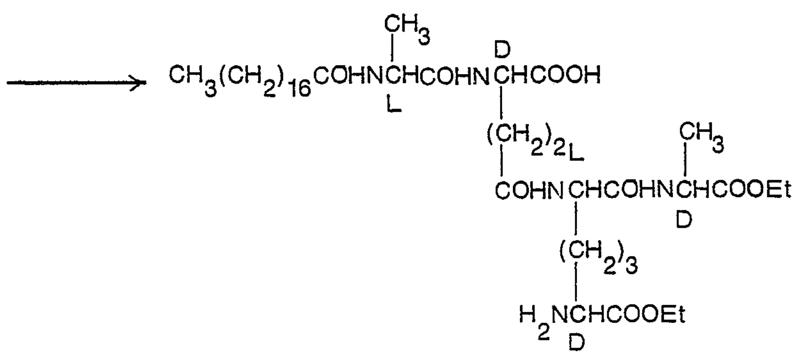

(4)

Stearoyl-L-Ala-$\gamma$-D-Glu($\alpha$-OBzl)-(L)-Z-(D)-mesoDAP-(D)-OEt-(L)-D-AlaOEt (3)(584 mg) was dissolved in acetic acid (20 ml) and hydrogenated under 2.5 atmospheric pressures of hydrogen over 10% palladium charcoal. After removal of the catalyst by filtration, the filtrate was evaporated in vacuo. To the residue was added hexane to give crystals which were washed with hot isopropylether to give stearoyl-L-Ala-$\gamma$-D-Glu($\alpha$-OH)-(L)-messoDAP-(D)-OEt-(L)-D-AlaOEt (4)(340 mg).

IR (Nujol): 3290, 1740, 1630 $cm^{-1}$

NMR ($CF_3COOH$), $\delta$: 0.87 (3H, m), 1.1—2.9 (54H, m), 4.13—5.00 (9H, m)

Example 21

(1) Step 1

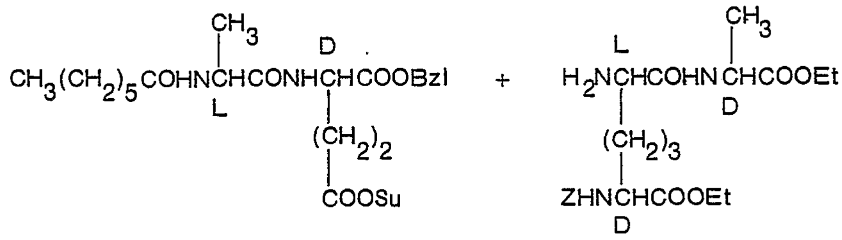

(1) (2)

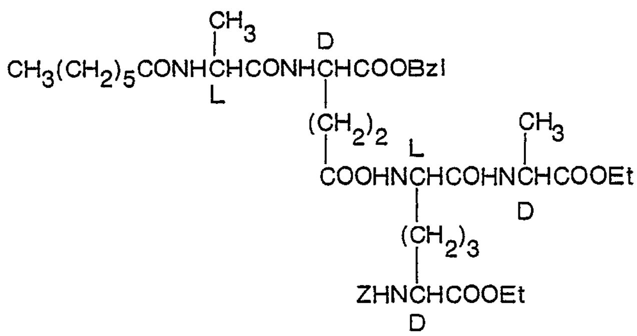

(3)

Heptanoyl-L-Ala-γ-D-Glu(α-OBzl)-(L)-Z-(D)-mesoDAP-(D)-OEt-(L)-D-AlaOEt (3) was prepared substantially in the same manner as Step 1 of Example 4.

IR (Nujol): 3260, 1730, 1680, 1620 $cm^{-1}$

NMR ($CDCl_3$), δ: 0.85 (3H, m), 1.05—2.40 (29H, m), 3.93—4.70 (8H, m), 5.06 (2H, s), 5.11 (2H, s), 5.58 (1H, d, J=8Hz), 6.60 (2H, d, J=8Hz), 7.20 (1H, d, J=8Hz), 7.31 (10H, s)

(2) Step 2

Compound (3)

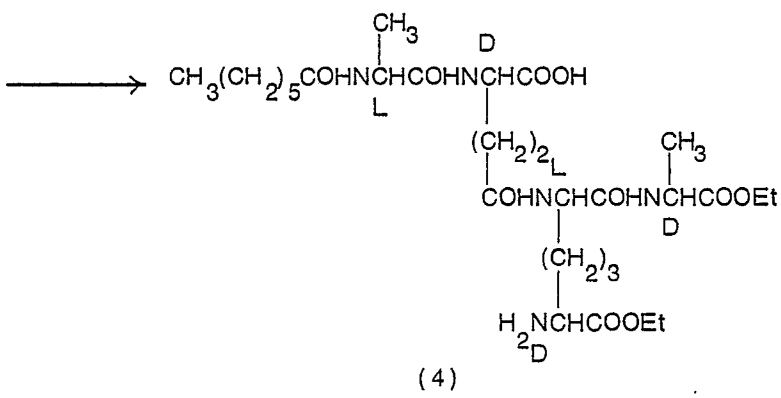

(4)

Heptanoyl-L-Ala-γ-D-Glu(α-OH)-(L)-mesoDAP-(D)-OEt-(L)-D-AlaOEt (4) was prepared substantially in the same manner as step 2 of Example 20.

IR (Nujol): 3280, 1740, 1635 $cm^{-1}$

NMR (DMSO-$d_6$), δ: 0.87 (3H, m), 1.02—2.33 (27H, m), 3.40 (1H, m)

Example 22

(1) Step 1

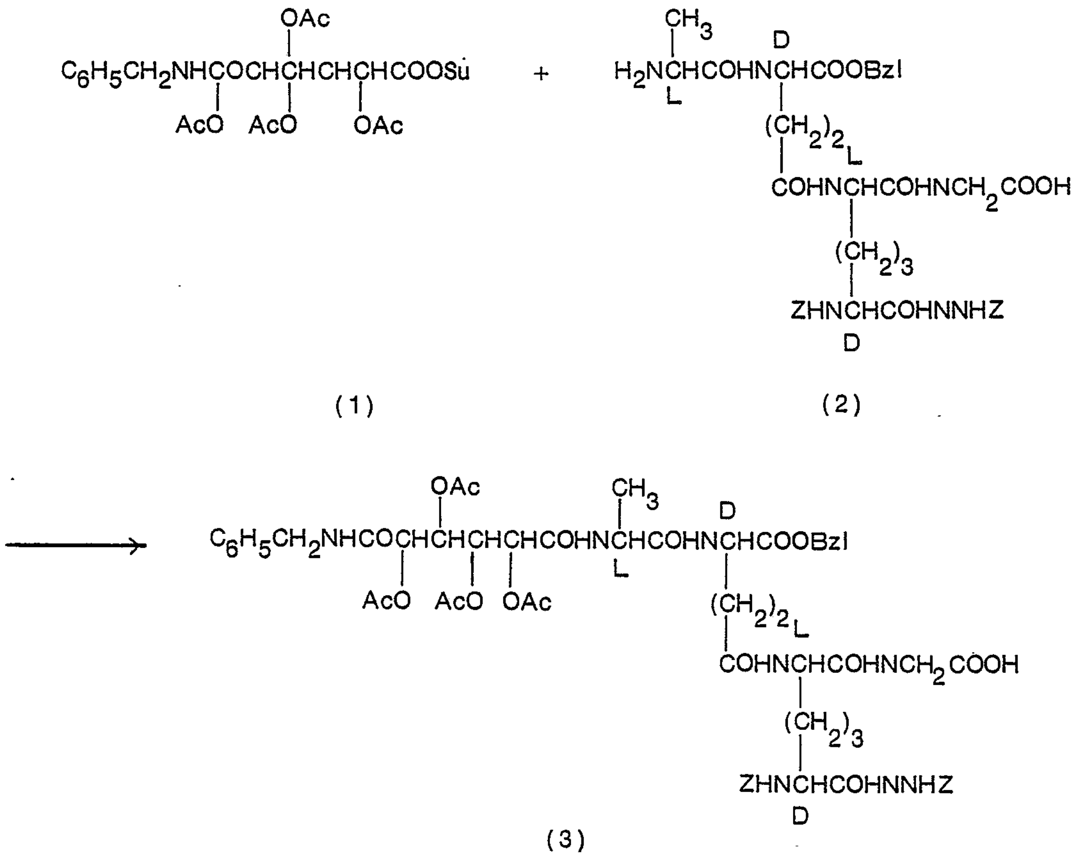

(1) (2)

(3)

1-N-Benzyl-2,3,4,5-O-tetraacetyl-D-glucarmoyl-L-Ala-$\gamma$-D-Glu($\alpha$-OBzl)-(L)-Z-(D)-mesoDAP-(D)-NHNHZ-(L)-GlyOH (3) was prepared in a similar manner to Step 1 of the Example 4.

mp. 176—178°C

IR (Nujol): 3350, 1750, 1670, 1530 $cm^{-1}$

NMR (DMSO-$d_6$): $\delta$ 1.0—2.2 (13H, m), 1.85 (3H, s), 1.94 (3H, s), 2.00 (3H, s), 2.10 (3H, s), 3.6—4.5 (8H, m), 4.95 (2H, s), 5.00 (2H, s), 5.06 (2H, s), 5.1—5.7 (2H, m), 7.20 (5H, s), 7.28 (15H, s), 7.6—8.6 (3H, m).

(2) Step 2

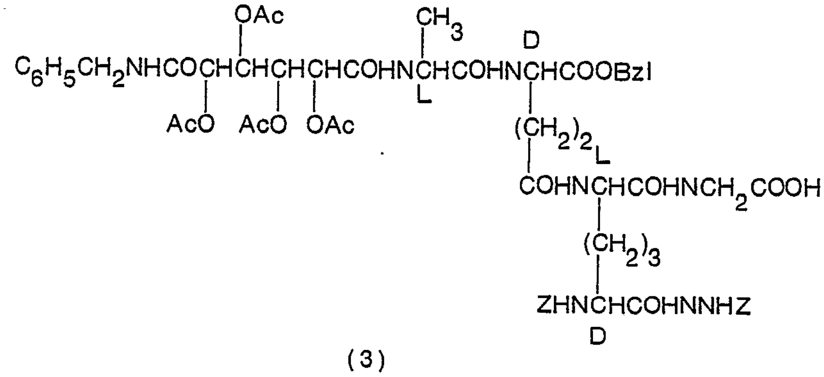

(3)

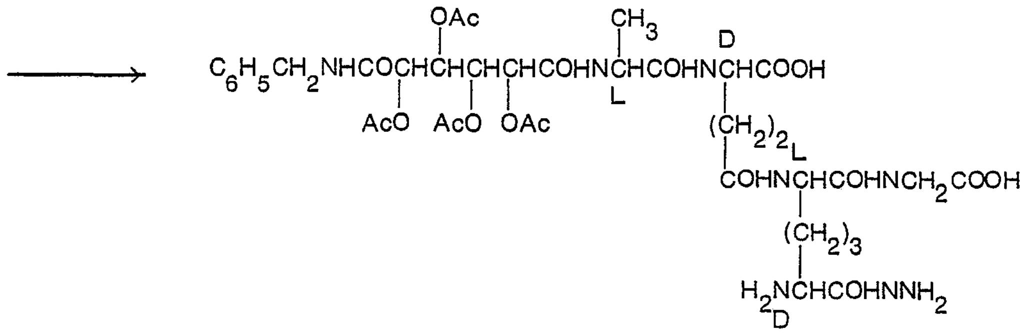

(4)

1-N-Benzyl-2,3,4,5-O-tetracetyl-D-glucaramoyl-L-Ala-$\gamma$-D-Glu($\alpha$-OH)-(L)-mesoDAP-(D)-$NHNH_2$-(L)-GlyOH (4) was prepared in a similar manner to the Example 1, Step 2.

mp. 178—181° (dec)

IR (Nujol): 3300, 1750, 1650, 1530 $cm^{-1}$

NMR ($CH_3OD$): $\delta$ 1.1—2.5 (13H, m), 1.83 (3H, s), 2.00 (3H, s), 2.13 (3H, s), 2.23 (3H, s), 3.85 (2H, s), 7.25 (5H, s).

(3) Step 3

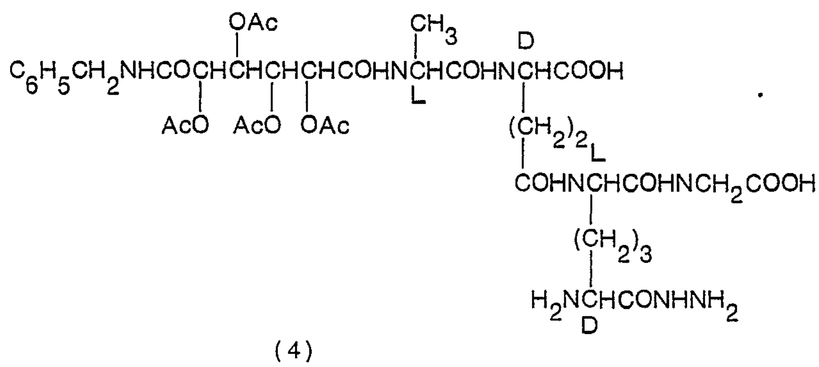

(4)

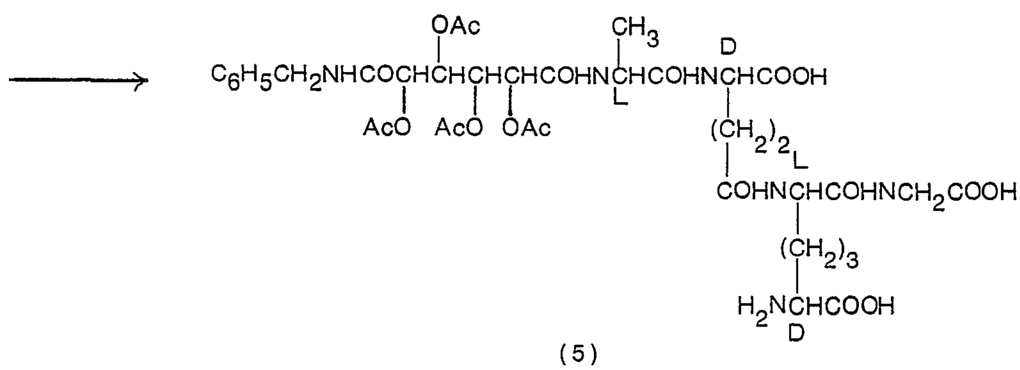

(5)

1-N-Benzyl-2,3,4,5-O-tetraacetyl-D-glucaramoyl-L-Ala-$\gamma$-D-Glu($\alpha$-OH)-(L)-mesoDAP-(L)-GlyOH (5) was prepared in a similar manner to the Example 14 Step 3.

mp. 145—150°C

IR (Nujol): 3350, 1760, 1660 (sh), 1650, 1530 $cm^{-1}$

NMR ($D_2O$): $\delta$ 1.43 (3H, d, J=7Hz), 1.3—2.5 (10H, m), 2.00 (3H, s), 2.10 (3H, s), 2.23 (6H, s), 3.7—4.0 (3H, m), 4.0—4.5 (3H, m), 5.1—6.0 (4H, m), 7.37 (5H, s)

(4) Step 4

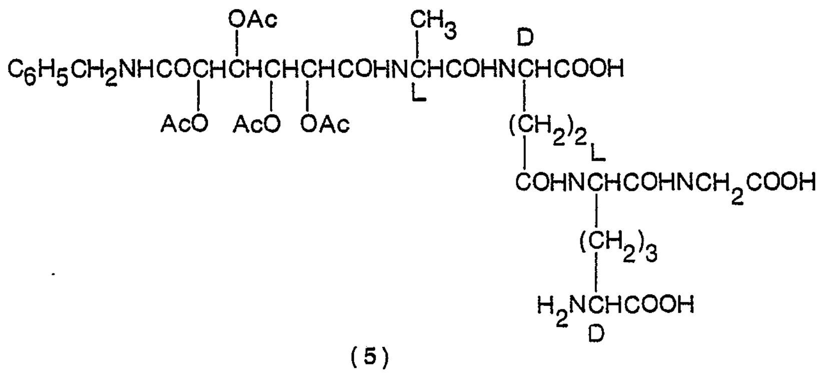

(5)

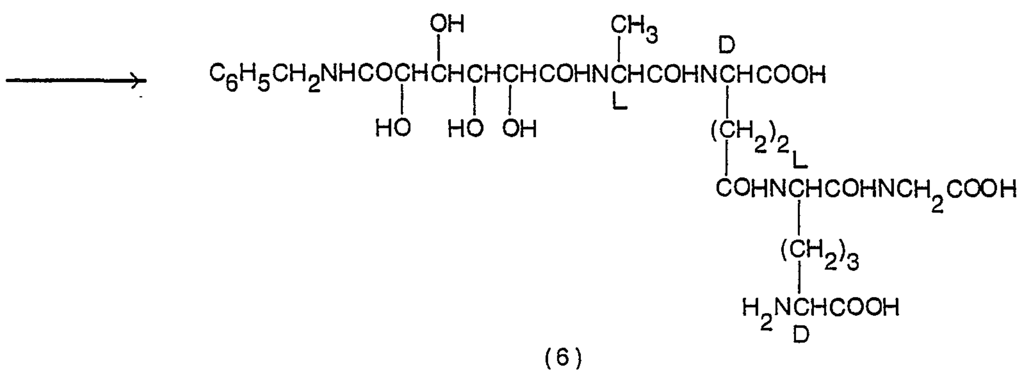

(6)

1-N-Benzyl-D-glucaramoyl-L-Ala-$\gamma$-Glu($\alpha$-OH)-(L)-mesoDAP-(L)-GlyOH (6) was prepared in a similar manner to the Example 14 Step 4.

mp. 117—120°C

IR (Nujol): 3350, 1730, 1640 (sh), 1540 $cm^{-1}$

NMR ($D_2O$): $\delta$1.42 (3H, d, J=7Hz), 1.2—2.5 (10H, m), 3.86 (2H, s), 3.5—5.0 (10H, m), 7.35 (5H, s).

Example 23

(1) Step 1

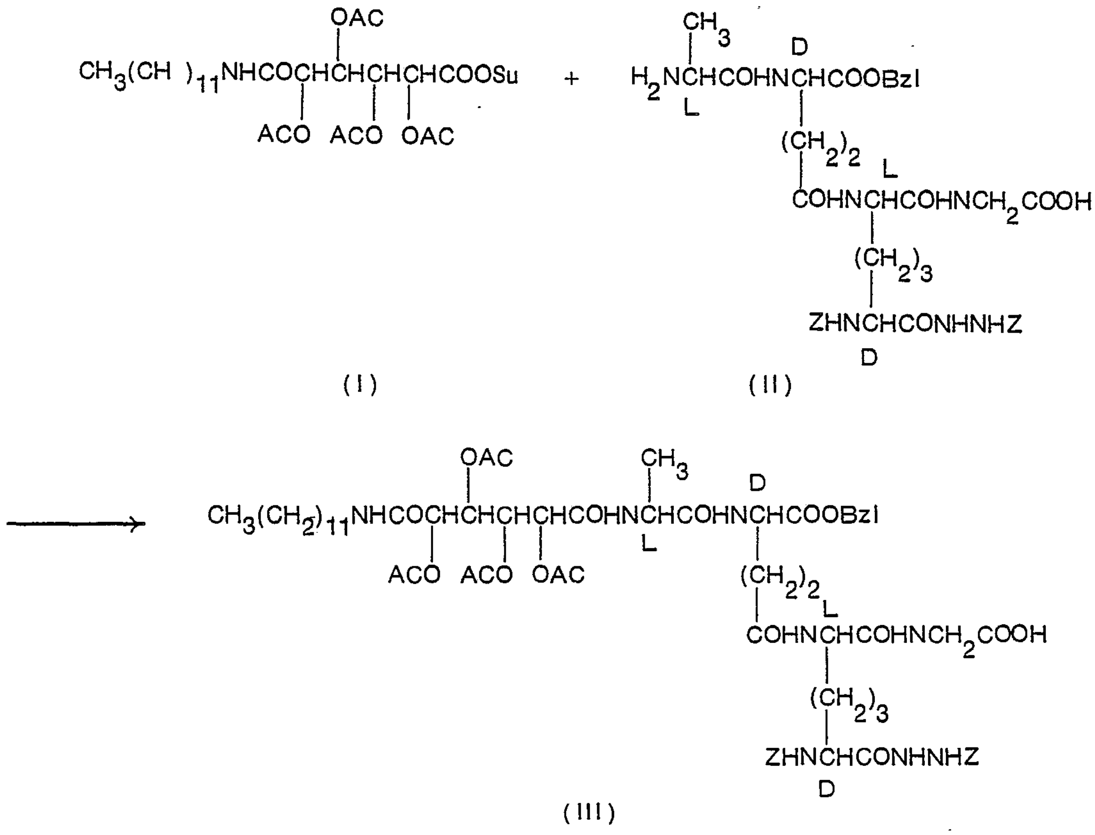

(I) (II)

(III)

1-N-lauryl-2,3,4-5-O-tetraacetyl-D-glucaramoyl-L-Ala-$\gamma$-D-Glu($\alpha$-OBzl)-(L)-Z-(D)-mesoDAP-(D)-NHNHZ-(D)-GlyOH (III) was prepared in a similar manner to the Example 4, step 1.

IR (Nujol): 3350, 1760, 1670, 1650, 1540 $cm^{-1}$

NMR ($DMSOD_6$): $\delta$ 0.9 (3H, m), 1.0—2.3 (33H, m), 1.95 (3H, s), 2.00 (3H, s), 2.07 (3H, s), 2.15 (3H, s), 3.00 (2H, m), 3.2—4.6 (10H, m), 5.08 (2H, s), 5.13 (2H, s), 5.18 (2H, s), 5.2—5.8 (2H, m), 7.37 (15H, s), 7.7—8.5 (5H, m), 9.20 (1H, m), 9.90 (1H, s).

(2) Step 2

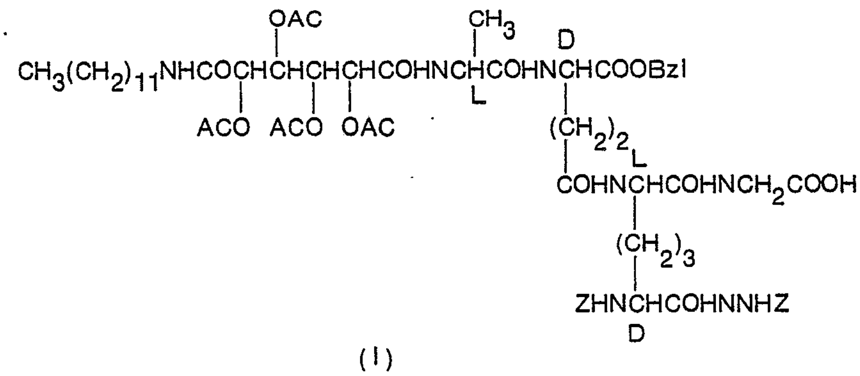

(I)

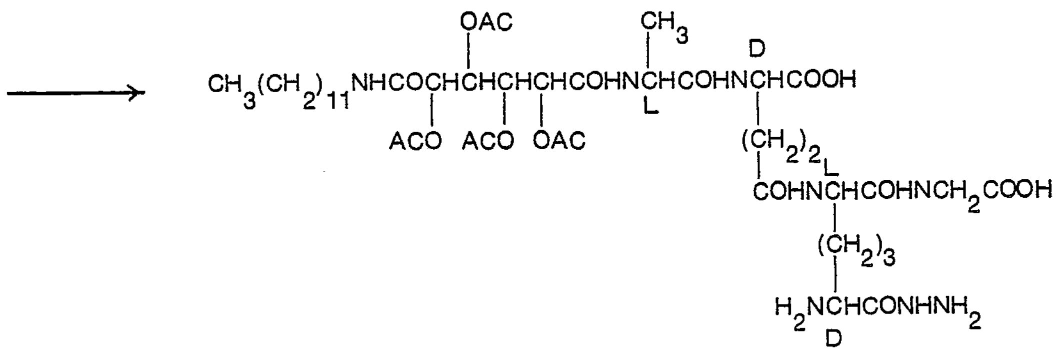

(II)

1-N-lauryl-2,3,4,5-O-tetraacetyl-D-glucaramoyl-L-Ala-γ-D-Glu(α-OH)-(L)-mesoDAP-(D)-$NHNH_2$-(L)-GlyOH (II) was prepared in a similar manner to the Example 1, step 2.

IR (Nujol): 3350, 1760, 1660, 1540 $cm^{-1}$

NMR ($CD_3OD$): δ 0.90 (3H, m), 1.0—2.4 (33H, m), 2.02 (6H, s), 2.13 (3H, s), 2.20 (3H, s), 2.9—3.3 (2H, m), 3.80 (2H, s).

(3) Step 3

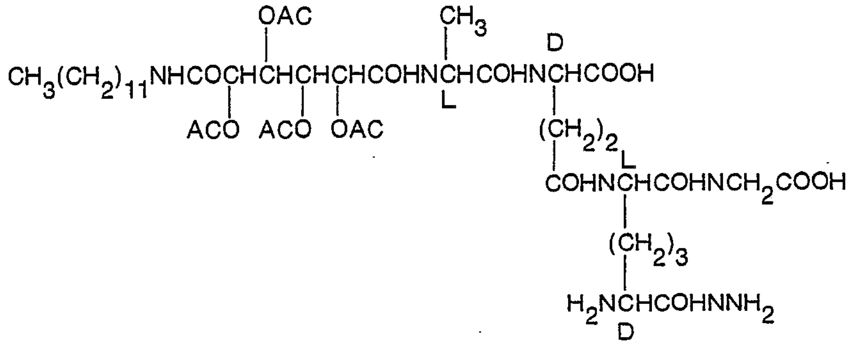

(I)

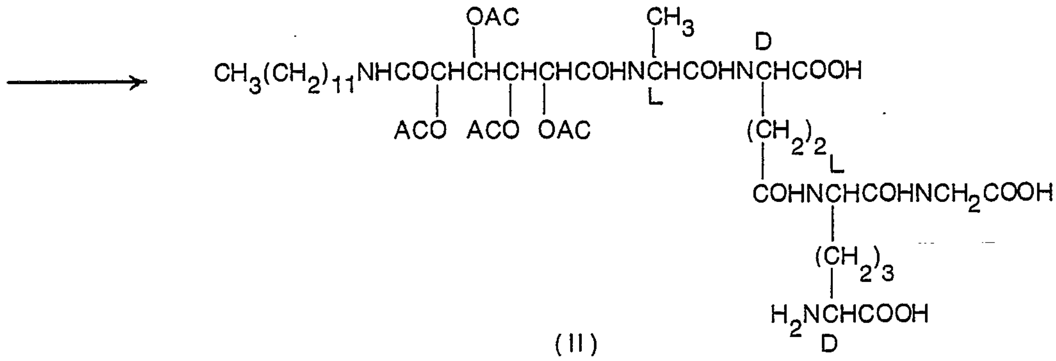

(II)

1-N-Lauryl-2,3,4,5-O-tetraacetyl-D-glucaramoyl-L-Ala-$\gamma$-D-Glu($\alpha$-OH)-(L)-mesoDAP-(L)-GlyOH (II) was prepared in a similar manner to the Example 14, step 3.

IR (Nujol): 3350, 1760, 1660, 1540 $cm^{-1}$

NMR ($D_2O$): $\delta$ 0.80 (3H, m), 1.0—2.5 (33H, m), 2.08 (6H, s), 2.16 (3H, s), 2.20 (3H, s), 3.10 (2H, m), 3.78 (2H, s).

(4) Step 4

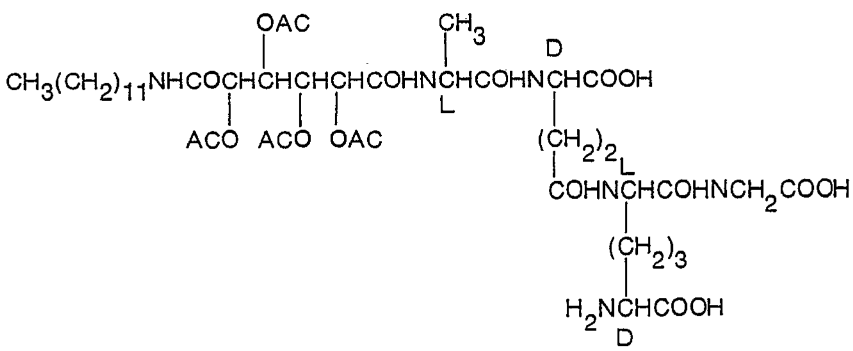

(I)

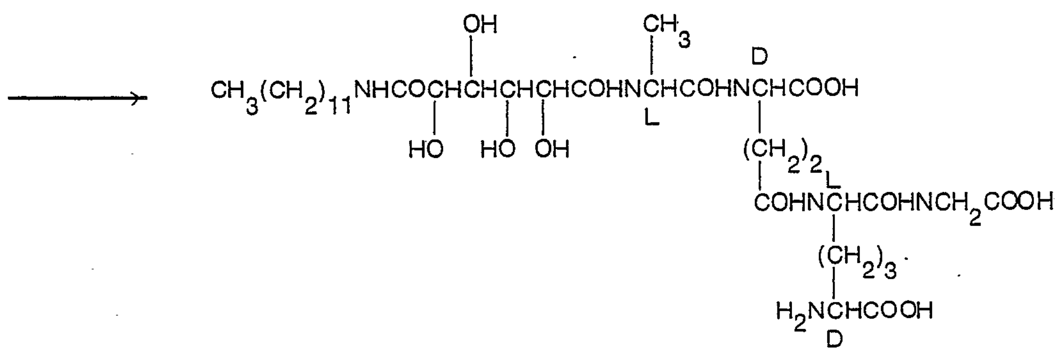

(II)

1-N-Lauryl-D-glucaramoyl-L-Ala-$\gamma$-D-Glu($\alpha$-OH)-(L)-mesoDAP-(L)-GlyOH (II) was prepared in a similar manner to the Example 4, step 4.

IR (Nujol): 3300, 1720, 1640, 1540 $cm^{-1}$

NMR ($D_2O$): $\delta$ 0.80 (3H, m), 1.0—2.5 (33H, m), 3.20 (2H, m), 3.80 (2H, m).

Example 24

(1) Step 1

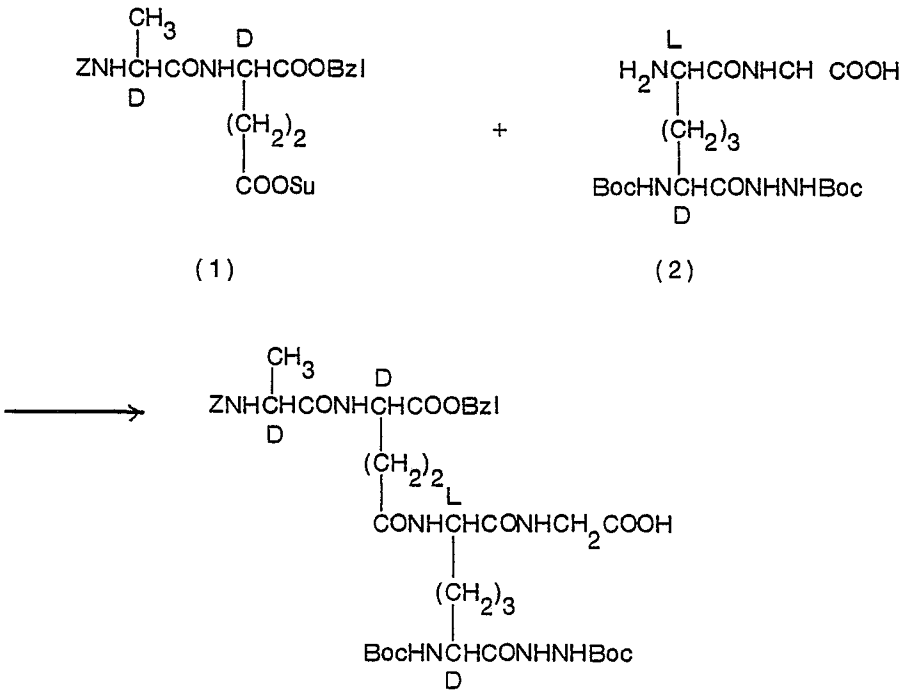

(1) (2)

(3)

Boc-(D)-mesoDAP-(L)-Gly-(D)-NHNHBoc (1)(2.31 g) was dissolved in a mixture of dioxane (60 ml) and water (50 ml) and triethylamine (610 mg) and Z-D-Ala-D-Glu($\alpha$-OBzl)-$\gamma$-OSu (1)(2.70 g) were added. The mixture was left for 18 hours at room temperature and concentrated at a reduced pressure. The mixture of water (30 ml), 1N-hydrochloric acid (10 ml) and ethyl acetate (100 ml) were added to the residue. The organic layer was separated and washed with brine, dried over magnesium sulfate, concentrated in vacuo. The resulting amorphous powder was washed with diisopropyl ether and collected to give a crude product, which was purified by silica gel chromatography (100 ml) eluting with chloroform-methanol (10:1—5:1, v/v). The fractions containing the object compound (3) were collected and concentrated to dryness to yield Z-D-Ala-$\gamma$-D-Glu-$\alpha$-OBzl-(L)-Boc-(D)-mesoDAP-(L)-Gly-(D)-NHNHBoc (3)(1.09 g). mp—125°C (dec.).

$[\alpha]_D$ −4.39 (C=0.21, $CHCl_3$)

IR (Nujol): 3300, 1720, 1670 $cm^{-1}$

(2) Step 2

Compound (3)

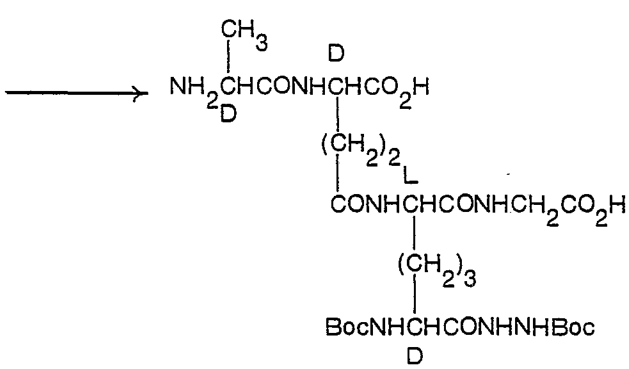

(4)

Z-D-Ala-$\gamma$-D-Glu($\alpha$-OBzl)-(L)-Boc-(D)-mesoDAP-(L)-Gly-(D)-NHNHBoc (3)(980 ml) was dissolved in acetic acid (20 ml) and hydrogenated under an atomospheric pressure of hydrogen over 10% palladium-charcoal (200 mg). After removal of the catalyst, the filtrate was concentrated at a reduced pressure. The resulting syrup was dissolved in water (5 ml) and applied for HP—20 column chromatography (50 ml) eluting with water-methanol (1:1, v/v). The fractions containing the object compound (4) were collected and evaporated to dryness to yield H-D-Ala-$\gamma$-D-Glu-(L)-Boc-(D)-mesoDAP-(L)-Gly-(D)-NHNHBoc (4) (610 mg). mp—159°C (dec.).

$[\alpha]_D$ −17.30 (C=0.1, meOH).

IR (Nujol): 3270, 1670 $cm^{-1}$

NMR ($CD_2OD$): $\delta$ 1.1—2.4 (31H, m), 3.70—4.55 (6H, m)

## Example 25

(1) Step 1

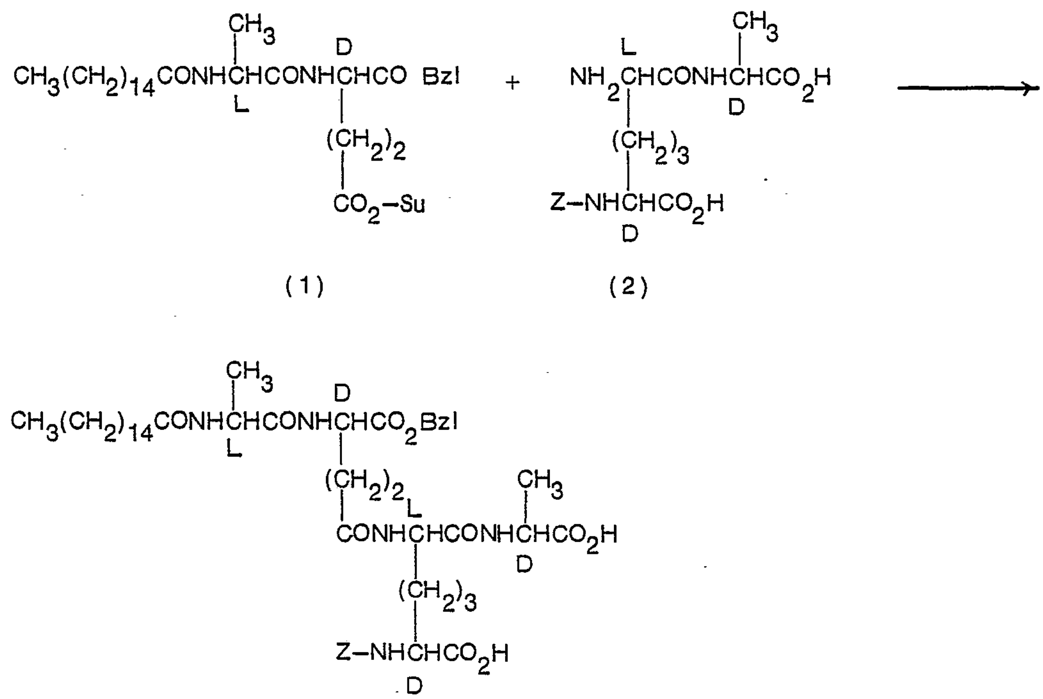

(1) (2)

(3)

To a solution of Z-(D)-meso-DAP-(L)-D-AlaOH (2) (918 mg) in the mixture of methylene chloride (25 ml) and methanol (6 ml) was added triethylamine (760 mg). After the solution turned clear, palmitoyl-L-Ala-D-Glu(OSu)OBzl (1) (1.61 g) was added to the solution. The mixture was kept for 18 hours at the room temperature and concentrated in vacuo. To the residue were added water (30 ml) and 1N-hydrochloric acid (8 ml). The precipitate was collected, washed with water and dried to give a crude product (2.06 g), which was washed with hot diisopropylether to give palmitoyl-L-Ala-$\gamma$-D-Glu-($\alpha$-OBzl)-(L)-Z-(D)-mesoDAP-(L)-D-AlaOH (3)(2.00 g). mp 185—187°C.

$[\alpha]_D$ −15.38°(C=0.21, AcOH)

IR (Nujol): 3300, 1725, 1690, 1630 $cm^{-1}$

NMR ($CDCl_3$-$CD_3OD$): $\alpha$ 0.87 (3H, m), 1.0—2.4 (44H, m), 4.1—4.6 (5H, m) 5.06 (2H, s)

(2) Step 2

Compound (3)

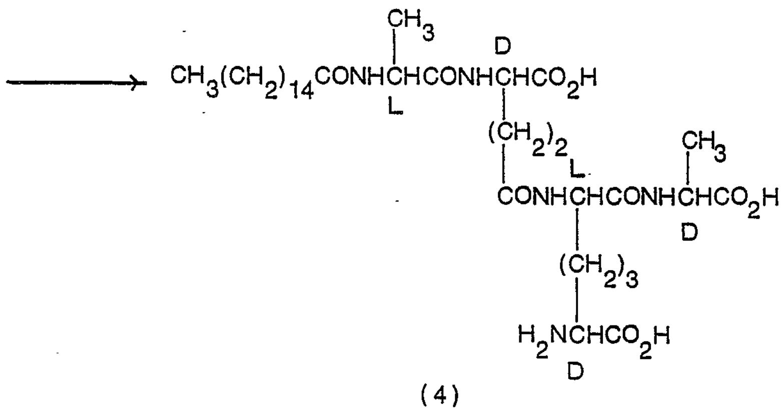

(4)

Palmitoyl-L-Ala-γ-D-Glu(α-OBzl)-(L)-Z-(D)-mesoDAP-(L)-D-Ala-OH (3)(1.86 g) was dissolved in acetic acid (40 ml) and hydrogenated under an atmospheric pressure of hydrogen over 10% palladium-charcoal (1.0 g). After removal of the catalyst, acetic acid was evaporated off in vacuo. To the residue was added diisopropylether and the crystalline mass was collected to give palmitoyl-L-Ala-γ-D-Glu-(L)-mesoDAP-(L)-D-AlaOH (4)(1.30 g). mp 185—190°C.

$[\alpha]_D - 14.07°$ (C=0.21, AcOH)

IR (Nujol): 3320, 1730(sh), 1710, 1625 $cm^{-1}$

NMR ($NaOD—D_2O$, δ): 0.87 (3H, m), 1.05—2.60 (44H, m), 3.91—4.54 (5H, m)

Example 26

(1) Step 1

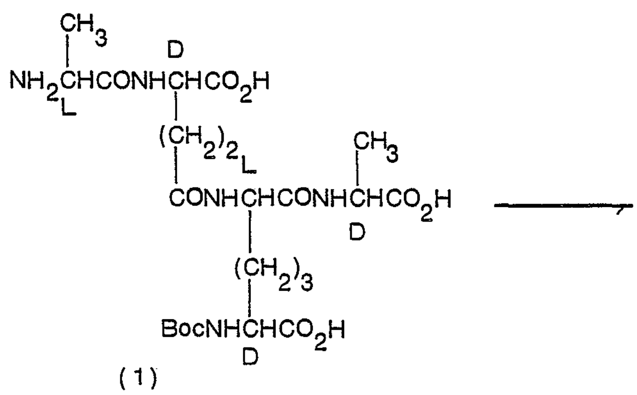

(1)

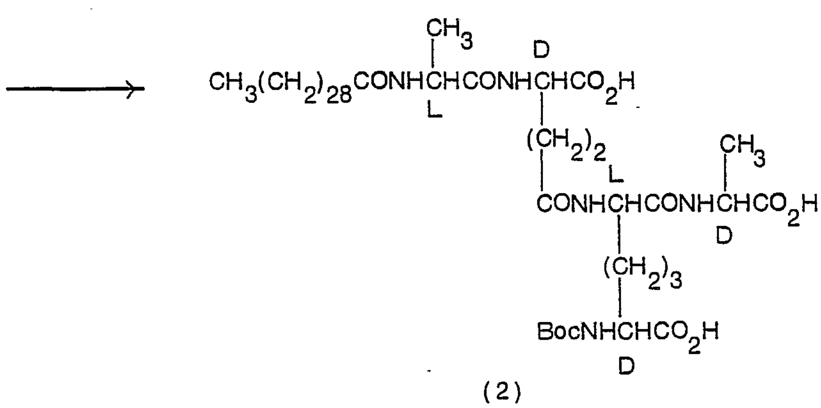

(2)

To the solution of H-L-Ala-γ-D-Glu(α-OH)-(L)-Boc-(D)-mesoDAP-(L)-Ala-OH (1)(528 mg) in a mixture of methylene chloride (20 ml) and methanol (6 ml) were added triethylamine (314 mg) and triacontanoic

acid-N-hydroxysuccinimide ester (518 mg). After stirring for 16 hours at room temperature, the reaction mixture was concentrated in vacuo and the mixture of water (10 ml) and 1N-hydrochloric acid (4 ml) was added to the residue. The precipitates were collected, washed with water and dried to give a crude product, which was washed with hot diisopropylether to give triacontanoyl-L-Ala-$\gamma$-D-Glu-($\alpha$-OH)-(L)-Boc(D)-mesoDAP-(L)-D-AlaOH (2) (610 mg).

mp 157°C (dec.).

$[\alpha]_D$ − 14.67° (C=0.20, AcOH).

IR (Nujol): 3280, 1720, 1625 $cm^{-1}$

NMR ($CDCl_2$—$CD_3OD$, $\delta$): 0.92 (3H, m), 1.1—2.1 (77H, m), 2.15—2.50 (4H, m).

(2) Step 2

Compound (2) 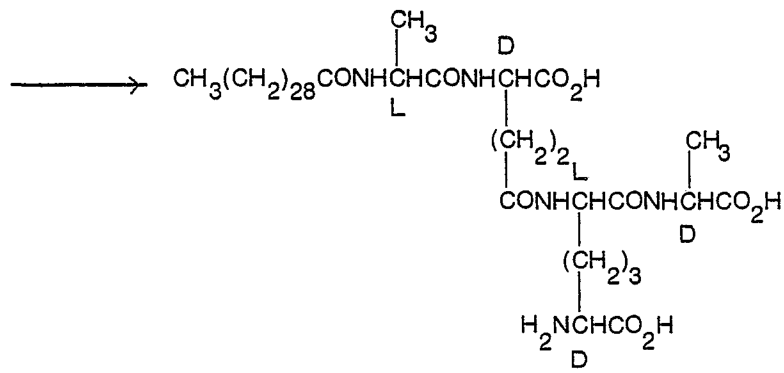

(3)

To the suspension of triacontanoyl-L-Ala-$\gamma$-D-Glu-($\alpha$-OH)-(L)-Boc-(D)-mesoDAP-(L)-D-Ala-OH (2) (500 mg) in acetic acid (20 ml) was added acetic acid saturated with hydrogen chloride (10 ml) and the mixture was stirred on a steam bath for 5 minutes and cooled to room temperature and kept for 1.5 hours at the same temperature. The reaction mixture was concentrated in vacuo and the residue was washed with water and the precipitates were collected and dried to give triacontanoyl-L-Ala-$\gamma$-D-Glu($\alpha$-OH)-(L)-mesoDAP-(L)-D-AlaOH (412 mg). mp—185°C (dec.).

IR (Nujol): 3300, 1720, 1625 $cm^{-1}$

NMR ($CF_3CO_2H$, $\delta$): 0.90 (3H, m).

Example 27

(1) Step 1

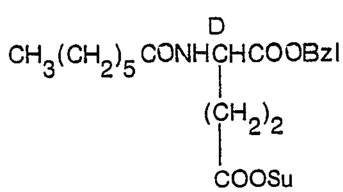

(1)

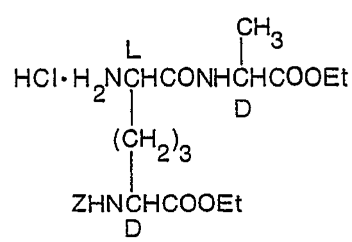

(2)

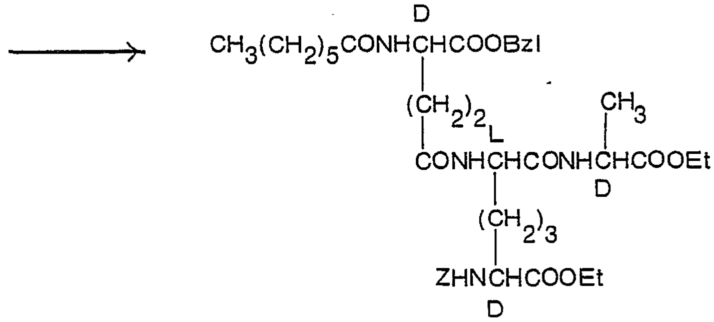

(3)

n-Heptanoyl-γ-D-Glu(α-OBzl)-(L)-Z-(D)-mesoDAP-(L)-D-AlaOEt-(D)OEt (3) was prepared substantially in the same manner as step 1 of Example 25.

mp 141—142°C.

$[\alpha]_D = -10.31°$ (C=0.22, $CHCl_3$)

NMR (($DMSO-d_6$), δ: 0.85 (3H, m), 1.05—2.40 (29H, m), 3.93—4.70 (8H, m), 5.06 (2H, s), 5.11 (2H, s), 5.58 (1H, d, J=8Hz), 6.60 (2H, d, J=8Hz), 7.20 (1H, d, J=8Hz), 7.31 (10H, s).

(2) Step 2

Compound (3)

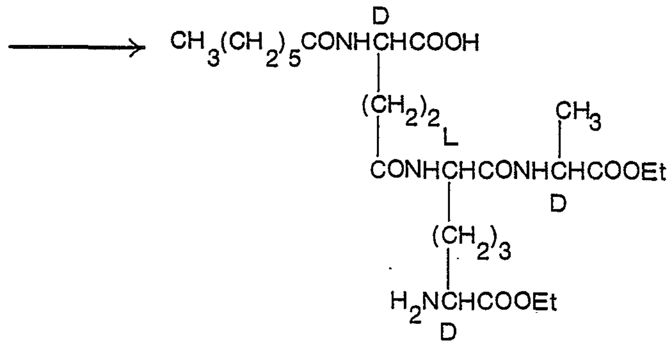

(4)

n-Heptanoyl-γ-D-Glu(α-OH)-(L)-mesoDAP-(L)-D-AlaOEt-(D)-OEt (4) was prepared substantially in the same manner as step 2 of Example 25, mp 179—182°C.

$[\alpha]_D = -13.85°$ (C=0.29, AcOH)

NMR (($DMSO-d_6$) :0.87 (3H, m), 1.02—2.33 (27H, m), 3.40 (1H, m).

Example 28

(1) Step 1

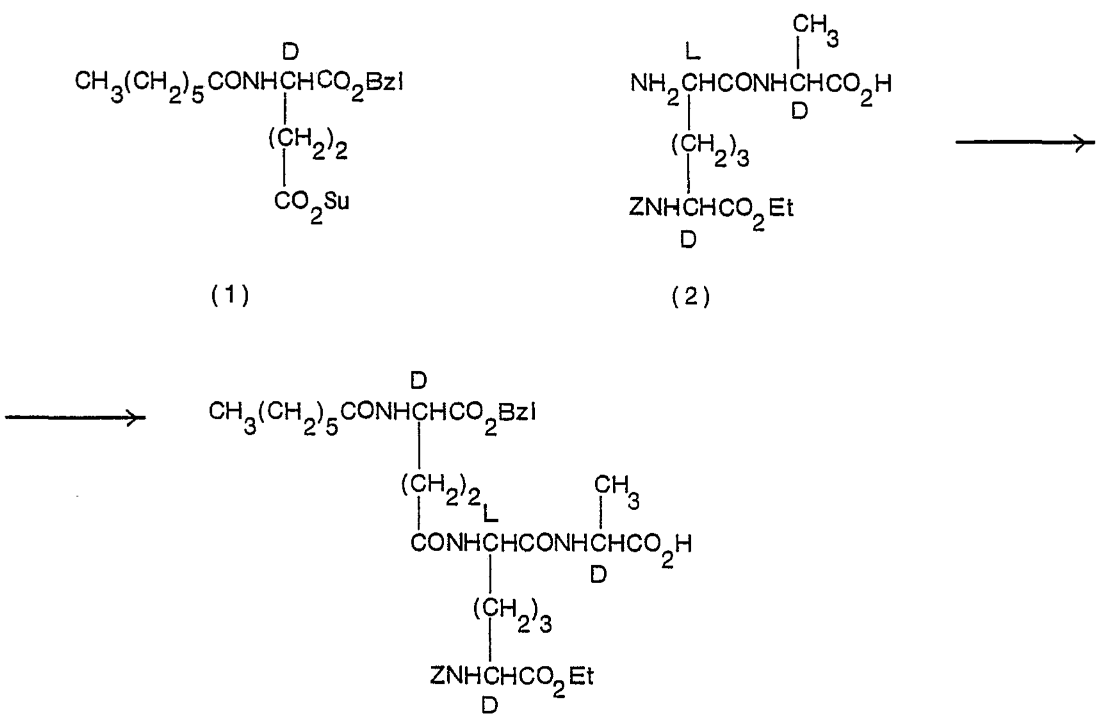

(1)

(2)

(3)

Heptanoyl-γ-D-Glu(α-OBzl)-(L)-Z-(D)-mesoDAP-(D)-OEt-(L)-D-AlaOH (3) was prepared substantially in the same manner as step 1 of Example 25, mp. 94—102°C.

$[\alpha]_D$ −1.06 (C=0.21, $CHCl_3$)

IR (Nujol): 3280, 1735, 1685, 1630 $cm^{-1}$

NMR ($CDCl_3$, δ): 0.86 (3H, m), 1.05—2.55 (26H, m), 3.9—4.9 (6H, m), 5.10 (2H, s), 5.16 (2H, s), 7.32 (10H, s).

(2) Step 2

Compound (3)

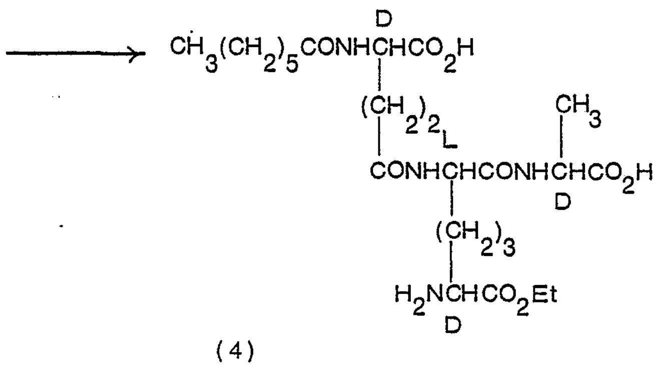

(4)

Heptanoyl-γ-D-Glu(α-OBzl)-(L)-Z-(D)-mesoDAP-(D)-OEt-(L)-D-AlaOH (3) (930 mg) was dissolved in acetic acid (20 ml) and hydrogenated under an atmospheric pressure of hydrogen over 10% palladium-charcoal (200 mg). After removal of the catalyst, acetic acid was evaporated in vacuo. The residue was purified by column chromatography of HP—20 (50 ml) with ethanol-H20 (3:7, v/v) as an eluent to give heptanoyl-γ-D-Glu(α-OH)-(L)-mesoDAP-(D)-OEt-(L)-D-AlaOH (4) (490 mg). mp 218°C.

$[\alpha]_D$ −14.63° (C=0.2, $H_2O$)

IR (Nujol): 3280, 1740, 1635 $cm^{-1}$

NMR ($D_2O$, δ): 0.90 (3H, m), 1.2—2.6 (26H, m), 4.1—4.55 (6H, m).

**Claims for the Contracting States: BE CH DE FR GB IT LI LU NL SE**

1. A compound of the formula or its pharmaceutically acceptable salt:

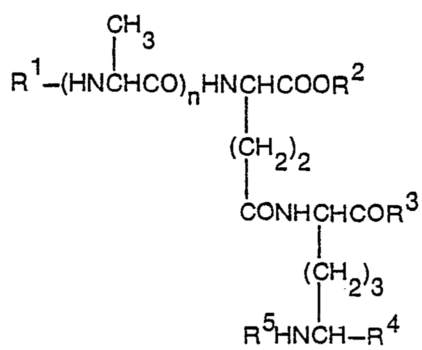

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is stearoyl, n is an integer of 0, $R^2$ is benzyl, $R^3$ is methoxy, $R^4$ is methoxycarbonyl and $R^5$ is hydrogen; or

$R^1$ is heptanoyl, n is an integer of 0, $R^2$ is hydrogen, $R^3$ is 1-carboxyethylamino, $R^4$ is carboxy and $R^5$ is t-butoxycarbonyl; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is octanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2-keto-L-gulonoyl, n is an integer of 0, $R^2$ is benzyl, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is 2-keto-L-gulonoyl, n is an integer of 0, $R^2$ is benzyl, $R^3$ is 1-carboxyethylamino, $R^4$ is carboxy and $R^5$ is hydrogen; or

$R^1$ is heptanoyl, n is an integer of 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl.

2. A compound of the formula or its pharmaceutically acceptable salt:

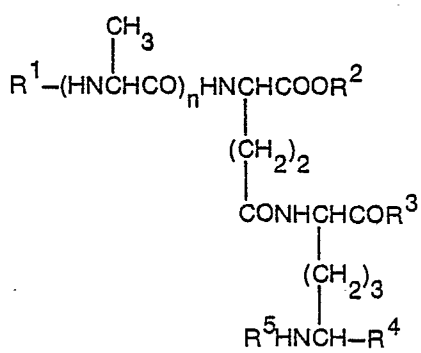

wherein

$R^1$ is n-docosanoyl, n is an integer of 1, $R^2$ is benzyl, $R^3$ is 1-carboxyethylamino, $R^4$ is carboxy and $R^5$ is t-butoxycarbonyl; or

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-tetracosanoyl, n is an integer of 1, $R^2$ is benzyl, $R^3$ is 1-carboxyethylamino, $R^4$ is carboxy and $R^5$ is t-butoxycarbonyl; or

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is 2-acetoxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 2-acetoxypropionyl, n is an integer of 1, $R^2$ is hydrogen, $R^3$ is carboxymethylamino, $R^4$ is methoxycarbonyl and $R^5$ is t-butoxycarbonyl; or

$R^1$ is 2-hydroxypropionyl, n is an integer of 1, $R^2$ is benzyl, $R^3$ is methoxycarbonylmethylamino, $R^4$ is carboxy and $R^5$ is t-butoxycarbonyl; or

$R^1$ is 2-hydroxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 1-N-n-hexyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is 1-N-benzyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4-5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is triacontanoyl, n is an integer of 1, $R^2$ is hydrogen, $R^3$ is 1-carboxyethylamino, $R^4$ is carboxy and $R^5$ is t-butoxycarbonyl.

3. A compound of the formula or its pharmaceutically acceptable salt:

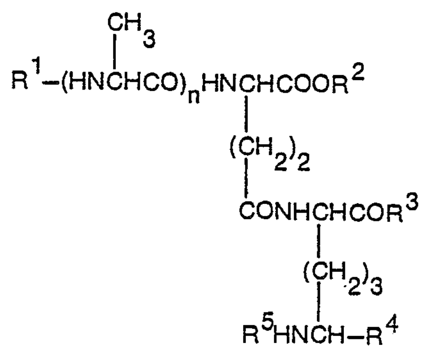

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$ is 1-carboxyethylamino, $R^4$ is carboxy and $R^5$ is hydrogen; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is hydrogen, $R^3$ is 1-carboxyethylamino, $R^4$ is ethoxycarbonyl and $R^5$ is hydrogen.

4. A compound of the formula or its pharmaceutically acceptable salt:

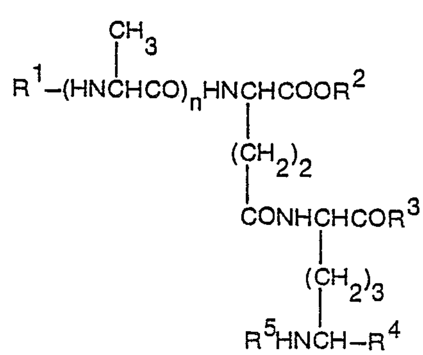

wherein

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is methoxycarbonylmethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is hydrogen.

5. A compound of the formula or its pharmaceutically acceptable salt:

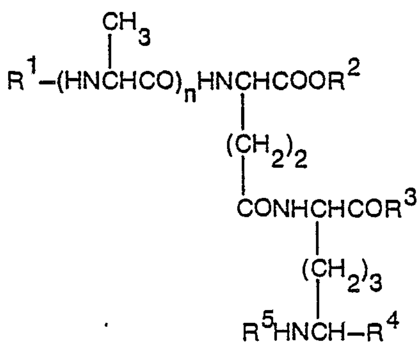

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen.

6. A process for preparation of a compound of the formula or its pharmaceutically acceptable salt:

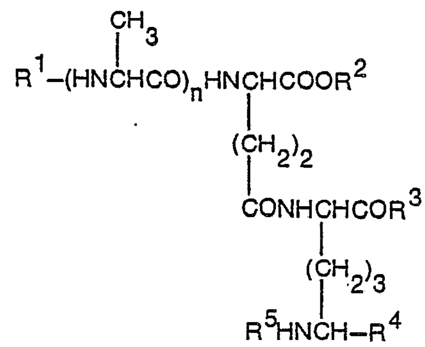

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is n-docosanoyl, n is an integer of 1, $R^2$ is benzyl, $R^3$ is 1-carboxyethylamino, $R^4$ is carboxy and $R^5$ is t-butoxycarbonyl; or

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-tetracosanoyl, n is an integer of 1, $R^2$ is benzyl, $R^3$ is 1-carboxyethylamino, $R^4$ is carboxy and $R^5$ is t-butoxycarbonyl; or

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is 2-acetoxypropionyl, n is an integer of 1, $R^2$ is benzyl, $R^3$ is carboxymethylamino, $R^4$ is methoxycarbonyl and $R^5$ is t-butoxycarbonyl; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is octanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is 2-hydroxypropionyl, n is an integer of 1, $R^2$ is benzyl, $R^3$ is methoxycarbonylmethylamino, $R^4$ is carboxy and $R^5$ is t-butoxycarbonyl; or

$R^1$ is 2-hydroxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 1-N-n-hexyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is methoxycarbonylmethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is 1-N-benzyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is triacontanoyl, n is an integer of 1, $R^2$ is hydrogen, $R^3$ is 1-carboxyethylamino, $R^4$ is carboxy and $R^5$ is t-butoxycarbonyl; or

$R^1$ is heptanoyl, n is an integer of 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl;

which comprises,

reacting a compound of the formula or its salt:

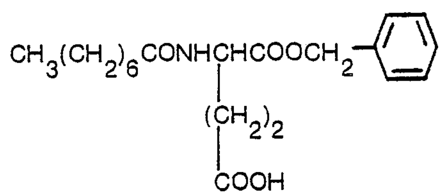

with a compound of the formula or its salt:

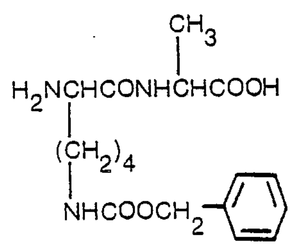

to give a compound of the formula or its salt:

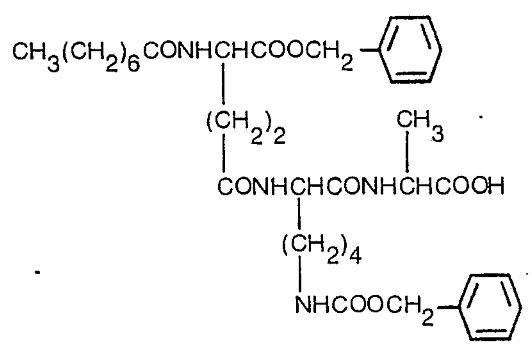

or

reacting of a compound of the formula or its salt:

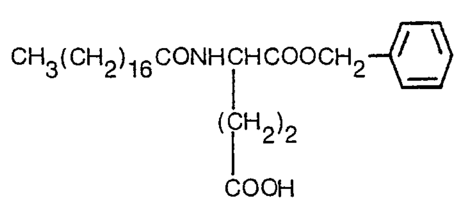

with a compound of the formula or its salt:

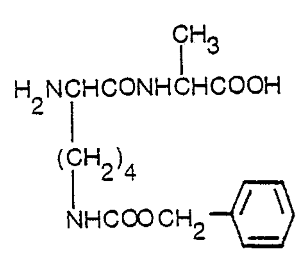

to give a compound of the formula or its salt:

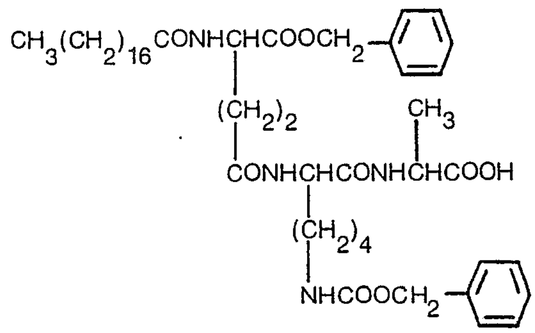

or

reacting a compound of the formula or its salt:

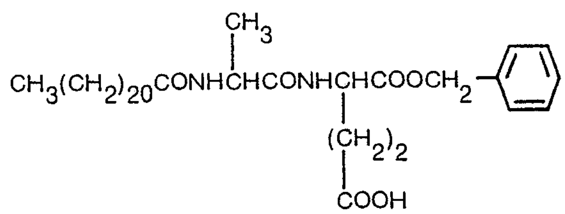

with a compound of the formula or its salt:

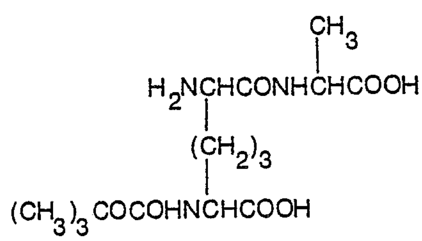

to give a compound of the formula or its salt:

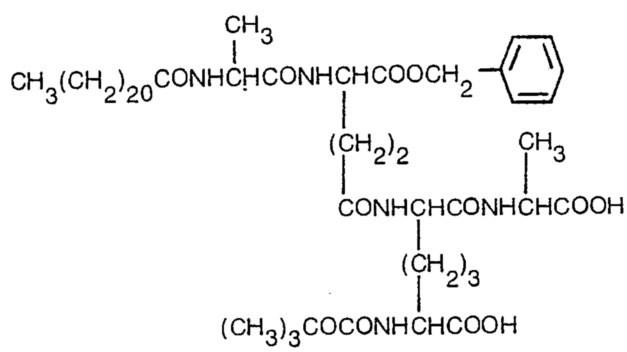

or

reacting a compound of the formula or its salts:

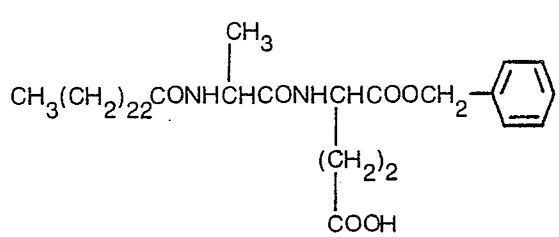

with a compound of the formula or its salt:

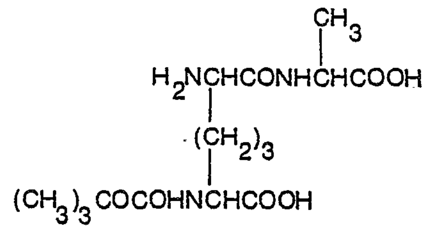

to give a compound of the formula or its salt:

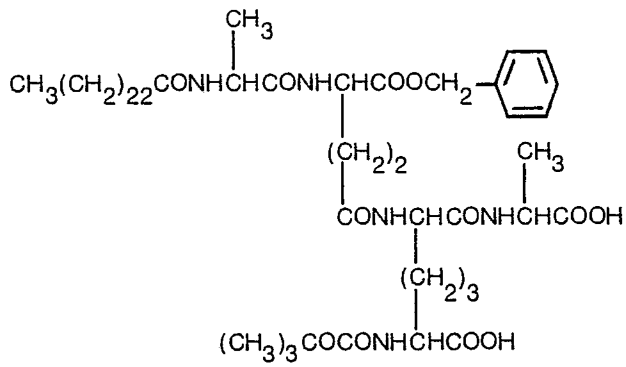

or

reacting a compound of the formula or its salt:

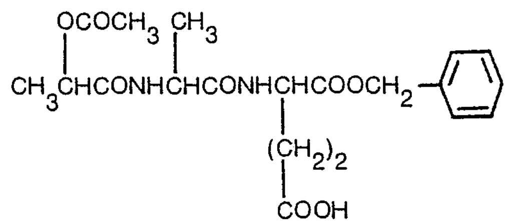

with a compound of the formula or its salt:

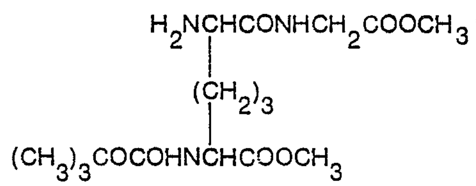

to give a compound of the formula or its salt:

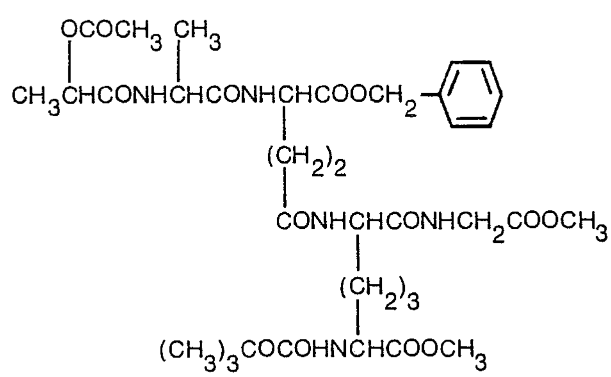

or

reacting a compound of the formula or its salt:

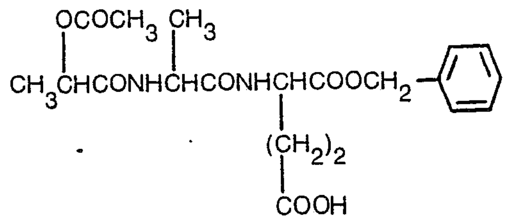

with a compound of the formula or its salt:

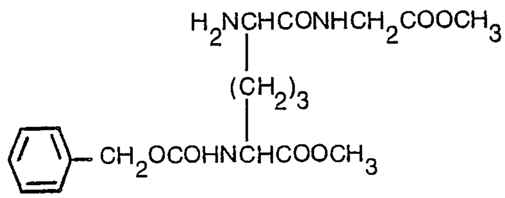

to give a compound of the formula or its salt:

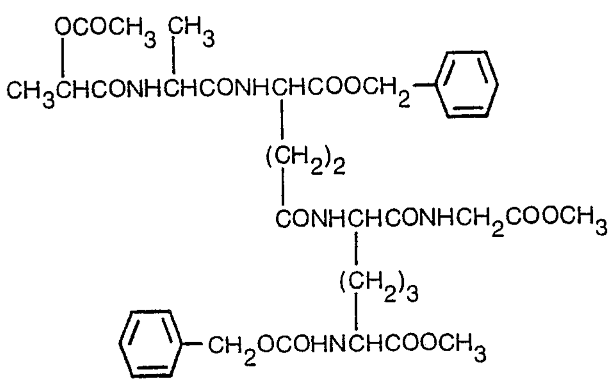

or

reacting a compound of the formula or its salt:

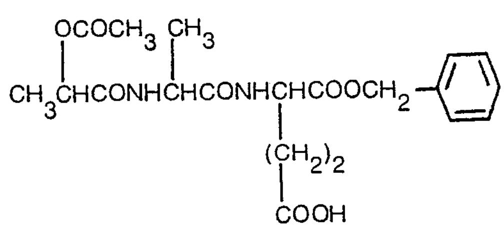

with a compound of the formula or its salt:

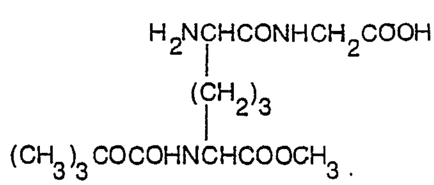

to give a compound of the formula or its salt:

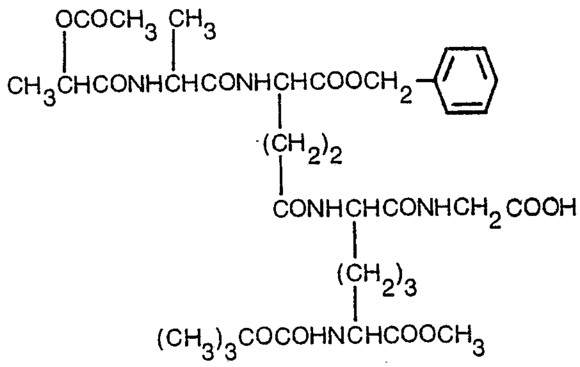

or

reacting a compound of the formula or its salt:

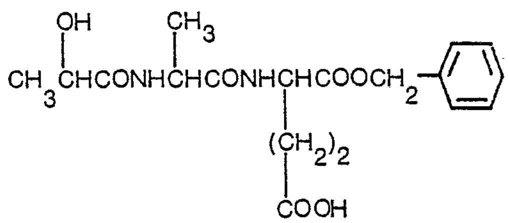

with a compound of the formula or its salt:

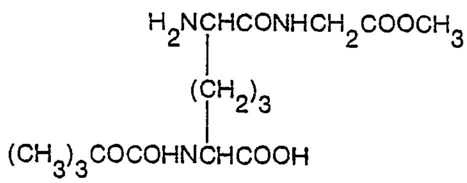

to give a compound of the formula or its salt:

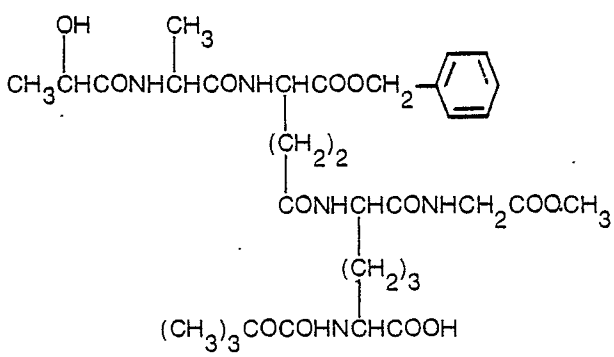

or

reacting a compound of the formula or its salt:

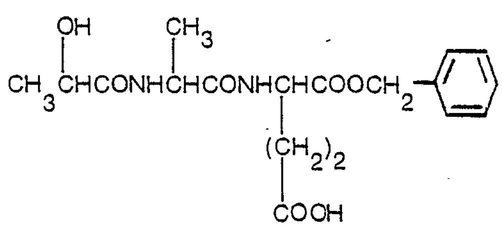

with a compound of the formula or its salt:

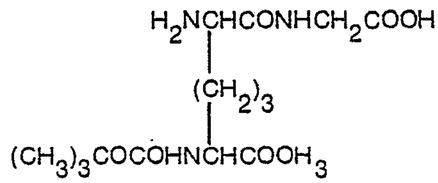

to give a compound of the formula or its salt:

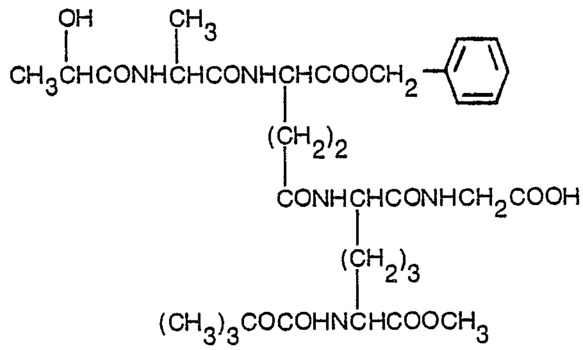

or

reacting a compound of the formula or its salt:

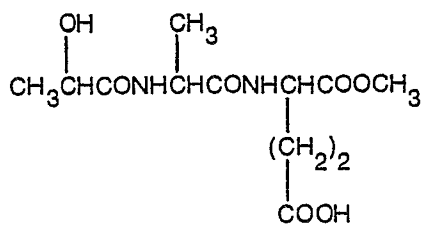

with a compound of the formula or its salt:

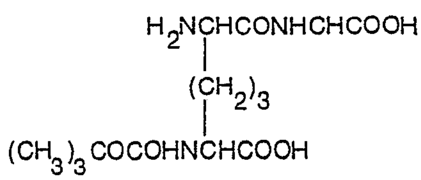

to give a compound of the formula or its salt:

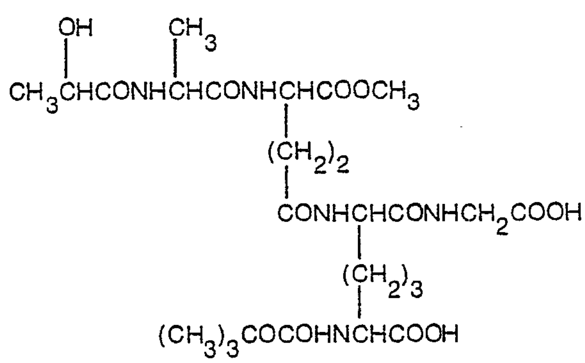

or

reacting a compound of the formula or its salt:

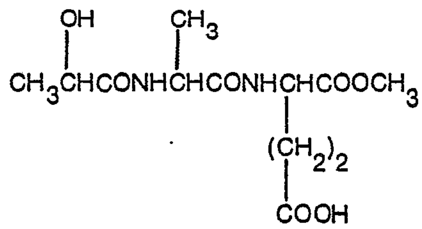

with a compound of the formula or its salt:

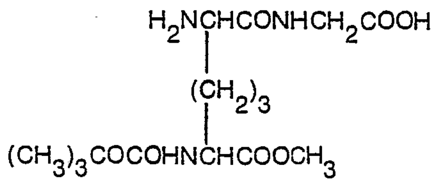

to give a compound of the formula or its salt:

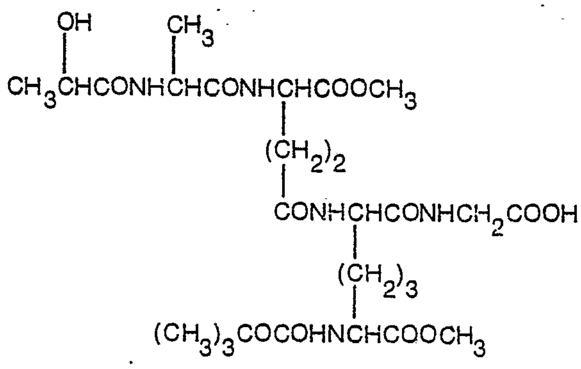

or

reacting a compound of the formula or its salt:

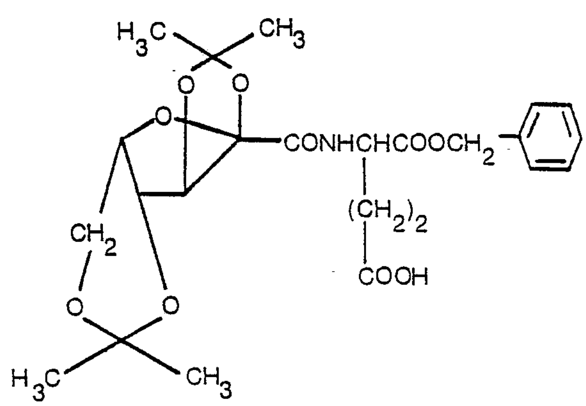

with a compound of the formula or its salt:

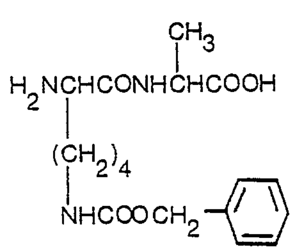

to give a compound of the formula or its salt:

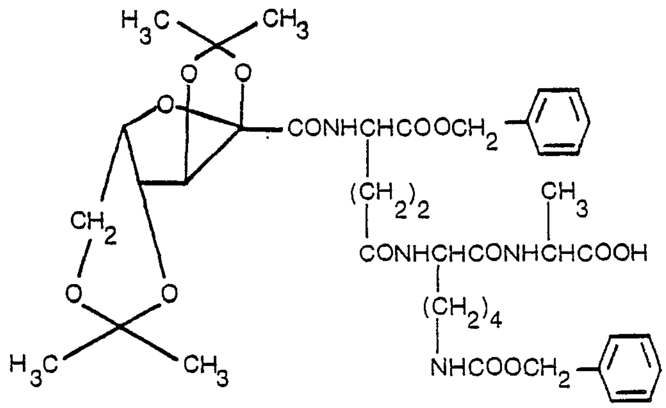

or

reacting a compound of the formula or its salt:

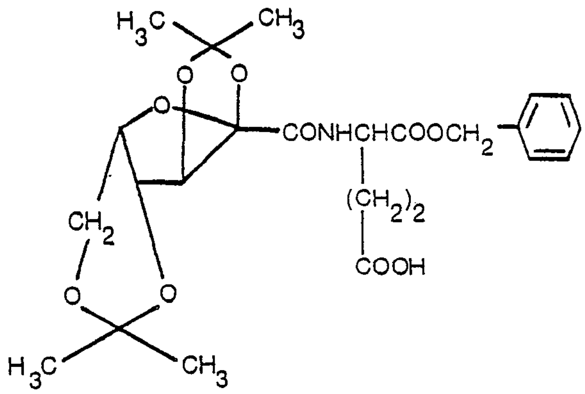

with a compound of the formula or its salt:

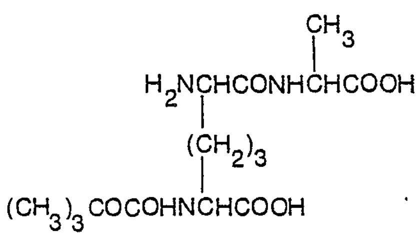

to give a compound of the formula or its salt:

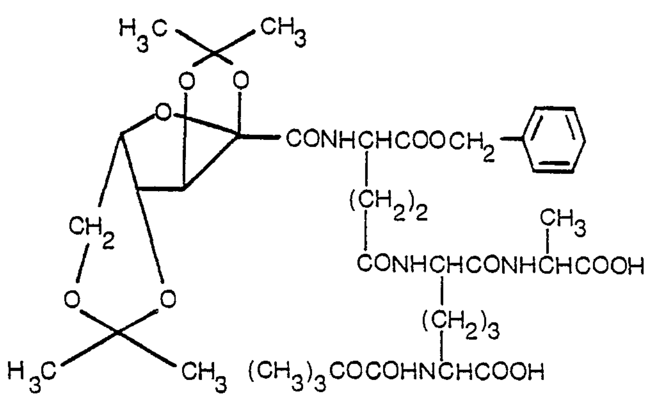

or

reacting a compound of the formula or its salt:

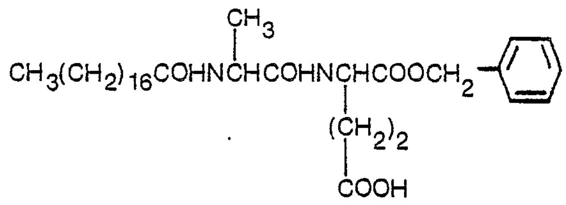

with a compound of the formula or its salt:

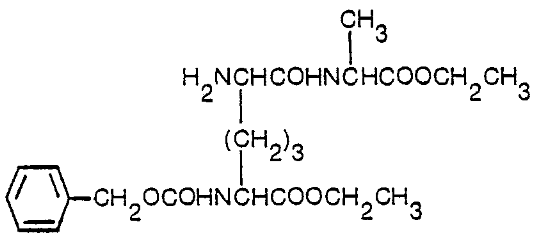

to give a compound of the formula or its salt:

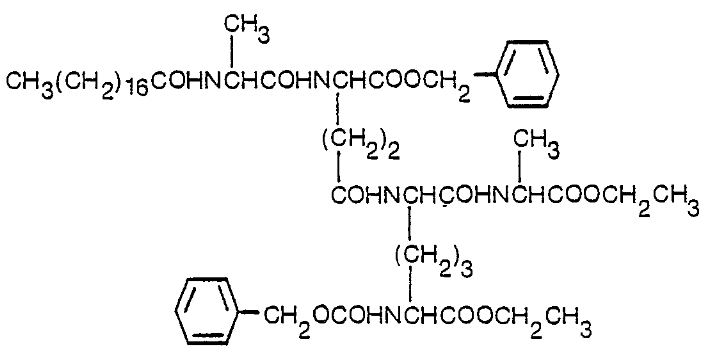

or

reacting a compound of the formula or its salt:

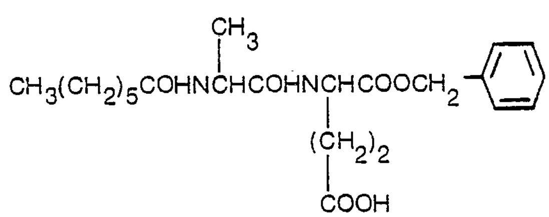

with a compound of the formula or its salt:

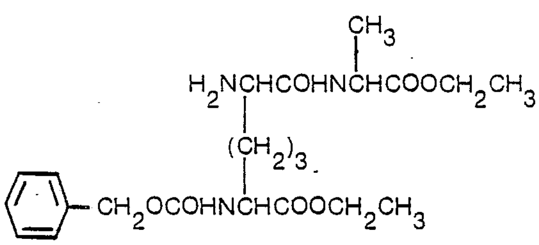

to give a compound of the formula or its salt:

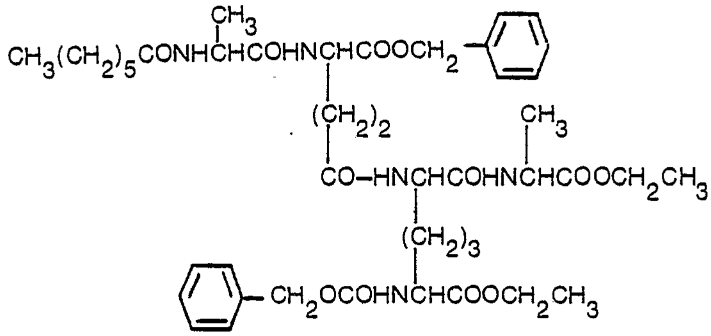

or

reacting a compound of the formula or its salt:

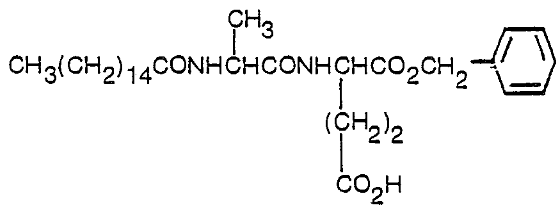

with a compound of the formula or its salt:

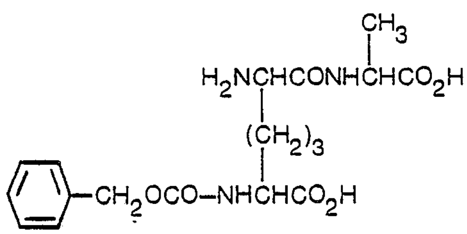

to give a compound of the formula or its salt:

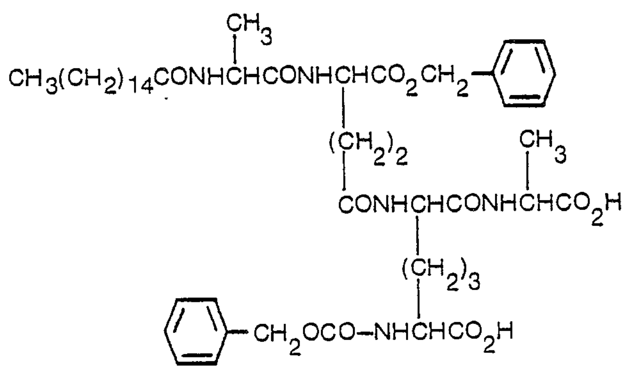

or

reacting a compound of the formula or its salt:

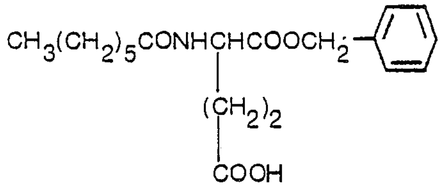

with a compound of the formula or its salt:

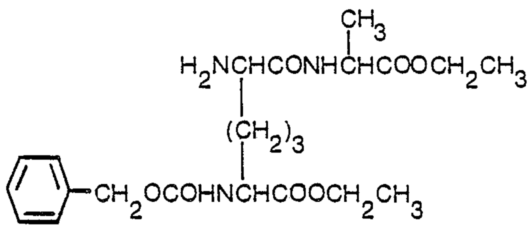

to give a compound of the formula or its salt:

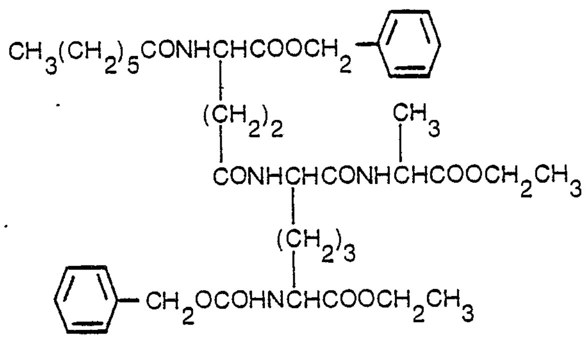

or

reacting a compound of the formula or its salt:

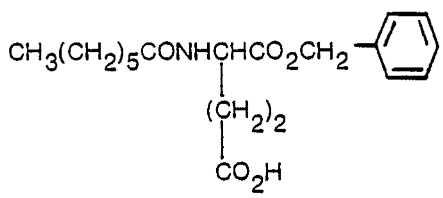

with a compound of the formula or its salt:

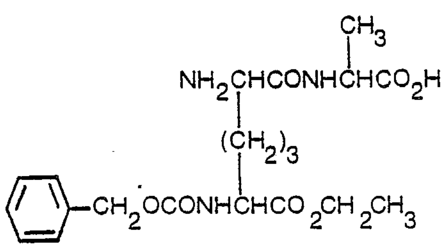

to give a compound of the formula or its salt:

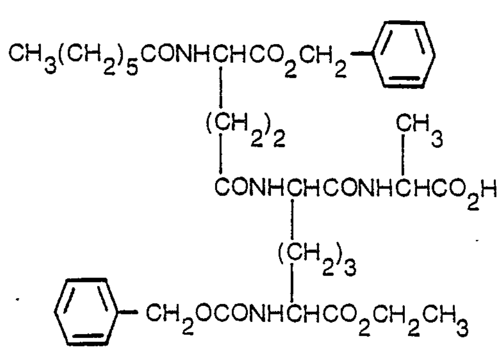

or

reacting a compound of the formula or its salt:

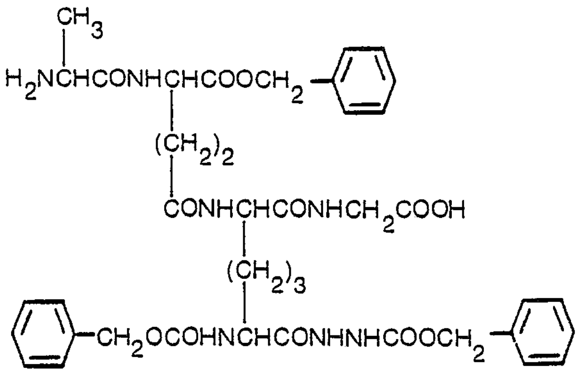

with 1 - N - n - hexyl - 2,3,4,5 - O - tetraacetyl - D - glucaramic acid, 1 - N - benzyl - 2,3,4,5 - O - tetraacetyl - D - glucaramic acid, 1 - N - lauryl - 2,3,4,5 - O - tetraacetyl - D - glucaramic acid or their reacting derivatives to give a compound of the formula or its salt:

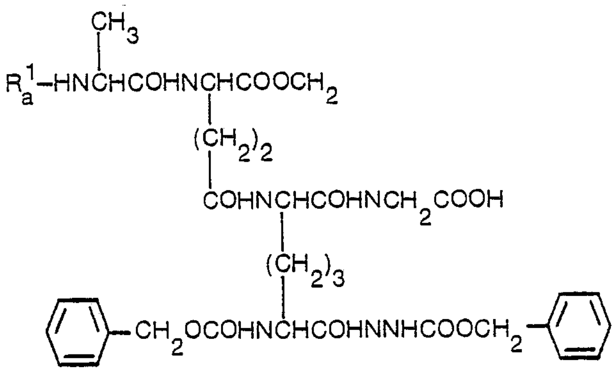

wherein $R_a^1$ is 1 - N - n - hexyl - 2,3,4,5 - O - tetraacetyl - D - glucaramoyl, 1 - N - benzyl - 2,3,4,5 - O - tetraacetyl - D - glucaramoyl or 1 - N - lauryl - 2,3,4,5 - O - tetraacetyl - D - glucaramoyl,

or

reacting a compound of the formula or its salt:

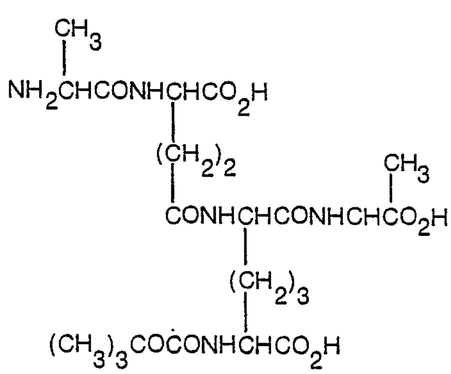

with triacontanoic acid or its reactive derivative to give a compound of the formula or its salt:

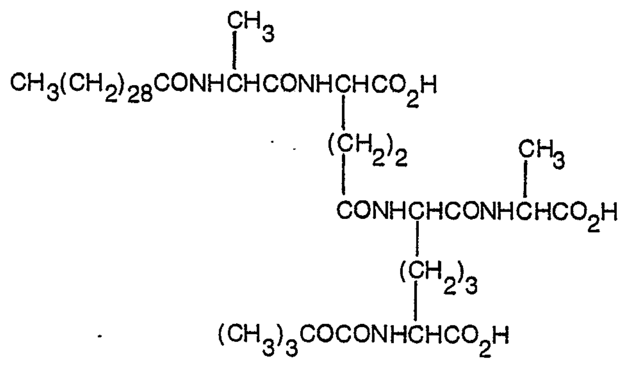

or

reacting a compound of the formula or its salt:

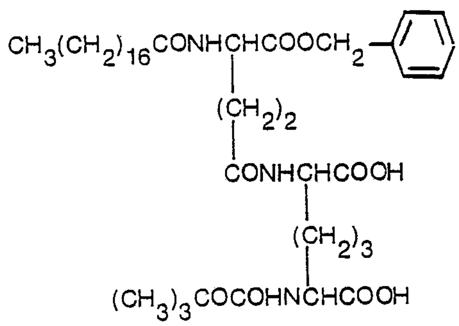

with a methylating agent to give a compound of the formula or its salt:

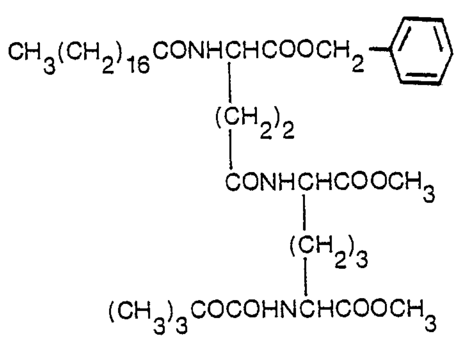

or

reacting a compound of the formula or its salt:

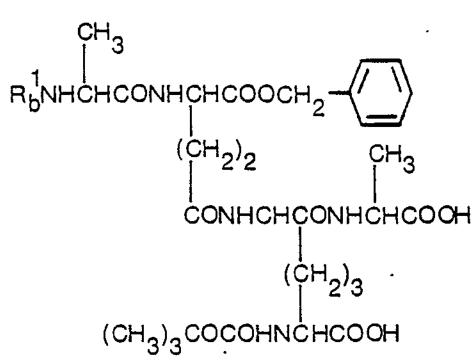

with a methylating agent to give a compound of the formula or its salt:

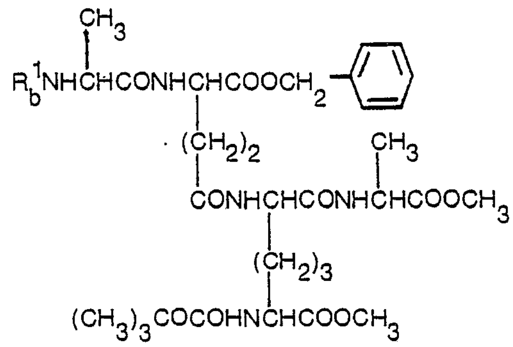

wherein $R_b^1$ is n-docosanoyl or n-tetracosanoyl,

or

reacting a compound of the formula or its salt:

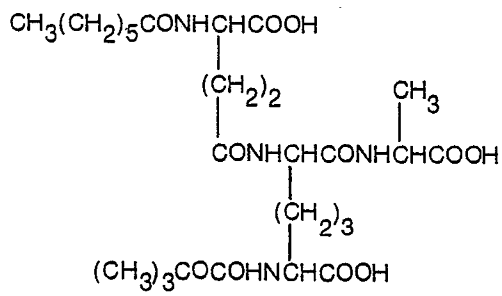

with a methylating agent to give a compound of the formula or its salt:

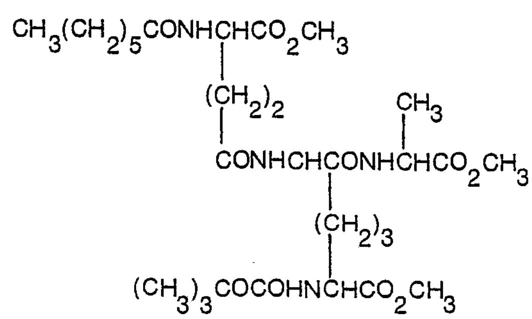

or

reacting a compound of the formula or its salt:

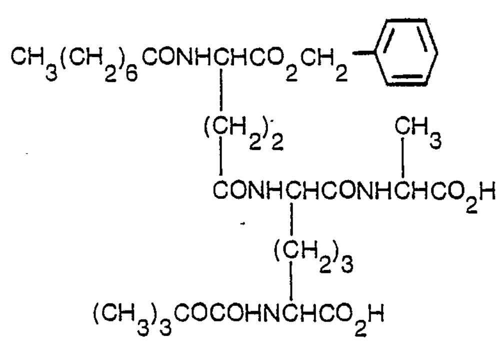

with a methylating agent to give a compound of the formula or its salt:

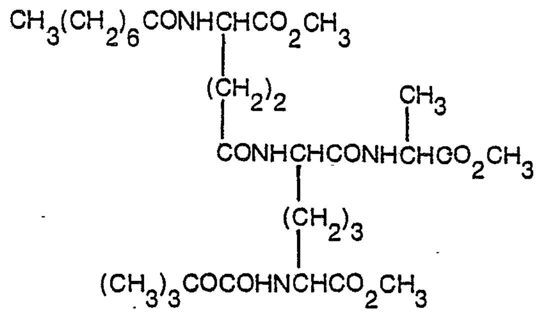

or

reacting a compound of the formula or its salt:

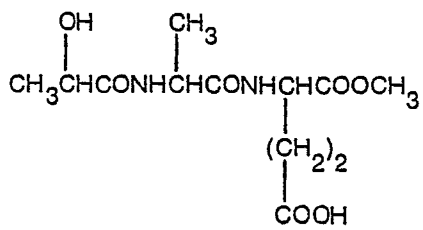

with a compound of the formula or its salt:

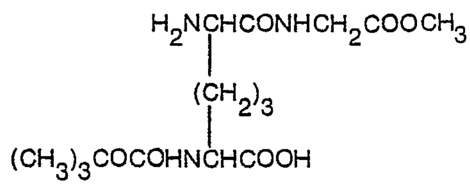

and subjecting the resulting compound to elimination reaction of the protective group to give a compound of the formula or its salt:

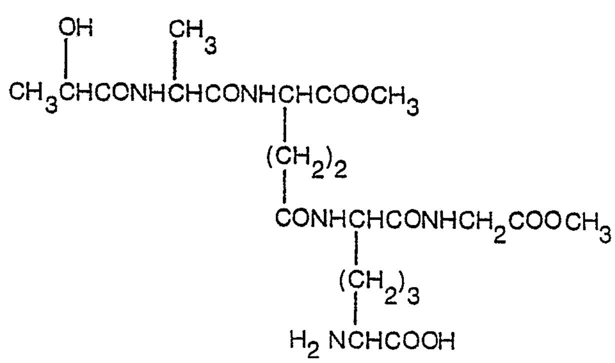

7. A process for preparation of a compound of the formula or its pharmaceutically acceptable salt:

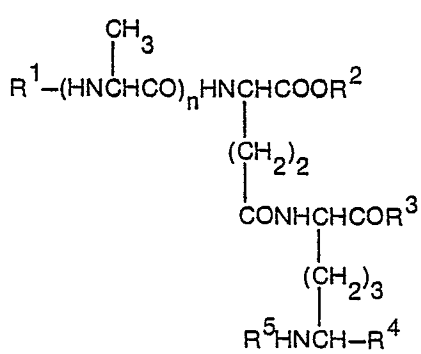

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is benzyl, $R^3$ is methoxy, $R^4$ is methoxycarbonyl and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is octanoyl, n is an integer of 0, $R^2$ is methyl, $R^3$ is 1-methoxycarbonylethylamino, $R^4$ is methoxycarbonyl and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$ is 1-carboxyethylamino, $R^4$ is carboxy and $R^5$ is hydrogen; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is hydrogen, $R^3$ is 1-carboxyethylamino, $R^4$ is ethoxycarbonyl and $R^5$ is hydrogen;

which comprises,

subjecting a compound of the formula or its salt:

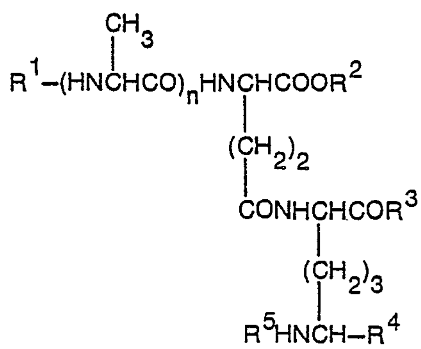

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is stearoyl, n is an integer of 0, $R^2$ is benzyl, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is octanoyl, n is an integer of 0, $R^2$ is methyl, $R^3$ is 1-methoxycarbonylethylamino, $R^4$ is methoxycarbonyl and $R^5$ is butoxycarbonyl; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2-keto-L-gulonoyl, n is an integer of 0, $R^2$ is benzyl, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is 2-keto-L-gulonoyl, n is an integer of 0, $R^2$ is benzyl, $R^3$ is 1-carboxyethylamino, $R^4$ is carboxy and $R^5$ is hydrogen; or

$R^1$ is heptanoyl, n is an integer of 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl,

to elemination reaction of protective group(s) to give a compound of the formula or its salt:

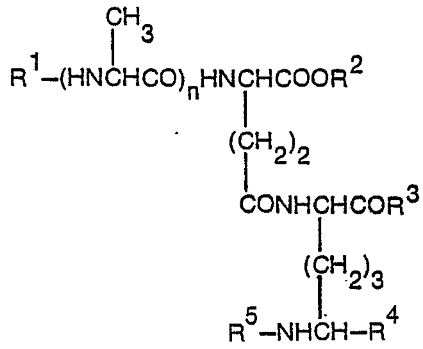

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is benzyl, $R^3$ is methoxy, $R^4$ is methoxycarbonyl and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is octanoyl, n is an integer of 0, $R^2$ is methyl, $R^3$ is 1-methoxycarbonylethylamino, $R^4$ is methoxycarbonyl and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$ is 1-carboxyethylamino, $R^4$ is carboxy and $R^5$ is hydrogen; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is hydrogen, $R^3$ is 1-carboxyethylamino, $R^4$ is ethoxycarbonyl and $R^5$ is hydrogen.

8. A process for preparation of a compound of the formula or its pharmaceutically acceptable salt:

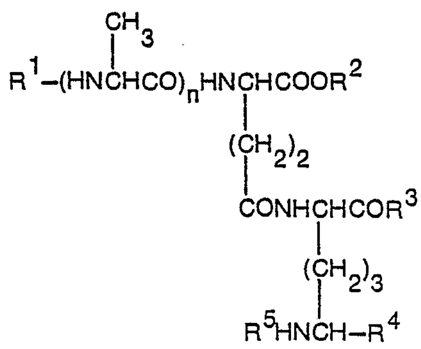

wherein

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-tetracosanoyl, n is an integer of 1, $R^2$ is hydrogen, $R^3$ is 1-carboxyethylamino, $R^4$ is carboxy and $R^5$ is hydrogen; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-n-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is hydrogen;

which comprises,

subjecting a compound of the formula or its salt:

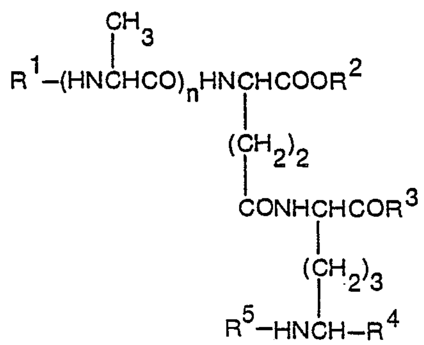

wherein

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-docosanoyl, n is an integer of 1, $R^2$ is benzyl, $R^3$ is 1-carboxyethylamino, $R^4$ is carboxy and $R^5$ is t-butoxycarbonyl; or

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is 2-hydroxypropionyl, n is an integer of 1, $R^2$ is benzyl, $R^3$ is methoxycarbonylmethylamino, $R^4$ is carboxy and $R^5$ is t-butoxycarbonyl; or

$R^1$ is 2-hydroxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 1-N-n-hexyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is 1-N-benzyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is triacontanoyl, n is an integer of 1, $R^2$ is hydrogen, $R^3$ is 1-carboxyethylamino, $R^4$ is carboxy and $R^5$ is t-butoxycarbonyl;

to elemination reaction of protective group(s) to give a compound of the formula or its salt:

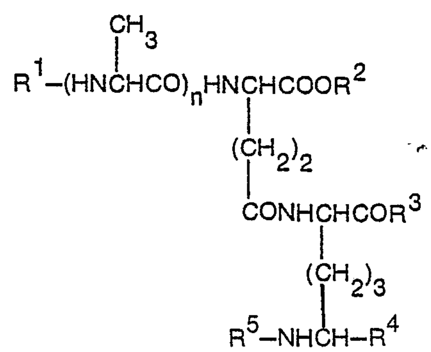

wherein

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-tetracosanoyl, n is an integer of 1, $R^2$ is hydrogen, $R^3$ is 1-carboxyethylamino, $R^4$ is carboxy and $R^5$ is hydrogen; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-n-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is hydrogen.

9. A pharmaceutical composition comprising a compound of claim 3 or 4 or their pharmaceutically acceptable salts in association with a pharmaceutically acceptable substantially nontoxic carrier or excipient.

10. A compound of claims 3 or 4 for use as a medicament.

11. A compound of claims 3 or 4 for use in treating infectious diseases and tumor.

**Claims for the Contracting State: AT**

1. A process for preparation of a compound of the formula or its pharmaceutically acceptable salt:

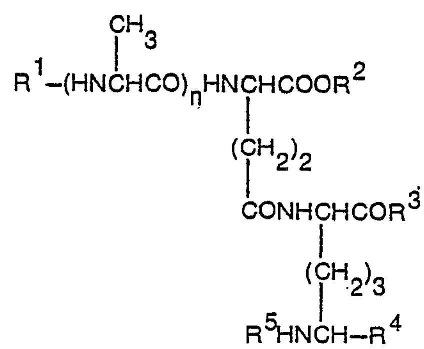

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is n-docosanoyl, n is an integer of 1, $R^2$ is benzyl, $R^3$ is 1-carboxyethylamino, $R^4$ is carboxy and $R^5$ is t-butoxycarbonyl; or

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-tetracosanoyl, n is an integer of 1, $R^2$ is benzyl, $R^3$ is 1-carboxyethylamino, $R^4$ is carboxy and $R^5$ is t-butoxycarbonyl; or

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is t-butoxycarbonyl; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is 2-acetoxypropionyl, n is an integer of 1, $R^2$ is benzyl, $R^3$ is carboxymethylamino, $R^4$ is methoxycarbonyl and $R^5$ is t-butoxycarbonyl; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is octanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is 2-hydroxypropionyl, n is an integer of 1, $R^2$ is benzyl, $R^3$ is methoxycarbonylmethylamino, $R^4$ is carboxy, and $R^5$ is t-butoxycarbonyl; or

$R^1$ is 2-hydroxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 1-N-n-hexyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is methoxycarbonylmethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is 1-N-benzyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is triacontanoyl, n is an integer of 1, $R^2$ is hydrogen, $R^3$ is 1-carboxyethylamino, $R^4$ is carboxy and $R^5$ is t-butoxycarbonyl; or

$R^1$ is heptanoyl, n is an integer of 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl;

which comprises,

reacting a compound of the formula or its salt:

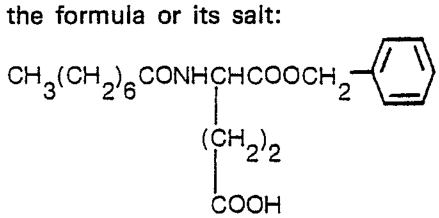

with a compound of the formula or its salt:

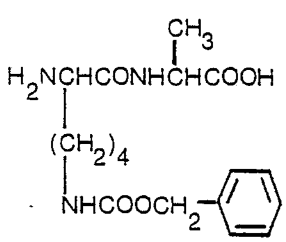

to give a compound of the formula or its salt:

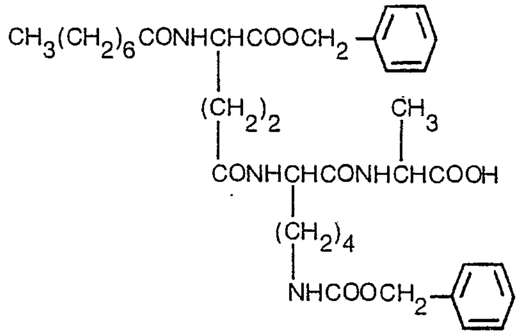

or

reacting of a compound of the formula or its salt:

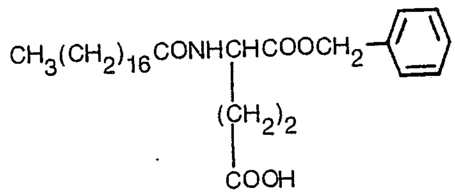

with a compound of the formula or its salt:

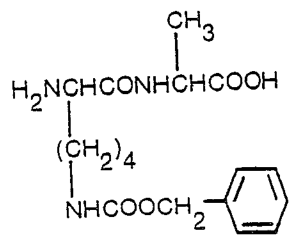

to give a compound of the formula or its salt:

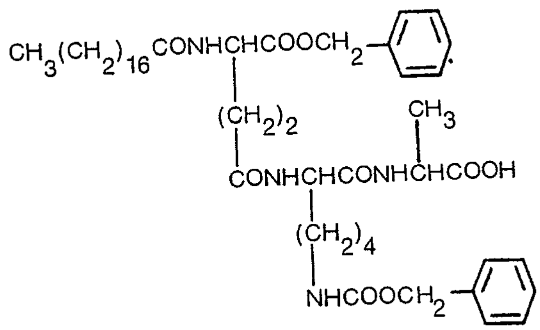

or

reacting a compound of the formula or its salt:

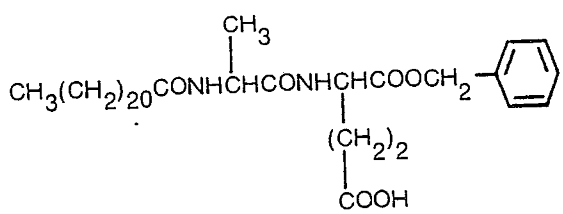

with a compound of the formula or its salt:

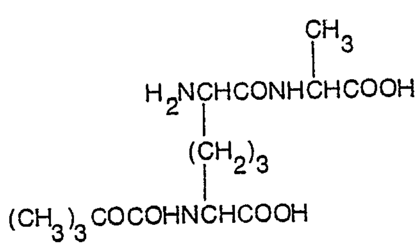

to give a compound of the formula or its salt:

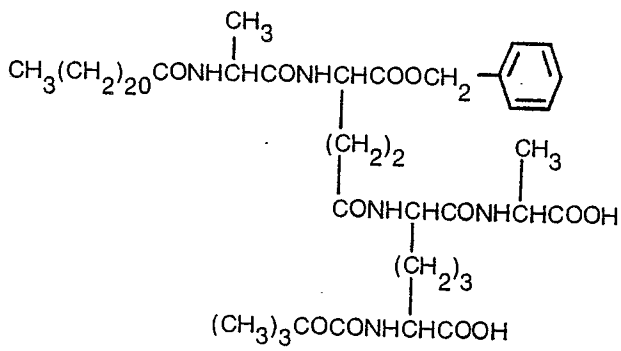

or

reacting a compound of the formula or its salts:

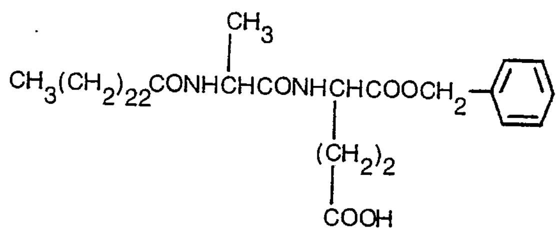

with a compound of the formula or its salt:

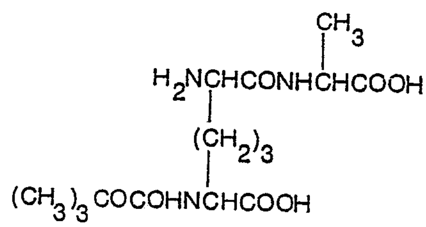

to give a compound of the formula or its salt:

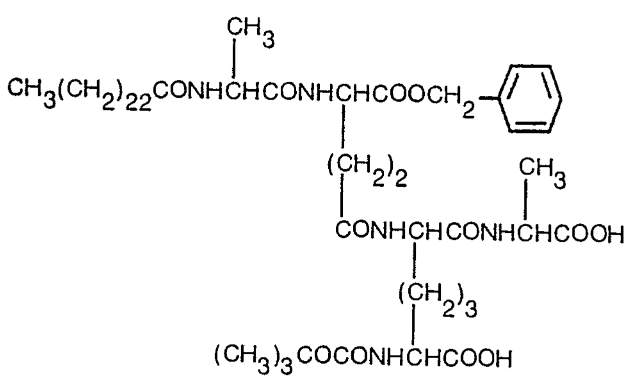

or

reacting a compound of the formula or its salt:

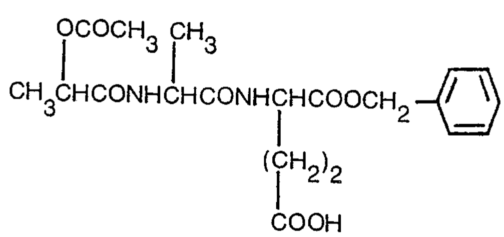

with a compound of the formula or its salt:

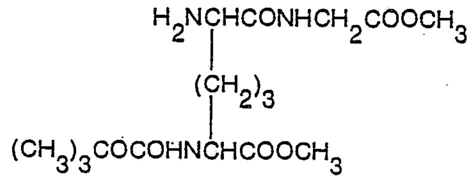

to give a compound of the formula or its salt:

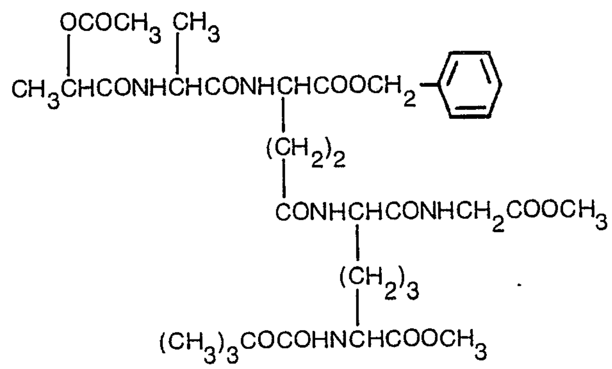

or
reacting a compound of the formula or its salt:

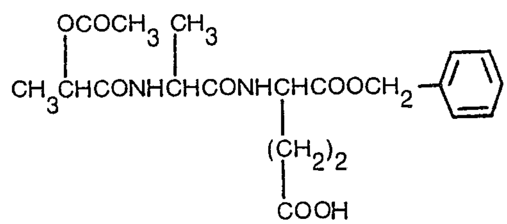

with a compound of the formula or its salt:

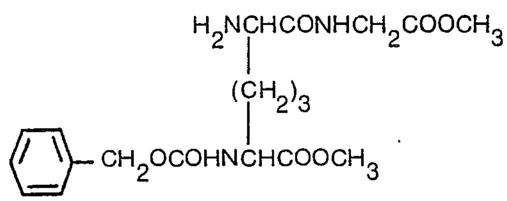

to give a compound of the formula or its salt:

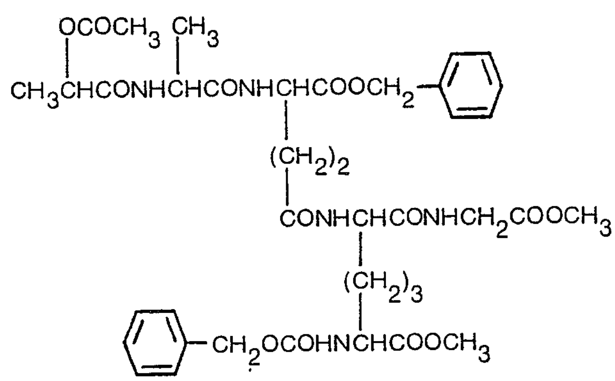

or

reacting a compound of the formula or its salt:

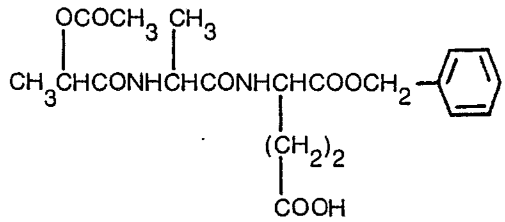

with a compound of the formula or its salt:

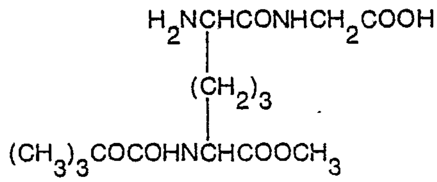

to give a compound of the formula or its salt:

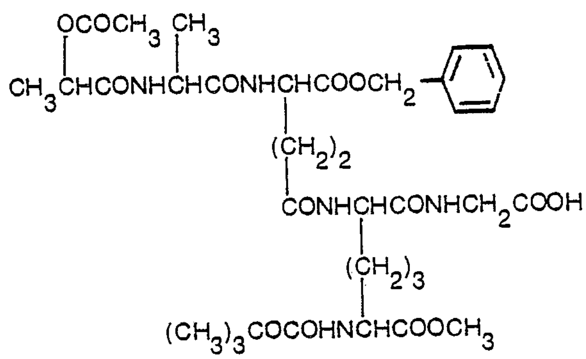

or

reacting a compound of the formula or its salt:

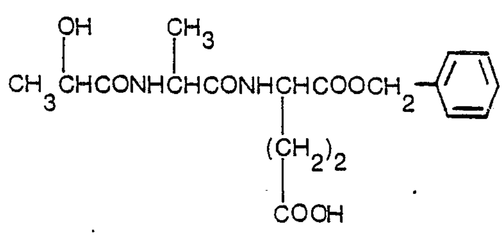

with a compound of the formula or its salt:

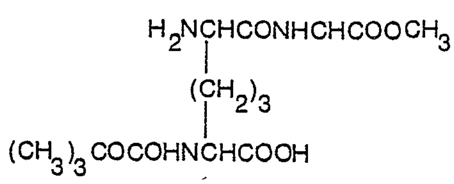

to give a compound of the formula or its salt:

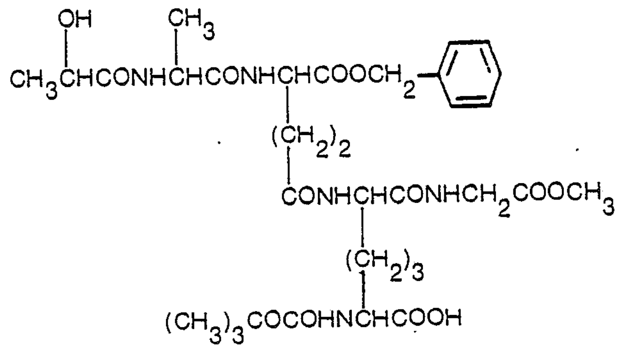

or

reacting a compound of the formula or its salt:

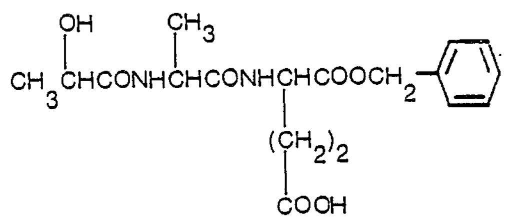

with a compound of the formula or its salt:

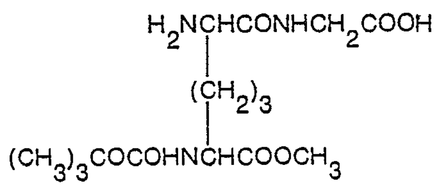

to give a compound of the formula or its salt:

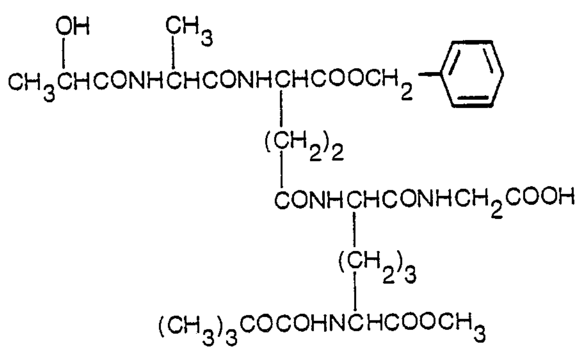

or

reacting a compound of the formula or its salt:

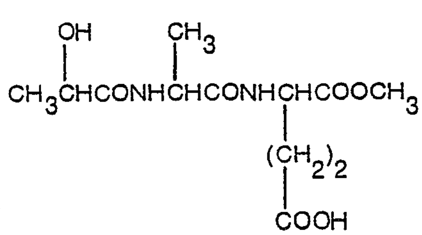

with a compound of the formula or its salt:

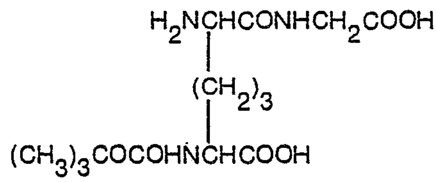

to give a compound of the formula or its salt:

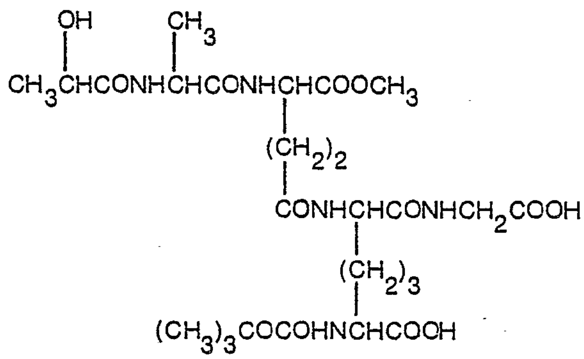

or
reacting a compound of the formula or its salt:

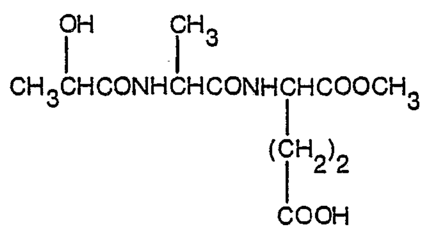

with a compound of the formula or its salt:

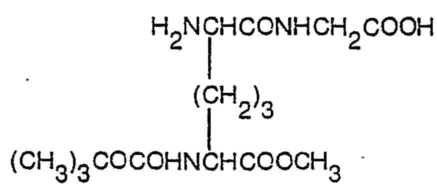

to give a compound of the formula or its salt:

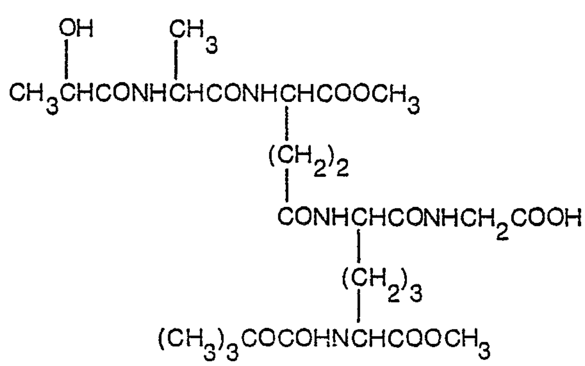

or

reacting a compound of the formula or its salt:

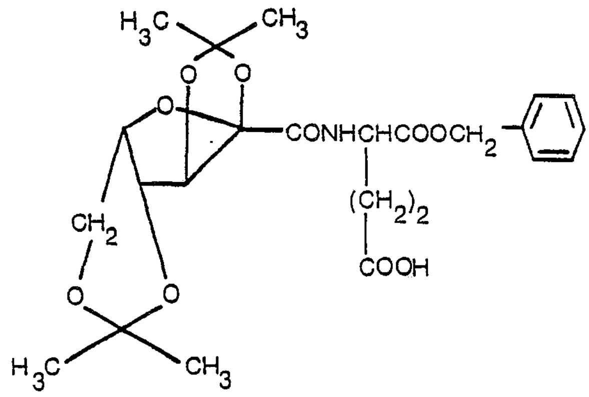

with a compound of the formula or its salt:

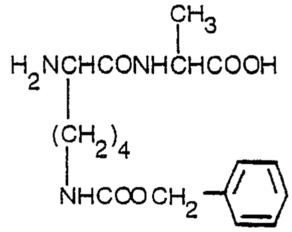

to give a compound of the formula or its salt:

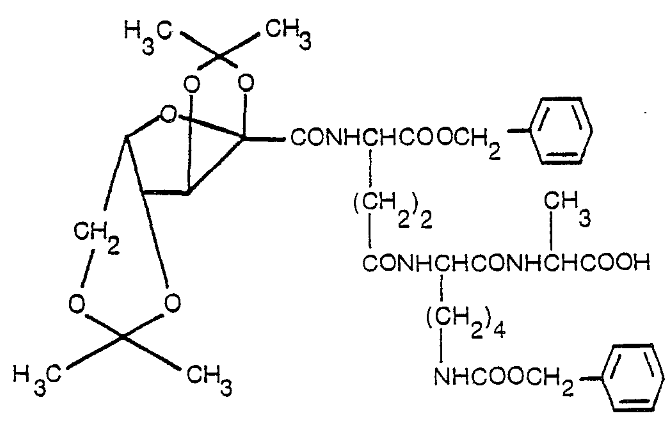

or

reacting a compound of the formula or its salt:

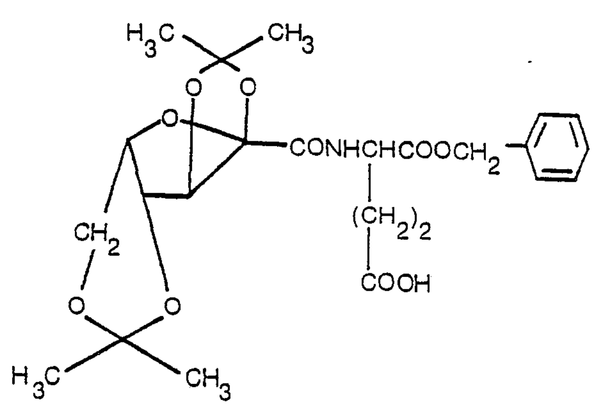

with a compound of the formula or its salt:

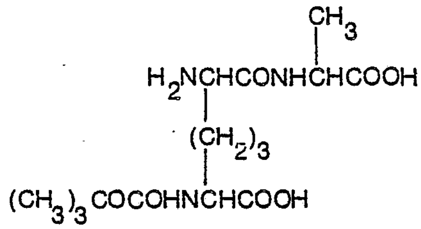

to give a compound of the formula or its salt:

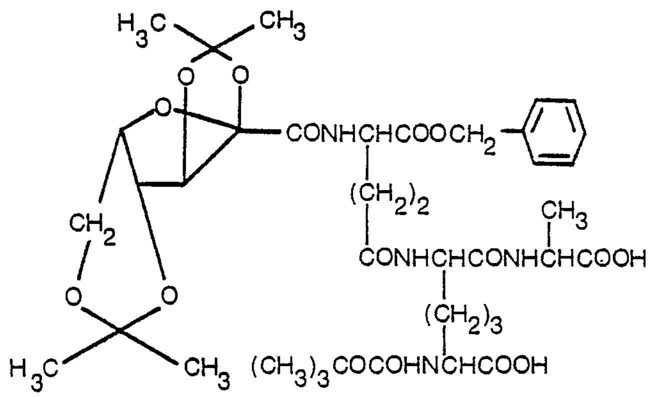

or
reacting a compound of the formula or its salt:

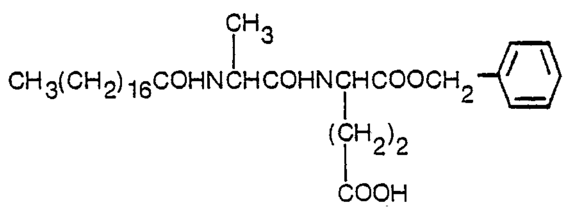

with a compound of the formula or its salt:

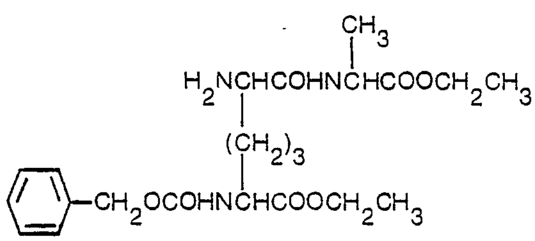

to give a compound of the formula or its salt:

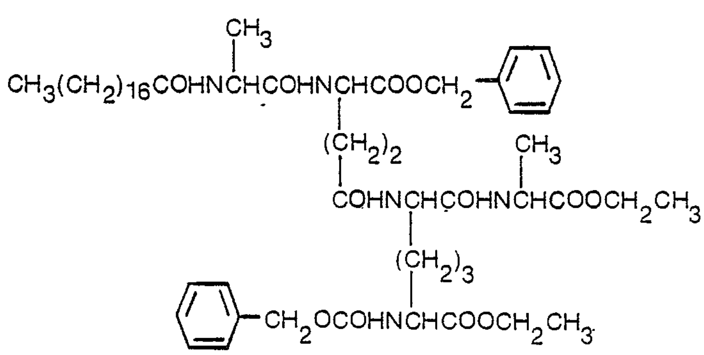

or

reacting a compound of the formula or its salt:

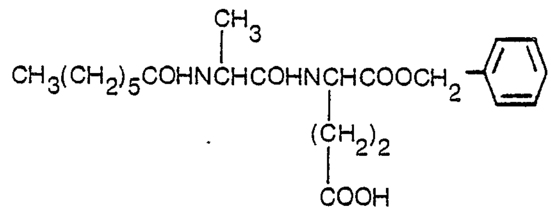

with a compound of the formula or its salt:

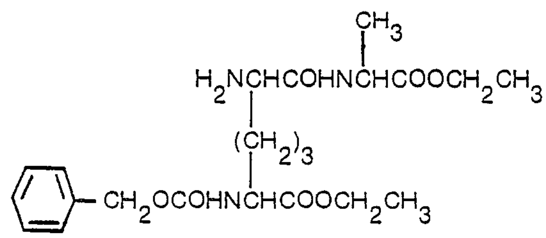

to give a compound of the formula or its salt:

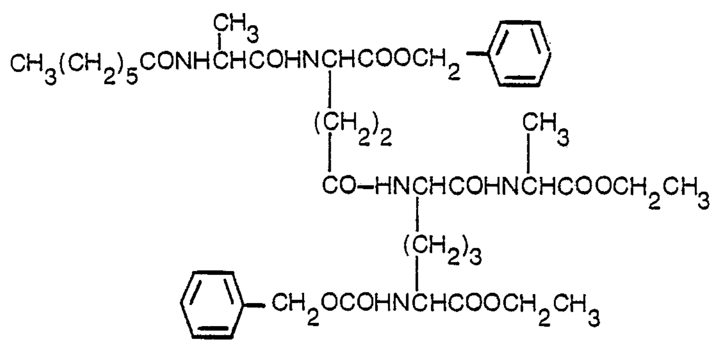

or
reacting a compound of the formula or its salt:

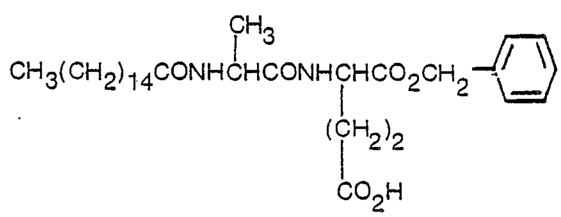

with a compound of the formula or its salt:

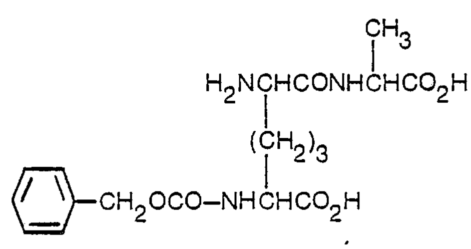

to give a compound of the formula or its salt:

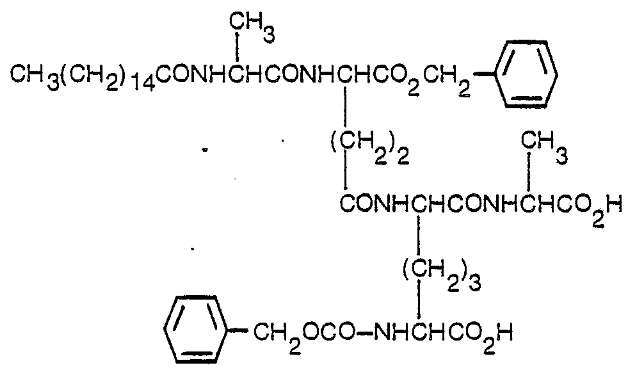

or
reacting a compound of the formula or its salt:

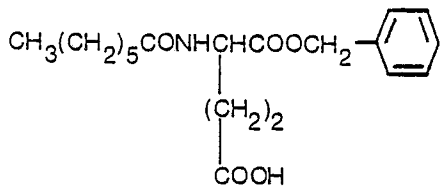

with a compound of the formula or its salt:

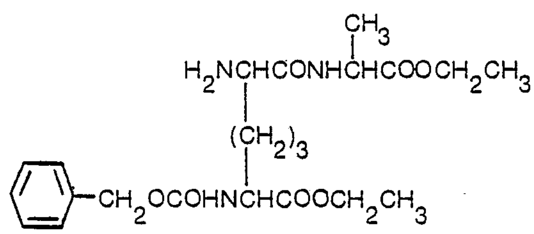

to give a compound of the formula or its salt:

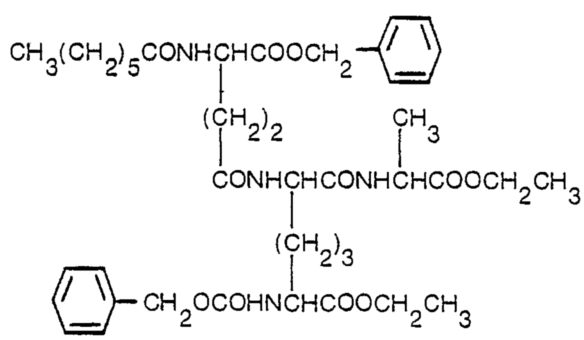

or
reacting a compound of the formula or its salt:

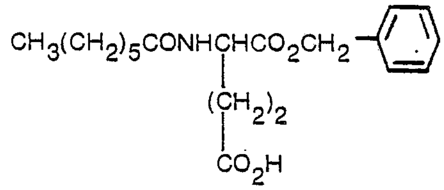

with a compound of the formula or its salt:

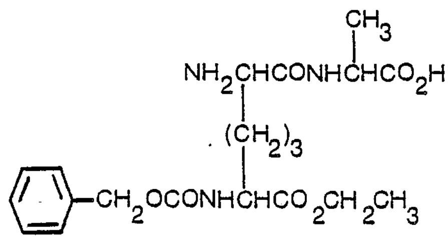

to give a compound of the formula or its salt:

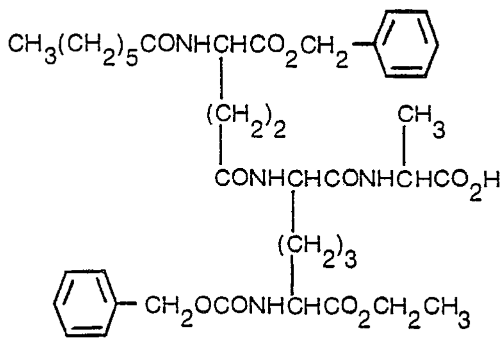

or
reacting a compound of the formula or its salt:

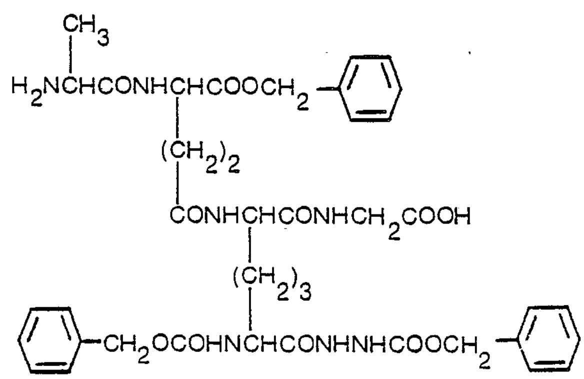

with 1-N-n-hexyl-2,3,4,5-O-tetraacetyl-D-glucaramic acid, 1 - N - benzyl - 2,3,4,5 - O - tetraacetyl - D - glucaramic acid, 1 - N - lauryl - 2,3,4,5 - O - tetraacetyl - D - glucaramic acid or their reacting derivatives to give a compound of the formula or its salt:

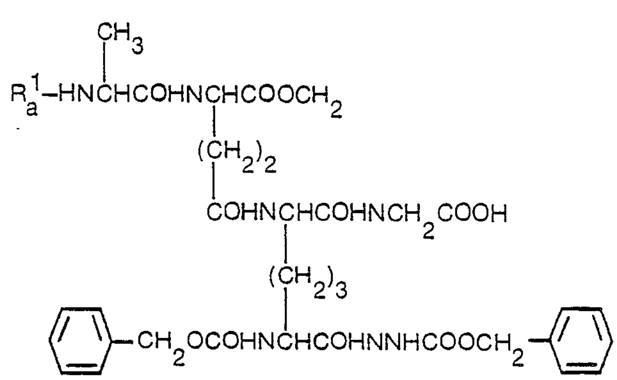

wherein $R_a^1$ is 1-N-n-hexyl-2,3,4,5-O-tetraacetyl-D-glucaramoyl, 1 - N - benzyl - 2,3,4,5 - O - tetraacetyl - D - glucaramoyl or 1 - N - lauryl - 2,3,4,5 - O - tetraacetyl - D - glucaramoyl,
or

reacting a compound of the formula or its salt:

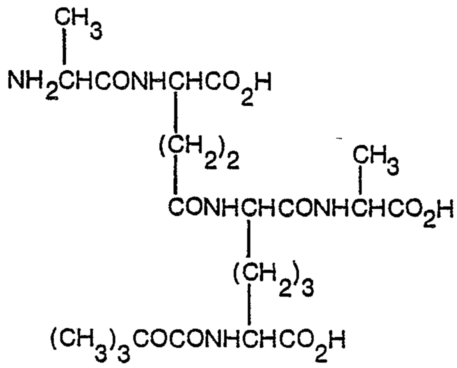

with triacontanoic acid or its reactive derivative to give a compound of the formula or its salt:

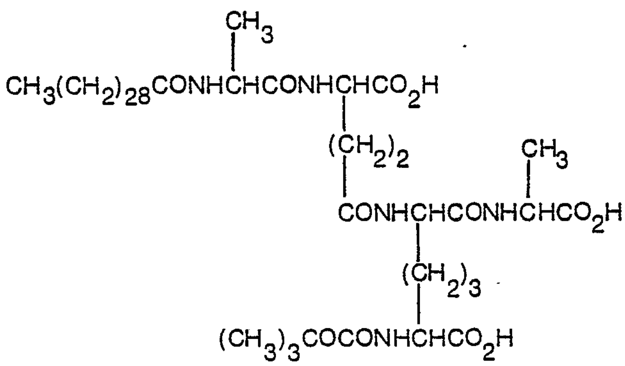

or

reacting a compound of the formula or its salt:

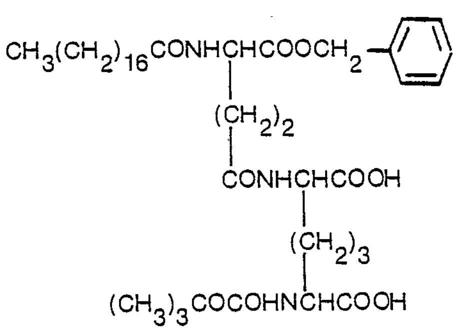

with a methylating agent to give a compound of the formula or its salt:

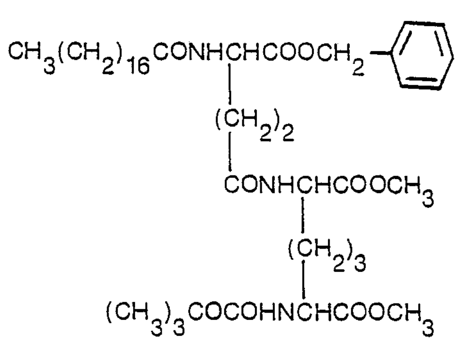

or

reacting a compound of the formula or its salt:

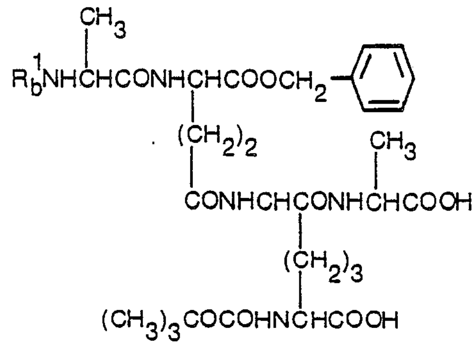

with a methylating agent to give a compound of the formula or its salt:

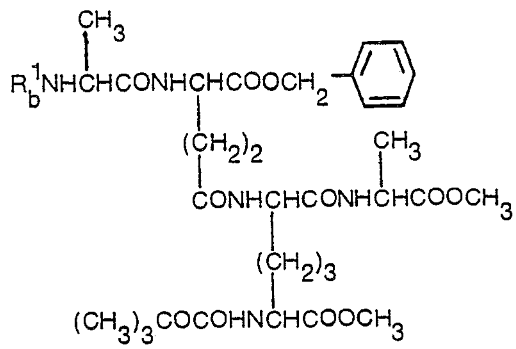

wherein $R_b^1$ is n-docosanoyl or n-tetracosanoyl,
or
reacting a compound of the formula or its salt:

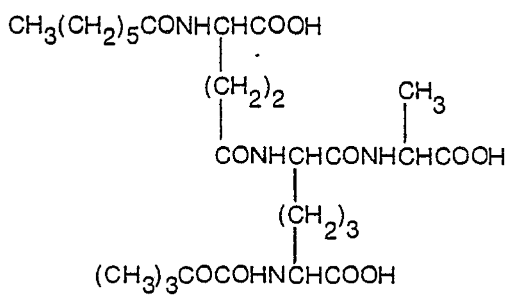

with a methylating agent to give a compound of the formula or its salt:

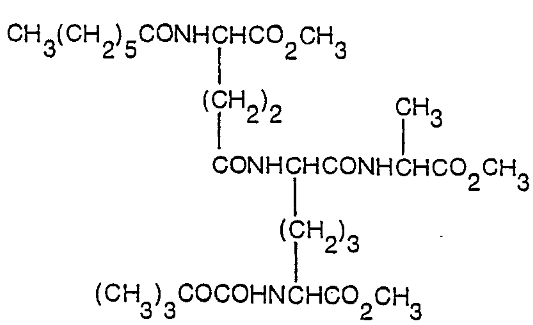

or

reacting a compound of the formula or its salt:

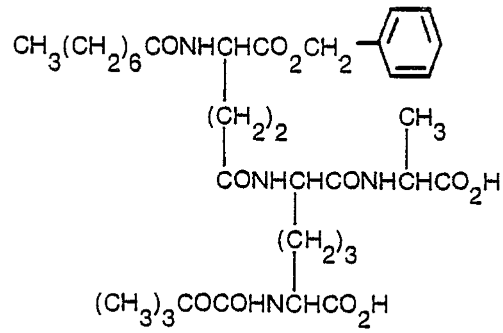

with a methylating agent to give a compound of the formula or its salt:

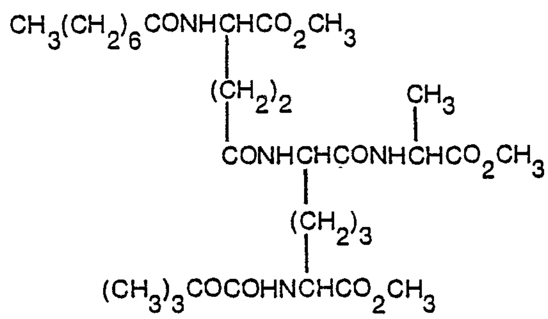

reacting a compound of the formula or its salt:

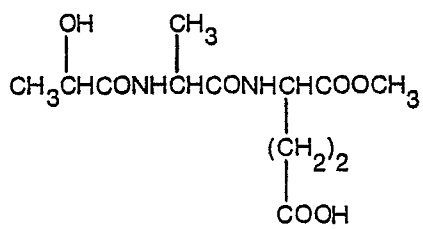

with a compound of the formula or its salt:

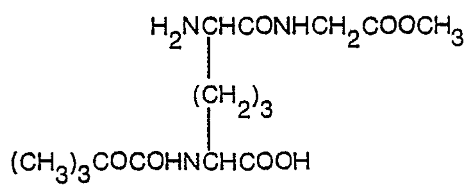

and subjecting the resulting compound to elimination reaction of the protective group to give a compound of the formula or its salt:

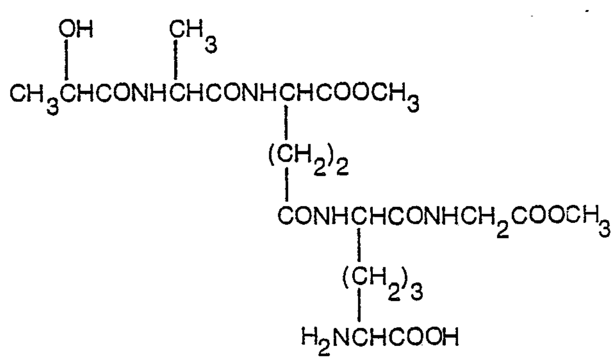

2. A process for preparation of a compound of the formula or its pharmaceutically acceptable salt:

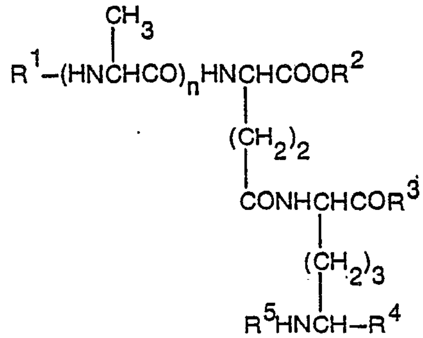

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen, and $R^5$ is hydrogen; or

$R^1$ is stearoyl, n is an integer of 0, $R^2$ is benzyl, $R^3$ is methoxy, $R^4$ is methoxycarbonyl and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is octanoyl, n is an integer of 0, $R^2$ is methyl, $R^3$ is 1-methoxycarbonylethylamino, $R^4$ is methoxycarbonyl and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$, is 1-carboxyethylamino, $R^4$ is carboxy and $R^5$ is hydrogen; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is hydrogen, $R^3$ is 1-carboxyethylamino, $R^4$ is ethoxycarbonyl and $R^5$ is hydrogen, which comprises,
subjecting a compound of the formula or its salt:

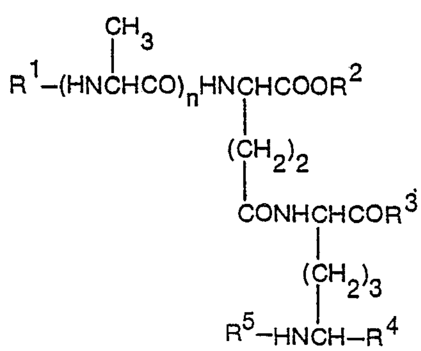

wherein

$R^1$ is n-octanoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is 1-carboxyethylamino, $R^4$ is hydrogen and $R^5$ is benzyloxycarbonyl; or

$R^1$ is stearoyl, n is an integer of 0, $R^2$ is benzyl, $R^3$ is methoxy, $R^4$ is methoxycarbonyl and $R^5$ is t-butoxycarbonyl; or

$R^1$ is stearoyl, n is an integer of 0, $R^2$ is benzyl, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is t-butoxycarbonyl; or

$R^1$ is octanoyl, n is an integer of 0, $R^2$ is methyl, $R^3$ is 1-methoxycarbonylethylamino, $R^4$ is methoxycarbonyl and $R^5$ is butoxycarbonyl; or

$R^1$ is 2,3; 4,6-diisopropylidene-L-2-ketogulonoyl, n is an integer of 0, $R^2$ is benzyl, $R^3$ is 1-carboxyethylamino, $R^4$ is hydrogen and $R^5$ is hydrogen; or

$R^1$ is 2-keto-L-gulonoyl, n is an integer of 0, $R^2$ is benzyl, $R^3$ is 1-carboxyethylamino, $R^4$ is hydrogen and $R^5$ is hydrogen; or

$R^1$ is 2,3; 4,6-diisopropylidene-2-keto-L-gulonoyl, n is an integer of 0, $R^2$ is benzyl, $R^3$ is 1-carboxyethylamino, $R^4$ is carboxy and $R^5$ is t-butoxycarbonyl; or

$R^1$ is 2-keto-L-gulonoyl, n is an integer of 0, $R^2$ is benzyl, $R^3$ is 1-carboxyethylamino, $R^4$ is carboxy and $R^5$ is hydrogen; or

$R^1$ is heptanoyl, n is an integer or 0, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 0, $R^2$ is benzyl, $R^3$ is 1-carboxyethylamino, $R^4$ is ethoxycarbonyl and $R^5$ is benzyloxycarbonyl,

to elimination reaction of protective group(s) to give a compound of the formula or its salt:

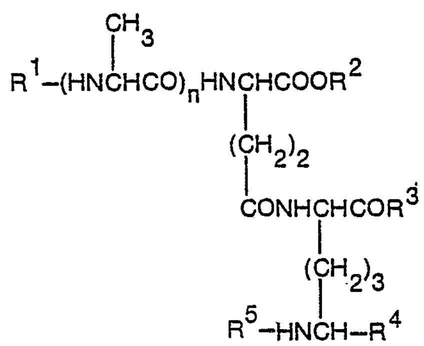

wherein

$R^1$ is n-octanoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is hydrogen, $R^3$ is 1-carboxyethylamino, $R^4$ is hydrogen and $R^5$ is hydrogen; or

$R^1$ is stearoyl, n is an integer of 0, $R^2$ is benzyl, $R^3$ is methoxy, $R^4$ is methoxycarbonyl and $R^5$ is hydrogen; or

$R^1$ is stearoyl, n is an integer of 0, $R^2$ is hydrogen, $R^3$ is methoxy, $R^4$ is methoxycarbonyl and $R^5$ is hydrogen; or

$R^1$ is heptanoyl, n is an integer of 0, $R^2$ is methyl, $R^3$ is 1-methoxycarbonylethylamino, $R^4$ is methoxycarbonyl and $R^5$ is hydrogen; or

$R^1$ is octanoyl, n is an integer of 0, $R^2$ is methyl, $R^3$ is 1-methoxycarbonylethylamino, $R^4$ is methoxycarbonyl and $R^5$ is hydrogen; or

$R^1$ is 2-keto-L-gulonoyl, n is an integer of 0, $R^2$ is hydrogen, $R^3$ is 1-carboxyethylamino, $R^4$ is hydrogen and $R^5$ is hydrogen; or

$R^1$ is 2-keto-L-gulonoyl, n is an integer of 0, $R^2$ is hydrogen, $R^3$ is 1-carboxyethylamino, $R^4$ is carboxy and $R^5$ is hydrogen; or

$R^1$ is heptanoyl, n is an integer of 0, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 0, $R^2$ is hydrogen, $R^3$ is 1-carboxyethylamino, $R^4$ is ethoxycarbonyl and $R^5$ is hydrogen.

3. A process for preparation of a compound of the formula or its pharmaceutically acceptable salt:

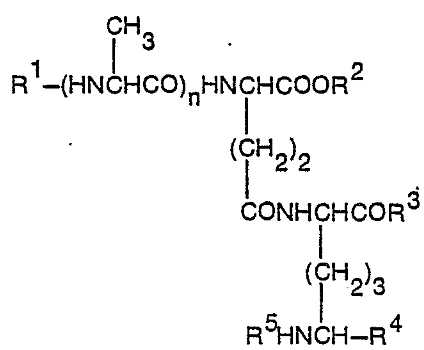

wherein

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-tetracosanoyl, n is an integer of 1, $R^2$ is hydrogen, $R^3$ is 1-carboxyethylamino, $R^4$ is carboxy and $R^5$ is hydrogen; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-n-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is hydrogen;

which comprises,

subjecting a compound of the formula or its salt:

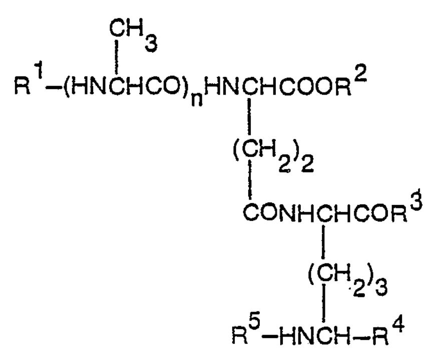

wherein

$R^1$ is n-docosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-docosanoyl, n is an integer of 1, $R^2$ is benzyl, $R^3$ is 1-carboxyethylamino, $R^4$ is carboxy and $R^5$ is t-butoxycarbonyl;

$R^1$ is n-tetracosanoyl, n is an integer of 1, $R^2$ is benzyl, $R^3$ is 1-methoxycarbonylethylamino, $R^4$ is methoxycarbonyl and $R^5$ is t-butoxycarbonyl; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is 2-acetoxypropionyl, n is an integer of 1, $R^2$ is benzyl, $R^3$ is methoxycarbonylmethylamino, $R^4$ is methoxycarbonyl and $R^5$ is benzyloxycarbonyl; or

$R^1$ is 2-hydroxypropionyl, n is an integer of 1, $R^2$ is benzyl, $R^3$ is methoxycarbonylmethylamino, $R^4$ is carboxy and $R^5$ is t-butoxycarbonyl; or

$R^1$ is 2-hydroxypropionyl, n is an integer of 1, $R^2$ is benzyl, $R^3$ is carboxymethylamino, $R^4$ is methoxycarbonyl and $R^5$ is t-butoxycarbonyl; or

$R^1$ is 1-N-n-hexyl-2,3,4,5,-O-tetraacetyl-D-glucaramoyl, n is an integer of 1, $R^2$ is benzyl, $R^3$ is carboxymethylamino, $R^4$ is 3-benzyloxycarbonylcarbazoly and $R^5$ is benzyloxycarbonyl; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is t-butoxycarbonyl; or

$R^1$ is 2-hydroxypropionyl, n is an integer of 1, $R^2$ is methyl, $R^3$ is carboxymethylamino, $R^4$ is methoxycarbonyl and $R^5$ is t-butoxycarbonyl; or

$R^1$ is stearoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is heptanoyl, n is an integer of 1, $R^2$ is benzyl, $R^3$ is 1-ethoxycarbonylethylamino, $R^4$ is ethoxycarbonyl and $R^5$ is benzyloxycarbonyl; or

$R^1$ is 1-N-benzyl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is benzyl, $R^3$ is carboxymethylamino, $R^4$ is 3-benzyloxycarbonylcarbazoyl and $R^5$ is benzyloxycarbonyl; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carbazoyl and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is benzyl, $R^3$ is 1-carboxyethylamino, $R^4$ is carboxy and $R^5$ is benzyloxycarbonyl; or

$R^1$ is triacontanoyl, n is an integer of 1, $R^2$ is hydrogen, $R^3$ is 1-carboxyethylamino, $R^4$ is carboxy and $R^5$ is t-butoxycarbonyl;

to elimination reaction of protective group(s) to give a compound of the formula or its salt:

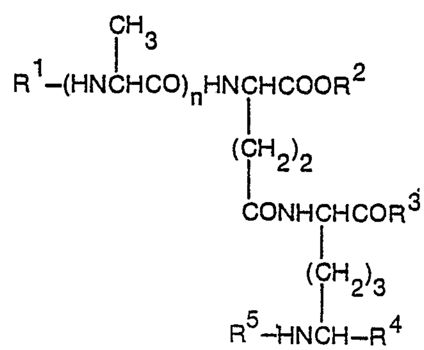

wherein

$R^1$ is n-docosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-docosanoyl, n is an integer of 1, $R^2$ is hydrogen, $R^3$ is 1-carboxyethylamino, $R^4$ is carboxy and $R^5$ is hydrogen; or

$R^1$ is n-tetracosanoyl, n is an integer of 1, $R^2$ is hydrogen, $R^3$ is 1-methoxycarbonylethylamino, $R^4$ is methoxycarbonyl and $R^5$ is hydrogen; or

$R^1$ is n-tetracosanoyl, n is an integer of 1, $R^2$ is hydrogen, $R^3$ is 1-carboxyethylamino, $R^4$ is carboxy and $R^5$ is hydrogen; or

$R^1$ is 2-acetoxypropionyl, n is an integer of 1, $R^2$ is hydrogen, $R^3$ is methoxycarbonylmethylamino, $R^4$ is methoxycarbonyl and $R^5$ is hydrogen; or

$R^1$ is 2-hydroxypropionyl, n is an integer of 1, $R^2$ is hydrogen, $R^3$ is methoxycarbonylmethylamino, $R^4$ is carboxy and $R^5$ is hydrogen; or

$R^1$ is 2-hydroxypropionyl, n is an integer of 1, $R^2$ is hydrogen, $R^3$ is carboxymethylamino, $R^4$ is methoxycarbonyl and $R^5$ is hydrogen; or

$R^1$ is 1-N-n-hexyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-n-hexyl-D-glucaramoyl, n is an integer of 1, $R^2$ is hydrogen, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is carboxy and $R^5$ is hydrogen; or

$R^1$ is 2-hydroxypropionyl, n is an integer of 1, $R^2$ is methyl, $R^3$ is carboxymethylamino, $R^4$ is methoxycarbonyl and $R^5$ is hydrogen; or

$R^1$ is stearoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is heptanoyl, n is an integer of 1, $R^2$ is hydrogen, $R^3$ is 1-ethoxycarbonylethylamino, $R^4$ is ethoxycarbonyl and $R^5$ is hydrogen; or

$R^1$ is 1-N-benzyl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-benzyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-2,3,4,5-O-tetraacetyl-D-glucaramoyl, n is an integer of 1, $R^2$ is hydrogen, $R^3$ is carboxymethylamino, $R^4$ is carboxy and $R^5$ is hydrogen; or

$R^1$ is 1-N-lauryl-D-glucaramoyl, n is an integer of 1, $R^2$ is hydrogen, $R^3$ is carboxymethylamino, $R^4$ is carboxy and $R^5$ is hydrogen; or

$R^1$ is palmitoyl, n is an integer of 1, $R^2$ is hydrogen, $R^3$ is 1-carboxyethylamino, $R^4$ is carboxy and $R^5$ is hydrogen; or

$R^1$ is triacontanoyl, n is an integer of 1, $R^2$ is hydrogen, $R^3$ is 1-carboxyethylamino, $R^4$ is carboxy and $R^5$ is hydrogen;

**Patentansprüche für die Vertragsstaaten: BE CH DE FR GB IT LI LU NL SE**

1. Verbindung mit der Formel

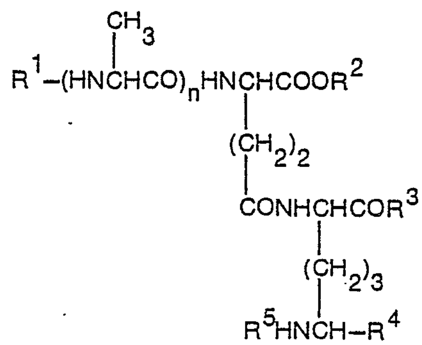

oder ihr pharmazeutisch brauchbares Salz, worin

$R^1$ n-Octanoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ t-Butoxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Wasserstoff ist, $R^3$ Carboxyethylamino ist, $R^4$ Carboxy ist und $R^5$ t-Butoxycarbony ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Butoxycarbonyl ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Butoxycarbonyl ist; oder

$R^1$ 2,3;4,6-Diisopropyliden-L-2-ketogulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2,3;4,6-Diisopropyliden-2-keto-L-gulonoyl ist, n eine ganze Zahl von 0 ist, $R^2$ Benzyl ist, $R^3$ 1-Carboxyethylamino ist, $R^4$ Carboxy ist und $R^5$ t-Butoxycarbonyl ist; oder

$R^1$ 2-Keto-L-gulonoyl ist, n eine ganze Zahl von 0 ist, $R^2$ Benzyl ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 0 ist, $R^2$ Benzyl ist, $R^3$ 1-Carboxyethylamino ist, $R^4$ Ethoxycarbonyl ist und $R^5$ Benzyloxycarbonyl ist.

2. Verbindung der Formel

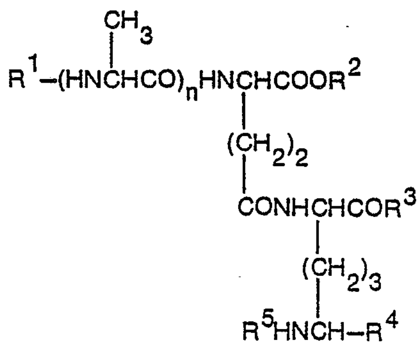

oder ihr pharmazeutisch brauchbares Salz, worin

$R^1$ n-Docosanoyl ist, n eine ganze Zahl von 1 ist, $R^2$ Benzyl ist, $R^3$ 1-Carboxyethylamino ist, $R^4$ Carboxy ist und $R^5$ t-Butoxycarbonyl ist; oder

$R^1$ n-Docosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Tetracosanoyl ist, n eine ganze Zahl von 1 ist, $R^2$ Benzyl ist, $R^3$ 1-Carboxyethylamino ist, $R^4$ Carboxy ist und $R^5$ t-Butoxycarbonyl ist; oder

$R^1$ n-Tetracosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ t-Butoxycarbonyl ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ Benzyloxycarbonyl ist; oder

$R^1$ 2-Acetoxypropionyl ist, n eine ganze Zahl von 1 ist, $R^2$ Wasserstoff ist, $R^3$ Methoxycarbonylmethylamino ist, $R^4$ Methoxycarbonyl ist und $R^5$ Wasserstoff ist; oder

$R^1$ 2-Acetoxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ 2-Acetoxypropionyl ist, n eine ganze Zahl von 1 ist, $R^2$ Wasserstoff ist, $R^3$ Carboxymethylamino ist, $R^4$ Methoxycarbonyl ist und $R^5$ t-Butoxycarbonyl ist; oder

$R^1$ 2-Hydroxypropionyl ist, n eine ganze Zahl von 1 ist, $R^2$ Benzyl ist, $R^3$ Methoxycarbonylmethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ 1-N-n-Hexyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ t-Butoxycarbonyl ist; oder

$R^1$ Stearoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-Benzyl-2,3-4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Benzyl ist,

$R^3$ Carboxymethylamino ist, $R^4$ 3-Benzyloxycarbonylcarbazoyl ist und $R^5$ Benzyloxycarbonyl ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Benzyl ist, $R^3$ 1-Carboxyethylamino ist, $R^4$ Carboxy ist und $R^5$ Benzyloxycarbonyl ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ t-Butoxycarbonyl ist.

3. Verbindung der Formel

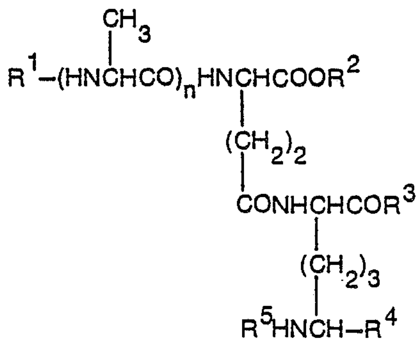

oder ihr pharmazeutisch brauchbares Salz, worin

$R^1$ n-Octanoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 0 ist, $R^2$ Wasserstoff ist, $R^3$ 1-Carboxyethylamino ist, $R^4$ Ethoxycarbonyl ist und $R^5$ Wasserstoff ist.

4. Verbindung der Formel

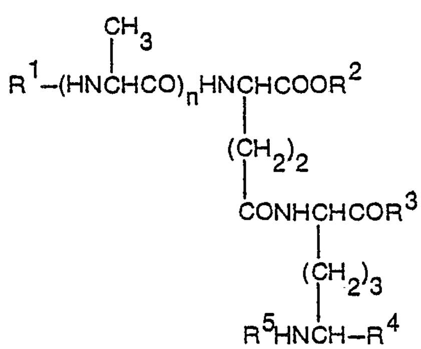

oder ihr pharmazeutisch brauchbares Salz, worin

$R^1$ n-Docosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Docosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ 2-Hydroxypropionyl ist, n eine ganze Zahl von 1 ist, $R^2$ Wasserstoff ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Wasserstoff ist, $R^3$ Carboxymethylamino ist, $R^4$ Methoxycarbonyl ist und $R^5$ Wasserstoff ist; oder

$R^1$ 1-N-Hexyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxyethylamino ist, $R^4$ Carboxy ist, und $R^5$ Wasserstoff ist; oder

$R^1$ 2-Hydroxypropionyl ist, n eine ganze Zahl von 1 ist, $R^2$ Methyl ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ Wasserstoff ist; oder

$R^1$ Stearoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ 1-N-Benzyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist.

5. Verbindung der Formel

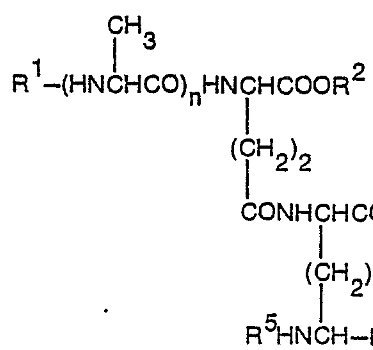

oder ihr pharmazeutisch brauchbares Salz, worin

$R^1$ n-Octanoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ Methoxy ist, $R^4$ Methoxycarbonyl ist und $R^5$ Wasserstoff ist; oder

$R^1$ n-Docosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ Stearoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Palmitoyl ist, n eine ganze Zahl von 1 ist, $R^2$ Wasserstoff ist, $R^3$ 1-Carboxyethylamino ist, $R^4$ Carboxy ist und $R^5$ Wasserstoff ist.

6. Verfahren zur Herstellung einer Verbindung der Formel

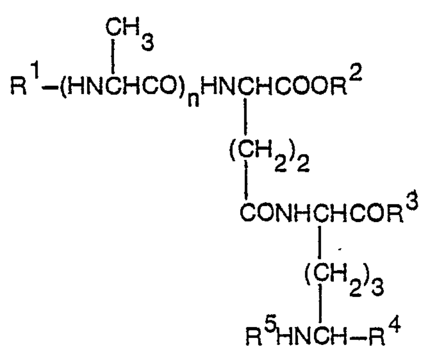

oder ihres pharmazeutisch brauchbaren Salzes, worin

$R^1$ n-Octanoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ t-Butoxycarbonyl ist; oder

$R^1$ n-Docosanoyl ist, n eine ganze Zahl von 1 ist, $R^2$ Benzyl ist, $R^3$ 1-Carboxyethylamino ist, $R^4$ Carboxy ist und $R^5$ t-Butoxycarbonyl ist; oder

$R^1$ n-Docosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Tetracosanoyl ist, n eine ganze Zahl von 1 ist, $R^2$ Benzyl ist, $R^3$ 1-Carboxyethylamino ist, $R^4$ Carboxy ist und $R^5$ t-Butoxycarbonyl ist; oder

$R^1$ n-Tetracosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ t-Butoxycarbonyl ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ Benzyloxycarbonyl ist; oder

$R^1$ 2-Acetoxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ Heptanoyl ist, n eine ganze Zahl von 0 ist, $R^2$ Methyl, $R^3$ 1-Methoxycarbonylethylamino ist, $R^4$ Methoxycarbonyl ist und $R^5$ t-Butoxycarbonyl ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Butoxycarbonyl ist; oder

$R^1$ 2-Hydroxypropionyl ist, n eine ganze Zahl von 1 ist, $R^2$ Benzyl ist, $R^3$ Methoxycarbonylmethylamino ist, $R^4$ Carboxy ist und $R^5$ t-Butoxycarbonyl ist; oder

$R^1$ 2-Hydroxpropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ 1-N-n-Hexyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Methyl ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ t-Butoxycarbonyl ist; oder

$R^1$ 2,3;4,6-Diisopropyliden-L-2-ketogulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2,3;4,6-Diisopropyliden-2-keto-L-gulonoyl ist, n eine ganze Zahl von 0 ist, $R^2$ Benzyl ist, $R^3$ 1-Carboxyethylamino ist, $R^4$ Carboxy ist und $R^5$ t-Butoxycarbonyl ist; oder

$R^1$ Stearoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Lauryl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ Palmitoyl ist, n eine ganze Zahl von 1 ist, $R^2$ Benzyl ist, $R^3$ 1-Carboxyethylamino ist, $R^4$ Carboxy ist und $R^5$ Benzyloxycarbonyl ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ t-Butoxycarbonyl ist; oder

$R^1$ Heptanoyl ist, n eine ganze Zahl von 0 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 0 ist, $R^2$ Benzyl ist, $R^3$ 1-Carboxyethylamino ist, $R^4$ Ethoxycarbonyl ist und $R^5$ Benzyloxycarbonyl ist;
durch Reaktion einer Verbindung der Formel

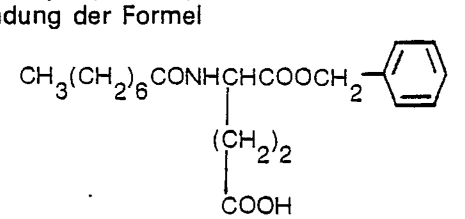

oder ihres Salzes

mit einer Verbindung der Formel

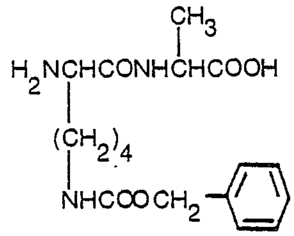

oder ihrem Salz,
unter Bildung einer Verbindung der Formel

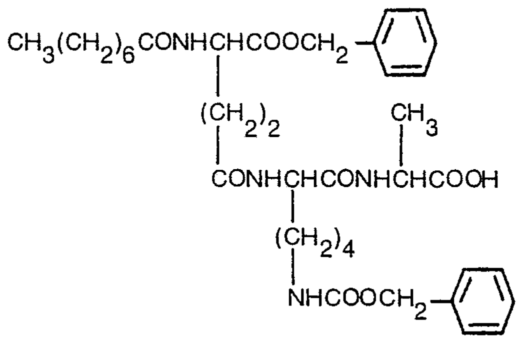

oder ihres Salzes oder
Reaktion einer Verbindung der Formel

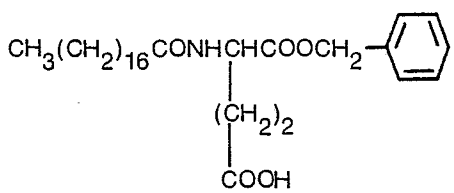

oder ihres Salzes,
mit einer Verbindung der Formel

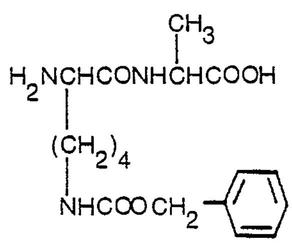

oder ihrem Salz,
unter Bildung einer Verbindung der Formel

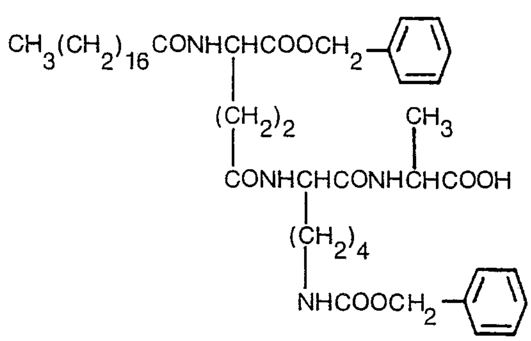

oder ihres Salzes,

oder Reaktion einer Verbindung der Formel

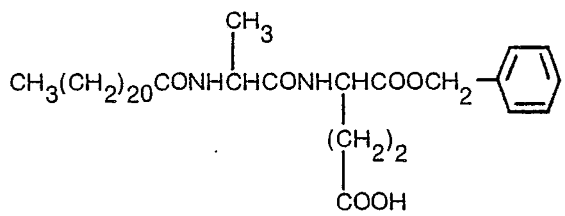

oder ihres Salzes,
mit einer Verbindung der Formel

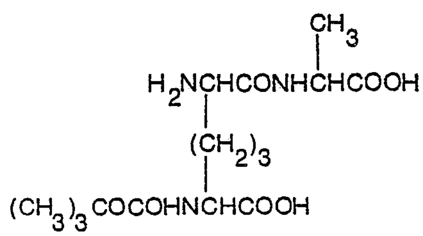

oder ihrem Salz,
unter Bildung einer Verbindung der Formel

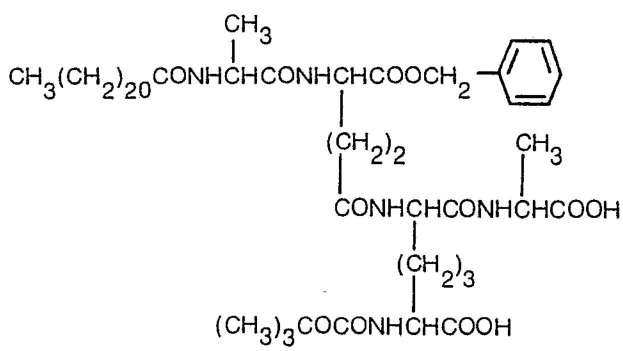

oder ihres Salzes
oder Reaktion einer Verbindung der Formel

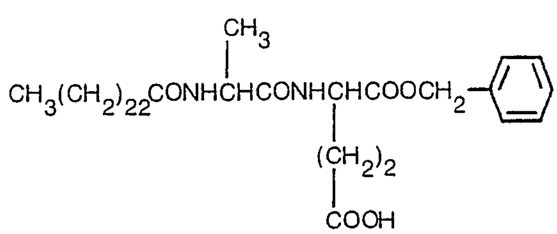

oder ihres Salzes
mit einer Verbindung der Formel

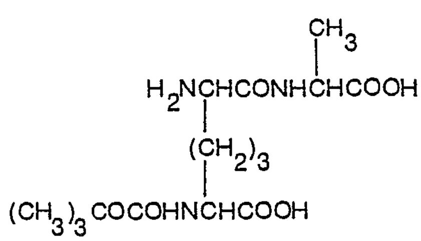

oder ihrem Salz

unter Bildung einer Verbindung der Formel

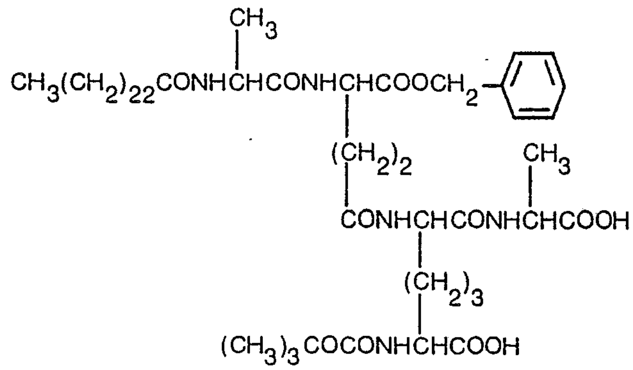

oder ihres Salzes
oder Reaktion einer Verbindung der Formel

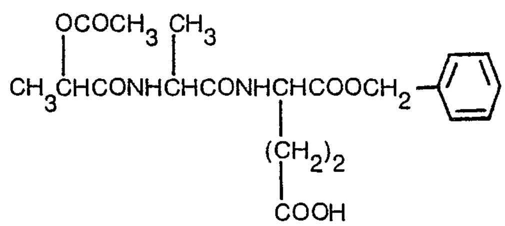

oder ihres Salzes
mit einer Verbindung der Formel

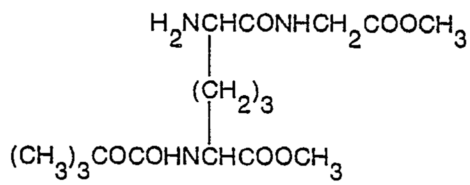

oder ihrem Salz
unter Bildung einer Verbindung der Formel

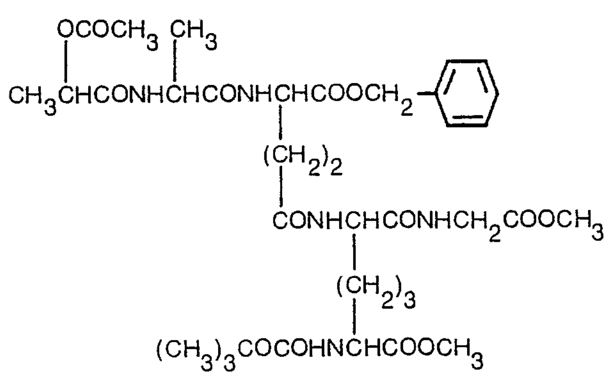

oder ihres Salzes
oder Reaktion einer Verbindung der Formel

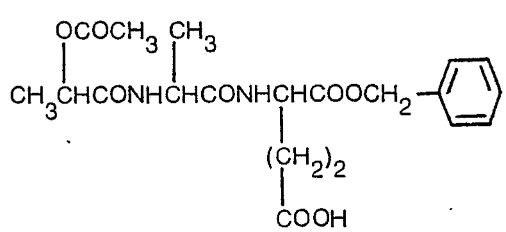

oder ihres Salzes

mit einer Verbindung der Formel

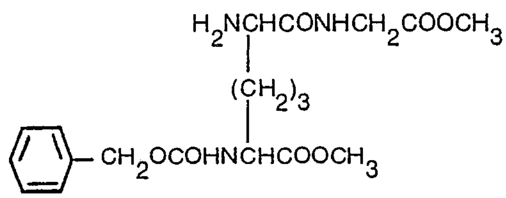

oder ihrem Salz
unter Bildung einer Verbindung der Formel

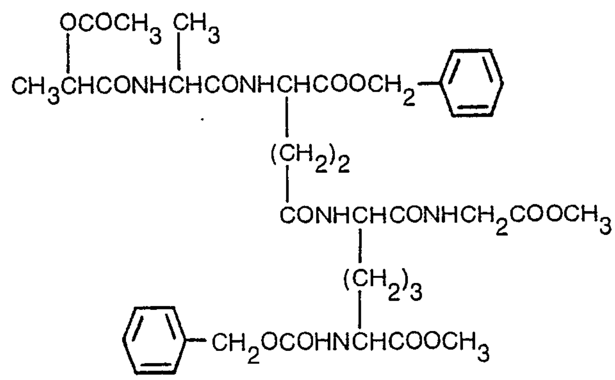

oder ihres Salzes
oder Reaktion einer Verbindung der Formel

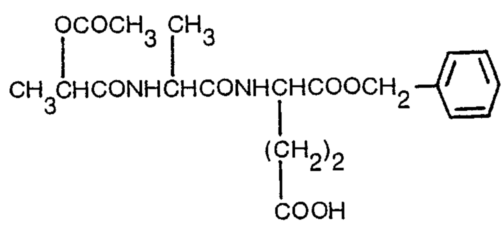

oder ihres Salzes
mit einer Verbindung der Formel

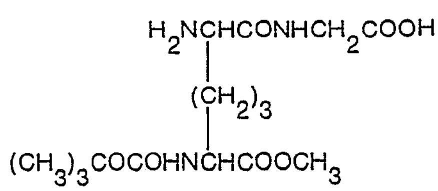

oder ihrem Salz
unter Bildung einer Verbindung der Formel

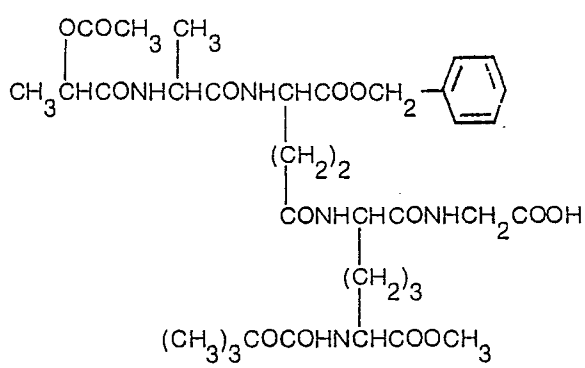

oder ihres Salzes

oder Reaktion einer Verbindung der Formel

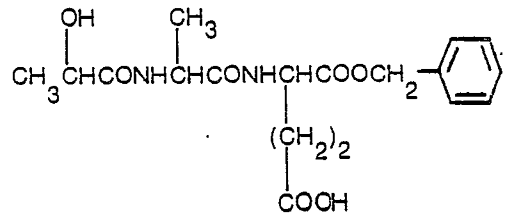

oder ihres Salzes
mit einer Verbindung der Formel

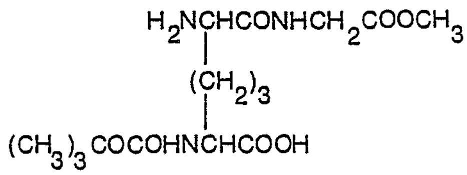

oder ihrem Salz
unter Bildung einer Verbindung der Formel

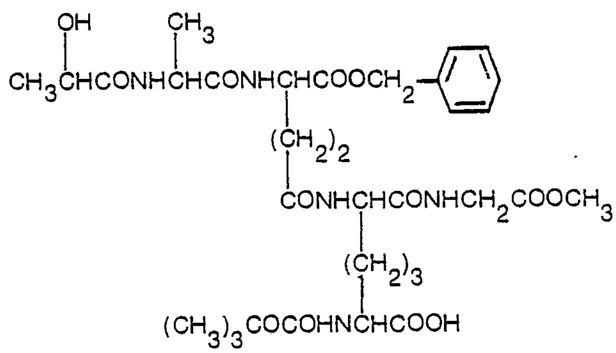

oder ihres Salzes
oder Reaktion einer Verbindung der Formel

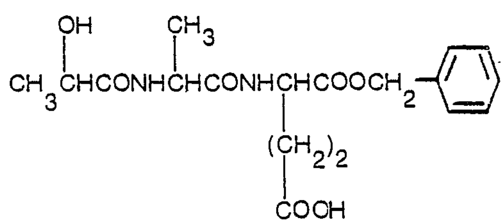

oder ihres Salzes
mit einer Verbindung der Formel

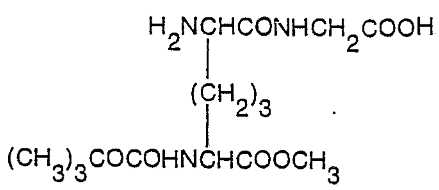

oder ihrem Salz

unter Bildung einer Verbindung der Formel

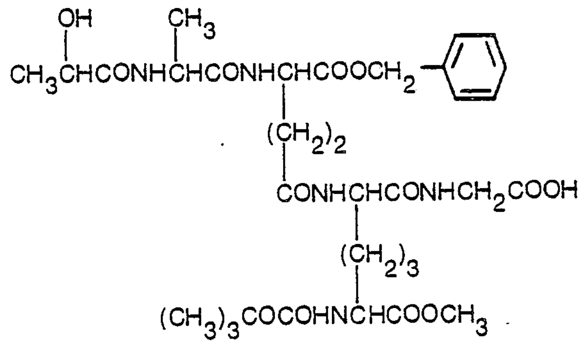

oder ihres Salzes
oder Reaktion einer Verbindung der Formel

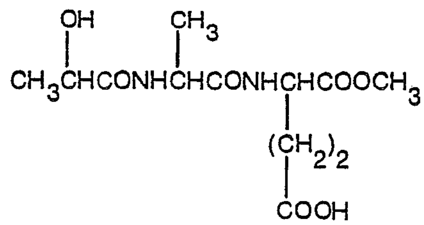

oder ihres Salzes
mit einer Verbindung der Formel

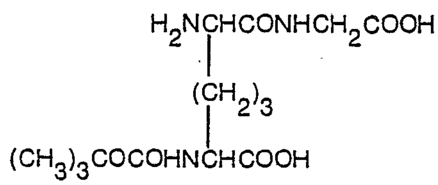

oder ihrem Salz
unter Bildung einer Verbindung der Formel

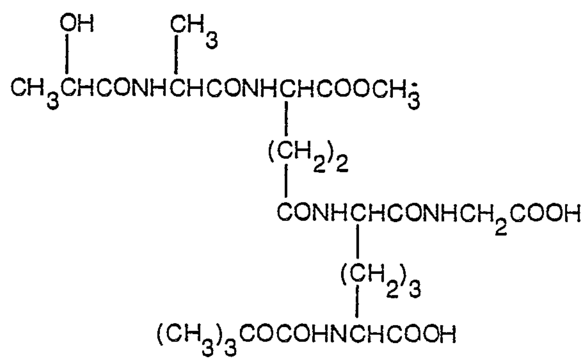

oder ihres Salzes
oder Reaktion einer Verbindung der Formel

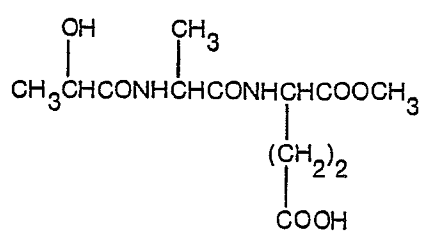

oder ihres Salzes

mit einer Verbindung der Formel

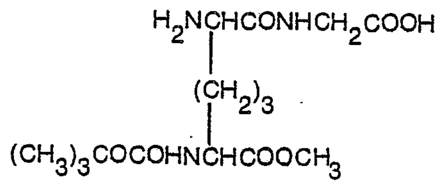

oder ihrem Salz
unter Bildung einer Verbindung der Formel

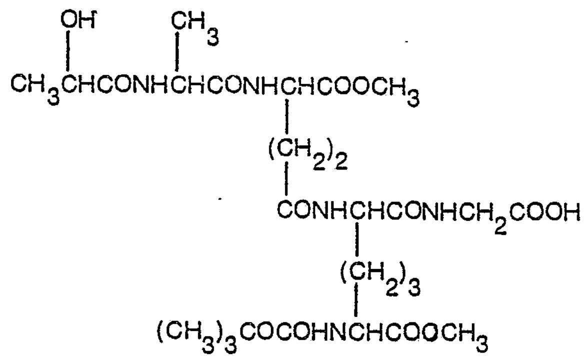

oder ihres Salzes
oder Reaktion einer Verbindung der Formel

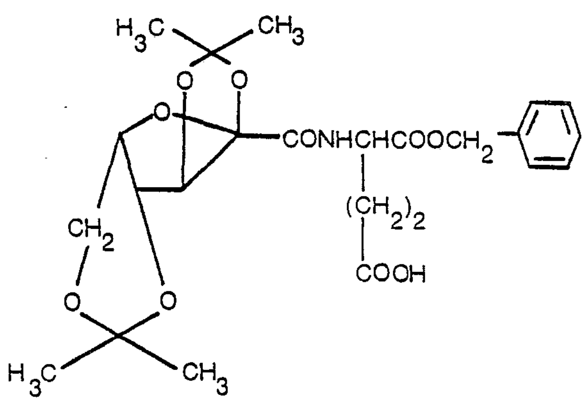

oder ihres Salzes
mit einer Verbindung der Formel

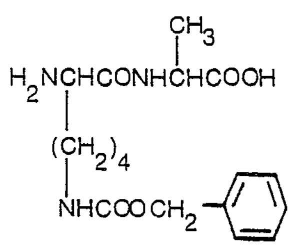

oder ihrem Salz

unter Bildung einer Verbindung der Formel

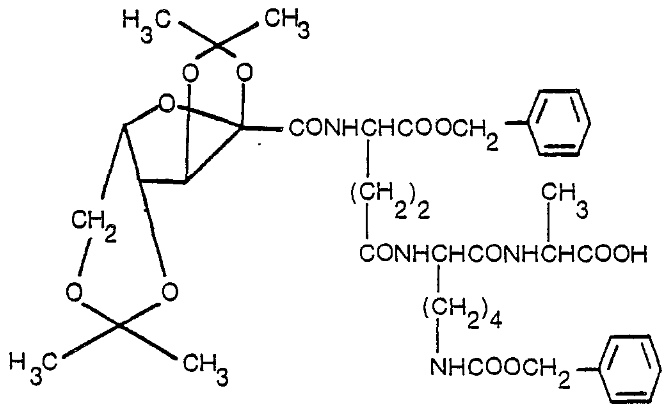

oder ihres Salzes
oder Reaktion einer Verbindung der Formel

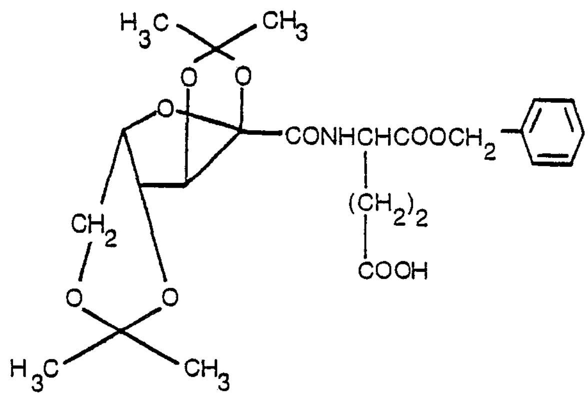

oder ihres Salzes
mit einer Verbindung der Formel

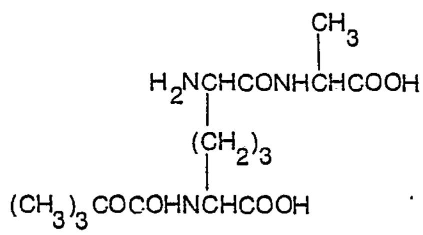

oder ihrem Salz
unter Bildung einer Verbindung der Formel

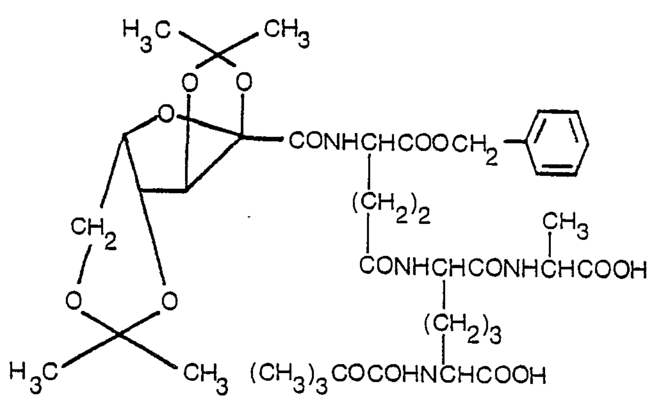

oder ihres Salzes

oder Reaktion einer Verbindung der Formel

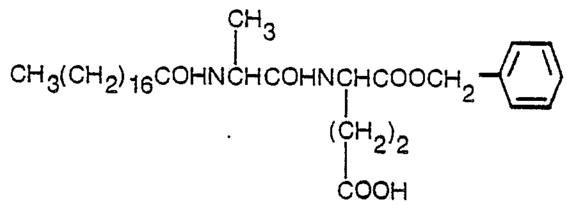

oder ihres Salzes
mit einer Verbindung der Formel

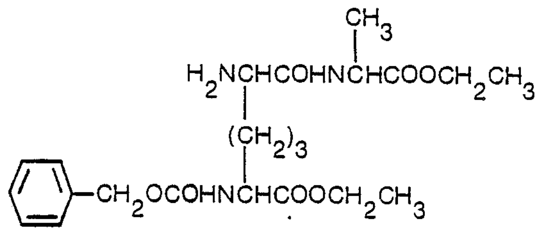

oder ihrem Salz
unter Bildung einer Verbindung der Formel

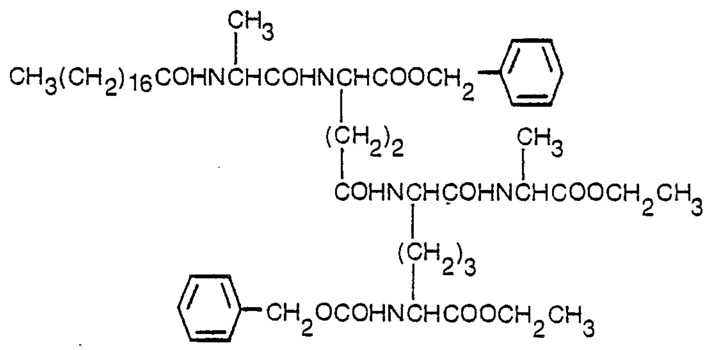

oder ihres Salzes
oder Reaktion einer Verbindung der Formel

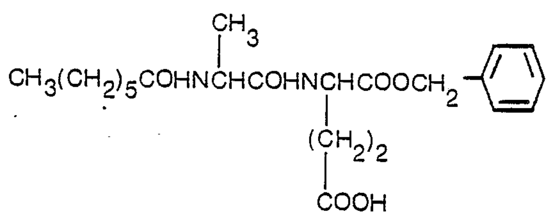

oder ihres Salzes
mit einer Verbindung der Formel

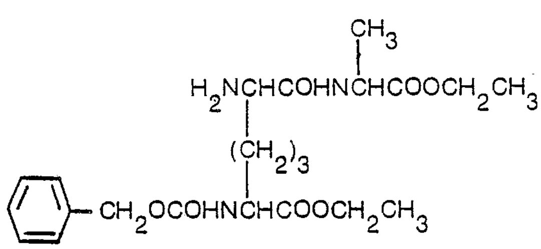

oder ihrem Salz

unter Bildung einer Verbindung der Formel

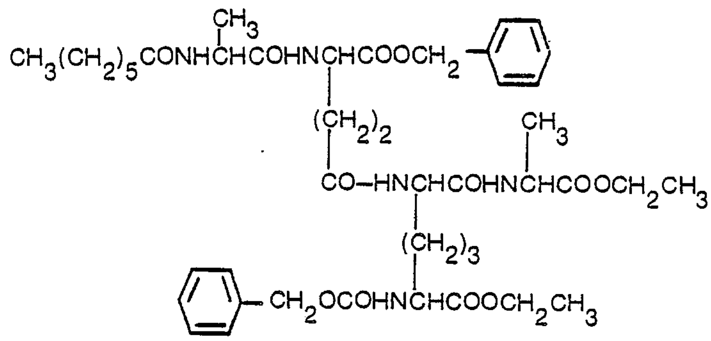

oder ihres Salzes
oder Reaktion einer Verbindung der Formel

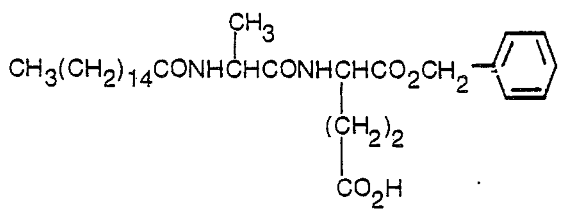

oder ihres Salzes
mit einer Verbindung der Formel

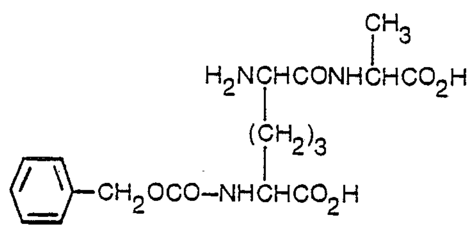

oder ihrem Salz
unter Bildung einer Verbindung der Formel

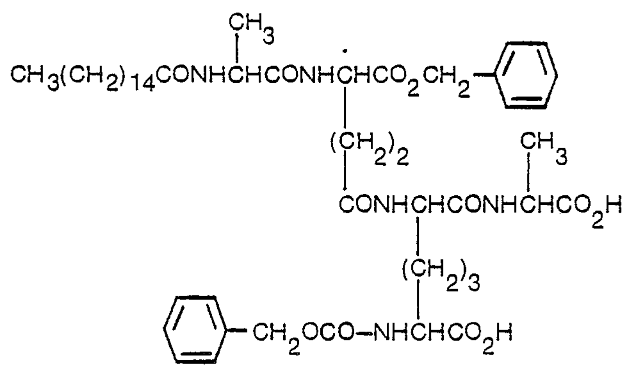

oder ihres Salzes
oder Reaktion einer Verbindung der Formel

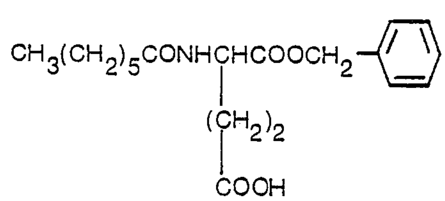

oder ihres Salzes

mit einer Verbindung der Formel

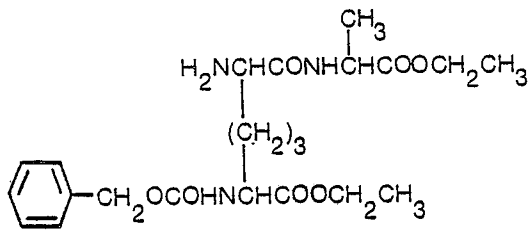

oder ihrem Salz
unter Bildung einer Verbindung der Formel

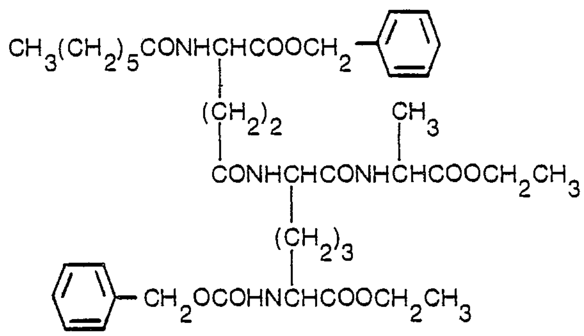

oder ihres Salzes
oder Reaktion einer Verbindung der Formel

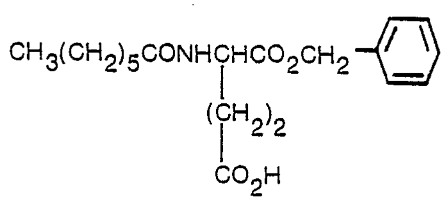

oder ihres Salzes
mit einer Verbindung der Formel

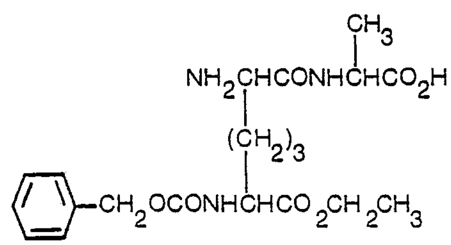

oder ihrem Salz
unter Bildung einer Verbindung der Formel

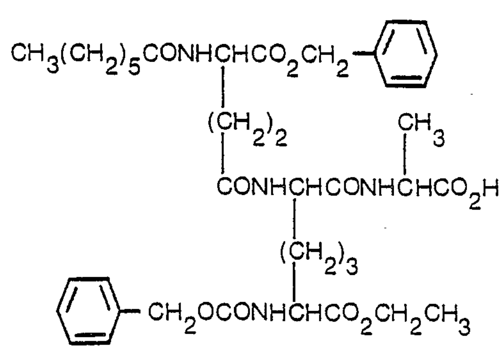

oder ihres Salzes

oder Reaktion einer Verbindung der Formel

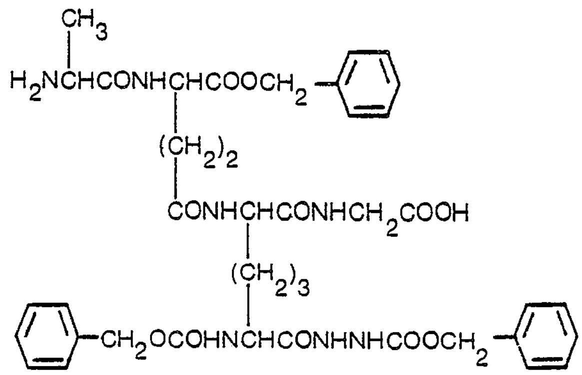

oder ihres Salzes
mit 1 - N - n - Hexyl - 2,3,4,5 - O - tetraacetyl - D - glucaramsäure, 1 - N - Benzyl - 2,3,4,5 - O - tetraacetyl - D - glucaramsäure, 1 - N - Laury - 2,3,4,5 - O - tetraacetyl - D - glucaramsäure oder ihren reaktionsfähigen Derivaten, unter Bildung einer Verbindung der Formel

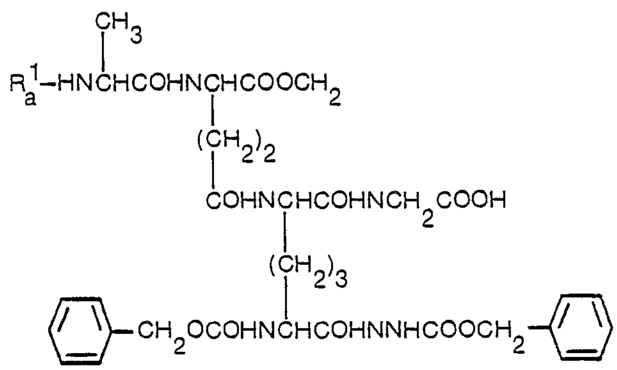

oder ihres Salzes,
worin
$R_a^1$ 1 - N - n - Hexyl - 2,3,4,5 - O - tetraacetyl - D - glucaramoyl, 1 - N - Benzyl - 2,3,4,5 - O - tetraacetyl - D - glucaramoyl oder 1 - N - Lauryl - 2,3,4,5 - O - tetraacetyl - D - glucaramoyl ist
oder Reaktion einer Verbindung der Formel

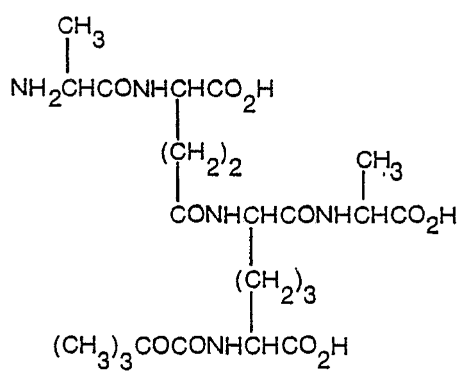

oder ihres Salzes

mit Triacontanoesäure oder ihrem reaktiven Derivat, unter Bildung einer Verbindung der Formel

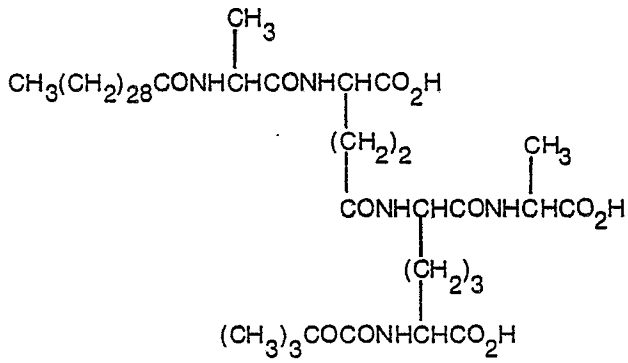

oder ihres Salzes
oder Reaktion einer Verbindung der Formel

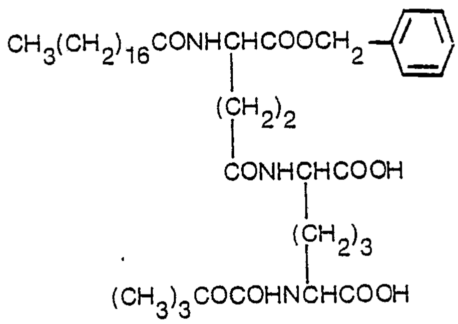

oder ihres Salzes,
mit einem Methylierungsmittel, unter Bildung einer Verbindung der Formel

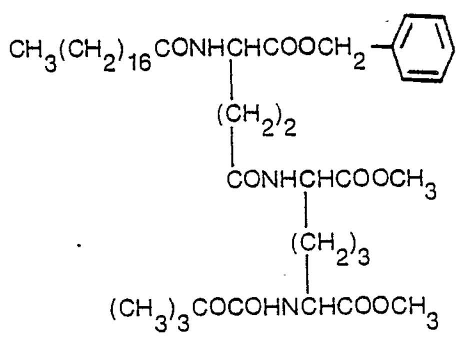

oder ihres Salzes,
oder Reaktion einer Verbindung der Formel

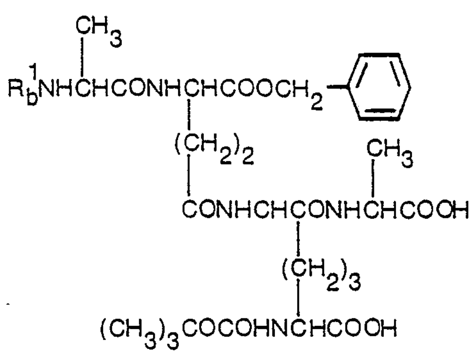

oder ihres Salzes

mit einem Methylierungsmittel unter Bildung einer Verbindung der Formel

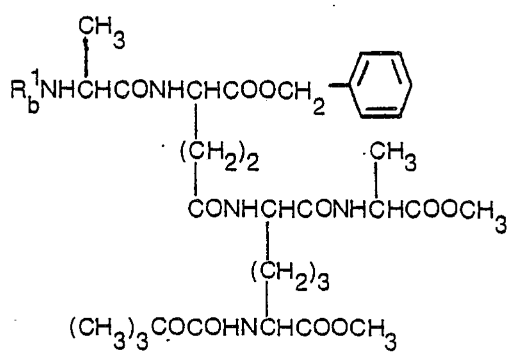

oder ihres Salzes
worin
$R_b^1$ n-Docosanoyl oder n-Tetracosanoyl ist oder Reaktion einer Verbindung der Formel

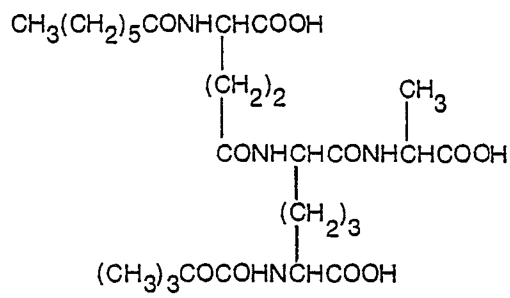

oder ihres Salzes
mit einem Methylierungsmittel unter Bildung einer Verbindung der Formel

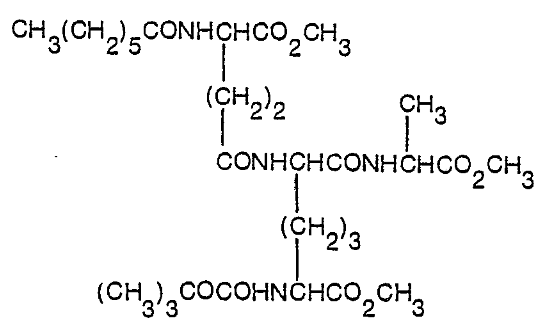

oder ihres Salzes
oder Reaktion einer Verbindung der Formel

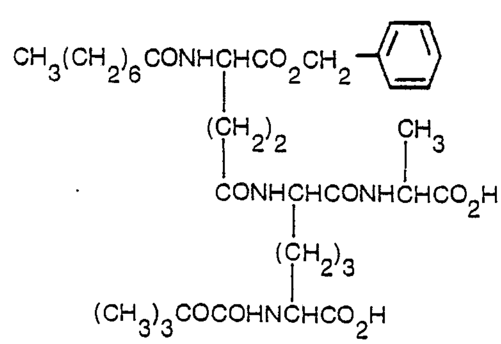

oder ihres Salzes

mit einem Methylierungsmittel unter Bildung einer Verbindung der Formel

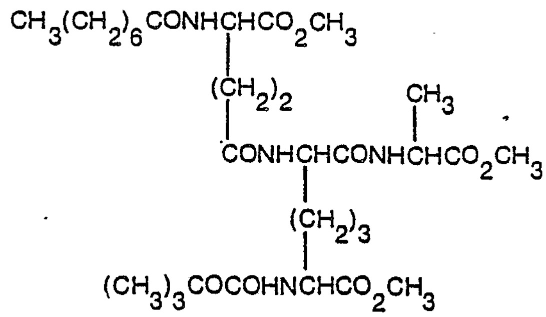

oder ihres Salzes
oder Reaktion einer Verbindung der Formel

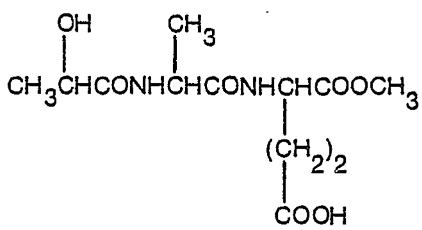

oder ihres Salzes
mit einer Verbindung der Formel

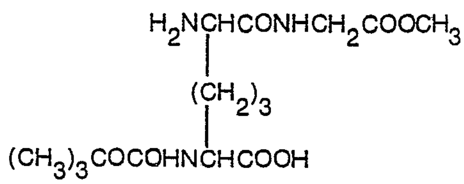

oder ihrem Salz
und Unterwerfen der resultierenden Verbindung der Eliminierungsreaktion für die Schutzgruppe, unter Bildung einer Verbindung der Formel

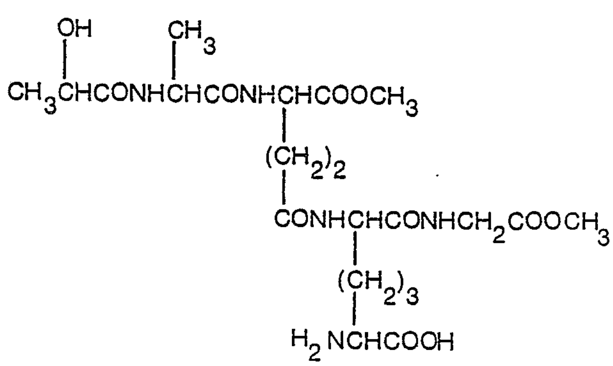

oder ihres Salzes.

7. Verfahren zur Herstellung einer Verbindung der Formel

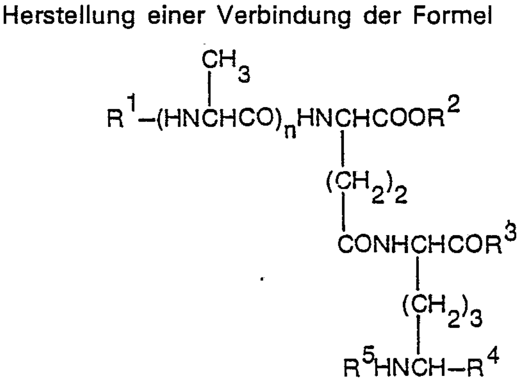

oder ihres pharmazeutisch brauchbaren Salzes, worin

$R^1$ n-Octanoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Stearoyl ist, n eine ganze Zahl von 0 ist, $R^2$ Wasserstoff ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 0 ist, $R^2$ Wasserstoff ist, $R^3$ 1-Carboxyethylamino ist, $R^4$ Ethoxycarbonyl ist und $R^5$ Wasserstoff ist,

durch Unterwerfen einer Verbindung der Formel

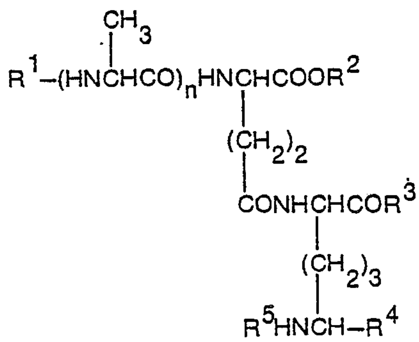

oder ihres Salzes, worin

$R^1$ n-Octanoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ t-Butoxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Butoxycarbonyl ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ Butoxycarbonyl ist; oder

$R^1$ 2,3;4,6-Diisopropyliden-L-2-ketogulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2,3;4,6-Diisopropyliden-2-keto-L-gulonoyl ist, n eine ganze Zahl von 0 ist, $R^2$ Benzyl ist, $R^3$ 1-Carboxyethylamino ist, $R^4$ Carboxy ist und $R^5$ t-Butoxycarbonyl ist; oder

$R^1$ 2-Keto-L-gulonoyl ist, n eine ganze Zahl von 0 ist, $R^2$ Benzyl ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 0 ist, $R^2$ Benzyl ist, $R^3$ 1-Carboxyethylamino ist, $R^4$ Ethoxycarbonyl ist und $R^5$ Benzyloxycarbonyl ist;

der Eliminierungsreaktion von einer oder mehreren Schutzgruppen, unter Bildung einer Verbindung der Formel

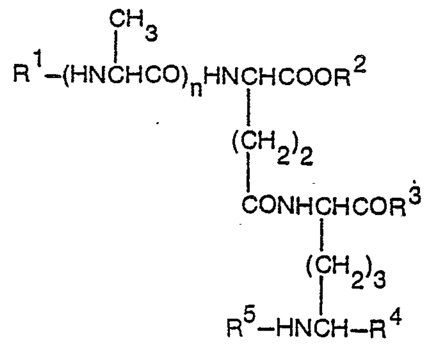

oder ihres Salzes, worin

$R^1$ Octanoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Stearoyl ist, n eine ganze Zahl von 0 ist, $R^2$ Wasserstoff ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 0 ist, $R^2$ Wasserstoff ist, $R^3$ 1-Carboxyethylamino ist, $R^4$ Ethoxycarbonyl ist und $R^5$ Wasserstoff ist.

8. Verfahren zur Herstellung einer Verbindung der Formel

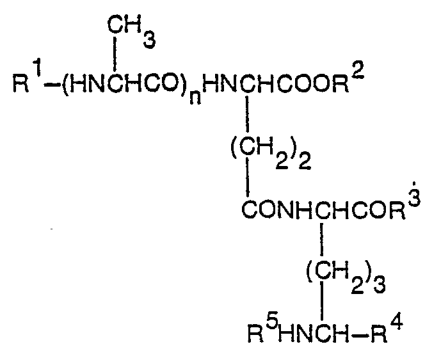

oder ihres pharmazeutisch brauchbaren Salzes, worin

$R^1$ n-Docosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Docosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Tetracosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ 2-Acetoxypropionyl ist, n eine ganze Zahl von 1 ist, $R^2$ Wasserstoff ist, $R^3$ Methoxycarbonylmethylamino ist, $R^4$ Methoxycarbonyl ist und $R^5$ Wasserstoff ist; oder

$R^1$ 2-Hydroxypropionyl ist, n eine ganze Zahl von 1 ist, $R^2$ Wasserstoff ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Wasserstoff ist, $R^3$ Carboxymethylamino ist, $R^4$ Methoxycarbonyl ist und $R^5$ Wasserstoff ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-n-Hexyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ Wasserstoff ist; oder

$R^1$ Stearoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Benzyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist;

durch Unterwerfen einer Verbindung der Formel

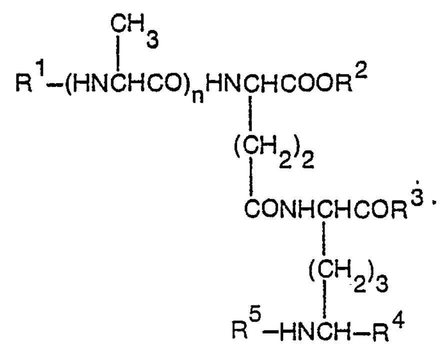

oder ihres Salzes, worin

$R^1$ n-Docosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Docosanoyl ist, n eine ganze Zahl von 1 ist, $R^2$ Benzyl ist, $R^3$ 1-Carboxyethylamino ist, $R^4$ Carboxy ist und $R^5$ t-Butoxycarbonyl ist; oder

$R^1$ n-Tetracosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ Benzyloxycarbonyl ist; oder

$R^1$ 2-Hydroxypropionyl ist, n eine ganze Zahl von 1 ist, $R^2$ Benzyl ist, $R^3$ Methoxycarbonylmethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ 1-N-n-Hexyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-n-Hexy-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ t-Butoxycarbonyl ist; oder

$R^1$ Stearoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Laury-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Benzyl ist, $R^3$ 1-Carboxyethylamino ist, $R^4$ Carboxy ist und $R^5$ Benzyloxycarbonyl ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ t-Butoxycarbonyl ist;

einer Eleminierungsreaktion für eine oder mehrere Schutzgruppen unter Bildung einer Verbindung der Formel

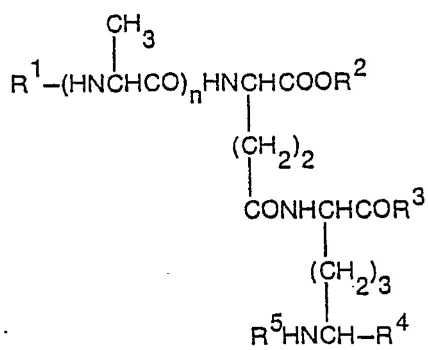

oder ihres Salzes, worin

$R^1$ n-Docosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Docosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Tetracosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ 2-Acetoxypropionyl ist, n eine ganze Zahl von 1 ist, $R^2$ Wasserstoff ist, $R^3$ Methoxycarbonylmethylamino ist, $R^4$ Methoxycarbonyl ist und $R^5$ Wasserstoff ist; oder

$R^1$ 2-Hydroxypropionyl ist, n eine ganze Zahl von 1 ist, $R^2$ Wasserstoff ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Wasserstoff ist, $R^3$ Carboxymethylamino ist, $R^4$ Methoxycarbonyl ist und $R^5$ Wasserstoff ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-n-Hexyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ Wasserstoff ist; oder

$R^1$ Stearoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 1 ist, $R^2$ Wasserstoff ist, $R^3$ 1-Ethoxycarbonylethylamino ist, $R^5$ Ethoxycarbonyl ist und $R^5$ Wasserstoff ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Benzyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist.

9. Pharmazeutische Zusammensetzung, enthaltend eine Verbindung nach Anspruch 3 oder 4, oder ihre pharmazeutisch brauchbaren Salze zusammen mit einem pharmazeutisch brauchbaren, im wesentlichen nichttoxischen Träger oder Exzipienten.

10. Verbindung nach Anspruch 3 oder 4 zur Verwendung als Arzneimittel.

11. Verbindung nach Anspruch 3 oder 4 zur Verwendung bei der Behandlung von Infektionserkrankungen und Tumor.

**Patentansprüche für den Vertragsstaat: AT**

1. Verfahren zur Herstellung einer Verbindung der Formel

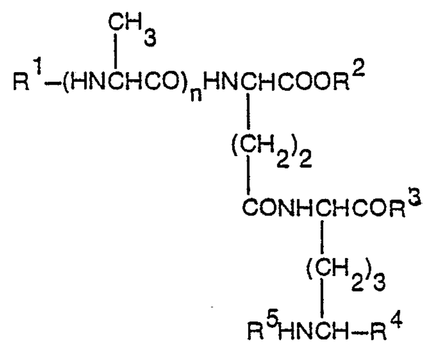

oder ihres pharmazeutisch brauchbaren Salzes, worin

$R^1$ n-Octanoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ t-Butoxycarbonyl ist; oder

$R^1$ n-Docosanoyl ist, n eine ganze Zahl von 1 ist, $R^2$ Benzyl ist, $R^3$ 1-Carboxyethylamino ist, $R^4$ Carboxy ist und $R^5$ t-Buxotycarbonyl ist; oder

$R^1$ n-Docosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Tetracosanoyl ist, n eine ganze Zahl von 1 ist, $R^2$ Benzyl ist, $R^3$ 1-Carboxyethylamino ist, $R^4$ Carboxy ist und $R^5$ t-Buxotycarbonyl ist; oder

$R^1$ n-Tetracosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Buxotycarbonyl ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ t-Butoxycarbonyl ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ Benzyloxycarbonyl ist; oder

$R^1$ 2-Acetoxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Buxotycarbonyl ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Butoxycarbonyl ist; oder

$R^1$ 2-Hydroxypropionyl ist, n eine ganze Zahl von 1 ist, $R^2$ Benzyl ist, $R^3$ Methoxycarbonylmethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ 1-N-n-Hexyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ t-Buxotycarbonyl ist; oder

$R^1$ 2-Hydroxypropionyl ist, n eine ganze Zahl von 1 ist, $R^2$ Methyl, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ t-Butoxycarbonyl ist; oder

$R^1$ 2,3; 4,6-Diisopropyliden-L-2-ketogulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2,3; 4,6-Diisopropyliden-2-keto-L-gulonoyl ist, n eine ganze Zahl von 0 ist, $R^2$ Benzyl ist, $R^3$ 1-Carboxyethylamino ist, $R^4$ Carboxy ist und $R^5$ t-Butoxycarbonyl ist; oder

$R^1$ Stearoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Lauryl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ Palmitoyl ist, n eine ganze Zahl von 1 ist, $R^2$ Benzyl ist, $R^3$ 1-Carboxyethylamino ist, $R^4$ Carboxy ist und $R^5$ Benzyloxycarbonyl ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ t-Buxotycarbonyl ist; oder

$R^1$ Heptanoyl ist, n eine ganze Zahl von 0 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 0 ist, $R^2$ Benzyl ist, $R^3$ 1-Carboxyethylamino ist, $R^4$ Ethoxycarbonyl ist und $R^5$ Benzyloxycarbonyl ist;

durch Reaktion einer Verbindung der Formel

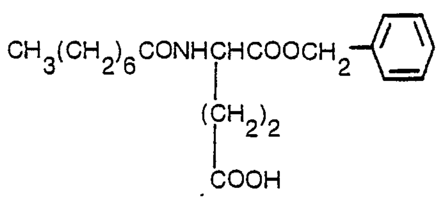

oder ihres Salzes,
mit einer Verbindung der Formel

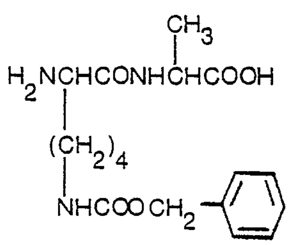

oder ihrem Salz,
unter Bildung einer Verbindung der Formel

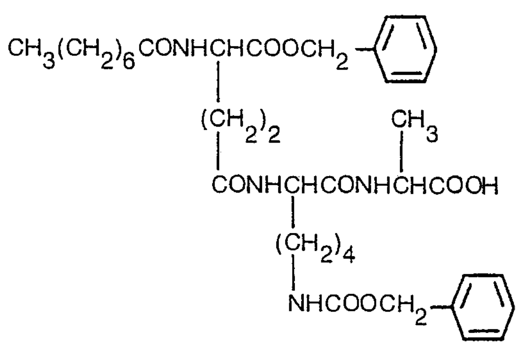

oder ihres Salzes oder

Reaktion einer Verbindung der Formel

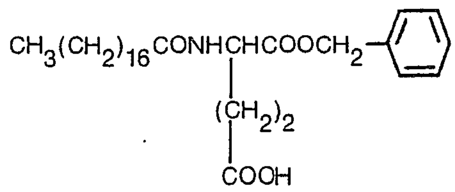

oder ihres Salzes, mit einer Verbindung der Formel

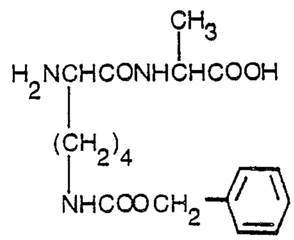

oder ihrem Salz, unter Bildung einer Verbindung der Formel

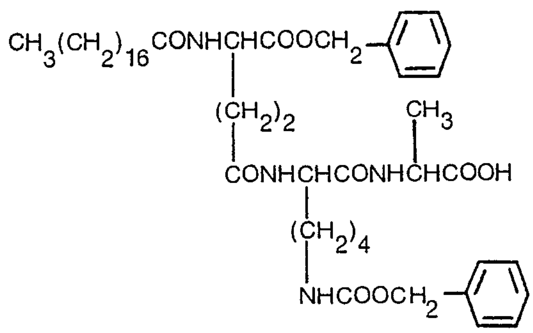

oder ihres Salzes, oder Reaktion einer Verbindung der Formel

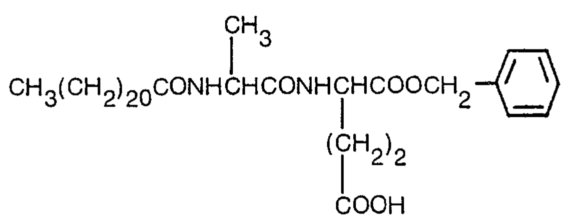

oder ihres Salzes, mit einer Verbindung der Formel

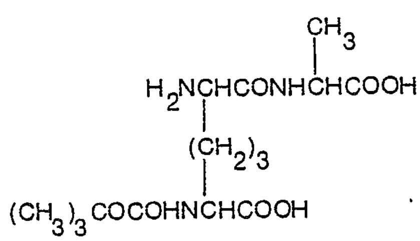

oder ihrem Salz,

unter Bildung einer Verbindung der Formel

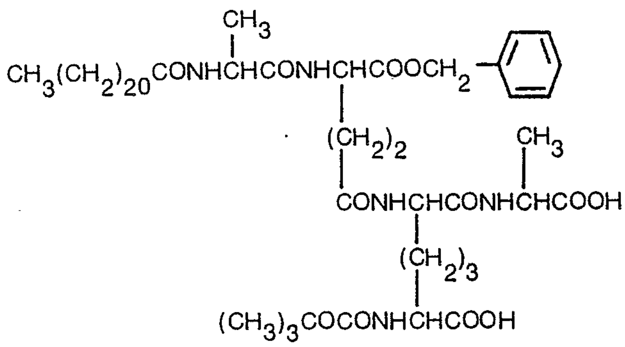

oder ihres Salzes
oder Reaktion einer Verbindung der Formel

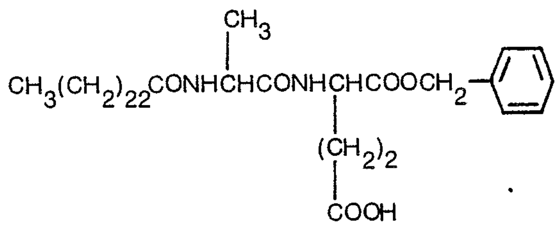

oder ihres Salzes
mit einer Verbindung der Formel

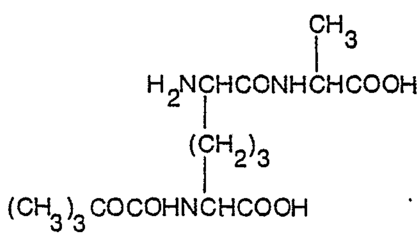

oder ihrem Salz
unter Bildung einer Verbindung der Formel

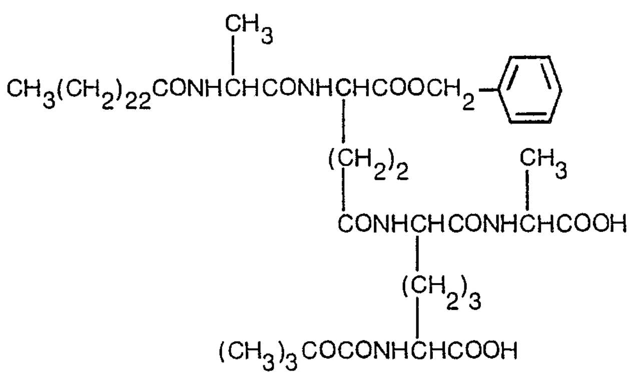

oder ihres Salzes
oder Reaktion einer Verbindung der Formel

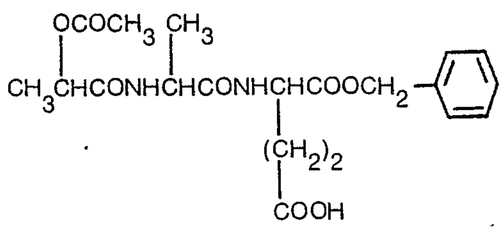

oder ihres Salzes

mit einer Verbindung der Formel

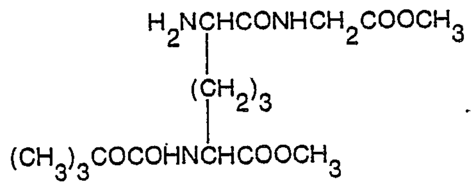

oder ihrem Salz
unter Bildung einer Verbindung der Formel

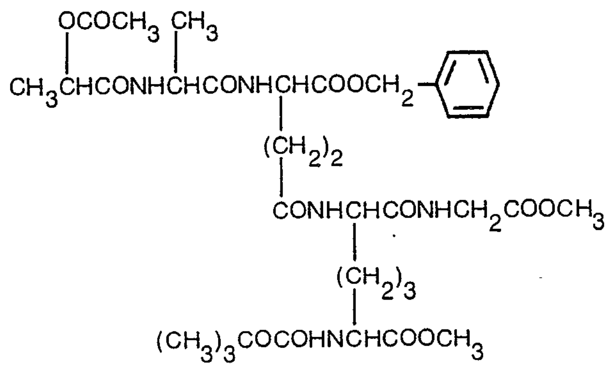

oder ihres Salzes
oder Reaktion einer Verbindung der Formel

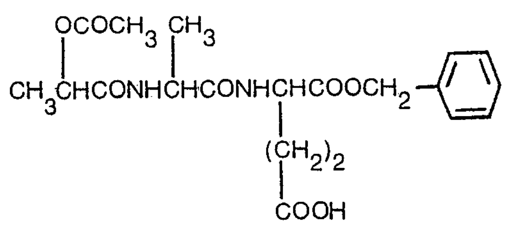

oder ihres Salzes
mit einer Verbindung der Formel

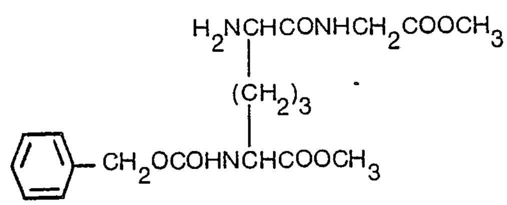

oder ihrem Salz
unter Bildung einer Verbindung der Formel

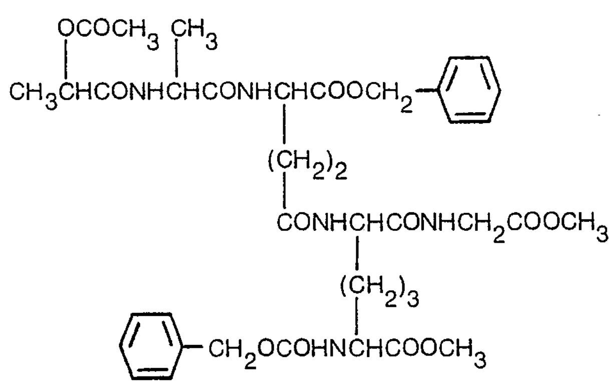

oder ihres Salzes

oder Reaktion einer Verbindung der Formel

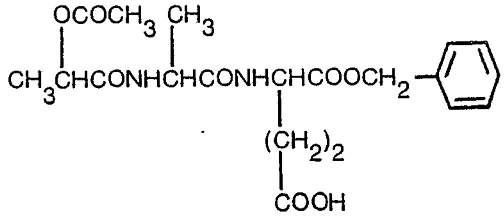

oder ihres Salzes
mit einer Verbindung der Formel

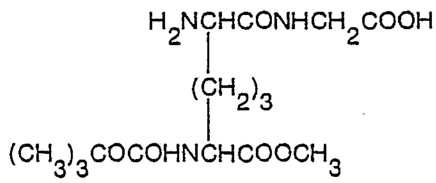

oder ihrem Salz
unter Bildung einer Verbindung der Formel

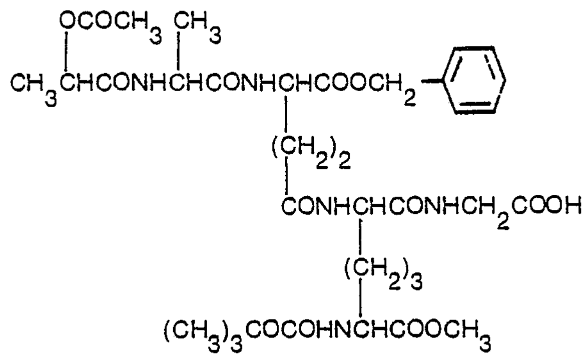

oder ihres Salzes
oder Reaktion einer Verbindung der Formel

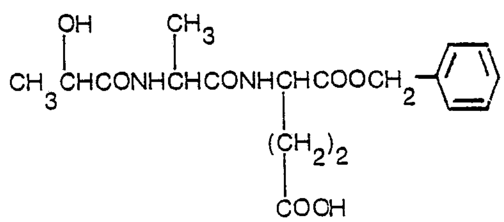

oder ihres Salzes
mit einer Verbindung der Formel

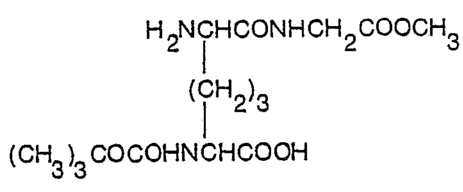

oder ihrem Salz

unter Bildung einer Verbindung der Formel

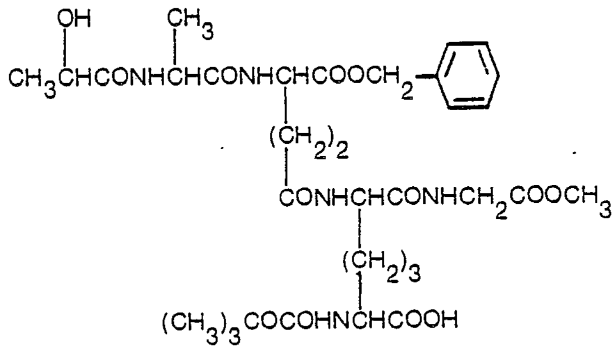

oder ihres Salzes
oder Reaktion einer Verbindung der Formel

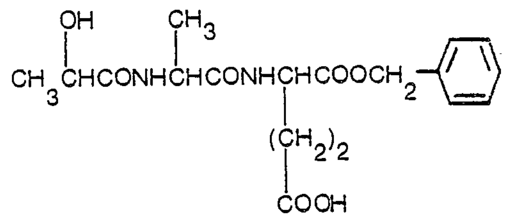

oder ihres Salzes
mit einer Verbindung der Formel

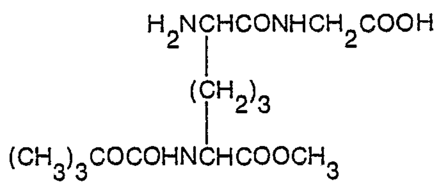

oder ihrem Salz
unter Bildung einer Verbindung der Formel

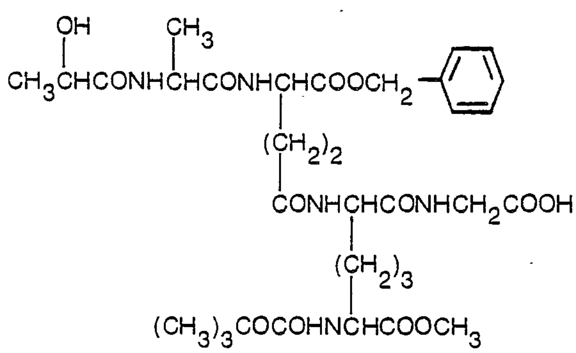

oder ihres Salzes
oder Reaktion einer Verbindung der Formel

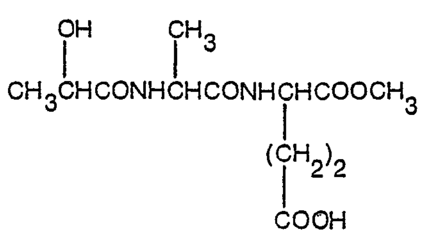

oder ihres Salzes

mit einer Verbindung der Formel

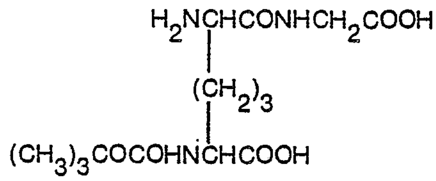

oder ihrem Salz
unter Bildung einer Verbindung der Formel

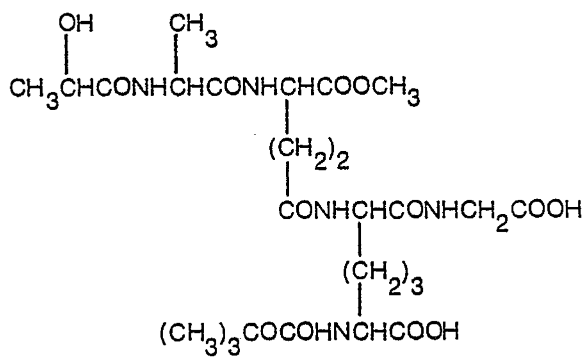

oder ihres Salzes
oder Reaktion einer Verbindung der Formel

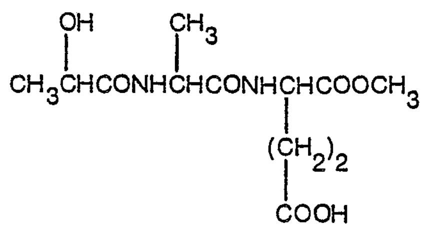

oder ihres Salzes
mit einer Verbindung der Formel

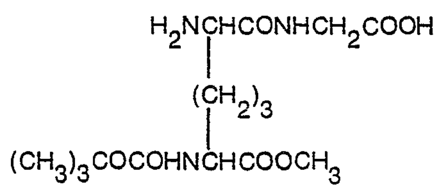

oder ihrem Salz
unter Bildung einer Verbindung der Formel

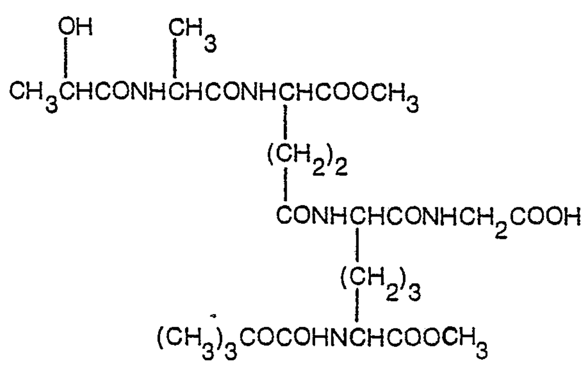

oder ihres Salzes

oder Reaktion einer Verbindung der Formel

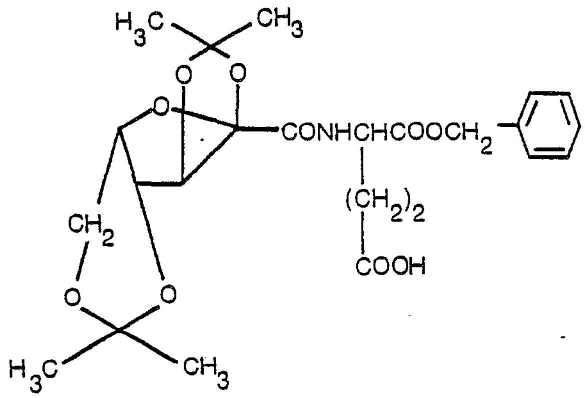

oder ihres Salzes
mit einer Verbindung der Formel

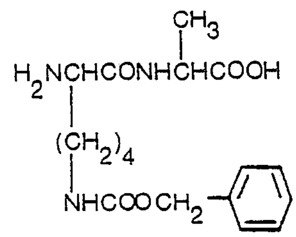

oder ihrem Salz
unter Bildung einer Verbindung der Formel

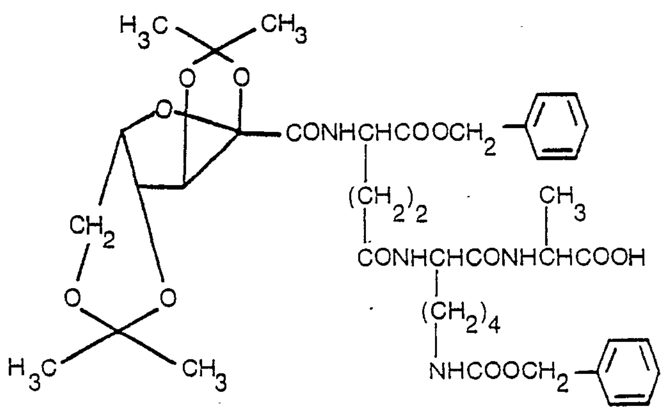

oder ihres Salzes
oder Reaktion einer Verbindung der Formel

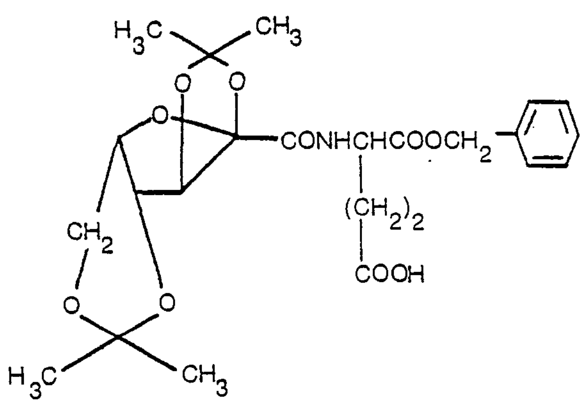

oder ihres Salzes

mit einer Verbindung der Formel

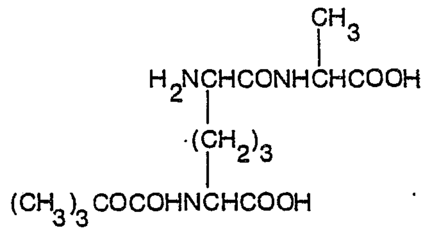

oder ihrem Salz
unter Bildung einer Verbindung der Formel

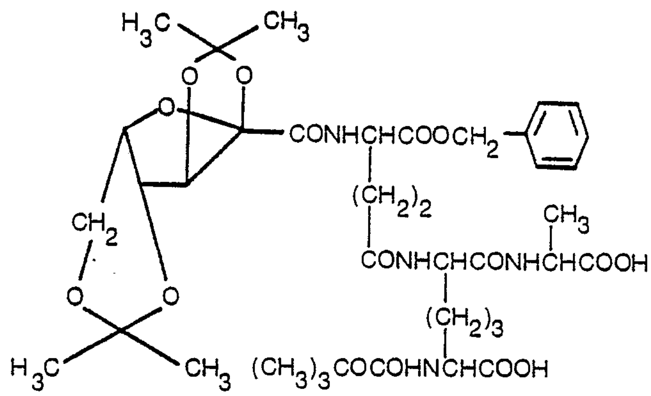

oder ihres Salzes
oder Reaktion einer Verbindung der Formel

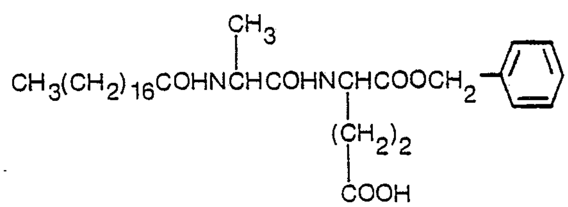

oder ihres Salzes
mit einer Verbindung der Formel

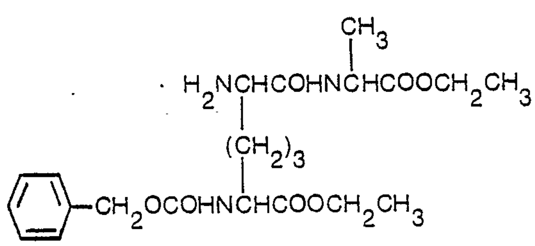

oder ihrem Salz
unter Bildung einer Verbindung der Formel

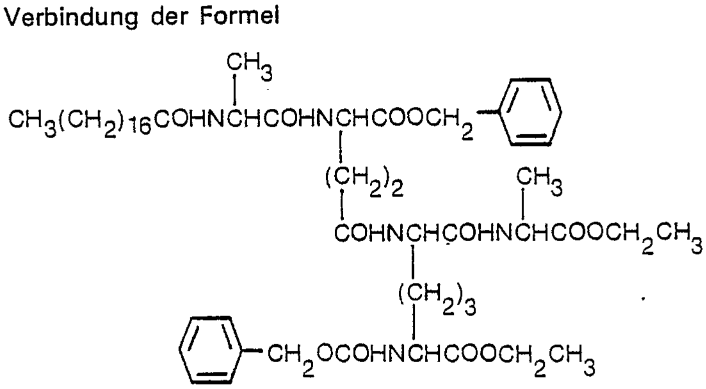

oder ihres Salzes

oder Reaktion einer Verbindung der Formel

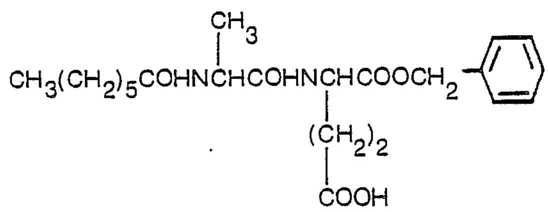

oder ihres Salzes
mit einer Verbindung der Formel

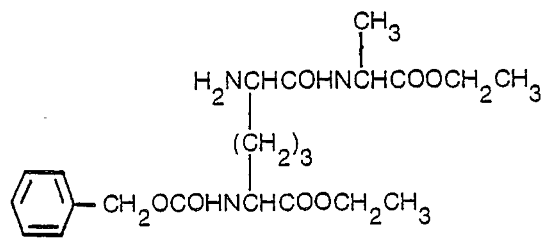

oder ihrem Salz
unter Bildung einer Verbindung der Formel

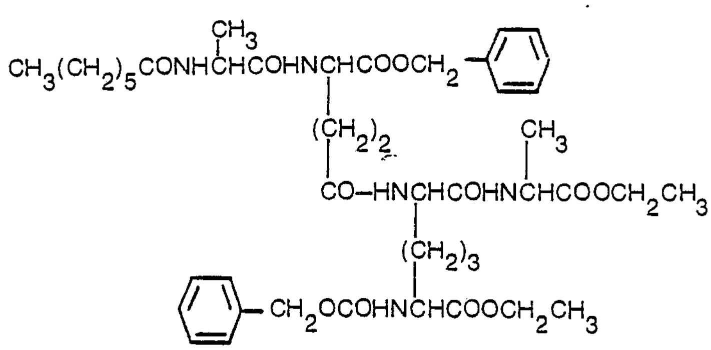

oder ihres Salzes
oder Reaktion einer Verbindung der Formel

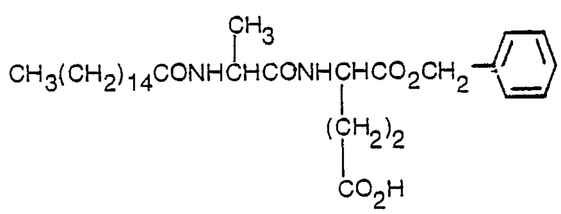

oder ihres Salzes
mit einer Verbindung der Formel

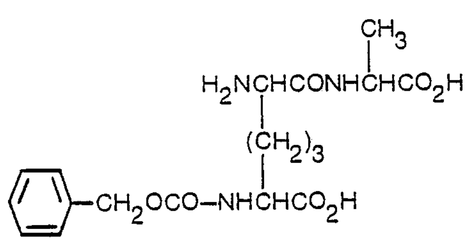

oder ihrem Salz

unter Bildung einer Verbindung der Formel

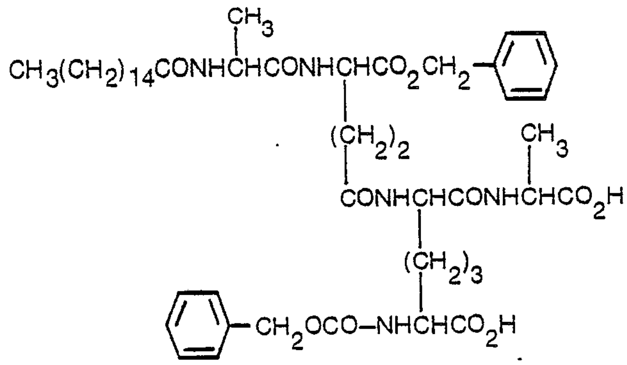

oder ihres Salzes
oder Reaktion einer Verbindung der Formel

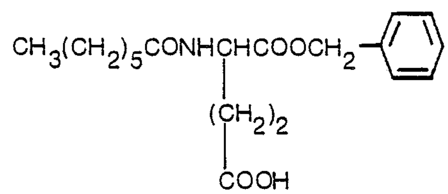

oder ihres Salzes
mit einer Verbindung der Formel

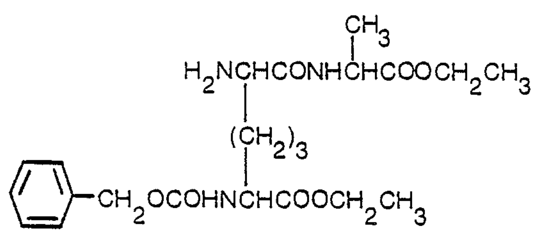

oder ihrem Salz
unter Bildung einer Verbindung der Formel

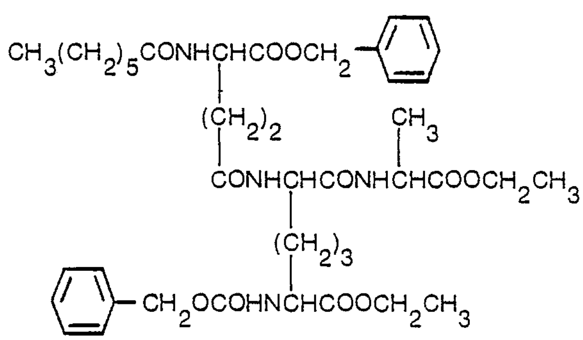

oder ihres Salzes
oder Reaktion einer Verbindung der Formel

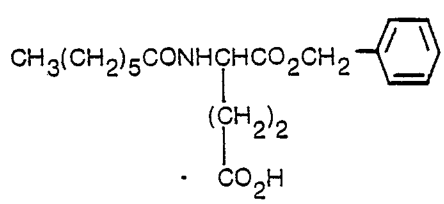

oder ihres Salzes

mit einer Verbindung der Formel

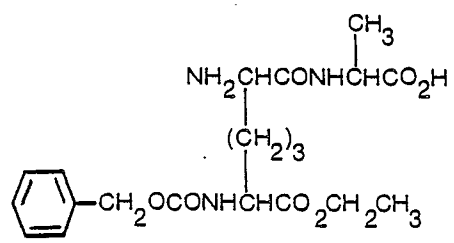

oder ihrem Salz
unter Bildung einer Verbindung der Formel

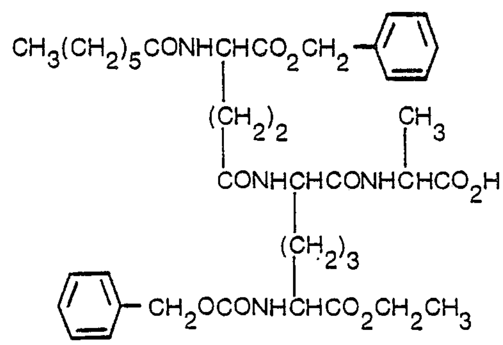

oder ihres Salzes
oder Reaktion einer Verbindung der Formel

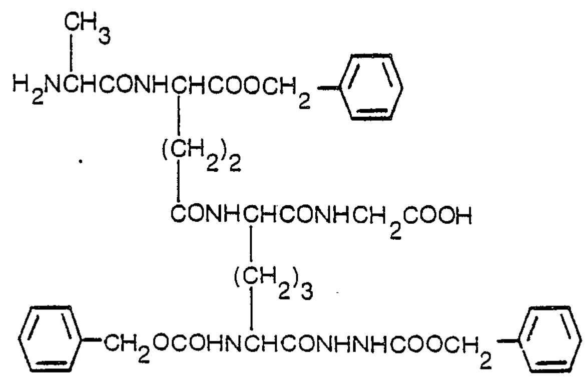

oder ihres Salzes
mit 1 - N - n - Hexyl - 2,3,4,5 - O - tetraacetyl - D - glucaramsäure, 1 - N - Benzyl - 2,3,4,5 - O - tetraacetyl - D - glucaramsäure, 1 - N - Laury - 2,3,4,5 - O - tetraacetyl - D - glucaramsäure oder ihren reaktionsfähigen Derivaten, unter Bildung einer Verbindung der Formel

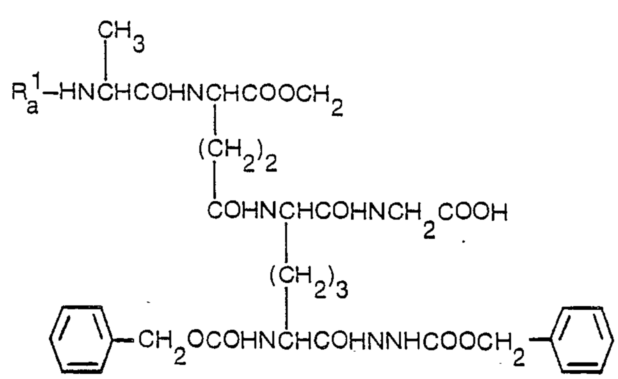

oder ihres Salzes,

worin $R_a^1$ 1 - N - n - Hexyl - 2,3,4,5 - O - tetraacetyl - D - glucaramoyl, 1 - N - Benzyl - 2,3,4,5 - O - tetraacetyl - D - glucaramoyl oder 1 - N - Lauryl - 2,3,4,5 - O - tetraacetyl - D - glucaramoyl ist oder Reaktion einer Verbindung der Formel

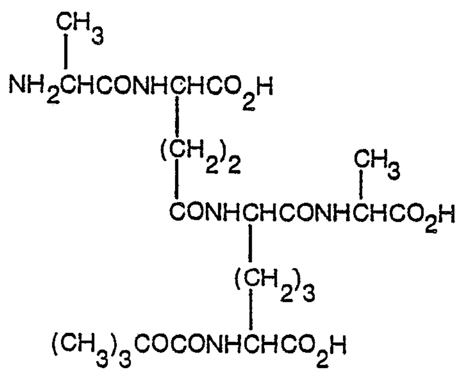

oder ihres Salzes mit Triacontanoesäure oder ihrem reaktiven Derivat, unter Bildung einer Verbindung der Formel

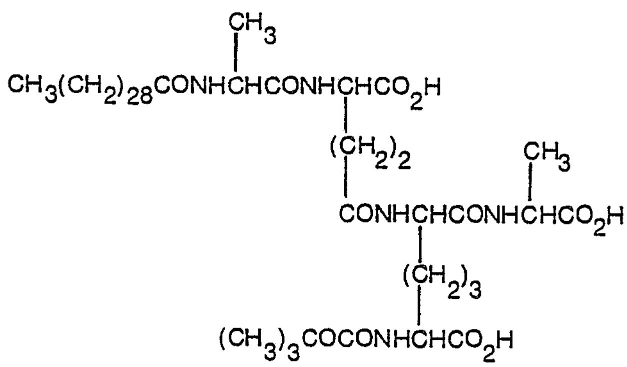

oder ihres Salzes
oder Reaktion einer Verbindung der Formel

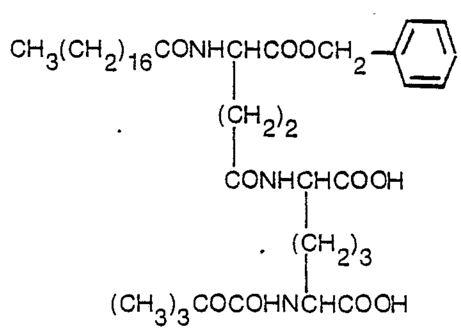

oder ihres Salzes, mit einer Methylierungsmittel, unter Bildung einer Verbindung der Formel

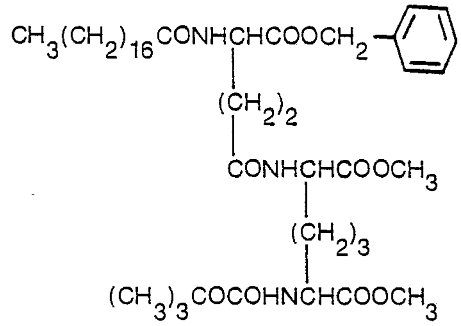

oder ihres Salzes,

oder Reaktion einer Verbindung der Formel

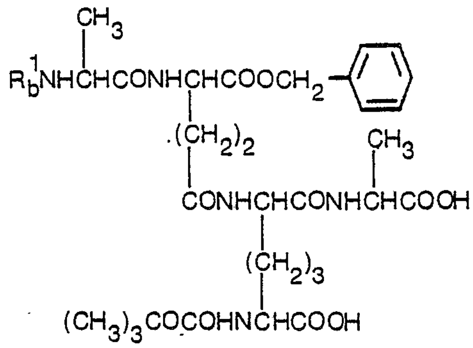

oder ihres Salzes mit einer Methylierungsmittel unter Bildung einer Verbindung der Formel

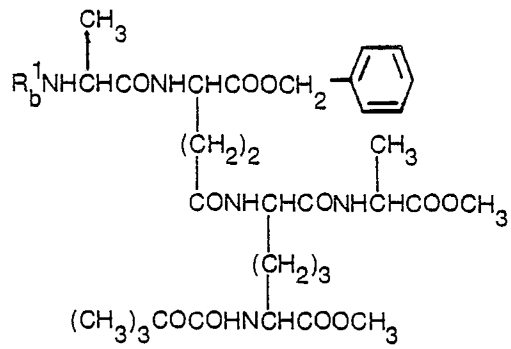

oder ihres Salzes worin $R_b^1$ n-Docosanoyl oder n-Tetracosanoyl ist oder Reaktion einer Verbindung der Formel

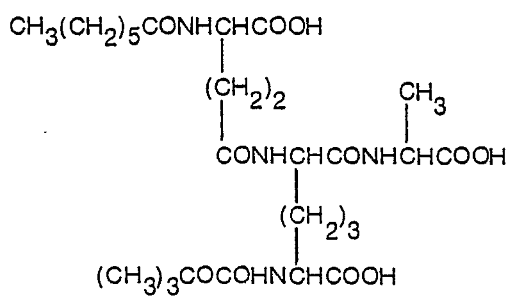

oder ihres Salzes mit einem Methylierungsmittel unter Bildung einer Verbindung der Formel

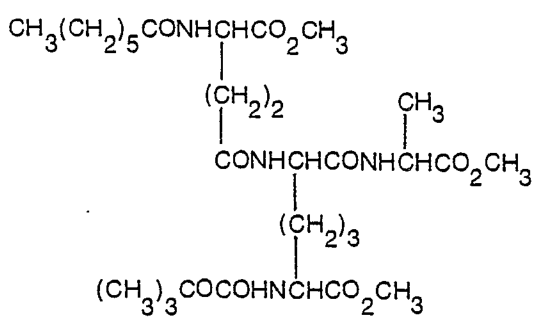

oder ihres Salzes

oder Reaktion einer Verbindung der Formel

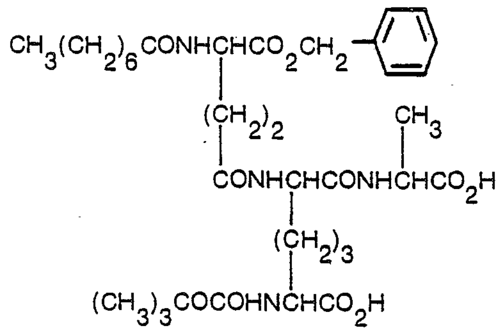

oder ihres Salzes mit einem Methylierungsmittel unter Bildung einer Verbindung der Formel

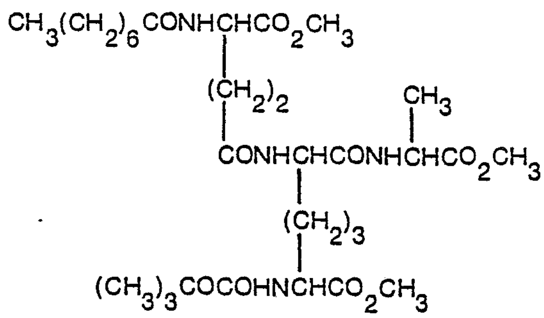

oder ihres Salzes
oder Reaktion einer Verbindung der Formel

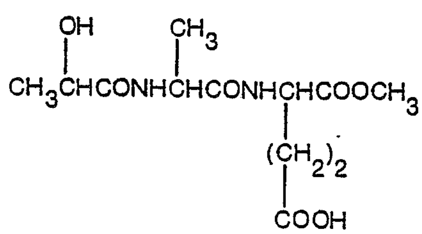

oder ihres Salzes mit einer Verbindung der Formel

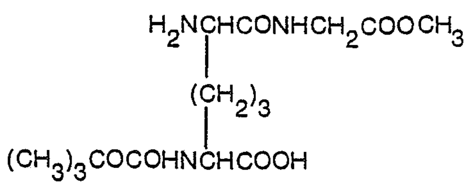

oder ihrem salz
und Unterwerfen der resultierenden Verbindung der Eliminierungsreaktion für die Schutzgruppe, unter Bildung einer Verbindung der Formel

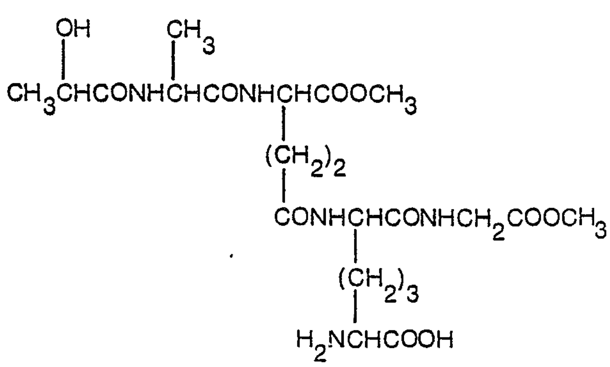

oder ihres Salzes.

2. Verfahren zur Herstellung einer Verbindung der Formel

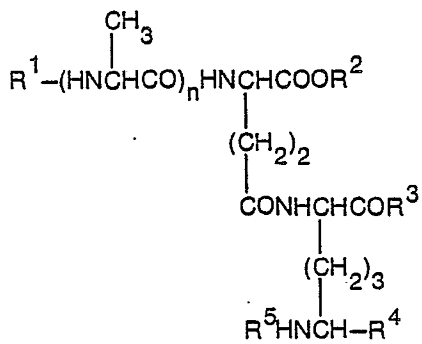

oder ihres pharmazeutisch brauchbaren Salzes,
worin

$R^1$ n-Octanoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Stearoyl ist, n eine ganze Zahl von 0 ist, $R^2$ Wasserstoff ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 0 ist, $R^2$ Wasserstoff ist, $R^3$ 1-Carboxyethylamino ist, $R^4$ Ethoxycarbonyl ist und $R^5$ Wasserstoff ist;

durch Unterwerfen einer Verbindung der Formel

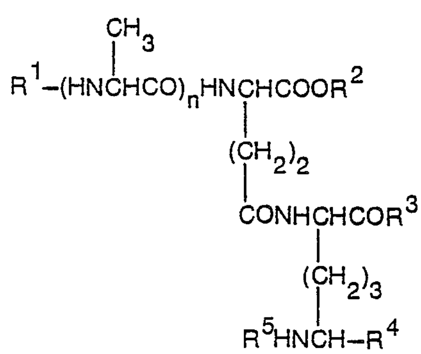

oder ihres Salzes,
worin

$R^1$ n-Octanoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ 1-Carboxyethylamino ist, $R^4$ Wasserstoff ist und $R^5$ Benzyloxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ t-Butoxycarbonyl ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ t-Butoxycarbonyl ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ Butoxycarbonyl ist; oder

$R^1$ 2,3; 4,6-Diisopropyliden-L-2-ketogulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Benzyl ist, $R^3$ 1-Carboxyethylamino ist, $R^4$ Wasserstoff ist und $R^5$ Wasserstoff ist; oder

$R^1$ 2,3; 4,6-Diisopropyliden-2-keto-L-gulonoyl ist, n eine ganze Zahl von 0 ist, $R^2$ Benzyl ist, $R^3$ 1-Carboxyethylamino ist, $R^4$ Carboxy ist und $R^5$ t-Buxotycarbonyl ist; oder

$R^1$ 2-Keto-L-gulonoyl ist, n eine ganze Zahl von 0 ist, $R^2$ Benzyl ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 0 ist, $R^2$ Benzyl ist, $R^3$ 1-Carboxyethylamino ist, $R^4$ Ethoxycarbonyl ist und $R^5$ Benzyloxycarbonyl ist;

der Eliminierungsreaktion von einen oder mehreren Schutzgruppen, unter Bildung einer Verbindung der Formel

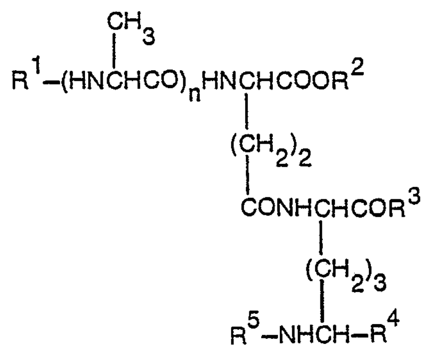

oder ihres Salzes,
worin

$R^1$ n-Octanoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ Stearoyl ist, n eine ganze Zahl von 0 ist, $R^2$ Benzyl ist, $R^3$ Methoxy ist, $R^4$ Methoxycarbonyl ist und $R^5$ Wasserstoff ist; oder

$R^1$ Stearoyl ist, n eine ganze Zahl von 0 ist, $R^2$ Wasserstoff ist, $R^3$ Methoxy ist, $R^4$ Methoxycarbonyl ist und $R^5$ Wasserstoff ist; oder

$R^1$ Heptanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ Octanoyl ist, n eine ganze Zahl von 0 ist, $R^2$ Methyl ist, $R^3$ 1-Methoxycarbonylethylamino ist, $R^4$ Methoxycarbonyl ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Wasserstoff ist und $R^5$ Wasserstoff ist; oder

$R^1$ 2-Keto-L-gulonoyl ist, n eine ganze Zahl von 0 ist, $R^2$ Wasserstoff ist, $R^3$ 1-Carboxyethylamino ist, $R^4$ Carboxy ist und $R^5$ Wasserstoff ist; oder

$R^1$ Heptanoyl ist, n eine ganze Zahl von 0 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 0 ist, $R^2$ Wasserstoff ist, $R^3$ 1-Carboxyethylamino ist, $R^4$ Ethoxycarbonyl ist und $R^5$ Wasserstoff ist.

3. Verfahren zur Herstellung einer Verbindung der Formel

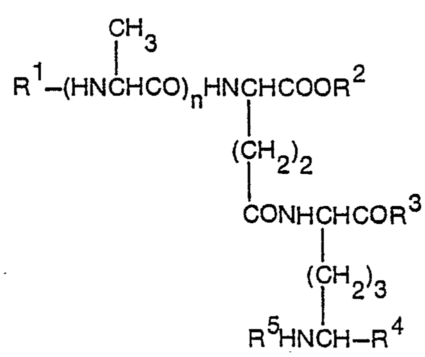

oder ihres pharmazeutisch brauchbaren Salzes,
worin

$R^1$ n-Docosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Docosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Tetracosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ 2-Acetoxypropionyl ist, n eine ganze Zahl von 1 ist, $R^2$ Wasserstoff ist, $R^3$ Methoxycarbonylmethylamino ist, $R^4$ Methoxycarbonyl ist und $R^5$ Wasserstoff ist; oder

$R^1$ 2-Hydroxypropionyl ist, n eine ganze Zahl von 1 ist, $R^2$ Wasserstoff ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Wasserstoff ist, $R^3$ Carboxymethylamino ist, $R^4$ Methoxycarbonyl ist und $R^5$ Wasserstoff ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-n-Hexyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ Wasserstoff ist; oder

$R^1$ Stearoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Benzyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist;

durch Unterwerfen einer Verbindung der Formel

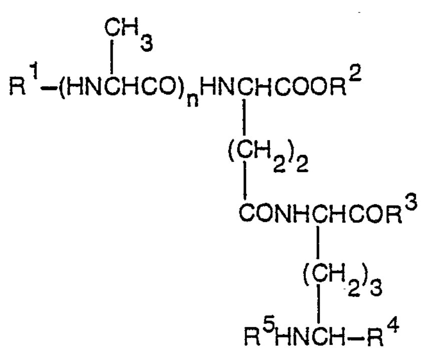

oder ihres Salzes,

worin

$R^1$ n-Docosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Docosanoyl ist, n eine ganze Zahl von 1 ist, $R^2$ Benzyl ist, $R^3$ 1-Carboxyethylamino ist, $R^4$ Carboxy ist und $R^5$ t-Butoxycarbonyl ist; oder

$R^1$ n-Tetracosanoyl ist, n eine ganze Zahl von 1 ist, $R^2$ Benzyl ist, $R^3$ 1-Methoxycarbonylethylamino ist, $R^4$ Methoxycarbonyl ist und $R^5$ t-Butoxycarbonyl ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ 2-Acetoxypropionyl ist, n eine ganze Zahl von 1 ist, $R^2$ Benzyl ist, $R^3$ Methoxycarbonylmethylamino ist, $R^4$ Methoxycarbonyl ist und $R^5$ Benzyloxycarbonyl ist; oder

$R^1$ 2-Hydroxypropionyl ist, n eine ganze Zahl von 1 ist, $R^2$ Benzyl ist, $R^3$ Methoxycarbonylmethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Benzyl ist, $R^3$ Carboxymethylamino ist, $R^4$ Methoxycarbonyl ist und $R^5$ t-Butoxycarbonyl ist; oder

$R^1$ 1-N-n-Hexyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-n-Hexy-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ t-Butoxycarbonyl ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ t-Butoxycarbonyl ist; oder

$R^1$ Stearoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ Heptanoyl ist, n eine ganze Zahl von 1 ist, $R^2$ Benzyl ist, $R^3$ 1-Ethoxycarbonylethylamino ist, $R^4$ Ethoxycarbonyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Benzyl ist, $R^3$ Carboxymethylamino ist, $R^4$ 3-Benzyloxycarbonylcarbazoyl ist und $R^5$ Benzyloxycarbonyl ist; oder

$R^1$ 1-N-Laury-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carbazoyl ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Benzyl ist, $R^3$ 1-Carboxyethylamino ist, $R^4$ Carboxy ist und $R^5$ Benzyloxycarbonyl ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ t-Butoxycarbonyl ist;

eine Eleminierungsreaktion für eine oder mehrere Schutzgruppen unter Bildung einer Verbindung der Formel

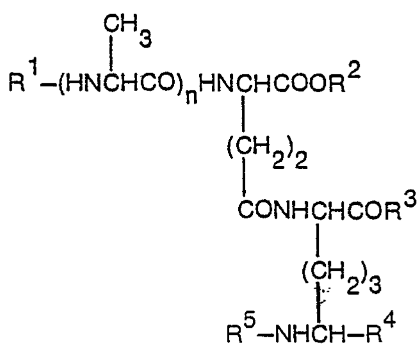

oder ihres Salzes,

worin

$R^1$ n-Docosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Docosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ n-Tetracosanoyl ist, n eine ganze Zahl von 1 ist, $R^2$ Wasserstoff ist, $R^3$ 1-Methoxycarbonylethylamino ist, $R^4$ Methoxycarbonyl ist und $R^5$ Wasserstoff ist; oder

$R^1$ n-Tetracosanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ 2-Acetoxypropionyl ist, n eine ganze Zahl von 1 ist, $R^2$ Wasserstoff ist, $R^3$ Methoxycarbonylmethylamino ist, $R^4$ Methoxycarbonyl ist und $R^5$ Wasserstoff ist; oder

$R^1$ 2-Hydroxypropionyl ist, n eine ganze Zahl von 1 ist, $R^2$ Wasserstoff ist, $R^3$ Methoxycarbonylmethylamino ist, $R^4$ Carboxy ist und $R^5$ Wasserstoff ist; oder

$R^1$ 2-Hydroxypropionyl ist, n eine ganze Zahl von 1 ist, $R^2$ Wasserstoff ist, $R^3$ Carboxymethylamino ist, $R^4$ Methoxycarbonyl ist und $R^5$ Wasserstoff ist; oder

$R^1$ 1-N-n-Hexyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-n-Hexyl-D-glucarmoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Carboxy ist und $R^5$ Wasserstoff ist; oder

$R^1$ 2-Hydroxypropionyl ist, n eine ganze Zahl von 1 ist, $R^2$ Methyl ist, $R^3$ Carboxymethylamino ist, $R^4$ Methoxycarbonyl ist und $R^5$ Wasserstoff ist; oder

$R^1$ Stearoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ Heptanoyl ist, n eine ganze Zahl von 1 ist, $R^2$ Wasserstoff ist, $R^3$ 1-Ethoxycarbonylethylamino ist, $R^4$ Ethoxycarbonyl ist und $R^5$ Wasserstoff ist; oder

$R^1$ 1-N-Benzyl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Benzyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-2,3,4,5-O-tetraacetyl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ 1-N-Lauryl-D-glucaramoyl ist, n eine ganze Zahl von 1 ist, $R^2$ Wasserstoff ist, $R^3$ Carboxymethylamino ist, $R^4$ Carboxy ist und $R^5$ Wasserstoff ist; oder

$R^1$ Palmitoyl ist, n eine ganze Zahl von 1 ist, $R^2$ Wasserstoff ist, $R^3$ 1-Carboxyethylamino ist, $R^4$ Carboxy ist und $R^5$ Wasserstoff ist; oder

$R^1$ Triacontanoyl ist, n eine ganze Zahl von 1 ist, $R^2$ Wasserstoff ist, $R^3$ 1-Carboxyethylamino ist, $R^4$ Carboxy ist und $R^5$ Wasserstoff ist.

**Revendications pour les Etats contractants: BE CH DE FR GB IT LI LU NL SE**

1. Composé de formule:

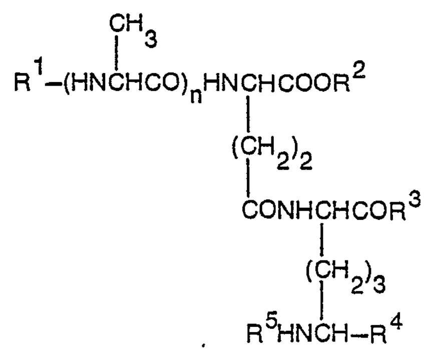

dans laquelle:

$R^1$ est un groupe n-octanoyle, n est un nombre entier nul, $R^2$ est un groupe benzyle, $R^3$ est un groupe carboxy-1-éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un groupe tertiobutoxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un atome d'hydrogène; ou

$R^1$ est un groupe heptanoyle, n est un nombre entier nul, $R^2$ est un groupe d'hydrogène, $R^3$ est un groupe carboxy-1 éthylamino, $R^4$ est un groupe carboxyle et $R^5$ est un groupe tertiobutoxycarbonyle; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe octanoyle, n est un nombre entier nul, $R^2$ est un groupe méthyle, $R^3$ est un groupe méthoxycarbonyl-1 éthylamino, $R^4$ est un groupe éthoxycarbonyle, et $R^5$ est un groupe tertiobutoxycarbonyle; ou

$R^1$ est un groupe diisopropylidène-2,3;4,6- L céto-2 gulonoyle, n est un nombre entier nul, $R^2$ est un groupe benzyle, $R^3$ est un groupe carboxy-1 éthylamino, $R^4$ est un atome d'hydrogène et $R^5$ est un atome d'hydrogène; ou

$R^1$ est un groupe céto-2 L-gulonoyle; n est un nombre entier nul, $R^2$ est un groupe benzyle, $R^3$ est un groupe carboxy-1-éthylamino, $R^4$ est un atome d'hydrogène et $R^5$ est un atome d'hydrogène; ou

$R^1$ est un groupe diisopropylidène-2,3;4,6 céto-2 L-gulonoyle, n est un nombre entier nul, $R^2$ est

un groupe benzyle, $R^3$ est un groupe carboxy-1 éthylamino, $R^4$ est un groupe carboxyle et $R^5$ est un groupe tertiobutoxycarbonyle; ou

$R^1$ est un groupe céto-2 L-gulonoyle, n est un nombre entier nul, $R^2$ est un groupe benzyle, $R^3$ est un groupe carboxy-1 éthylamino, $R^4$ est un groupe carboxyle et $R^5$ est un atome d'hydrogène; ou

$R^1$ est un groupe heptanoyle, n est un nombre entier nul, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe heptanoyle, n est un nombre entier nul, $R^2$ est un groupe benzyle, $R^3$ est un groupe carboxy-1 éthylamino, $R^4$ est un groupe éthoxycarbonyle et $R^5$ est un groupe benzyloxycarbonyle), ou son sel pharmaceutiquement acceptable.

2. Composé de formule:

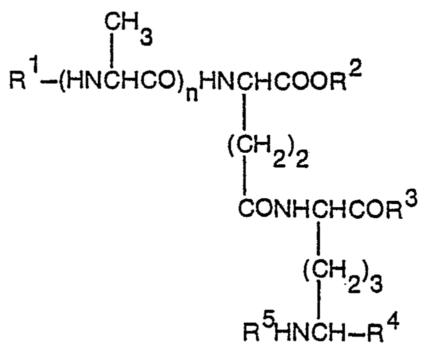

(dans laquelle:

$R^1$ est un groupe n-docosanoyle, n est un nombre entier valant 1, $R^2$ est un groupe benzyle, $R^3$ est groupe carboxy-1 éthylamino, $R^4$ est un groupe carboxyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe n-tétracosanoyle, n est un nombre entier valant 1, $R^2$ est un groupe benzyle, $R^3$ est un groupe carboxy-1 éthylamino, $R^4$ est un groupe carboxyle et $R^5$ est un groupe tertiobutoxycarbonyle; ou

$R^1$ est un groupe n-tétracosanoyle, n est un nombre entier valant 1, $R^2$ est un groupe benzyle, $R^3$ est un groupe méthoxycarbonyl-1 éthylamino, $R^4$ est un groupe méthoxycarbonyle, et $R^5$ est un groupe tertiobutoxycarbonyle; ou

$R^1$ est un groupe acétoxy-2 propionyle, n est nombre entier valant 1, $R^2$ et un groupe benzyle, $R^3$ est un groupe méthoxycarbonylméthylamino, $R^4$ est un groupe méthoxycarbonyle, et $R^5$ est un groupe tertiobutoxycarbonyle; ou

$R^1$ est un groupe acétoxy-2 propionyle, n est nombre entier valant 1, $R^2$ est un groupe benzyle, $R^3$ est un groupe méthoxycarbonylméthylamino, $R^4$ est un groupe méthoxycarbonyle et $R^5$ est un groupe benzyloxycarbonyle; ou

$R^1$ est un groupe acétoxy-2 propionyle, n est un nombre entier valant 1, $R^2$ est un atome d'hydrogène, $R^3$ est un groupe méthoxycarbonylméthylamino, $R^4$ est un groupe méthoxycarbonyle et $R^5$ est un atome d'hydrogène; ou

$R^1$ est un groupe acétoxy-2 propionyle, n est un nombre entier valant 1, $R^2$ est un groupe benzyle, $R^3$ est un groupe carboxyméthylamino, $R^4$ est un groupe méthoxycarbonyle, et $R^5$ est un groupe tertiobutoxycarbonyle; ou

$R^1$ est un groupe acétoxy-2 propionyle, n est un nombre entier valant 1, $R^2$ est un atome d'hydrogène, $R^3$ est un groupe carboxyméthylamino, $R^4$ est un groupe méthoxycarbonyle, et $R^5$ est un groupe tertiobutoxycarbonyle; ou

$R^1$ est un groupe hydroxy-2 propionyle, n est un nombre entier valant 1, $R^2$ est un groupe benzyle, $R^3$ est un groupe méthoxycarbonylméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe benzyle, $R^3$ est un groupe carboxyméthylamino, $R^4$ est un groupe méthoxycarbonyle, et $R^5$ est un groupe tertiobutoxycarbonyle; ou

$R^1$ est un groupe N-n-hexyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle, et $R^5$ est un groupe benzyloxycarbonyle; ou

$R^1$ est un groupe N-n-hexyl-1 O-tétraacétyl-2,3,4,5 D-glucaramoyle, n est nombre entier valant 1, $R^2$ est un atome d'hydrogène, $R^3$ est un groupe carboxyméthylamino, $R^4$ est un groupe carbazoyle et $R^5$ est un atome d'hydrogène; ou

$R^1$ est un groupe N-n-hexyl-1 O-tétraacétyl-2,3,4,5 D-glucaramoyle, n est un nombre entier valant

1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est nombre entier valant 1, $R^2$ est un groupe méthyle, $R^3$ est un groupe carboxyméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle, et $R^5$ est un groupe tertiobutoxycarbonyle; ou

$R^1$ est un groupe stéraoyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un heptanoyle, n est nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-2 carbazoyle, et $R^5$ est un groupe benzyloxycarbonyle; ou

$R^1$ est un groupe N-benzyl-1 O-tétraacétyl-2,3,4,5 D-glucaramoyle, n est nombre entier valant 1, $R^2$ est un atome d'hydrogène, $R^3$ est un groupe carboxyméthylamino, $R^4$ est un groupe carbazoyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-2 carbazoyle, et $R^5$ est un groupe benzyloxycarbonyle; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carbazoyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un groupe benzyle, $R^3$ est un groupe carboxy-1 éthylamino, $R^4$ est un groupe carboxyle et $R^5$ est un groupe benzyloxycarbonyle; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un groupe tertiocarbonyle), ou son sel pharmaceutiquement acceptable.

3. Composé de formule:

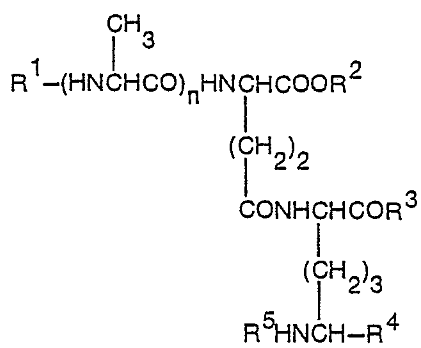

(dans laquelle

$R^1$ est un groupe n-octanoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe méthoxy, $R^4$ est un groupe méthoxycarbonyle et $R^5$ est un atome d'hydrogène; ou

$R^1$ est un groupe heptanoyle, n est nombre entier nul, $R^2$ est un groupe méthyle, $R^3$ est un groupe méthoxycarbonyl-1 éthylamino, $R^4$ est un groupe méthoxycarbonyle et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe heptanoyle, n est nombre entier nul, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est nombre entier nul, $R^2$ est un atome d'hydrogène, $R^3$ est un groupe carboxy-1 méthylamino, $R^4$ est un groupe éthoxycarbonyle et $R^5$ est un atome d'hydrogène) ou son sel pharmaceutiquement acceptable.

4. Composé de formule:

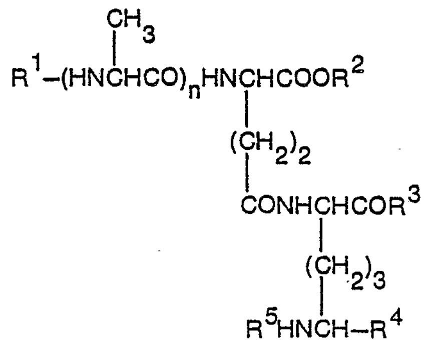

(dans laquelle

$R^1$ est un groupe docosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe n-docosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe n-tétracosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe méthoxycarbonylméthylamino, $R^4$ est un groupe carboxyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe carboxyméthylamino, $R^4$ est un groupe méthoxycarbonyle et $R^5$ est un atome d'hydrogène; ou

$R^1$ est un groupe N-hexyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe méthoxycarbonylméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un atome d'hydrogène; ou

$R^1$ est un groupe stéaroyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe N-benzyl-1 D-glucaromoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ un groupe N lauryl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène) ou son sel pharmaceutiquement acceptable.

5. Composé de formule

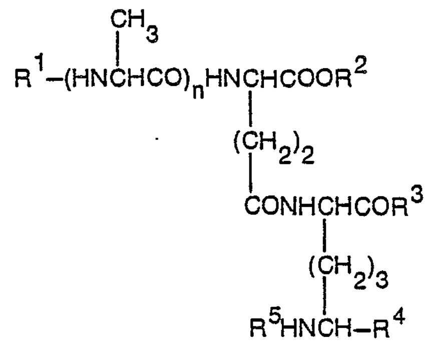

(dans laquelle

$R^1$ est un groupe n-octanoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe méthoxy, $R^4$ est un groupe méthoxycarbonyle, et $R^5$ est un atome d'hydrogène; ou

$R^1$ est un groupe n-docosanoyle, n est un nombre entier valant 1, $R^2$ est un atome d'hydrogène, $R^3$ est un groupe carboxy-1 éthylamino, $R^4$ est un groupe carboxyle, et $R^5$ est un atome d'hydrogène; ou

$R^1$ est un group stéaroyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe palmitoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène), ou son sel pharmaceutiquement acceptable.

6. Procédé pour préparer un composé de formule:

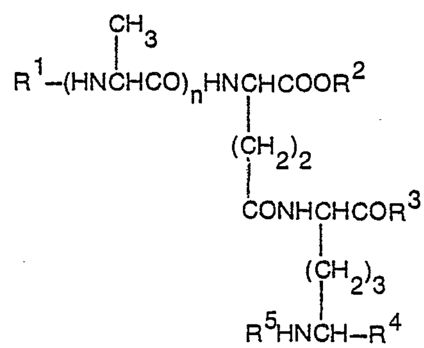

(dans laquelle

$R^1$ est un groupe n-octanoyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe carboxy-1 éthylamino, $R^4$ est un groupe carboxyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe n-tétracosanoyle, n est un nombre entier valant 1, $R^2$ est un groupe benzyle, $R^3$ est un groupe carboxy-1-éthylamino, $R^4$ est un groupe carboxyle et $R^5$ est un groupe tertiobutoxycarbonyle; ou

$R^1$ est un groupe n-tétracosanoyle, n est nombre entier valant 1, $R^2$ est un group benzyle, $R^3$ est un groupe éthoxycarbonyl-1 éthylamino, $R^4$ est un groupe méthoxycarbonyle et $R^5$ est un groupe tertiobutoxycarbonyle; ou

$R^1$ est un groupe acétoxy-2 propionyle, n est un nombre entier valant 1, $R^2$ est un groupe benzyle, $R^3$ est un groupe méthoxycarbonyléthylamino, $R^4$ est un groupe méthoxycarbonyle et $R^5$ est un groupe tertiobutoxycarbonyle; ou

$R^1$ est un groupe acétoxy-2 propionyle, n est un nombre entier valant 1, $R^2$ est un groupe benzyle, $R^3$ est un groupe méthoxycarbonylméthylamino, $R^4$ est un groupe méthoxycarbonyle et $R^5$ est un groupe benzyloxycarbonyle; ou

$R^1$ est un groupe acétoxy-2 propionyle, n est un nombre entier valant 1, $R^2$ est un groupe benzyle, $R^3$ est un groupe carboxyméthylamino, $R^4$ est un groupe méthoxycarbonyle et $R^5$ est un groupe butoxycarbonyle; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe hydroxy-2 propionyle, n est nombre entier valant 1, $R^2$ est un groupe benzyle, $R^3$ est un groupe méthoxycarbonylméthylamino, $R^4$ est un groupe carboxyle, et $R^5$ est un groupe tertiobutoxycarbonyle; ou

$R^1$ est un groupe hydroxy-2 propionyle, n est un nombre entier valant 1, $R^2$ est un groupe benzyle, $R^3$ est un groupe carboxyméthylamino, $R^4$ est un groupe méthoxycarbonyle et $R^5$ est un groupe tertiobutoxycarbonyle; ou

$R^1$ est un groupe N-n-hexyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle, et $R^5$ est un groupe benzyloxycarbonyle; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe méthyle, $R^3$ est un groupe méthoxycarbonylméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un groupe tertiobutoxycarbonyle; ou

$R^1$ est un groupe diisopropylidène-2,3; 4,6- L-céto-2 gulonoyle, n est un nombre entier nul, $R^2$ est un groupe benzyle, $R^3$ est un groupe carboxy-1 éthylamino, $R^4$ est un atome d'hydrogène et $R^5$ est un atome d'hydrogène; ou

$R^1$ est un groupe diisopropylidène-2,3; 4,6 céto-2 L-gulonoyle, n est un nombre entier nul, $R^2$ est un groupe benzoyle, $R^3$ est un groupe carboxy-1 éthylamino, $R^4$ est un groupe carboxyle et $R^5$ est un groupe tertiobutoxycarbonyle; ou

$R^1$ est un groupe stéaroyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe heptanoyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle, et $R^5$ est un groupe benzyloxycarbonyle; ou

$R^1$ est un groupe N-lauryl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle, et $R^5$ est un groupe benzyloxycarbonyle; ou

$R^1$ est un groupe palmitoyle, n est un nombre entier valant 1, $R^2$ est un groupe benzyle, $R^3$ est un groupe carboxy-1 éthylamino, $R^4$ est un groupe carboxyle et $R^5$ est un groupe benzyloxycarbonyle; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un groupe tertiobutoxycarbonyle; ou

$R^1$ est un groupe heptanoyle, n est un nombre entier nul, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe heptanoyle, n est un nombre entier nul, $R^2$ est un groupe benzyle, $R^3$ est un groupe carboxy-1 éthylamino, $R^4$ est un groupe éthoxycarbonyle et $R^5$ est un groupe benzyloxycarbonyle),

ou son sel pharmaceutiquement acceptable,
procédé caractérisé en ce que:

on fait réagir un composé de formule:

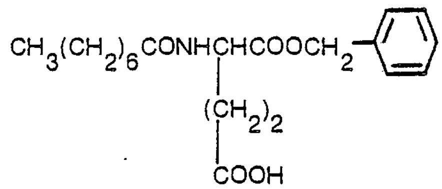

ou son sel, avec un composé de formule:

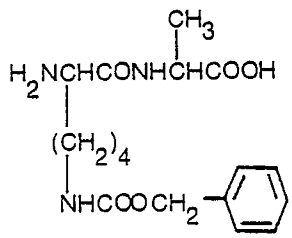

ou son sel, pour obtenir un composé de formule:

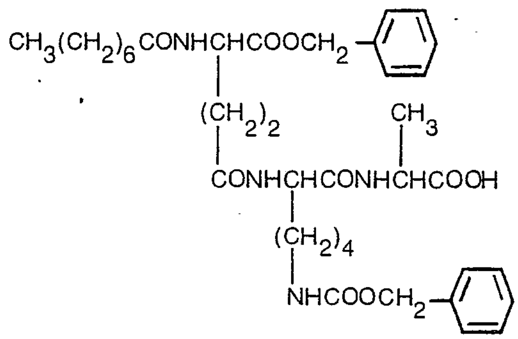

ou son sel, ou bien
on fait réagir un composé de formule:

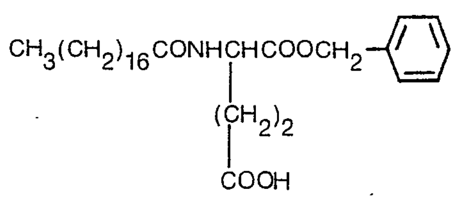

ou son sel, avec un composé de formule:

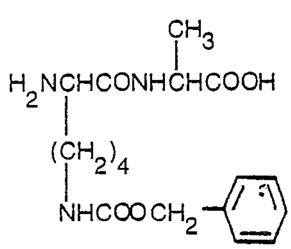

ou son sel, pour obtenir un composé de formule:

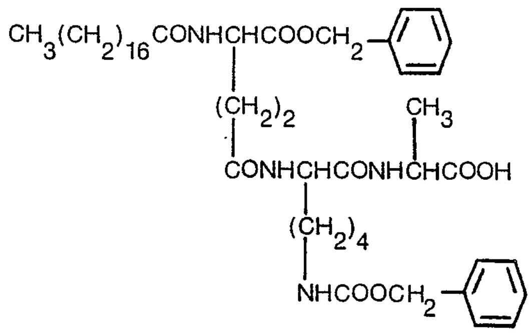

ou son sel; ou bien

on fait réagir un composé de formule:

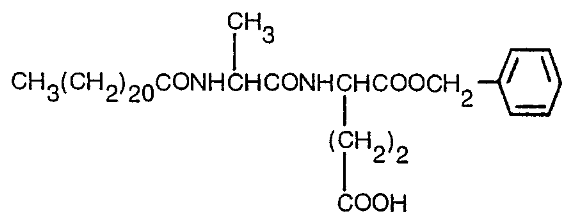

ou son sel, avec un composé de formule:

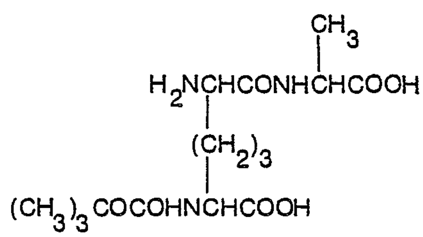

ou son sel, pour obtenir un composé de formule:

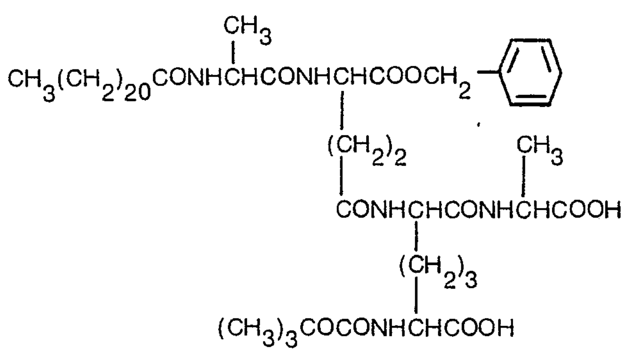

ou son sel; ou bien

on fait réagir un composé de formule:

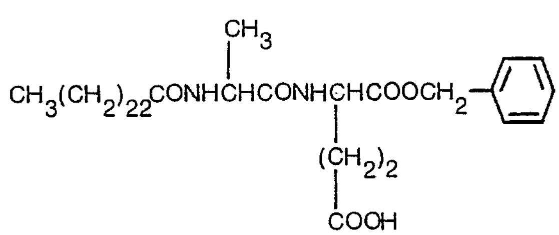

ou son sel, avec un composé de formule:

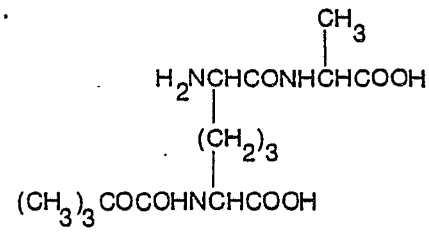

ou son sel, pour obtenir un composé de formule:

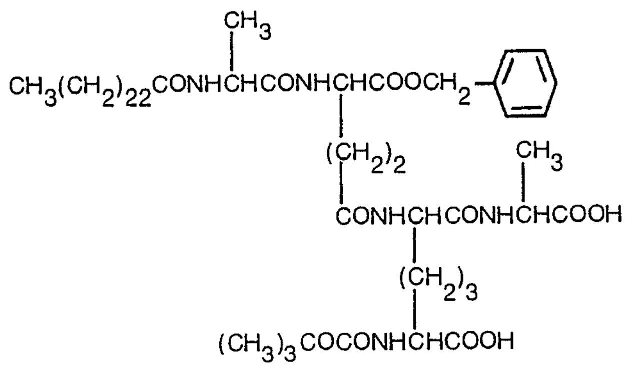

ou son sel, ou bien

on fait réagir un composé de formule:

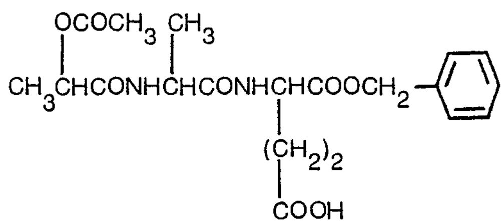

ou son sel, avec un composé de formule:

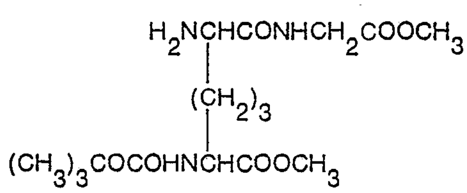

ou son sel, pour obtenir un composé de formule:

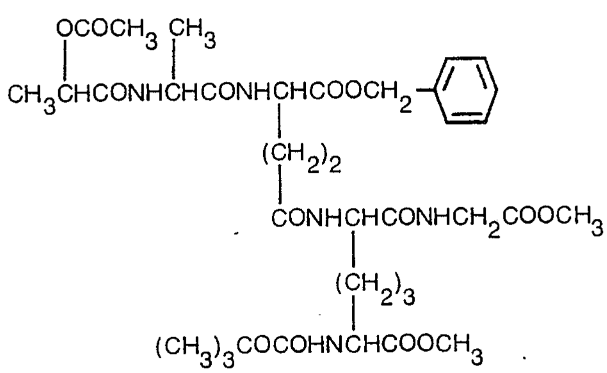

ou son sel, ou bien

on fait réagir un composé de formule:

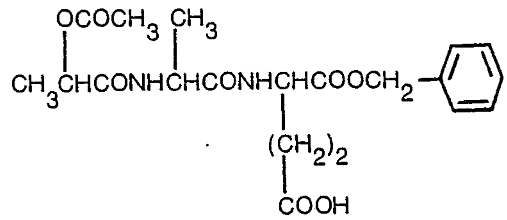

ou son sel, avec un composé de formule:

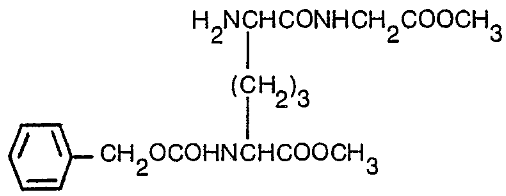

ou son sel, pour obtenir un composé de formule:

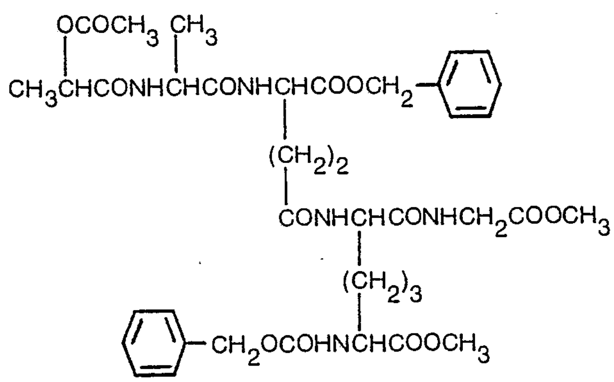

ou son sel, ou bien,
on fait réagir un composé de formule:

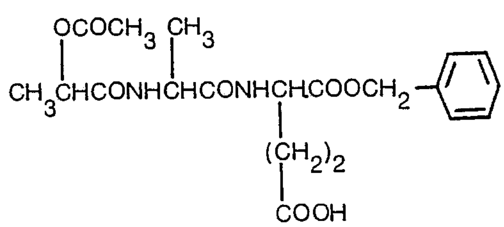

ou son sel, avec un composé de formule:

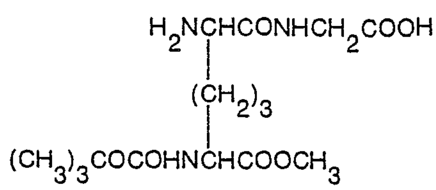

ou son sel, pour obtenir un composé de formule:

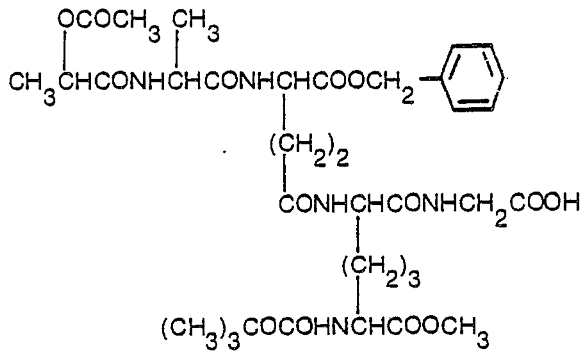

ou son sel, ou bien

on fait réagir un composé de formule:

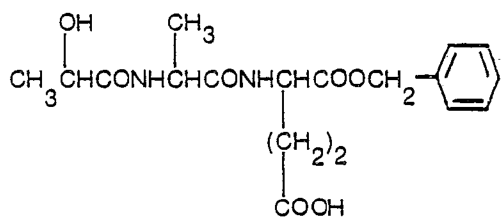

ou son sel, avec un composé de formule:

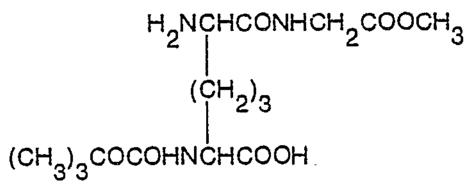

ou son sel, pour obtenir un composé de formule:

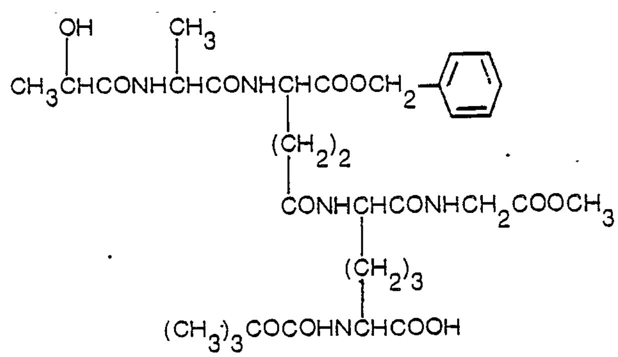

ou son sel; ou bien

on fait réagir un composé de formule:

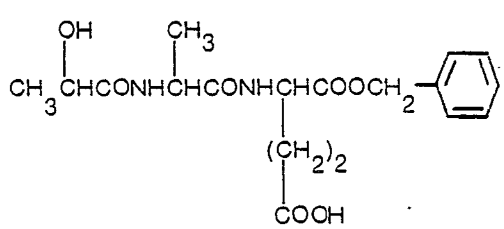

ou son sel, avec un composé de formule:

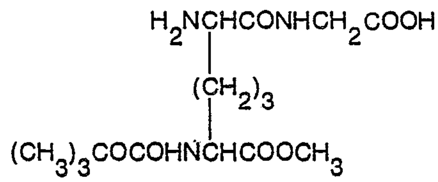

ou son sel, pour obtenir un composé de formule:

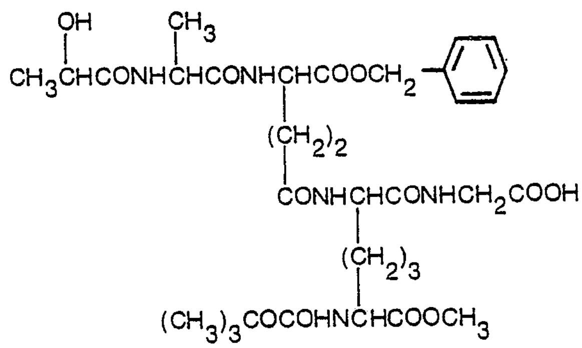

ou son sel; ou bien

on fait réagir un composé de formule:

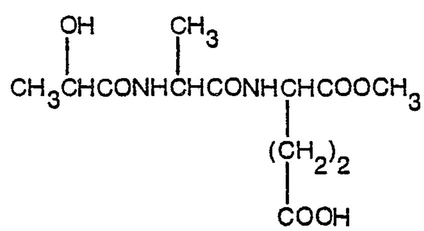

ou son sel, avec un composé de formule:

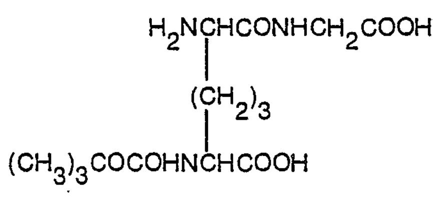

ou son sel, pour obtenir un composé de formule:

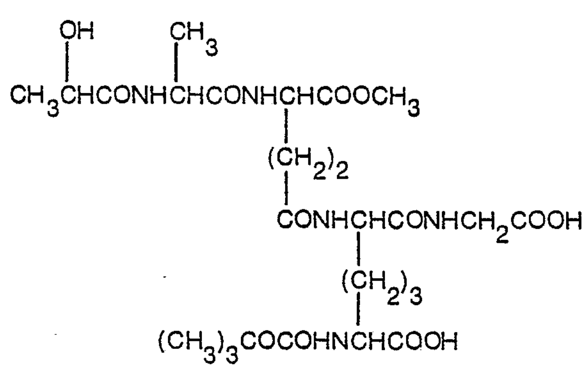

ou son sel; ou bien

on fait réagir un composé de formule:

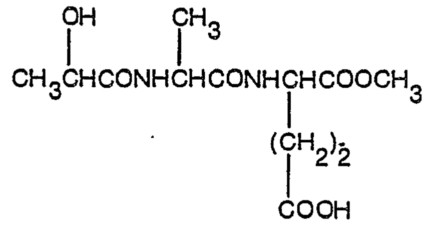

ou son sel, avec un composé de formule:

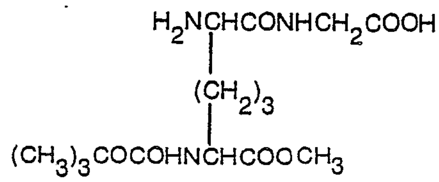

ou son sel, pour obtenir un composé de formule:

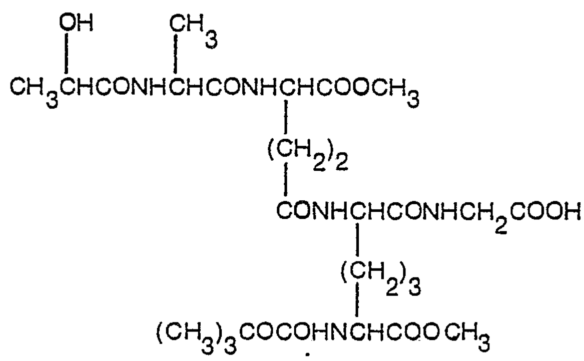

ou son sel, ou bien

on fait réagir un composé de formule:

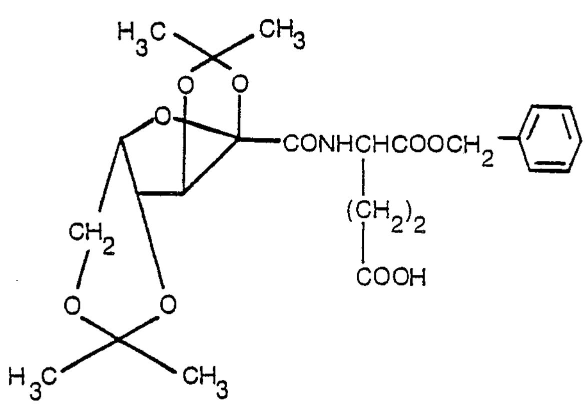

ou son sel, avec un composé de formule:

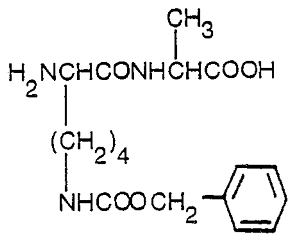

ou son sel, pour obtenir un composé de formule:

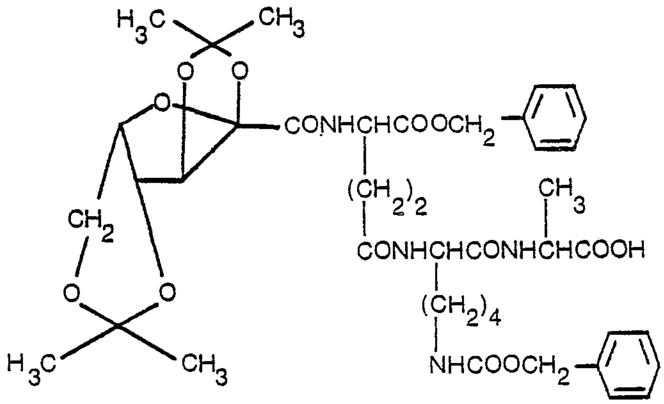

ou son sel; ou bien

on fait réagir un composé de formule:

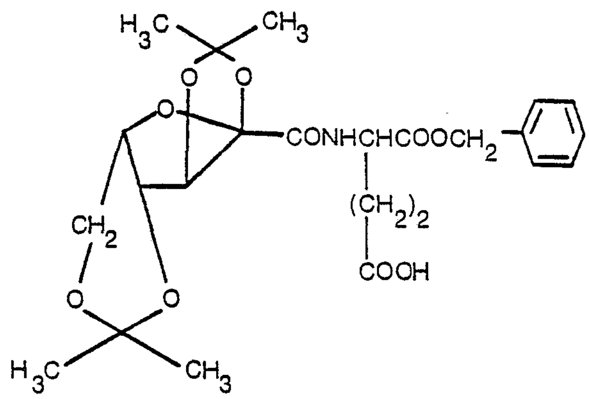

ou son sel, avec un composé de formule:

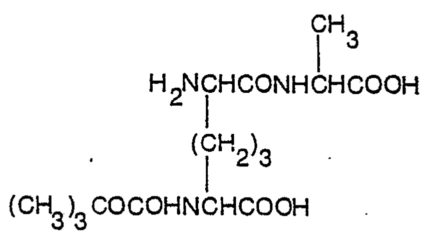

ou son sel, pour obtenir un composé de formule:

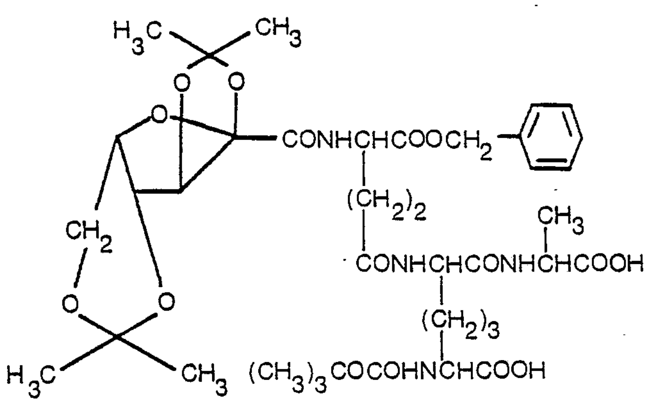

ou son sel, ou bien

on fait réagir un composé de formule:

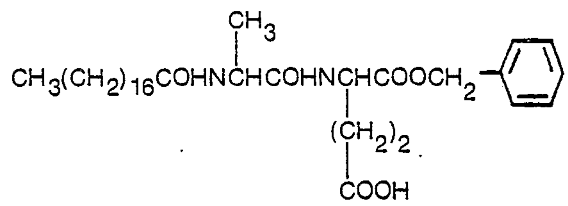

ou son sel, avec un composé de formule:

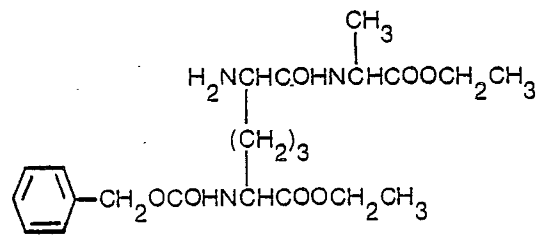

ou son sel, pour obtenir un composé de formule:

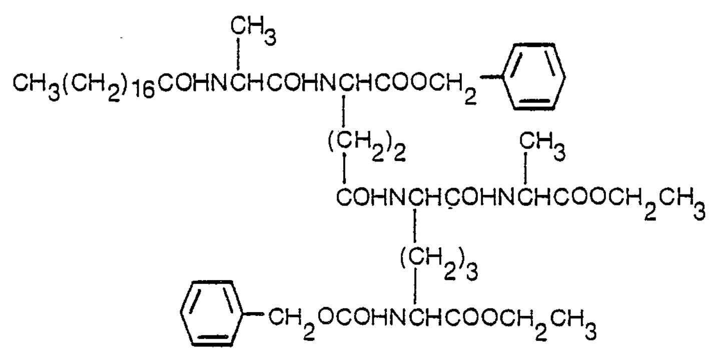

ou son sel, ou bien

on fait réagir un composé de formule:

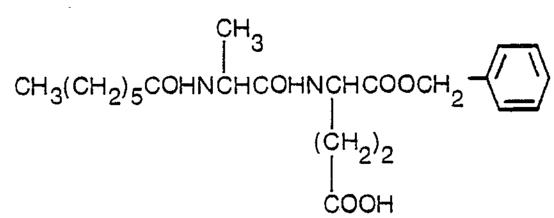

ou son sel, avec un composé de formule:

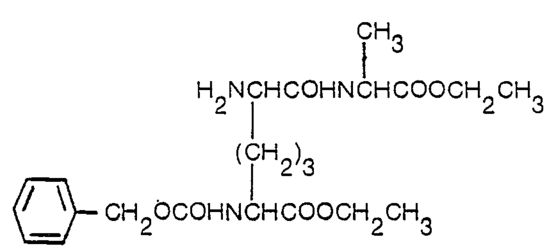

ou son sel, pour obtenir un composé de formule:

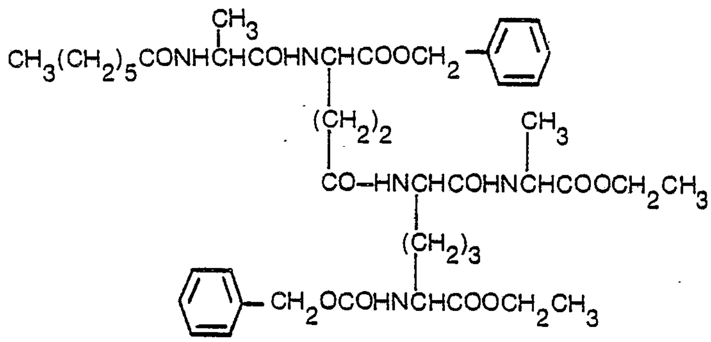

ou son sel; ou bien

on fait réagir un composé de formule:

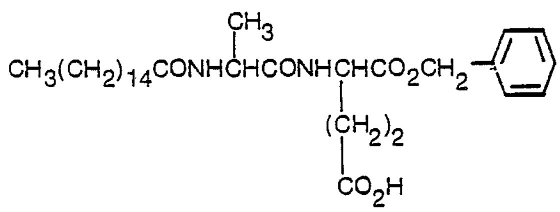

ou son sel, avec un composé de formule:

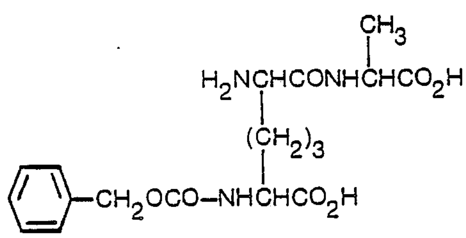

ou son sel, pour obtenir un composé de formule:

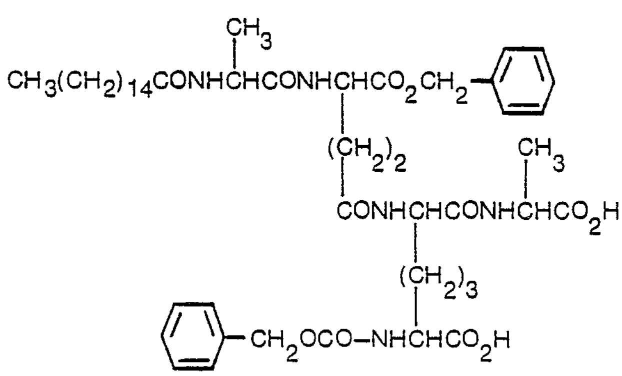

ou son sel; ou bien

on fait réagir un composé de formule:

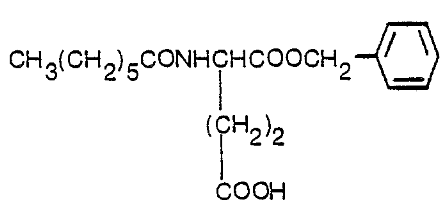

ou son sel, avec un composé de formule:

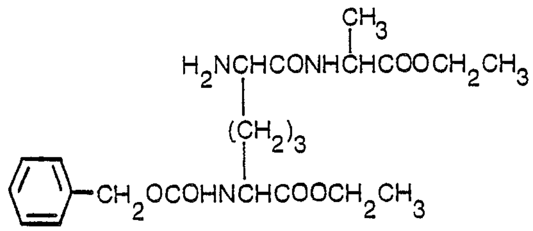

ou son sel, pour obtenir un composé de formule:

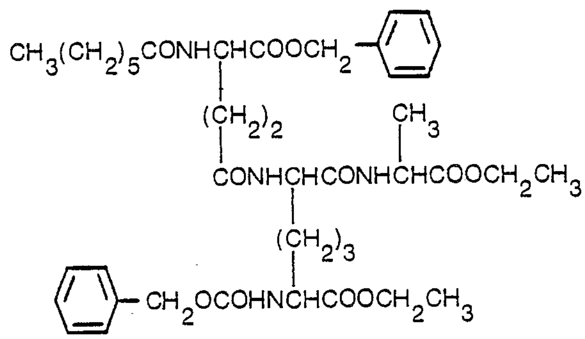

ou son sel; ou bien

on fait réagir un composé de formule:

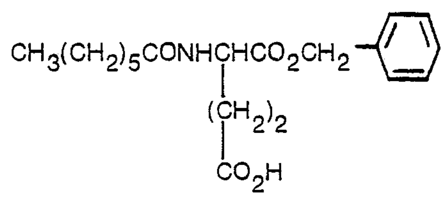

ou son sel, avec un composé de formule:

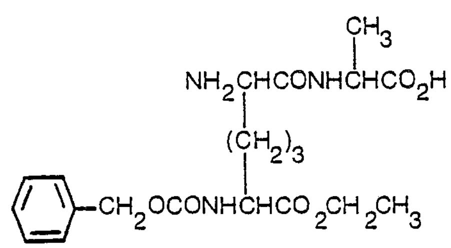

ou son sel, pour obtenir un composé de formule:

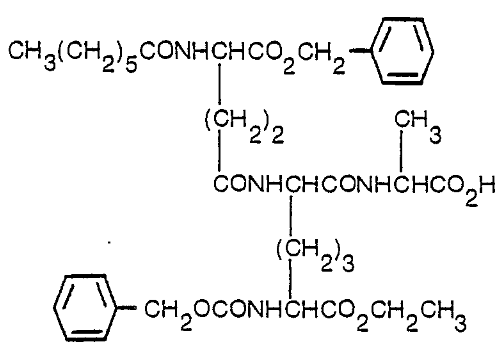

ou son sel; ou bien

on fait réagir un composé de formule:

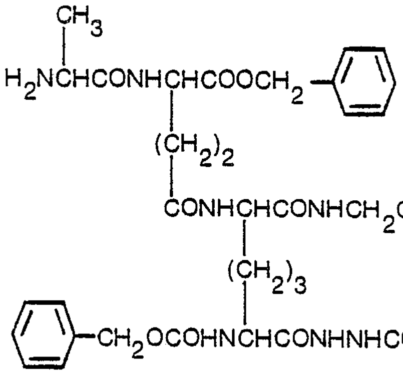

ou son sel, avec l'acide N-n-hexyl-1 O-tétradécyl-2,3,4,5 D-glucaramique, l'acide N-benzyl-1 O-tétraacétyl-2,3,4,5 D-glucaramique, l'acide N-lauryl-1 O-tétraacétyl-2,3,4,5 D-glucaramique, ou leurs dérivés réactifs, pour obtenir un composé de formule

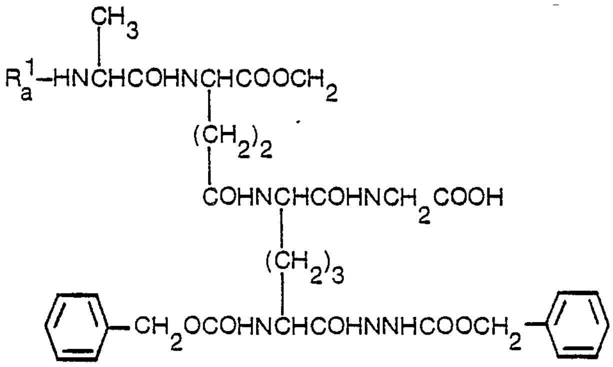

(dans laquelle $R_a^1$ est un groupe N-n-hexyl-1 O-tétraacétyl-2,3,4,5-D glucaramoyle, N-benzyl-1 O-tétraacétyl-2,3,4,5 D-glucaramoyle ou N-lauryl O-tétraacétyl-2,3,4,5 D-glucaramoyle) ou son sel; ou bien on fait réagir un composé de formule:

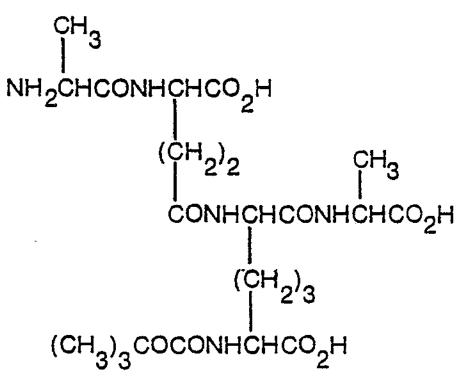

ou son sel, avec l'acide triacontanoïque ou son dérivé réactif, pour obtenir un composé de formule

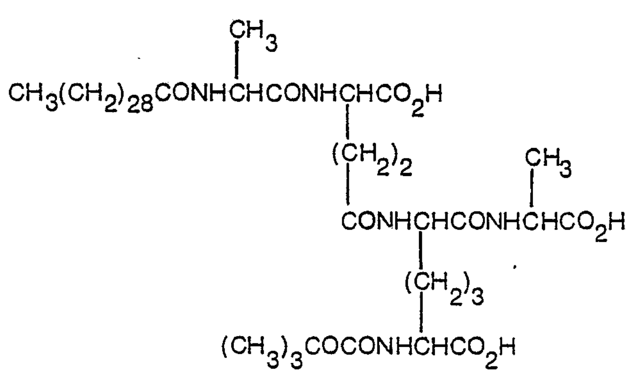

ou son sel; ou bien

on fait réagir un composé de formule:

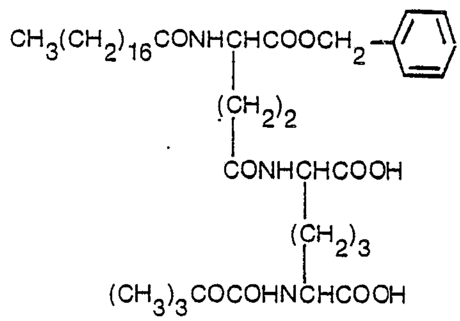

ou son sel, avec un agent de méthylation pour obtenir un composé de formule:

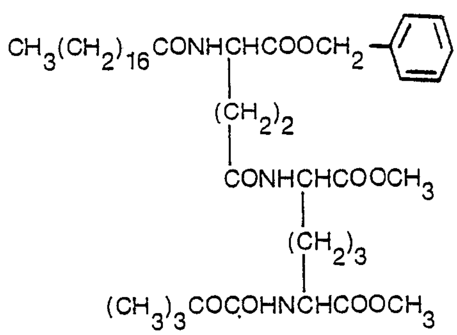

ou son sel, ou bien

on fait réagir un composé de formule:

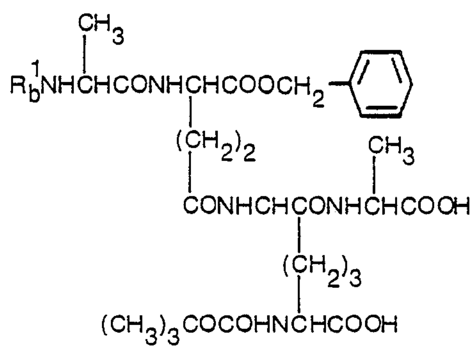

ou son sel, avec un agent de méthylation pour obtenir un composé de formule:

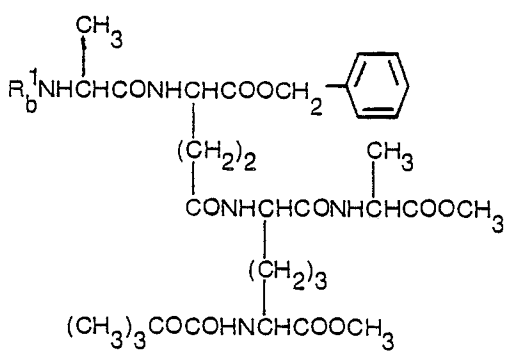

(dans laquelle $R_b^1$ est un groupe n-docosanoyle ou n-tétracosanoyle) ou son sel; ou bien on fait réagir un composé de formule:

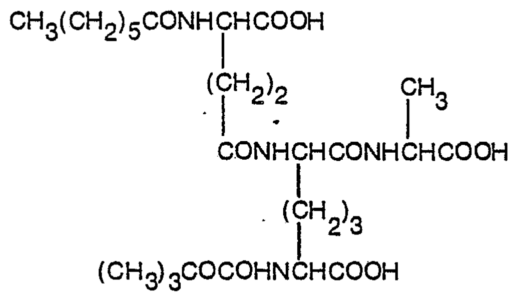

ou son sel, avec un agent de méthylation pour obtenir un composé de formule:

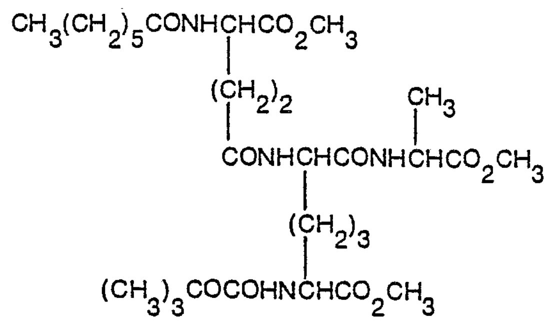

ou son sel; ou bien on fait réagir un composé de formule:

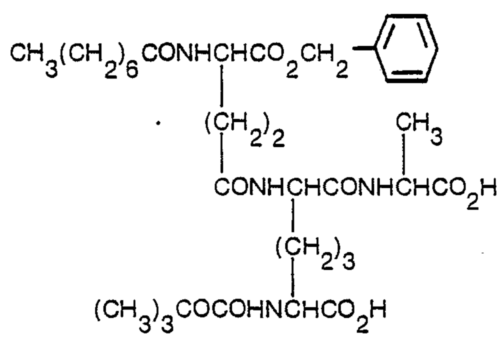

ou son sel, avec un agent de méthylation pour obtenir un composé de formule:

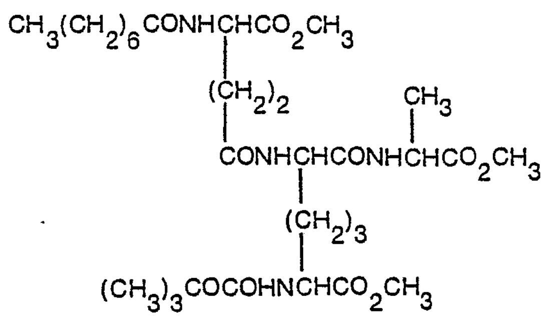

ou son sel;

on fait réagir un composé de formule:

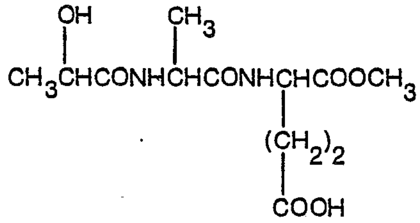

ou son sel, avec un composé de formule:

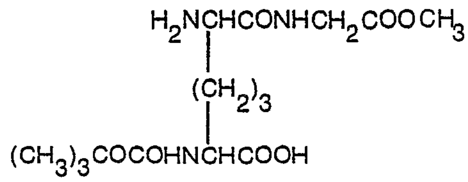

ou son sel, et l'on soumet le composé résultant à une réaction d'élimination du groupe protecteur, ce qui donne un composé de formule:

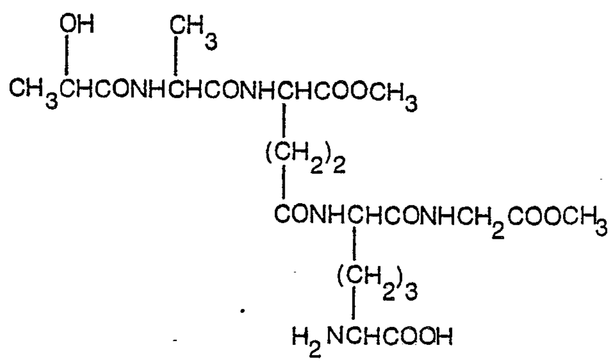

ou son sel.

7. Procédé de préparation d'un composé de formule:

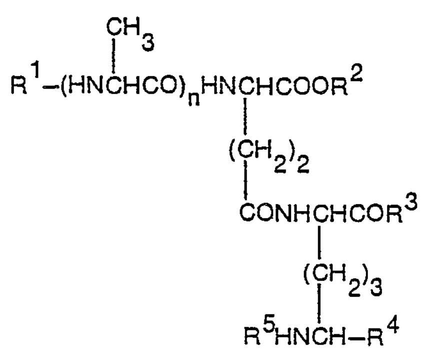

(dans laquelle

$R^1$ est un groupe n-octanoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un groupe benzyle, $R^3$ est un groupe méthoxy, $R^4$ est un groupe méthoxycarbonyle, et $R^5$ est un atome d'hydrogène; ou

$R^1$ est un groupe stéaroyle, n est un nombre entier, $R^2$ est un atome d'hydrogène, $R^3$ est un groupe méthoxy, $R^4$ est un groupe méthoxycarbonyle et $R^5$ est un atome d'hydrogène; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un atome d'hydrogène; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe heptanoyle, n est un nombre entier nul, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier nul, $R^2$ est un atome d'hydrogène, $R^3$ est un groupe carboxy-1 éthylamino, $R^4$ est un groupe éthoxycarbonyle et $R^5$ est un atome d'hydrogène), ou son sel, procédé caractérisé en ce qu'on soumet un composé de formule:

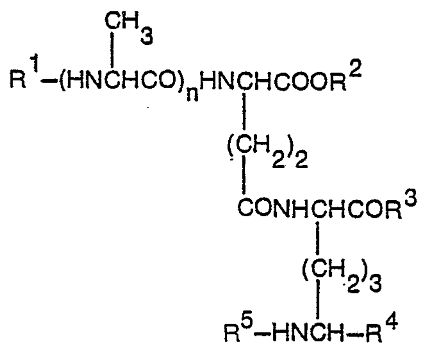

(dans laquelle

$R^1$ est un groupe n-octanoyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe carboxy-1 éthylamino, $R^4$ est un atome d'hydrogène et $R^5$ et un groupe benzyloxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle, $R^5$ est un groupe tertiobutoxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un atome d'hydrogène; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe butoxycarbonyle; ou

$R^1$ est un groupe diisopropylidène-2,3; 4,6 céto-2 L-gulonoyle, n est un nombre entier nul, $R^2$ est un groupe benzyle, $R^3$ est un groupe carboxy-1 éthylamino, $R^4$ est un atome d'hydrogène et $R^5$ est un atome d'hydrogène; ou

$R^1$ est un groupe céto-2 L-gulonoyle, n est un nombre entier nul, $R^2$ est un groupe benzyle, $R^3$ est un groupe carboxy-1 éthylamino, $R^4$ est un atome d'hydrogène et $R^5$ est un atome d'hydrogène; ou

$R^1$ est un groupe diisopropylidène-2,3; 4,6 céto-L-gulonoyle, n est un nombre entier nul, $R^2$ est un groupe benzyle, $R^3$ est un groupe carboxyl-1 éthylamino, $R^4$ est un carboxyle et $R^5$ est un groupe tertiobutoxycarbonyle; ou

$R^1$ est un groupe céto-2 L-gulonoyle, n est un nombre entier nul, $R^2$ est un groupe benzyle, $R^3$ est un groupe carboxy-1 éthylamino, $R^4$ est un groupe carboxyle et $R^5$ est un atome d'hydrogène; ou

$R^1$ est un groupe heptanoyle, n est un nombre entier nul, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe heptanoyle, n est un nombre entier nul, $R^2$ est un groupe benzyle, $R^3$ est un groupe carboxy-1 éthylamino, $R^4$ est un groupe éthoxycarbonyle et $R^5$ est un groupe benzyloxycarbonyle),

ou son sel, à une réaction d'élimination du ou des groupes protecteurs, ce qui donne un composé de formule:

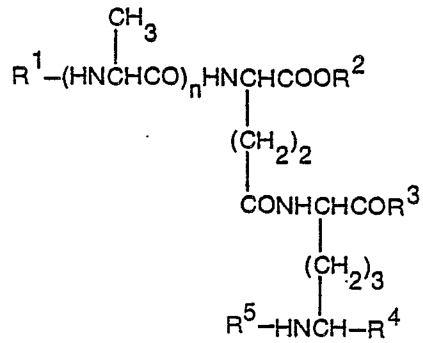

(dans laquelle

$R^1$ est un groupe n-octanoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un atome d'hydrogène; ou

$R^1$ est un groupe stéaroyle, n est un nombre entier nul, $R^2$ est un atome d'hydrogène, $R^3$ est un groupe méthoxy, $R^4$ est un groupe méthoxycarbonyle, et $R^5$ est un atome d'hydrogène; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un atome d'hydrogène; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle, et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un groupe carboxyle et $R^5$ un atome d'hydrogène; ou

$R^1$ est un groupe heptanoyle, n est un nombre entier nul, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier nul, $R^2$ est un atome d'hydrogène, $R^3$ est un groupe carboxy-1 éthylamino, $R^4$ est un groupe éthoxycarbonyle et $R^5$ est un atome d'hydrogène) ou son sel.

8. Procédé de préparation d'un composé de formule:

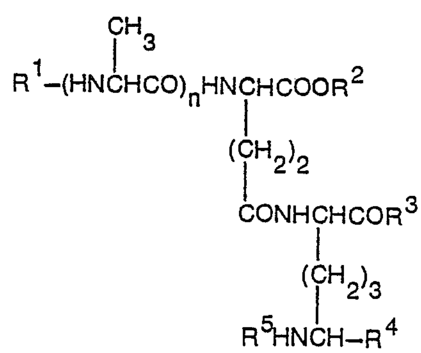

(dans laquelle

$R^1$ est un groupe n-docosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe n-docosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe n-tétracosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe n-tétracosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe acétoxy-2 propionyle, n est un nombre entier valant 1, $R^2$ est un atome d'hydrogène, $R^3$ est un groupe méthoxycarbonylméthylamino, $R^4$ est un groupe méthoxycarbonyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe méthoxycarbonylméthylamino, $R^4$ est un groupe carboxyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe carboxyméthylamino, $R^4$ est un groupe méthoxycarbonyle et $R^5$ est un atome d'hydrogène; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-n-hexyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un atome d'hydrogène; ou

$R^1$ est un groupe stéaroyle, n est un nombre entier valant 1, $R^2$ est un atome d'hydrogène, $R^3$ est un groupe éthoxycarbonyl-1 éthylamino, $R^4$ est un atome éthoxycarbonyle et $R^5$ est un atome d'hydrogène; ou

$R^1$ est groupe heptanoyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-benzyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène), ou son sel pharmaceutiquement acceptable,

procédé caractérisé en ce qu' on soumet un composé de formule:

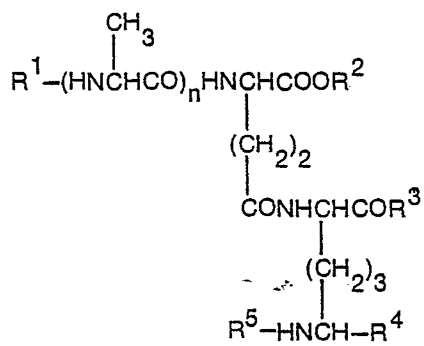

(dans laquelle

$R^1$ est un groupe n-docosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe carboxy-1 éthylamino, $R^4$ est un groupe carboxyle et $R^5$ est un groupe tertiobutoxycarbonyle; ou

$R^1$ est un groupe n-tétracosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe t-butoxycarbonyle; ou

$R^1$ est un groupe n-tétracosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe acétoxy-2 propionyle, n est un nombre entier valant 1, $R^2$ est un groupe benzyle, $R^3$ est un groupe méthoxycarbonylméthylamino, $R^4$ est un groupe méthoxycarbonyle et $R^5$ est un groupe benzyloxycarbonyle; ou

$R^1$ est un groupe hydroxy-2 propionyle, n est un nombre entier valant 1, $R^2$ est un groupe benzyle, $R^3$ est un groupe méthoxycarbonylméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe benzyle, $R^3$ est un groupe carboxyméthylamino, $R^4$ est un groupe méthoxycarbonyle et $R^5$ est un groupe tertiobutoxycarbonyle; ou

$R^1$ est un groupe N-n-hexyl-1 O-tétraacétyl-2,3,4,5 D-glucaramoyle, n est un nombre entier valant 1, $R^2$ est un groupe benzyl, $R^3$ est un groupe carboxyméthylamino, $R^4$ est un groupe benzoyloxycarbonyl-3 carbazoyle et $R^5$ est un groupe benzyloxycarbonyle; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carbazoyle et $R^5$ est un atome d'hydrogène; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un groupe tertiobutoxycarbonyle; ou

$R^1$ est un groupe stéaroyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzoyloxycarbonyle; ou

$R^1$ est un groupe heptanoyle, n est un nombre entier valant 1, $R^2$ est un groupe benzyle, $R^3$ est un groupe éthoxycarbonyle-1 éthylamino, $R^4$ est un groupe éthoxycarbonyle et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle, et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carbaxoyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzoyloxycarbonyl-3 carbazoyle et $R^5$ est un groupe benzyloxycarbonyle; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carbazoyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un groupe benzyle, $R^3$ est un groupe carboxy-1 éthylamino, $R^4$ est un groupe carboxyle et $R^5$ est un groupe benzyloxycarbonyle; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un groupe tertiobutoxycarbonyle)

ou son sel, à une réaction d'élimination du ou des groupes protecteurs, pour obtenir un composé de formule:

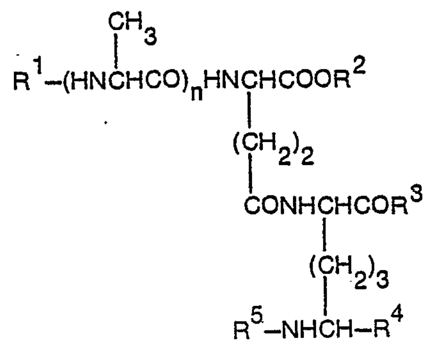

(dans laquelle

$R^1$ est un groupe n-docosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe n-docosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe n-tétracosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe n-tétracosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe acétoxy-2 propionyle, n est un nombre entier valant 1, $R^2$ est un atome d'hydrogène, $R^3$ est un groupe méthoxycarbonylméthylamino, $R^4$ est un groupe méthoxycarbonyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe méthoxycarbonylméthylamino, $R^4$ est un groupe carboxyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe carboxyméthylamino, $R^4$ est un groupe méthoxycarbonyle et $R^5$ est un atome d'hydrogène; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-n-hexyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un atome d'hydrogène; ou

$R^1$ est un groupe stéaroyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-benzyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe triacontanoyle, n est un nombre entier valant 1, $R^2$ est un atome d'hydrogène, $R^3$ est un groupe carboxy-1 éthyleamino, $R^4$ est un groupe carboxyle et $R^5$ est un atome d'hydrogène), ou son sel.

9. Composition pharmaceutique comprenant un composé selon la revendication 3 ou 4, ou ses sels pharmaceutiquement acceptables, en association avec un véhicule ou excipient pharmaceutiquement acceptable et essentiellement non toxique.

10. Composé selon les revendications 3 ou 4, destiné à servir de médicament.

11. Composé selon la revendication 3 ou 4, destiné à servir au traitement de maladies infectieuses et d'une tumeur.

**Revendications pour l'Etat contractant: AT**

1. Procédé pour préparer un composé de formule:

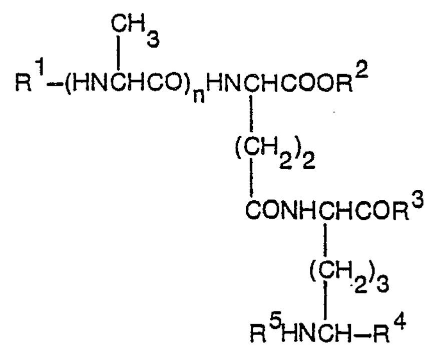

dans laquelle

$R^1$ est un groupe n-octanoyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe carboxy-1 éthylamino, $R^4$ est un groupe carboxyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe n-tétracosanoyle, n est un nombre entier valant 1, $R^2$ est un groupe benzyle, $R^3$ est un groupe carboxy-1-éthylamino, $R^4$ est un groupe carboxyle et $R^5$ est un groupe tertiobutoxycarbonyle; ou

$R^1$ est un groupe n-tétracosanoyle, n est nombre entier valant 1, $R^2$ est un groupe benzyle, $R^3$ est un groupe éthoxycarbonyl-1 éthylamino, $R^4$ est un groupe méthoxycarbonyle et $R^5$ est un groupe tertiobutoxycarbonyle; ou

$R^1$ est un groupe acétoxy-2 propionyle, n est un nombre entier valant 1, $R^2$ est un groupe benzyle, $R^3$ est un groupe méthoxycarbonyléthylamino, $R^4$ est un groupe méthoxycarbonyle et $R^5$ est un groupe tertiobutoxycarbonyle; ou

$R^1$ est un groupe acétoxy-2 propionyle, n est un nombre entier valant 1, $R^2$ est un groupe benzyle, $R^3$ est un groupe méthoxycarbonylméthylamino, $R^4$ est un groupe méthoxycarbonyle et $R^5$ est un groupe benzyloxycarbonyle; ou

$R^1$ est un groupe acétoxy-2 propionyle, n est un nombre entier valant 1, $R^2$ est un groupe benzyle, $R^3$ est un groupe carboxyméthoxylamino, $R^4$ est un groupe méthoxycarbonyle et $R^5$ est un groupe butoxycarbonyle; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe hydroxy-2 propionyle, n est nombre entier valant 1, $R^2$ est un groupe benzyle,

$R^3$ est un groupe méthoxycarbonylméthylamino, $R^4$ est un carboxyle et $R^5$ est un groupe tertiobutoxycarbonyle; ou

$R^1$ est un groupe hydroxy-2 propionyle, n est un nombre entier valant 1, $R^2$ est un groupe benzyle, $R^3$ est un groupe carboxyméthylamino, $R^4$ est un groupe méthoxycarbonyle et $R^5$ est un groupe tertiobutoxycarbonyle; ou

$R^1$ est un groupe N-n-hexyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle et $R^5$ est un groupe benzyloxycarbonyle; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe méthyle, $R^3$ est un groupe méthoxycarbonylméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un groupe tertiobutoxycarbonyle; ou

$R^1$ est un groupe diisopropylidène-2,3; 4,6 L-céto-2 gulonoyle, n est un nombre entier nul, $R^2$ est un groupe benzyle, $R^3$ est un groupe carboxy-1 éthylamino, $R^4$ est un atome d'hydrogène et $R^5$ est un atome d'hydrogène; ou

$R^1$ est un groupe diisopropylidène-2,3; 4,6 céto-2 L-gulonoyle, n est un nombre entier nul, $R^2$ est un groupe benzoyle, $R^3$ est un groupe carboxy-1 éthylamino, $R^4$ est un groupe carboxyle et $R^5$ est un groupe tertiobutoxycarbonyle; ou

$R^1$ est un groupe stéaroyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe heptanoyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle et $R^5$ est un groupe benzyloxycarbonyle; ou

$R^1$ est un groupe N-lauryl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle et $R^5$ est un groupe benzyloxycarbonyle; ou

$R^1$ est un groupe palmitoyle, n est un nombre entier valant 1, $R^2$ est un groupe benzyle, $R^3$ est un groupe carboxy-1 éthylamino, $R^4$ est un groupe carboxyle et $R^5$ est un groupe benzyloxycarbonyle; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un groupe tertiobutoxycarbonyle; ou

$R^1$ est un groupe heptanoyle, n est un nombre entier nul, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe heptanoyle, n est un nombre entier nul, $R^2$ est un groupe benzyle, $R^3$ est un groupe carboxy-1 éthylamino, $R^4$ est un groupe éthoxycarbonyle et $R^5$ est un groupe benzyloxycarbonyle,

ou son sel pharmaceutiquement acceptable, procédé caractérisé en ce que:

on fait réagir un composé de formule:

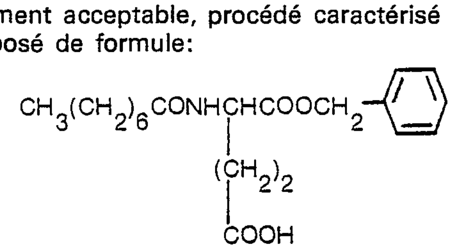

ou son sel, avec un composé de formule:

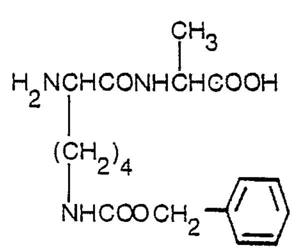

ou son sel, pour obtenir un composé de formule:

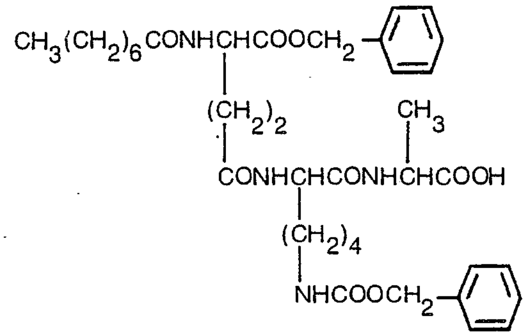

ou son sel, ou bien on fait réagir un composé de formule:

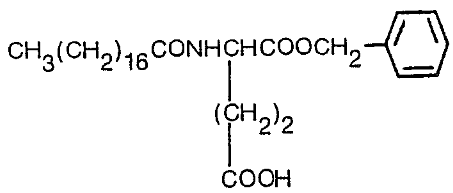

ou son sel, avec un composé de formule:

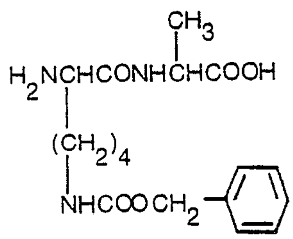

ou son sel, pour obtenir un composé de formule:

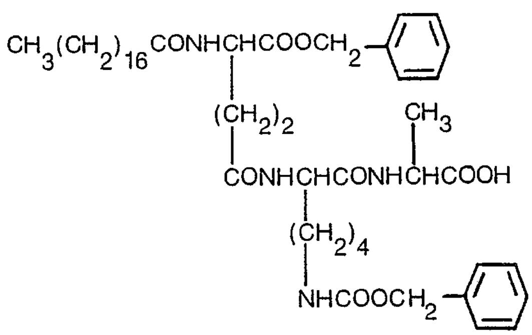

ou son sel; ou bien on fait réagir un composé de formule:

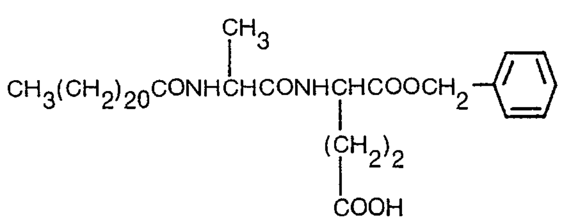

ou son sel, avec un composé de formule:

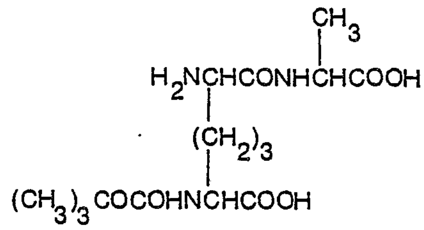

ou son sel, pour obtenir un composé de formule:

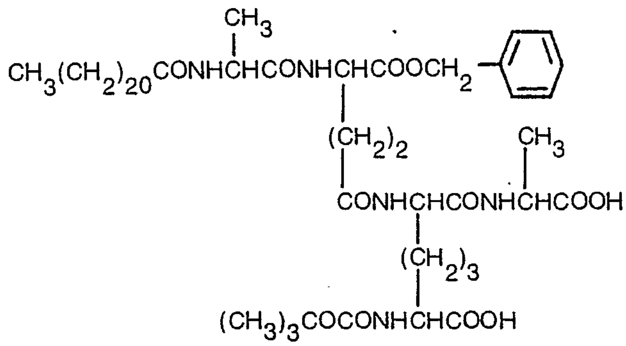

ou son sel, ou bien

on fait réagir un composé de formule:

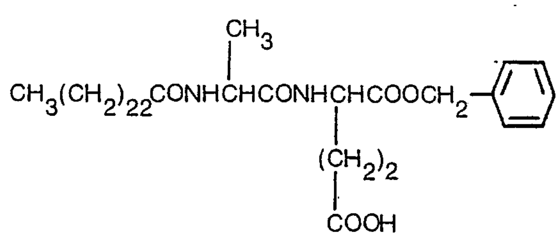

ou son sel, avec un composé de formule:

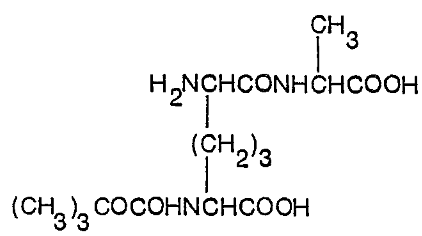

ou son sel, pour obtenir un composé de formule:

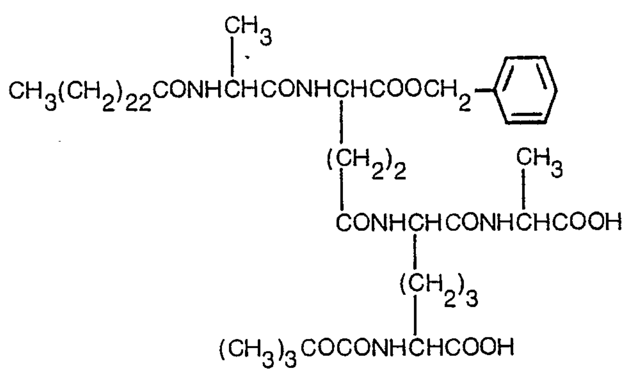

ou son sel, ou bien

on fait réagir un composé de formule:

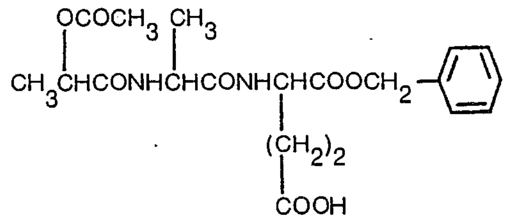

ou son sel, avec un composé de formule:

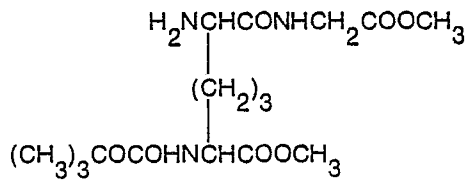

ou son sel, pour obtenir un composé de formule:

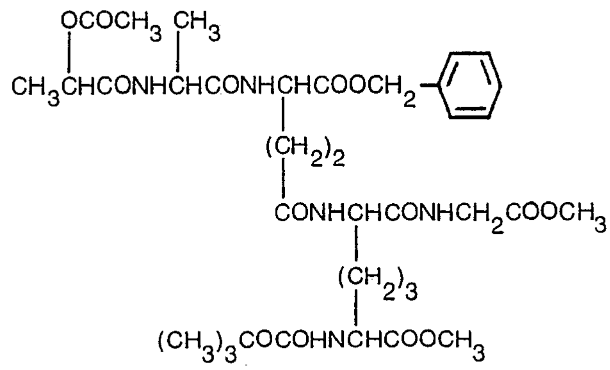

ou son sel, ou bien

on fait réagir un composé de formule:

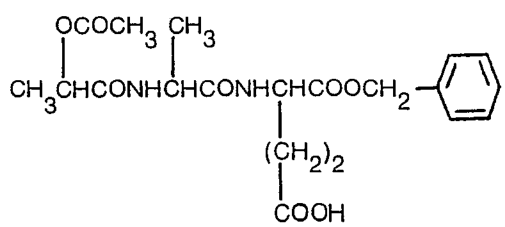

ou son sel, avec un composé de formule:

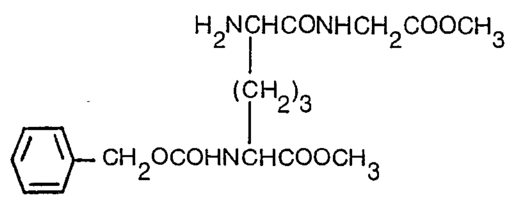

ou son sel, pour obtenir un composé de formule:

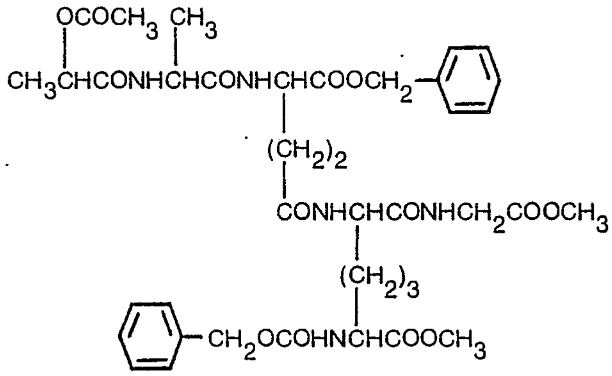

ou son sel, ou bien,
on fait réagir un composé de formule:

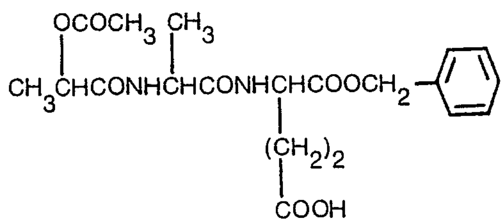

ou son sel, avec un composé de formule:

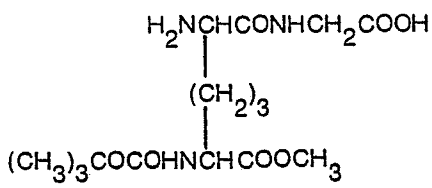

ou son sel, pour obtenir un composé de formule:

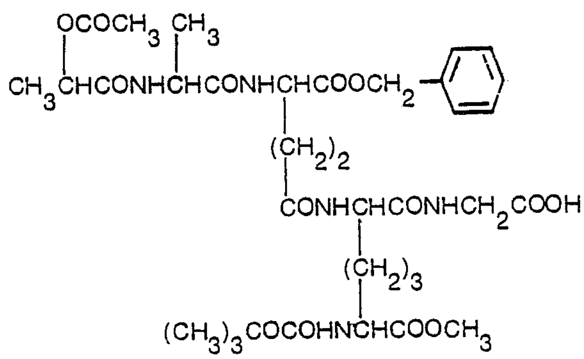

ou son sel, ou bien
on fait réagir un composé de formule:

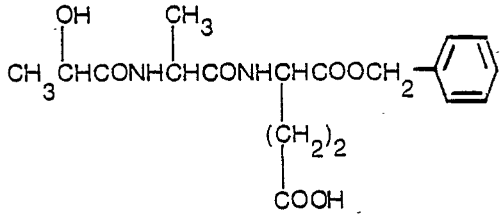

ou son sel, avec un composé de formule:

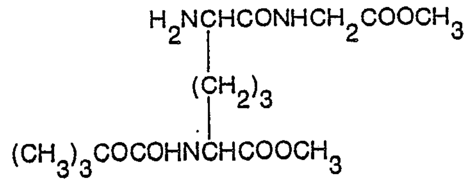

ou son sel, pour obtenir un composé de formule:

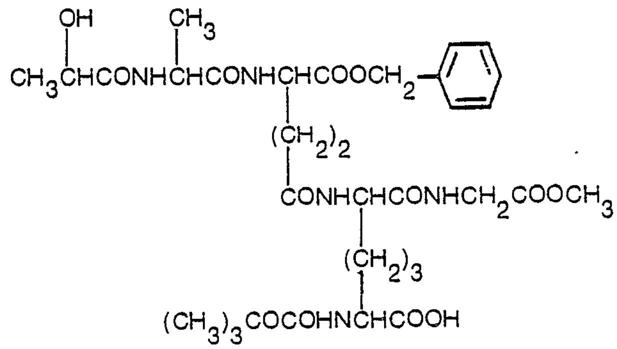

ou son sel; ou bien
on fait réagir un composé de formule:

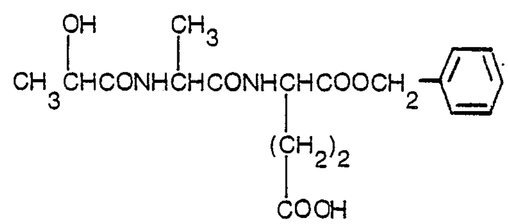

ou son sel, avec un composé de formule:

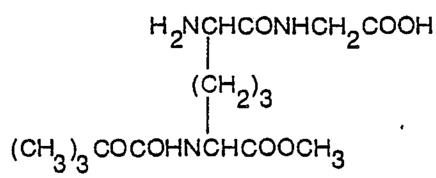

ou son sel, pour obtenir un composé de formule:

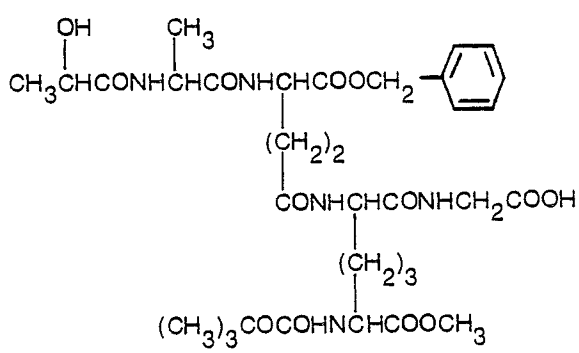

ou son sel; ou bien

on fait réagir un composé de formule:

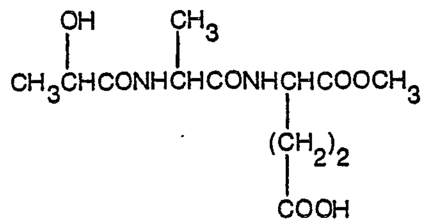

ou son sel, avec un composé de formule:

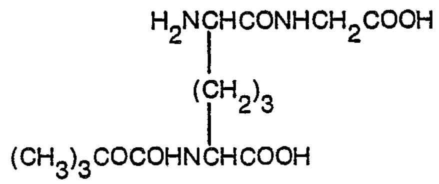

ou son sel, pour obtenir un composé de formule:

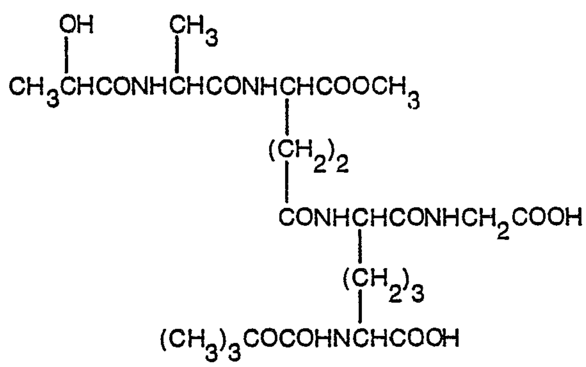

ou son sel; ou bien

on fait réagir un composé de formule:

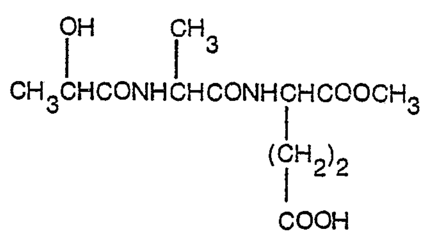

ou son sel, avec un composé de formule:

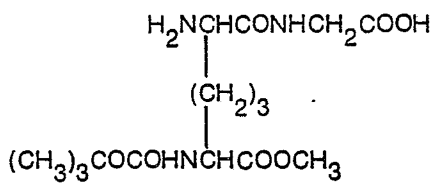

ou son sel, pour obtenir un composé de formule:

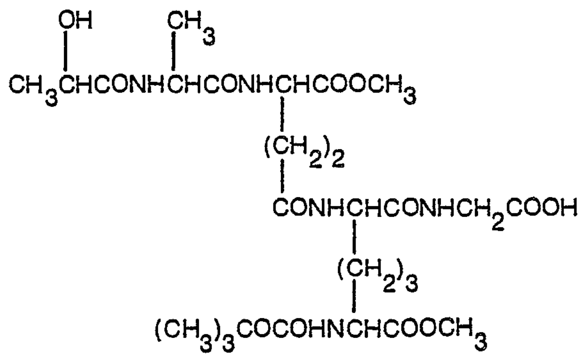

ou son sel, ou bien

on fait réagir un composé de formule:

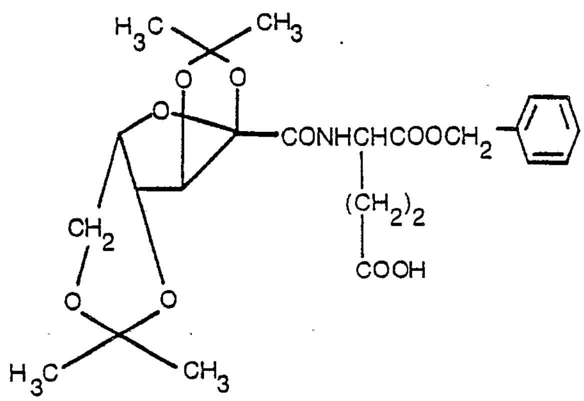

ou son sel, avec un composé de formule:

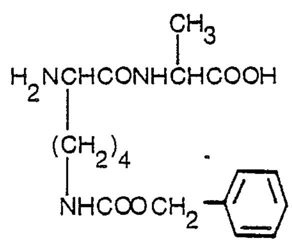

ou son sel, pour obtenir un composé de formule:

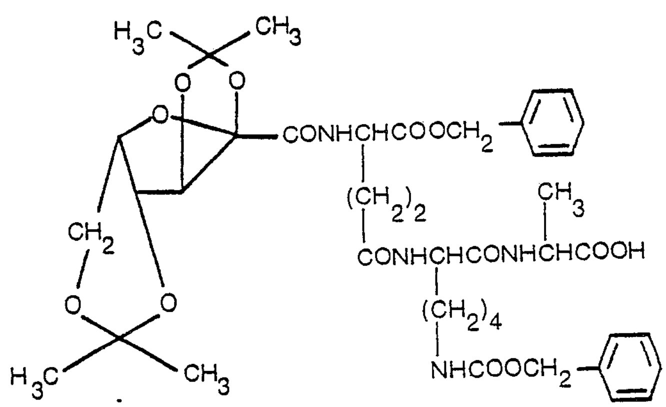

ou son sel, ou bien

on fait réagir un composé de formule:

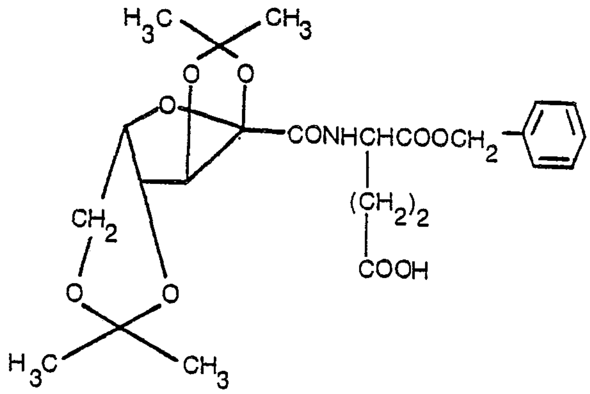

ou son sel, avec un composé de formule:

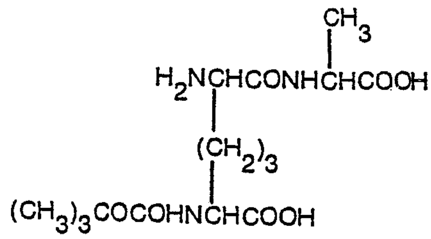

ou son sel, pour obtenir un composé de formule:

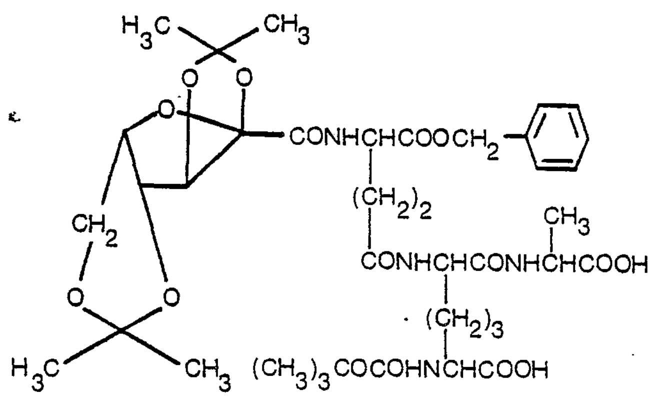

ou son sel, ou bien

on fait réagir un composé de formule:

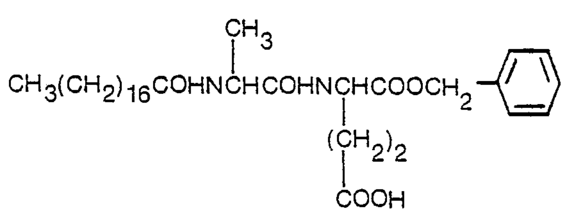

ou son sel, avec un composé de formule:

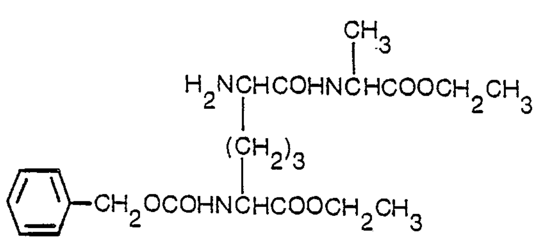

ou son sel, pour obtenir un composé de formule:

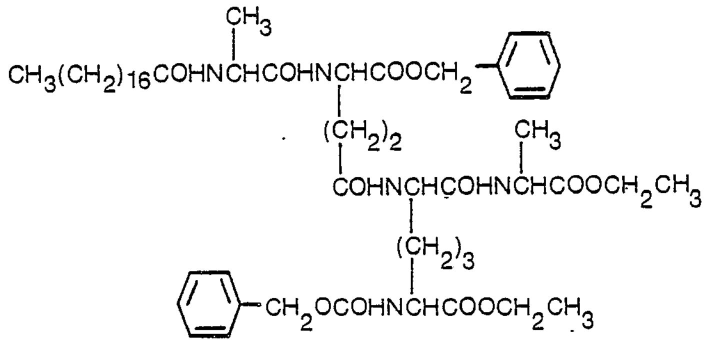

ou son sel, ou bien

on fait réagir un composé de formule:

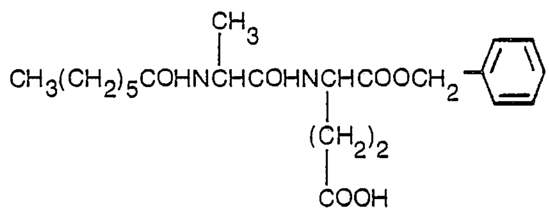

ou son sel, avec un composé de formule:

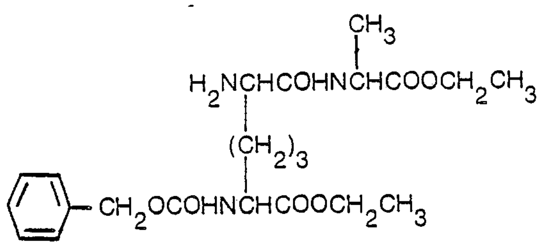

ou son sel, pour obtenir un composé de formule:

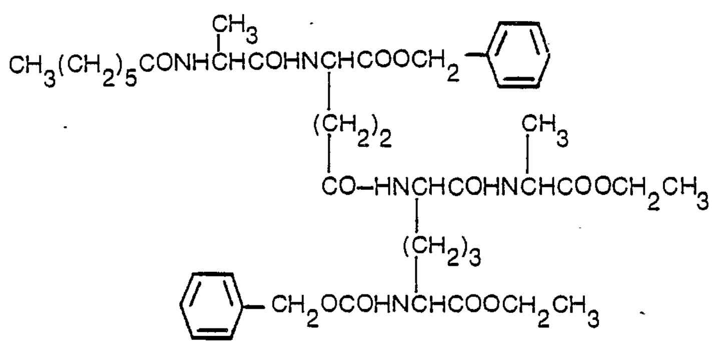

ou son sel; ou bien

on fait réagir un composé de formule:

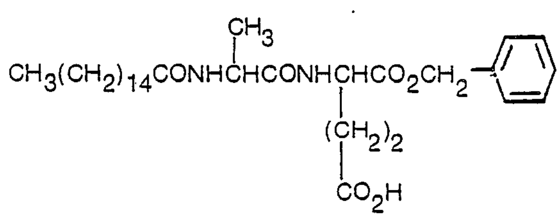

ou son sel, avec un composé de formule:

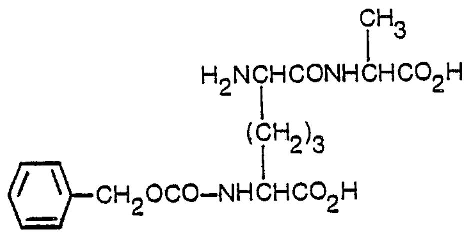

ou son sel, pour obtenir un composé de formule:

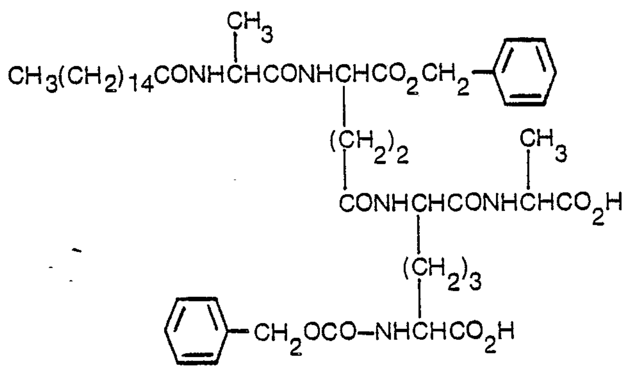

ou son sel; ou bien.

on fait réagir un composé de formule:

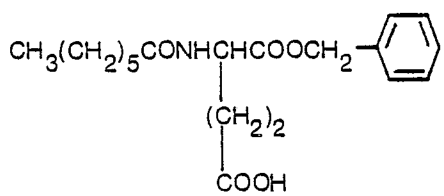

ou son sel, avec un composé de formule:

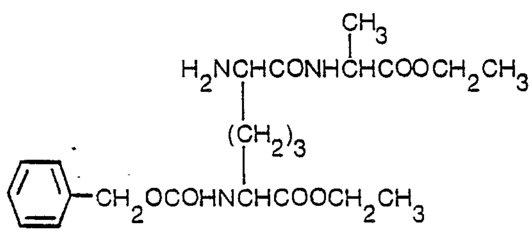

ou son sel, pour obtenir un composé de formule:

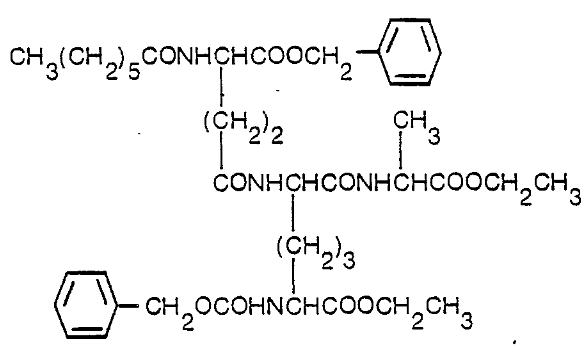

ou son sel; ou bien

on fait réagir un composé de formule:

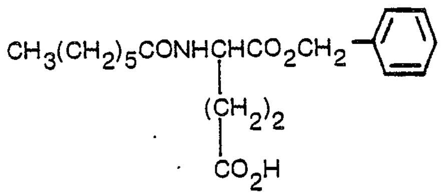

ou son sel, avec un composé de formule:

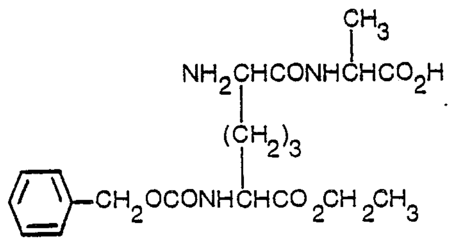

ou son sel, pour obtenir un composé de formule:

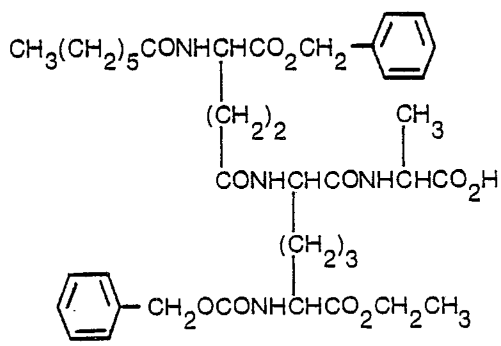

ou son sel; ou bien

on fait réagir un composé de formule:

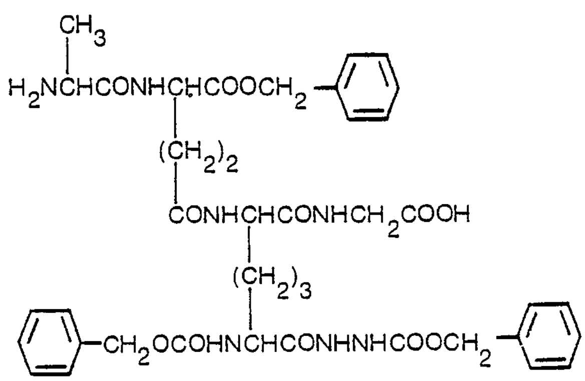

ou son sel, avec l'acide N-n-hexyl-1 O-tétradécyl-2,3,4,5 D-glucaramique, l'acide N-benzyl-1 O-tétraacétyl-2,3,4,5 D-glucaramique, l'acide N-lauryl-1 O-tétraacétyl-2,3,4,5 D-glucaramique, ou leurs dérivés réactifs, pour obtenir un composé de formule

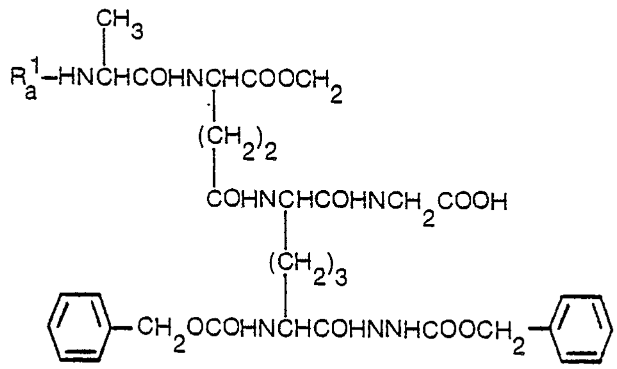

(dans laquelle $R_a^1$ est un groupe N-n-hexyl-1 O-tétraacétyl-2,3,4,5-D glucaramoyle N-benzyl-1 O-téra-acétyl-2,3,4,5 D-glucaramoyle ou N-lauryl O-tétraacétyl-2,3,4,5 D-glucaramoyle) ou son sel; ou bien on fait réagir un composé de formule:

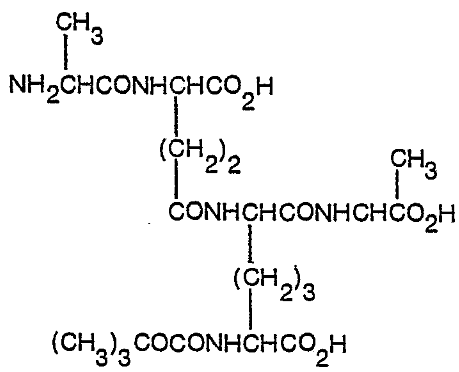

ou son sel, avec lacide triacontanoïque ou son dérivé réactif, pour obtenir un composé de formule:

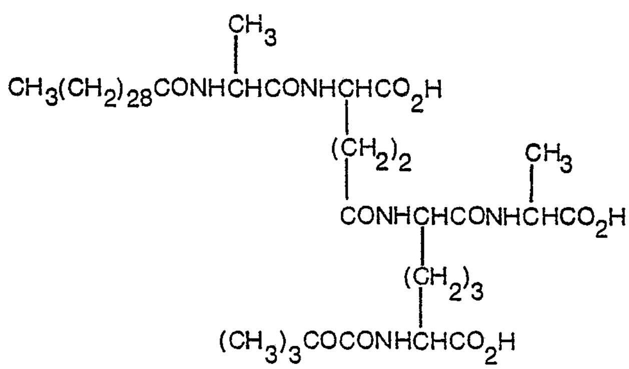

ou son sel; ou bien

on fait réagir un composé de formule:

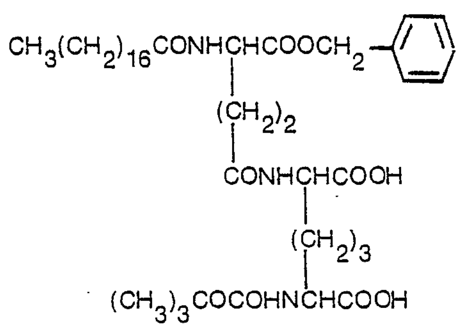

ou son sel, avec un agent de méthylation pour obtenir un composé de formule:

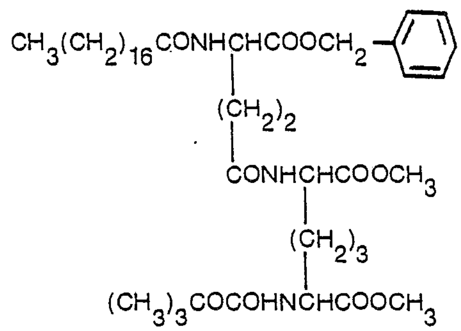

ou son sel, ou bien

on fait réagir un composé de formule:

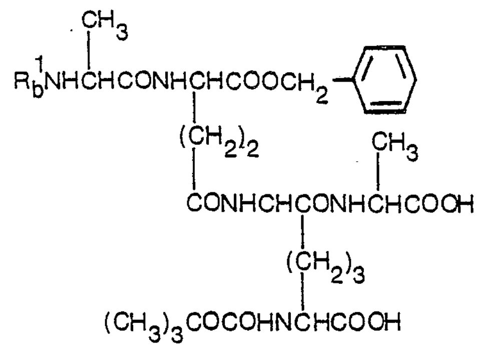

ou son sel, avec un agent de méthylation pour obtenir un composé de formule:

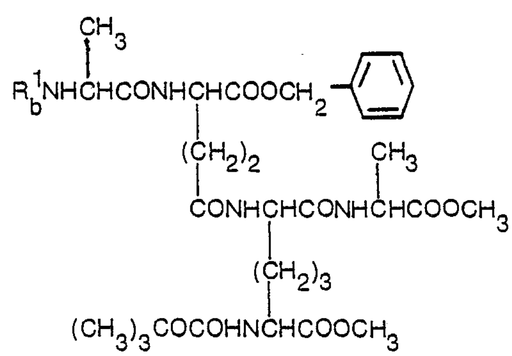

(dans laquelle $R_b^1$ est un groupe n-docosanoyle ou n-téracosanoyle) ou son sel; ou bien

on fait réagir un composé de formule:

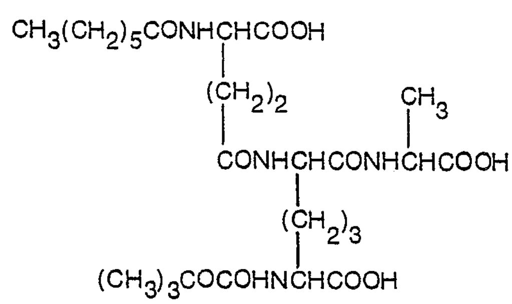

ou son sel, avec un agent de méthylation pour obtenir un composé de formule:

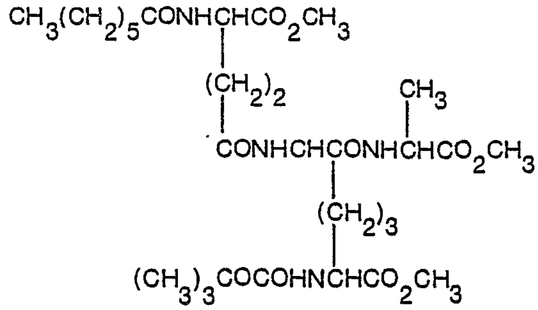

ou son sel; ou bien

on fait réagir un composé de formule:

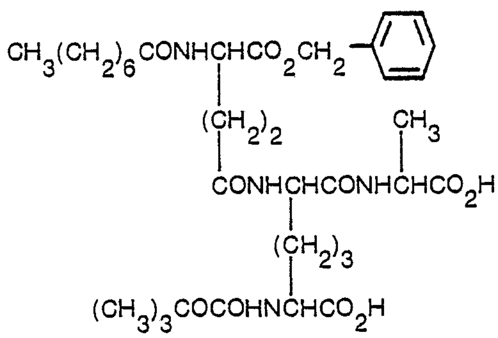

ou son sel, avec un agent de méthylation pour obtenir un composé de formule:

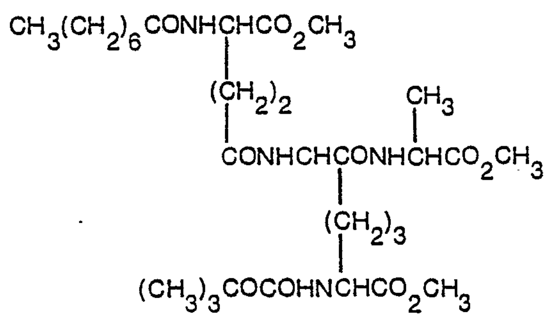

ou son sel,

on fait réagir un composé de formule:

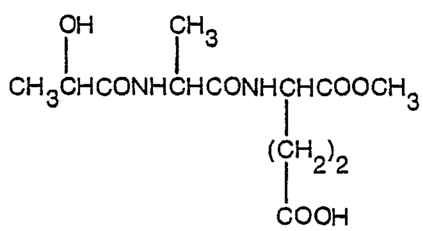

ou son sel, avec un composé de formule:

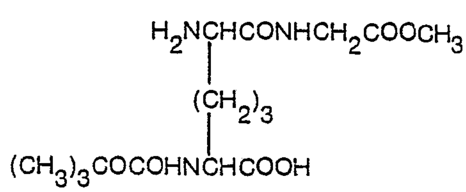

ou son sel, et l'on soumet le composé résultant à une réaction d'élimination du groupe protecteur, ce qui donne un composé de formule:

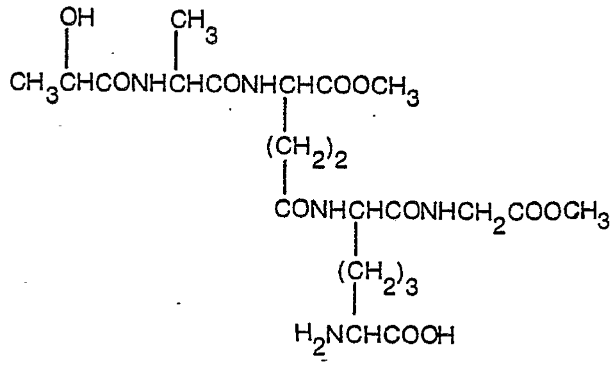

ou son sel.

2. Procédé de préparation d'un composé de formule:

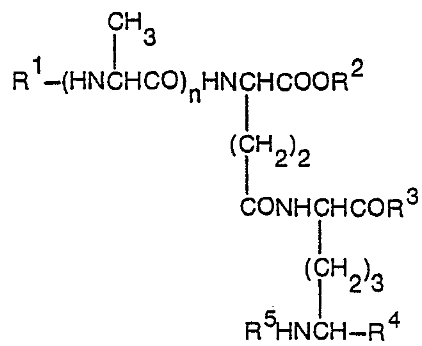

(dans laquelle

$R^1$ est un groupe n-octanoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéarole, n est un nombre entier nul, $R^2$ est un groupe benzyle, $R^3$ est un groupe méthoxy, $R^4$ est un groupe méthoxycarbonyle et $R^5$ est un atome d'hydrogène; ou

$R^1$ est un groupe stéaroyle, n est un nombre entier, $R^2$ est un atome d'hydrogène, $R^3$ est un groupe méthoxy, $R^4$ est un groupe méthoxycarbonyle et $R^5$ est un atome d'hydrogène; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un atome d'hydrogène; ou

$R^1$ est un groupe octanoyle, n est un nombre entier nul, $R^2$ est un groupe méthyle, $R^3$ est un groupe méthoxycarboxyl-1 éthylamino, $R^4$ est un groupe méthoxycarbonyle et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe heptanoyle, n est un nombre entier nul, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier nul, $R^2$ est un atome d'hydrogène, $R^3$ est un groupe carboxy-1 éthylamino, $R^4$ est un groupe éthoxycarbonyle et $R^5$ est un atome d'hydrogène), ou son sel, procédé caractérisé en ce qu'on soumet un composé de formule:

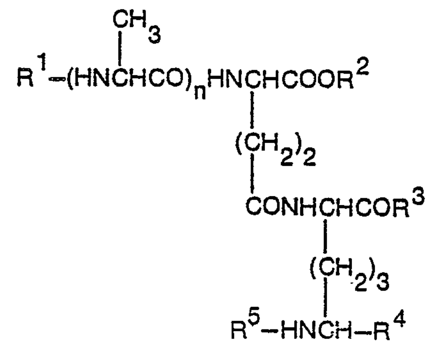

(dans laquelle

$R^1$ est un groupe n-octanoyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe carboxy-1 éthylamino, $R^4$ est un atome d'hydrogène et $R^5$ est un groupe benzyloxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle, $R^5$ est un groupe tertiobutoxycarbonyle; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un atome d'hydrogène; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe tertio-butoxycarbonyle; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un groupe butoxy-carbonyle; ou

$R^1$ est un groupe diisopropylidène-2,3; 4,6 céto-2 L-gulonoyle, n est un nombre entier nul, $R^2$ est un groupe benzyle, $R^3$ est un groupe carboxy-1 éthylamino $R^4$ est un atome d'hydrogène et $R^5$ est un atome d'hydrogène; ou

$R^1$ est un groupe céto-2 L-gulonoyle, n est un nombre entier nul, $R^2$ est un groupe benzyle, $R^3$ est un groupe carboxy-1 éthylamino, $R^4$ est un atome d'hydrogène et $R^5$ est un atome d'hydrogène; ou

$R^1$ est un groupe diisopropylidène-2,3; 4,6 céto-2 L-gulonoyle, n est un nombre entier nul, $R^2$ est un groupe benzyle, $R^3$ est un groupe carboxyl-1 éthylamino, $R^4$ est un carboxyle et $R^5$ est un groupe tertiobutoxycarbonyle; ou

$R^1$ est un groupe céto-2 L-gulonoyle, n est un nombre entier nul, $R^2$ est un groupe benzyle, $R^3$ est un groupe carboxy-1 éthylamino, $R^4$ est un groupe carboxyle et $R^5$ est un atome d'hydrogène; ou

$R^1$ est un groupe heptanoyle, n est un nombre entier nul, $R^2$ est un groupe benzyle, $R^3$ est un groupe éthoxycarbonyl-1 éthylamino, $R^4$ est un groupe éthoxycarbonyle et $R^5$ est un groupe benzyloxy-carbonyle; ou

$R^1$ est un groupe heptanoyle, n est un nombre entier nul, $R^2$ est un groupe benzyle, $R^3$ est un groupe carboxy-1 éthylamino, $R^4$ est un groupe éthoxycarbonyle et $R^5$ est un groupe benzyloxy-carbonyle),

ou son sel, à une réaction d'élimination du ou des groupes protecteurs, ce qui donne un composé de formule:

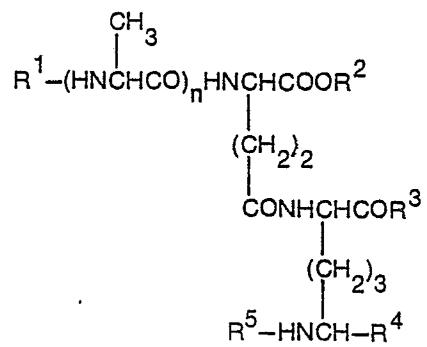

(dans laquelle

$R^1$ est un groupe n-octanoyle, n est un nombre entier nul, $R^2$ est un groupe d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe stéaroyle, n est un nombre entier nul, $R^2$ est un groupe benzyle, $R^3$ est un groupe méthoxy, $R^4$ est un groupe méthoxycarbonyle et $R^5$ est un atome d'hydrogène; ou

$R^1$ est un groupe stéaroyle, n est un nombre entier nul, $R^2$ est un atome d'hydrogène, $R^3$ est un groupe méthoxy, $R^4$ est un groupe méthoxy-carbonyle et $R^5$ est un atome d'hydrogène; ou

$R^1$ est un groupe heptanoyle, n est un nombre entier nul, $R^2$ est un groupe méthyle, $R^3$ est un groupe méthoxycarbonyl-1 éthylamino, $R^4$ est un groupe méthoxycarbonyle et $R^5$ est un atome d'hydrogène; ou

$R^1$ est un groupe octanoyle, n est un nombre entier nul, $R^2$ est un groupe méthyle, $R^3$ est un groupe méthoxycarbonyl-1 éthylamino, $R^4$ est un groupe méthoxycarbonyle, et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un atome d'hydrogène et $R^5$ est un atome d'hydrogène; ou

$R^1$ est un groupe céto-2 L-gulonoyle, n est un nombre entier nul, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe heptanoyle, n est un nombre entier nul, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier nul, $R^2$ est un atome d'hydrogène, $R^3$ est un groupe carboxy-1 éthylamino, $R^4$ est un groupe éthoxycarbonyle et $R^5$ est un atome d'hydrogène) ou son sel.

3. Procédé de préparation d'un composé de formule:

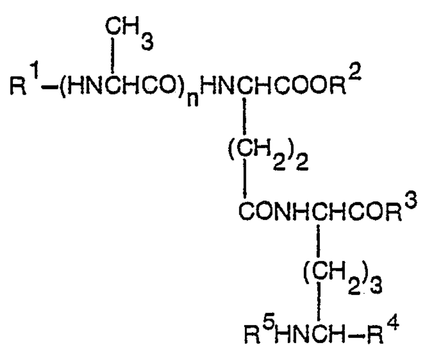

(dans laquelle

$R^1$ est un groupe n-docosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou.

$R^1$ est un groupe n-docosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe n-tétracosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe n-tétracosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe acétoxy-2 propionyle, n est un nombre entier valant 1, $R^2$ est un atome d'hydrogène, $R^3$ est un groupe méthoxycarbonylméthylamino, $R^4$ est un groupe méthoxycarbonyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe méthoxycarbonylméthyl amino, $R^4$ est un groupe carboxyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe carboxyméthylamino, $R^4$ est un groupe méthoxycarbonyle et $R^5$ est un atome d'hydrogène; ou

$R^1$ est un groupe N-n-hexyl-1 O-tétraacétyl-2,3,4,5-D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-n-hexyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un atome d'hydrogène; ou

$R^1$ est un groupe stéaroyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe heptanoyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-benzyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un atome d'hydrogène, $R^3$ est un groupe carboxy-1 éthylamino, $R^4$ est un carboxyle et $R^5$ est un atome d'hydrogène; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène), ou son sel pharmaceutique acceptable, procédé caractérisé en ce qu' on soumet un composé de formule:

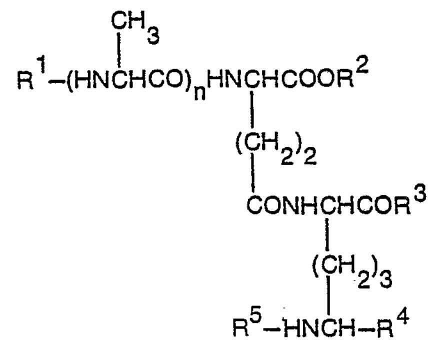

(dans laquelle

$R^1$ est un groupe n-docosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe tertiobutoxycarbonyle; ou

$R^1$ est un groupe n-docosanoyle, n est un nombre entier valant 1, $R^2$ est un groupe benzyle, $R^3$ est un groupe carboxy-1 éthylamino, $R^4$ est un groupe carboxyle et $R^5$ est un groupe tertiobutoxycarbonyle; ou

$R^1$ est un groupe n-tétracosanoyle, n est un nombre entier valant 1, $R^2$ est un groupe benzyle, $R^3$ est un groupe méthoxycarbonyl-1 éthylamino, $R^4$ est un groupe méthoxycarbonyle et $R^5$ est un groupe t-butoxycarbonyle; ou

$R^1$ est un groupe n-tétracosanoyle, n est un nombre entier valant 1, $R^2$ est un atome d'hydrogène, $R^3$ est un groupe méthoxycarbonyl-1 éthylamino, $R^4$ est un groupe méthoxycarbonyle et $R^5$ est un atome d'hydrogène; ou

$R^1$ est un groupe acétoxy-2 propionyle, n est un nombre entier valant 1, $R^2$ est un groupe benzyle, $R^3$ est un groupe méthoxycarbonylméthylamino, $R^4$ est un groupe méthoxycarbonyle et $R^5$ est un groupe benzyloxycarbonyle; ou

$R^1$ est un groupe hydroxy-2 propionyle, n est un nombre entier valant 1, $R^2$ est un groupe benzyle, $R^3$ est un groupe méthoxycarbonylméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe benzyle, $R^3$ est un groupe carboxyméthylamino, $R^4$ est un group méthoxycarbonyle et $R^5$ est un groupe tertiobutoxycarbonyle; ou

$R^1$ est un groupe N-n-hexyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzoyloxycarbonyl-3 carbazoyle et $R^5$ est un groupe benzyloxycarbonyle; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carbazoyle et $R^5$ est un atome d'hydrogène; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un groupe tertiobutoxycarbonyle; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un groupe tertiobutoxycarbonyle; ou

$R^1$ est un groupe stéaroyle, n est un nombre entier valant 1, $R^2$ est un groupe benzyle, $R^3$ est un groupe éthoxycrbonyl-1 éthylamino, $R^4$ est un groupe éthoxycarbonyle et $R^5$ est un groupe benzoyloxycarbonyle; ou

$R^1$ est un groupe heptanoyle, n est un nombre entier valant 1, $R^2$ est un groupe benzyle, $R^3$ est un groupe éthoxycarbonyl-1 éthylamino, $R^4$ est un groupe éthoxycarbonyle et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzyloxycarbonyl-3 carbazoyle, et $R^5$ est un groupe benzyloxycarbonyle; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carbaxoyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un groupe benzyle, $R^3$ est un groupe carboxyméthylamino, $R^4$ est un groupe benzoyloxycarbonyl-3 carbazoyle et $R^5$ est un groupe benzyloxycarbonyle; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un carbazoyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un groupe benzyle, $R^3$ est un groupe carboxy-1 éthylamino, $R^4$ est un groupe carboxyle et $R^5$ est un groupe benzyloxycarbonyle; ou

$R^1$ est un groupe triacontanoyle, n est un nombre entier valant 1, $R^2$ est un atome d'hydrogène, $R^3$ est un groupe carboxy-1 éthylamino, $R^4$ est un carboxyle et $R^5$ est un groupe tertiobutoxycarbonyle) ou son sel, à une réaction d'élimination du ou des groupes protecteurs, pour obtenir un composé de formule:

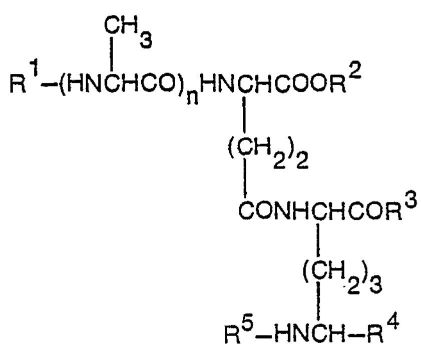

(dans laquelle

$R^1$ est un groupe n-docosanoyle, n est un nombre entier valant 1, $R^2$ est un atome d'hydrogène, $R^3$ est un groupe méthoxycarbonyl-1 éthylamino, $R^4$ est un méthoxycarbonyle et $R^5$ est un atome d'hydrogène; ou

$R^1$ est un groupe n-docosanoyle, n est un nombre entier valant 1, $R^2$ est un atome d'hydrogène, $R^3$ est un groupe carboxy-1 éthylamino, $R^4$ est un carboxyle et $R^5$ est un atome d'hydrogène; ou

$R^1$ est un groupe n-tétracosanoyle, n est un nombre entier valant 1, $R^2$ est un atome d'hydrogène,

$R^3$ est un groupe méthoxycarbonyl-1 éthylamino, $R^4$ est un groupe méthoxycarbonyle et $R^5$ est un atome d'hydrogène; ou

$R^1$ est un groupe n-tétracosanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène; ou

$R^1$ est un groupe acétoxy-2 propionyle, n est un nombre entier valant 1, $R^2$ est un atome d'hydrogène, $R^3$ est un groupe méthoxycarbonylméthylamino, $R^4$ est un groupe méthoxycarbonyle et $R^5$ est un atome d'hydrogène; ou

$R^1$ est un groupe hydroxy-2 propionyle, n est un nombre entier valant 1, $R^2$ est un atome d'hydrogène, $R^3$ est un groupe méthoxycarboxylméthylamino, $R^4$ est un groupe carboxyle et $R^5$ est un atome d'hydrogène; ou

$R^1$ est un groupe hydroxy-2 propionyle, n est un nombre entier valant, $R^2$ est un atome d'hydrogène, $R^3$ est un groupe carboxyméthylamino, $R^4$ est un groupe méthoxycarbonyle et $R^5$ est un groupe atome d'hydrogène; ou

$R^1$ est un groupe N-n-hexyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-n-hexyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe carboxyle et $R^5$ est un atome d'hydrogène; ou

$R^1$ est un groupe hydroxy-2 propionyle, n est un nombre entier valant 1, $R^2$ est un groupe méthyle, $R^3$ est un groupe carboxyméthylamino, $R^4$ est un groupe méthoxycarbonyle et $R^5$ est un atome d'hydrogène; ou

$R^1$ est un groupe stearoyle, n est un nombre entier valant 1, $R^2$ est un atome d'hydrogène, $R^3$ est un groupe éthoxycarbonyl-1 éthylamino, $R^4$ est un group éthoxycarbonyle et $R^5$ est un atome d'hydrogène; ou

$R^1$ est un groupe heptanoyle, n est un nombre entier valant 1, $R^2$ est un atome d'hydrogène, $R^3$ est un groupe éthoxycarbonyl-1 éthylamino, $R^4$ est un groupe éthoxycarbonyle et $R^5$ est un atome d'hydrogène; ou

$R^1$ est un groupe N-benzyl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-benzyl-1 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 O-tétraacétyl-2,3,4,5 D-glucaramoyle, n est un nombre entier valant 1, $R^2$ est un atome d'hydrogène, $R^3$ est un groupe carboxyméthylamino, $R^4$ est un groupe carboxyle et $R^5$ est un atome d'hydrogène; ou

$R^1$ est un groupe N-lauryl-1 D-glucaramoyle, n est un nombre entier valant 1, $R^2$ est un atome d:hydrogène, $R^3$ est un groupe carboxyméthylamino, $R^4$ est un groupe carboxyle et $R^5$ est un atome d'hydrogène; ou

$R^1$ est un groupe palmitoyle, n est un nombre entier valant 1, $R^2$ est un atome d'hydrogène, $R^3$ est un groupe carboxy-1 éthylamino, $R^4$ est un carboxyle et $R^5$ est un atome d'hydrogène; ou

$R^1$ est un groupe triacontanoyle, n est un nombre entier valant 1, $R^2$ est un atome d'hydrogène, $R^3$ est un groupe carboxy-1 éthylamino, $R^4$ est un groupe carboxyle et $R^5$ est un atome d'hydrogène), ou son sel.